(19) Europäisches Patentamt
European Patent Office
Office européen des brevets

(11) EP 4 736 883 A1

(12) EUROPEAN PATENT APPLICATION
published in accordance with Art. 153(4) EPC

(43) Date of publication:
**06.05.2026 Bulletin 2026/19**

(21) Application number: **24831046.8**

(22) Date of filing: **28.06.2024**

(51) International Patent Classification (IPC):
***A61K 47/64*** *(2017.01)* ***A61P 31/16*** *(2006.01)*

(52) Cooperative Patent Classification (CPC):
**A61K 31/351; A61K 31/4025; A61K 47/64; A61K 47/68; A61P 31/16**

(86) International application number:
**PCT/CN2024/102634**

(87) International publication number:
**WO 2025/002421 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 CN 202310790115**

(71) Applicant: **Shanghai Ark Biopharmaceutical Co. Ltd.**
**Pudong New Area**
**Shanghai 201203 (CN)**

(72) Inventors:
- **SONG, Guowei**
  **Shanghai 201203 (CN)**
- **SONG, Zhidong**
  **Shanghai 201203 (CN)**
- **PENG, Cheng**
  **Shanghai 201203 (CN)**
- **GAO, Guanglin**
  **Shanghai 201203 (CN)**
- **WU, Jin**
  **Shanghai 201203 (CN)**
- **GAO, Fan**
  **Shanghai 201203 (CN)**
- **GAO, Zhao**
  **Shanghai 201203 (CN)**
- **ZOU, Gang**
  **Shanghai 201203 (CN)**
- **YIE, Junmin**
  **Shanghai 201203 (CN)**
- **WU, Jimzhen**
  **Shanghai 201203 (CN)**
- **CHUN, Sophia Siufei**
  **Shanghai 201203 (CN)**

(74) Representative: **Bianchetti & Minoja with Trevisan & Cuonzo IPS SRL**
**Via Plinio, 63**
**20129 Milano (IT)**

Remarks:
The complete document including Reference Table(s) and the Sequence Listing(s) can be downloaded from the EPO website

# (54) CONJUGATE FOR PREVENTING AND TREATING VIRAL INFECTION AND USE THEREOF

(57) The present invention relates to conjugates for the preventing and treating of viral infections and use thereof, and more specifically to a conjugate of protein and anti-influenza compound of formula I-A or I-B, or a pharmaceutically acceptable salt, ester, isomer, solvate, prodrug, or isotopically labeled compound thereof, wherein each symbol is as defined herein. The conjugates or intermediate compounds of the present invention exhibit significant anti-influenza virus activity, while also have excellent *in vitro*/*in vivo* pharmacokinetic properties and safety, indicating high prospects for clinical application.

EP 4 736 883 A1

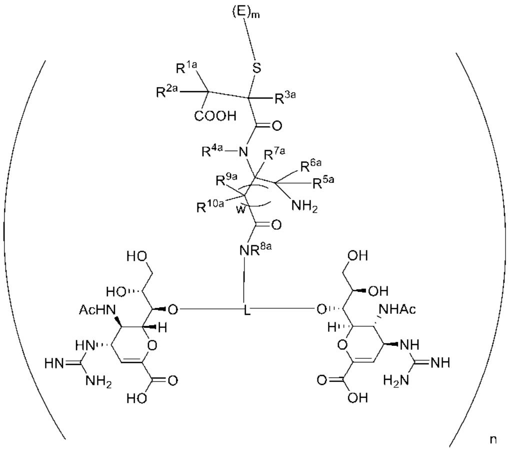

Formula I-A

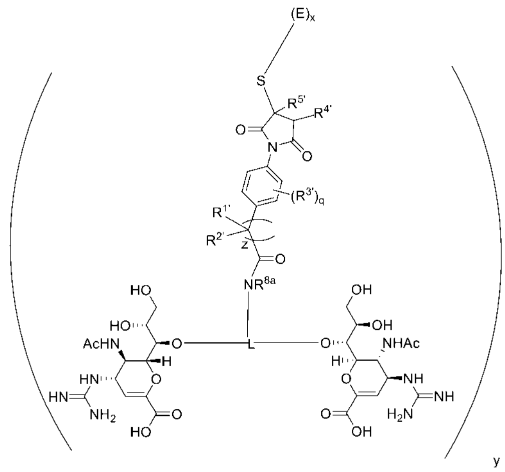

Formula I-B

## Description

### Field of the invention

The present invention relates to conjugates of antiviral drugs against influenza (Flu) viruses and antibodies or their constant region Fc. Specifically, the present invention relates to compounds comprising antibodies or their constant region Fc covalently conjugated to small molecule inhibitors of viral surface proteins or inhibitor peptide drugs and intermediate compounds thereof, as well to pharmaceutical compositions or drugs comprising these compounds, and their use for the prevention and/or treatment of related viral infections.

### Background of the invention

Influenza virus causes approximately 3 to 5 million severe infections globally each year, with around 500,000 deaths (Luliano et al., 2018, Lancet 391: 1285-1300). Although most healthy individuals recover spontaneously from the infection within one to two weeks, for the elderly, those with chronic diseases, and individuals with compromised immune systems, influenza virus infection can progress to life-threatening severe infections and complications, such as pneumonia.

Developing treatments against influenza viruses remains a persistent challenge for humanity. Although antiviral drugs and preventive vaccines are currently available on the market, small molecule anti-influenza drugs typically need to be administered within 48 hours of symptom onset to achieve clinical benefits. Furthermore, due to the high variability of influenza viruses, resistant strains to commonly used drugs have been identified. Therefore, there is a need to develop more effective and long-lasting therapies for the treatment and/or prevention of influenza virus infection.

Influenza viruses are a type of negative strand, segmented RNA viruses belonging to the Orthomyxoviridae family, and include influenza virus A, B, and C. Among these, infections are primarily caused by influenza A and B viruses in humans. Influenza viruses infect respiratory epithelial cells. The first step of this process involves the adsorption to host cells mediated by the viral surface receptor binding protein (hemagglutinin (HA protein), in the case of influenza proteins). Subsequently, under the action of the hemagglutinin, the viral envelope and cell membrane fuse, and the influenza virus genome segments and the viral RNA-dependent RNA polymerase complex are subsequently released into the cells. The RNA-dependent RNA polymerase complex of the virus uses genome fragments as templates to synthesize new progeny virus particles within the cell. The newly synthesized virus particles are released outside the cell through cell lysis or budding. For influenza viruses, the complete release of progeny viruses relies on the neuraminidase (NA) to cleave sialic acid residues on the cell surface. Neuraminidase inhibitors, which target the neuraminidase of influenza viruses to reduce virus spread, have been approved for clinical use, including oseltamivir (Tamiflu™), zanamivir (Relenza™), and peramivir (Rapivab™).

Patients who have undergone organ transplants or those with cancer, due to their suppressed immune systems, are unable to effectively eliminate viruses. After being infected with the influenza viruses, the viruses tend to replicate for a longer period in their bodies, thereby increasing the likelihood of developing drugresistant strains. Such findings have already been observed clinically. Meanwhile, the problem of influenza drug resistance in ordinary patients is also a significant challenge in medication use. Therefore, more effective new methods and therapeutic agents for treating influenza are needed.

### Description of the invention

In one aspect, the present invention provides a conjugate, or a pharmaceutically acceptable salt, ester, isomer, solvate, prodrug, or isotopically labeled compound thereof, comprising an anti-influenza small molecule compound conjugated to a protein (also referred to as "conjugate of protein and anti-influenza compound "), which exhibits significant anti-influenza virus activity.

In some embodiments, the present invention provides a conjugate represented by a structure of Formula I-A or Formula I-B, or a pharmaceutically acceptable salt, ester, isomer, solvate, prodrug, or isotopically labeled compound thereof:

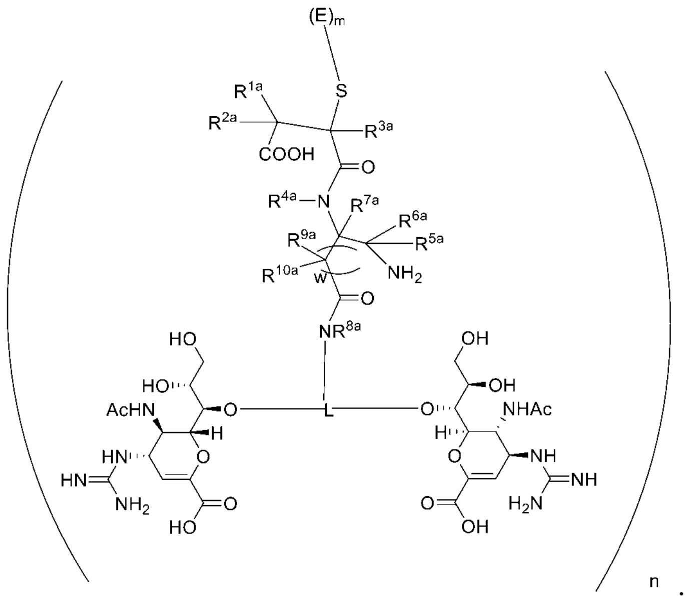

Formula I-A

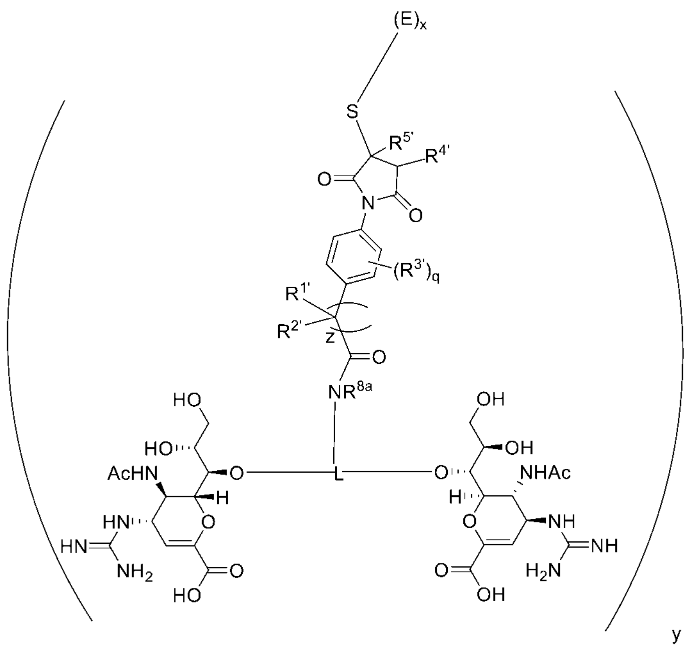

Formula I-B

wherein,

m is 1 or 2;

n is an integer from 1 to 20, and preferably an integer of 1, 2, 3, 4, 5, 6, 7, or 8;

w is an integer from 0 to 8; for example, 0, 1, 2, 3, 4, or 5;

L is a linker;

E is selected from an antibody or antibody fragment, Fc domain monomer, Fc domain, Fc binding peptide, albumin, or

albumin-binding peptide;

each of $R^{1a}$, $R^{2a}$, $R^{3a}$, $R^{5a}$, $R^{6a}$, $R^{7a}$, $R^{9a}$ and $R^{10a}$ is independently selected from H, deuterium, hydroxyl, - O-$C_{1-6}$ alkyl, hydroxyl$C_{1-6}$ alkyl-, $C_{1-6}$ alkyl-, $C_{3-8}$ cycloalkyl-, $C_{3-8}$ heterocycloalkyl-, $C_6$-$C_{15}$ aryl, 5 to 15-membered heteroaryl, halogen, cyano, and amino; $R^{1a}$ and $R^{2a}$ may together form $C_{3-8}$ cycloalkyl or $C_{3-8}$ heterocycloalkyl;

or $R^{5a}$ and $R^{6a}$ may together form $C_{3-8}$ cycloalkyl or $C_{3-8}$ heterocycloalkyl;

or $R^{9a}$ and $R^{10a}$ may together form $C_{3-8}$ cycloalkyl or $C_{3-8}$ heterocycloalkyl, wherein said $C_{3-8}$ cycloalkyl or $C_{3-8}$ heterocycloalkyl may be optionally substituted with a substituent selected from halogen and hydroxyl;

each of $R^{4a}$ and $R^{8a}$ is independently selected from H, $C_{1-6}$ alkyl, $C_{3-8}$ cycloalkyl or $C_{3-8}$ heterocycloalkyl;

x is 1 or 2;

y is an integer from 1 to 20, and preferably an integer of 1, 2, 3, 4, 5, 6, 7, or 8;

z is an integer from 0 to 8; for example, 0, 1, 2, 3, 4, or 5;

q is 0, 1, 2, 3 or 4;

each of $R^{1'}$, $R^{2'}$, $R^{3'}$, $R^{4'}$ and $R^{5'}$ is independently selected from hydrogen, deuterium, hydroxyl, -O-$C_{1-6}$ alkyl, hydroxyl$C_{1-6}$ alkyl, $C_{1-6}$ alkyl, $C_{3-8}$ cycloalkyl, $C_{3-8}$ heterocycloalkyl, $C_6$-$C_{15}$ aryl, 5 to 15-membered heteroaryl, halogen, cyano, and amino; $R^{1'}$ and $R^{2'}$ may together form $C_{3-8}$ cycloalkyl or $C_{3-8}$ heterocycloalkyl; alternatively, two adjacent $R^{3'}$ may form $C_{3-8}$ cycloalkyl, $C_{3-8}$ heterocycloalkyl, $C_6$-$C_{15}$ aryl, or 5 to 15-membered heteroaryl.

It should be understood that, in Formulas I-A and I-B, the parentheses with subscripts n or y do not encompass the $(E)_m$ structural part, but encompass the S atom and the structure below it.

In some embodiments, L is a linker represented by the structure of formula I-3a, comprising $L^1$ and $L^{2a}$. The left and right ends of $L^1$ are connected to the drug moiety (D), and the upper end of $L^{2a}$ is connected to - $NR^{8a}$ in formulas I-A and I-B,

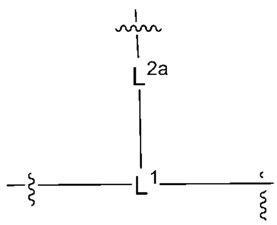

Formula I-3a

wherein, $L^1$ is selected from the following structure:

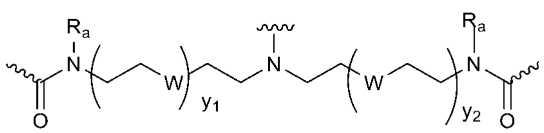

wherein,

W is selected from O, S, $NR^b$, $CH_{2-}$ or absence;

each of $R^a$ and $R^b$ is independently selected from H, optionally substituted $C_1$-$C_{20}$ alkyl, and optionally substituted $C_2$-$C_{20}$ alkenyl; and preferably selected from H and methyl, and more preferably methyl;

each of $y_1$ and $y_2$ is independently 0, 1, 2, 3, 4, 5, or 6;

It should be understood that the bonds at the left and right ends of the $L^1$ structure are connected to the oxygen atom of the drug ($D^1$), and the bond marked by the wavy line drawn on the intermediate amine group is connected to $L^{2a}$.

Preferably, $L^1$ is selected from the following structures:

| Structure of $L^1$ |
|---|
| 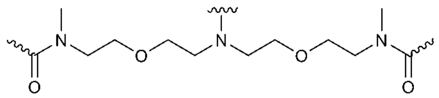 |
| 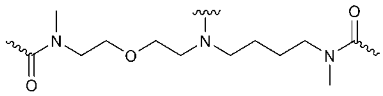 |
| 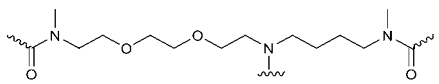 |
| 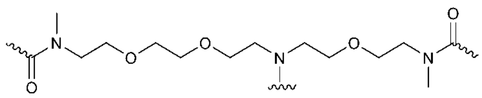 |
| 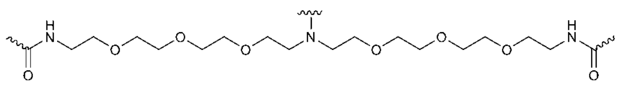 |
| 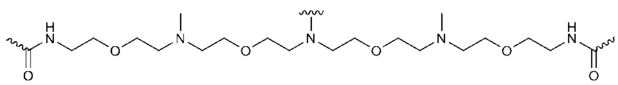 |
| 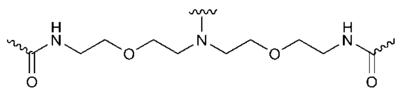 |
| 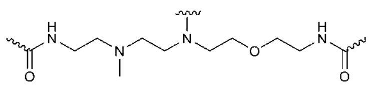 |
| 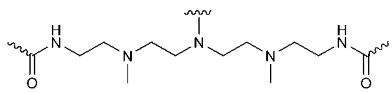 |
| 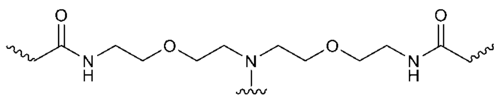 |
| 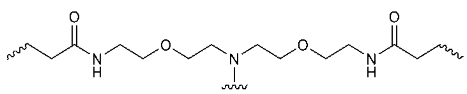 |
| 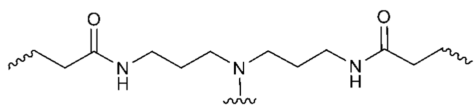 |
| 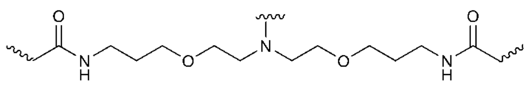 |
| 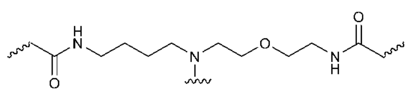 |

(continued)

| Structure of $L^1$ |
|---|
| 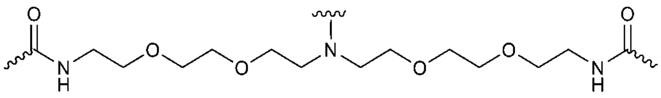 |
| 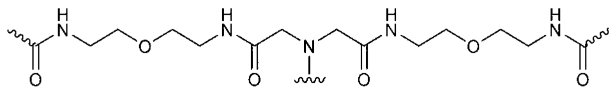 |
| 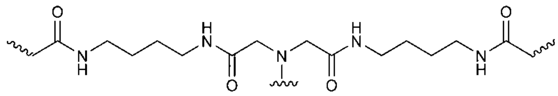 |
| 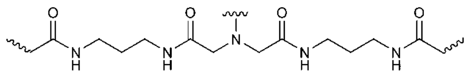 |
| 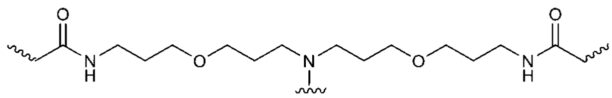 |
| 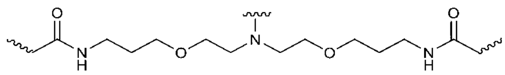 |
| 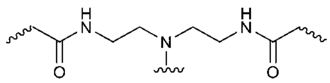 |
| 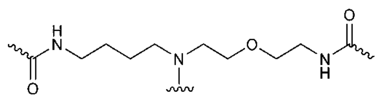 |
| 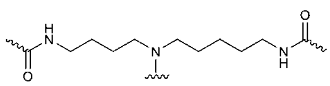 |
| 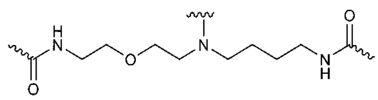 |
| 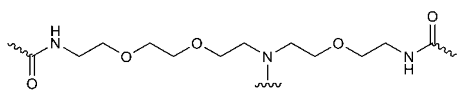 |

In some embodiments, wherein $L^{2a}$ is represented by a structure of the formula L2-1a to L2-7a:

| $L^{2a}$ structure No. | Structure |
|---|---|
| Formula L2-1a | 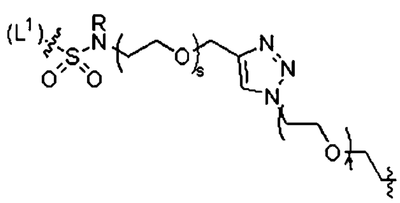 |

(continued)

| $L^{2a}$ structure No. | Structure |
|---|---|
| Formula L2-2a | 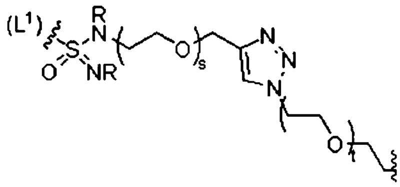 |
| Formula L2-3a | 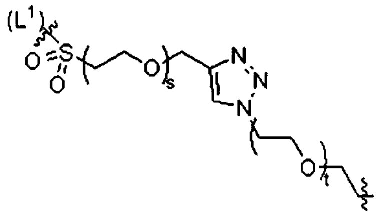 |
| Formula L2-4a | 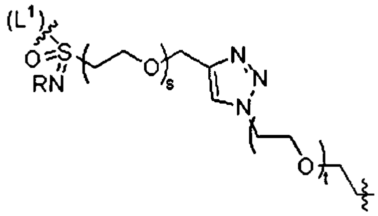 |
| Formula L2-5a | 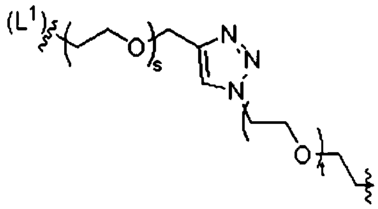 |
| Formula L2-6a | 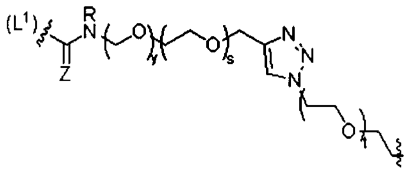 |
| Formula L2-7a | 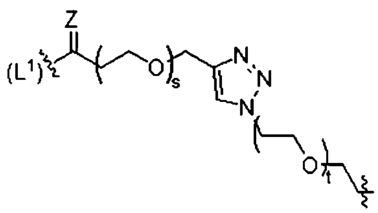 |

wherein,

Z is selected from NR, S and O;

The R groups are each independently selected from hydrogen, deuterium, optionally substituted $C_1$-$C_{20}$ alkyl, optionally substituted $C_2$-$C_{20}$ alkenyl, optionally substituted $C_3$-$C_{20}$ cycloalkyl, optionally substituted 3 to 20-membered heterocycloalkyl, optionally substituted $C_6$-$C_{15}$ aryl, and optionally substituted 5 to 15-membered heteroaryl;

s and t are integers from 1 to 20; for example, 1, 2, 3, 4, 5, 6, 7, 8, 9, 10, 11, or 12; and y is 0 or 1.

It should be understood that in formulas L2-1a to L2-7a, $L^{2a}$ represents the portion between the two wavy lines; the ($L^1$) moiety is not a component of $L^{2a}$, and indicates the end of $L^{2a}$ linked to $L^1$, to more clearly show the connection direction of $L^{2a}$. The right end of $L^{2a}$ is linked to -$NR^{8a}$.

In some embodiments, $L^{2a}$ is selected from formula L2-1a, L2-5a, and L2-7a,

Z is O,

R is H,

s and t are integers selected from 3 to 10, such as 4, 5, 6, 7, or 8. Preferably, s and t are 4 or 8.

In some embodiments, the S atom on E comes from cysteine (cys) residues or disulfide bonds in E;

Preferably, E is an antibody or an antibody fragment thereof, a Fc domain monomer, a Fc domain, a Fc binding peptide, albumin, or an albumin-binding peptide. For example, the Fc domain monomer comprises or consists of: any of the amino acid sequences set forth in SEQ ID Nos. 1-81, or an amino acid sequence having at least 95%, 96%, 97%, 98%, or 99% identity to any of these sequences.

Preferably, it comprises or consists of: an amino acid sequence set forth in any one of SEQ ID NO: 9, SEQ ID NO: 10, SEQ ID NO: 11, SEQ ID NO: 33, SEQ ID NO: 36, SEQ ID NO: 37, SEQ ID NO: 38, SEQ ID NO: 41, SEQ ID NO: 42, SEQ ID NO: 43, SEQ ID NO: 44, SEQ ID NO: 45, SEQ ID NO: 46, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 59, SEQ ID NO: 65, SEQ ID NO: 66, SEQ ID NO: 67, SEQ ID NO: 68, SEQ ID NO: 69, SEQ ID NO: 70, SEQ ID NO: 71, and SEQ ID NO: 72.

In some embodiments, the conjugate has the structure of formula I-A.

In some embodiments, the conjugate has the structure of formula I-B.

In some embodiments, the structure between L and E is selected from the following structure:

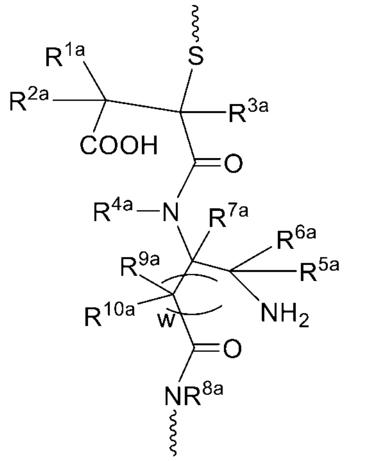

Formula I-4a

wherein, each of $R^{1a}$, $R^{2a}$, $R^{3a}$, $R^{5a}$, $R^{6a}$, $R^{7a}$, $R^{9a}$ and $R^{10a}$ is independently selected from hydrogen, deuterium, -O-$C_{1-6}$ alkyl, hydroxyl$C_{1-6}$ alkyl-, $C_{1-6}$ alkyl- and $C_{3-8}$ cycloalkyl-; alternatively, $R^{1a}$ and $R^{2a}$ may together form $C_{3-8}$ cycloalkyl;

alternatively, $R^{5a}$ and $R^{6a}$ may together form $C_{3-8}$ cycloalkyl;

alternatively, $R^{9a}$ and $R^{10a}$ may together form $C_{3-8}$ cycloalkyl, wherein said $C_{3-8}$ cycloalkyl is optionally substituted with a substituent selected from halogen and hydroxyl;

each of $R^{4a}$ and $R^{8a}$ is independently selected from H, $C_{1-6}$ alkyl, and $C_{3-8}$ cycloalkyl;

w is 0, 1 or 2;

preferably, each of $R^{1a}$, $R^{3a}$, $R^{4a}$, $R^{5a}$, $R^{7a}$, $R^{9a}$, and $R^{10a}$ is independently hydrogen;

each of $R^{2a}$, $R^{6a}$ and $R^{8a}$ is independently selected from hydrogen, $C_{1-6}$ alkyl, and $C_{3-8}$ cycloalkyl; and w is 0;

more preferably, the structure of formula I-4a is selected from:

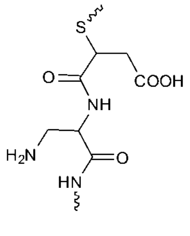
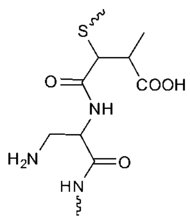
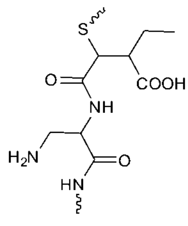
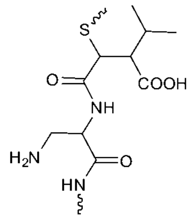
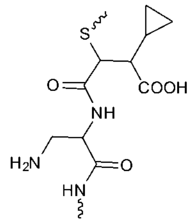
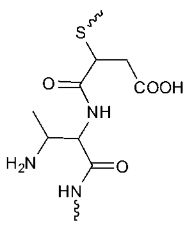
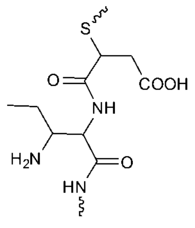
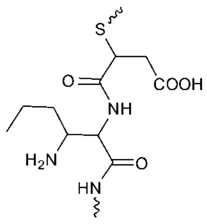
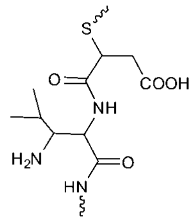
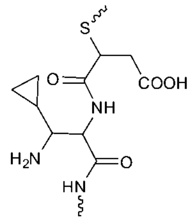
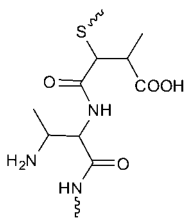
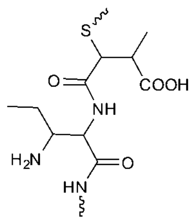
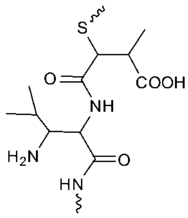
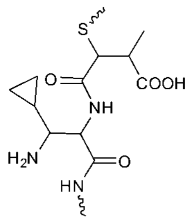
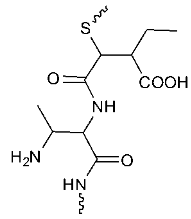
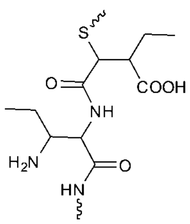
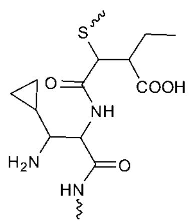
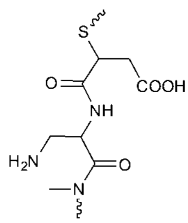
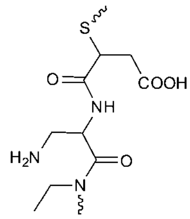
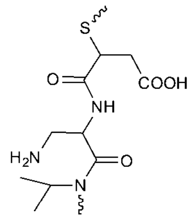
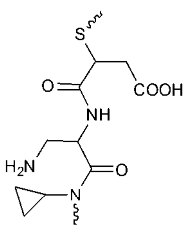
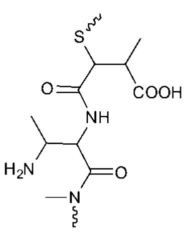
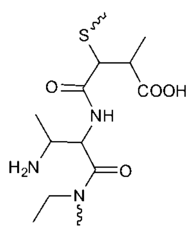
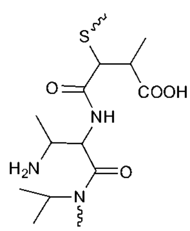
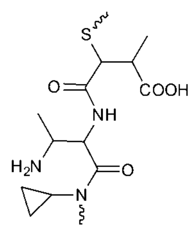
.

It should be understood that the S atom with a wavy line is linked to E, and the N atom with a wavy line is linked to L or $L^{2a}$.

In some embodiments, the conjugate is selected from compounds represented by Formula C-1a to Formula C-27a:

| No. | The structure of conjugate |
|---|---|
| C-1a | 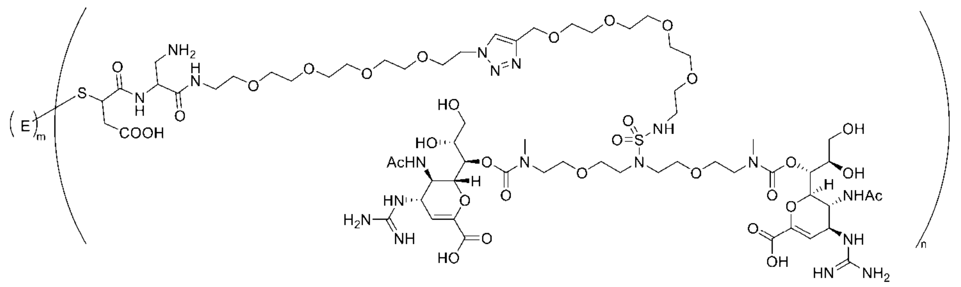 |
| C-2a | 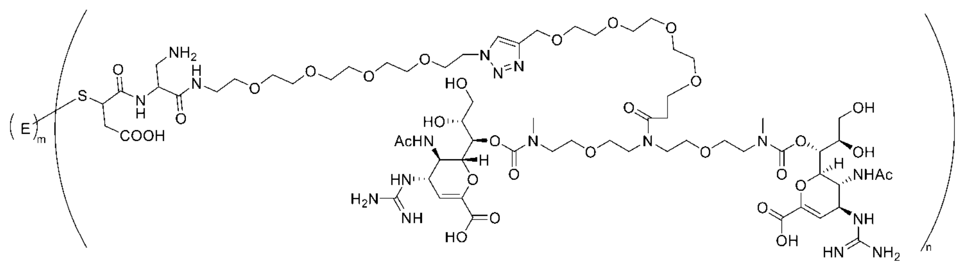 |
| C-3a | 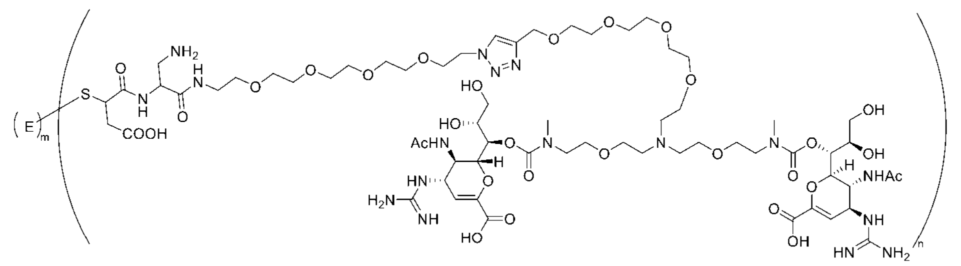 |
| C-4a | 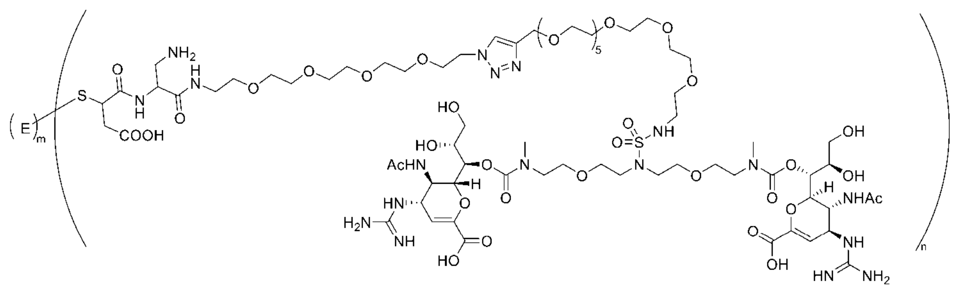 |
| C-5a | 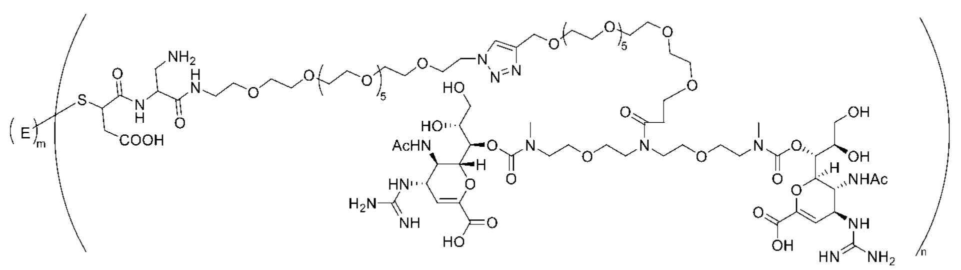 |
| C-6a | 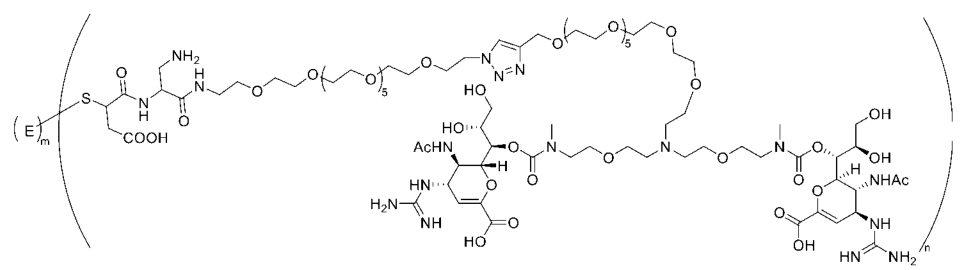 |

(continued)

| No. | The structure of conjugate |
|---|---|
| C-7a | 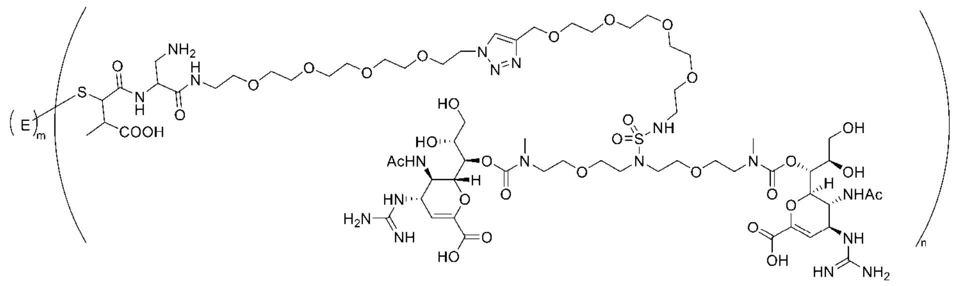 |
| C-8a | 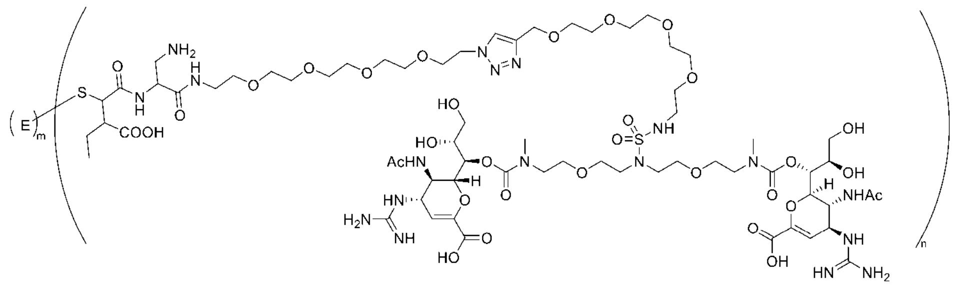 |
| C-9a | 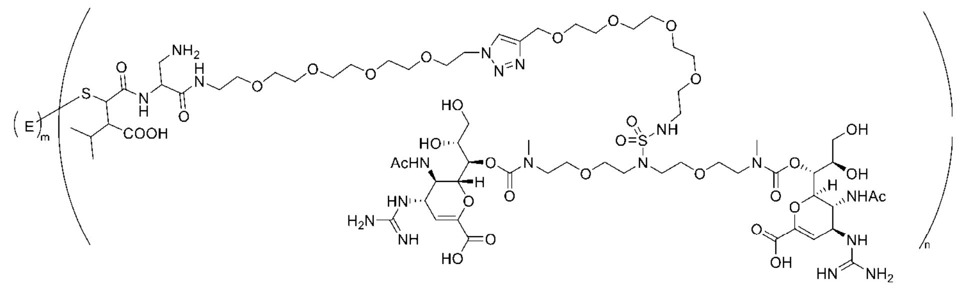 |
| C-10a | 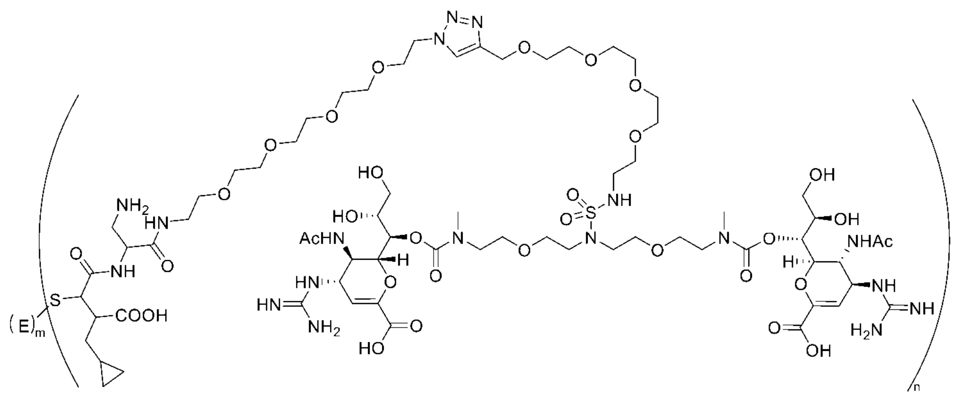 |
| C-11a | 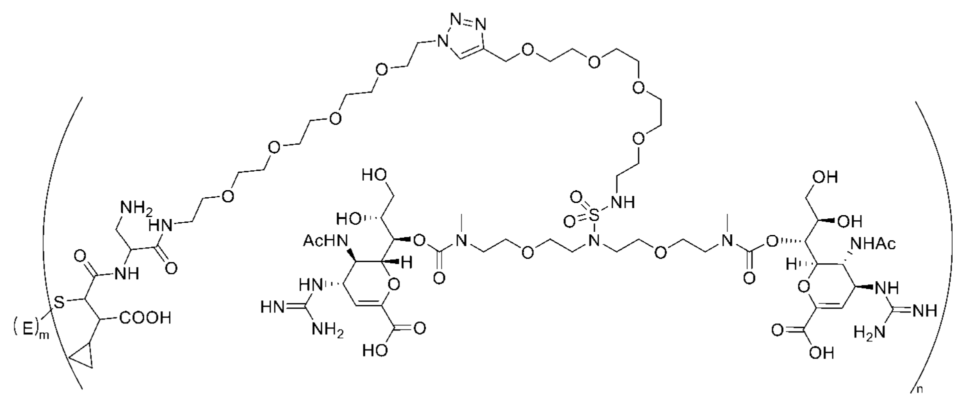 |

(continued)

| No. | The structure of conjugate |
|---|---|
| C-12a | 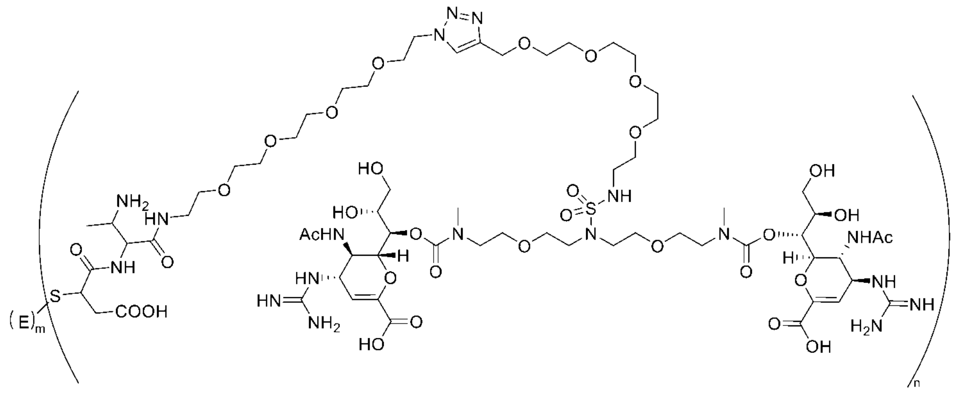 |
| C-13a | 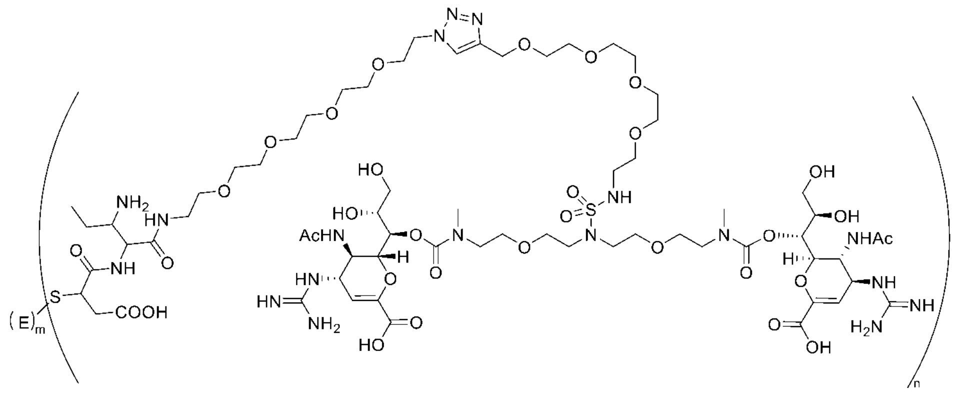 |
| C-14a | 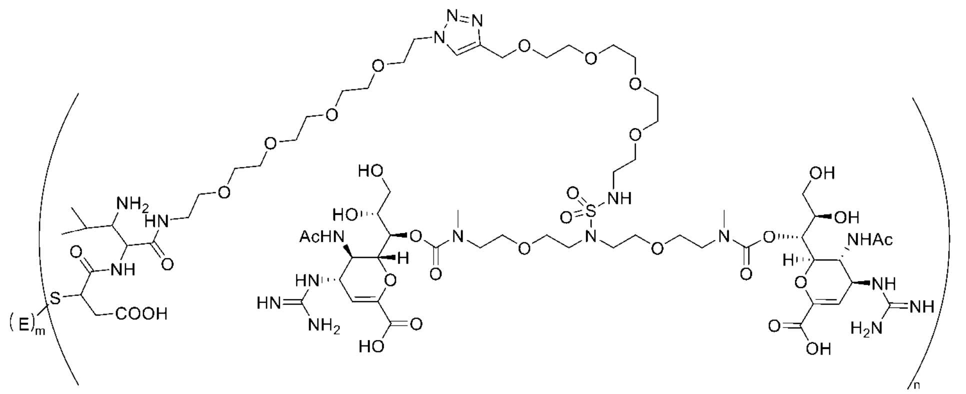 |
| C-15a | 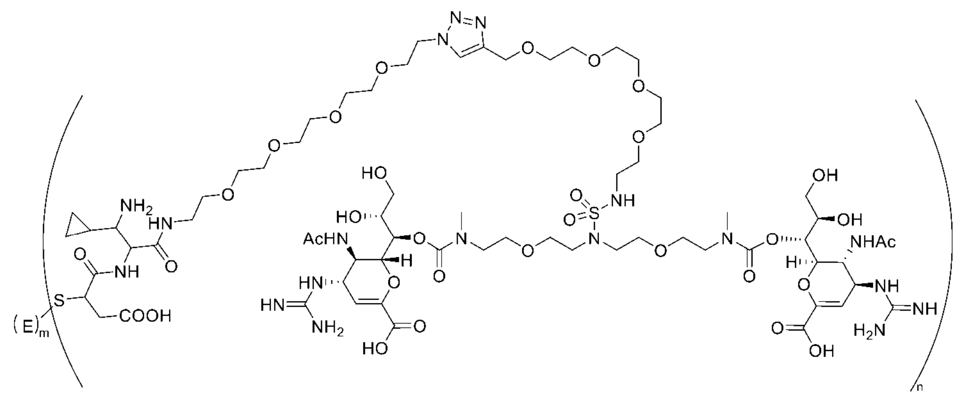 |

(continued)

| No. | The structure of conjugate |
|---|---|
| C-16a | 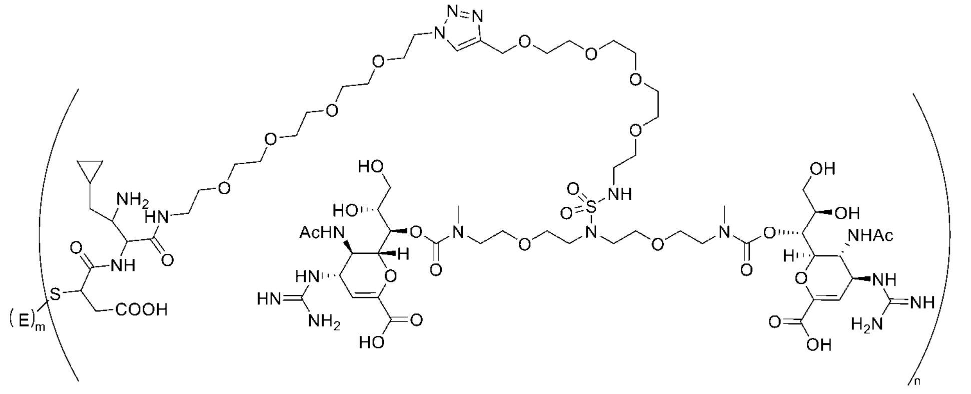 |
| C-17a | 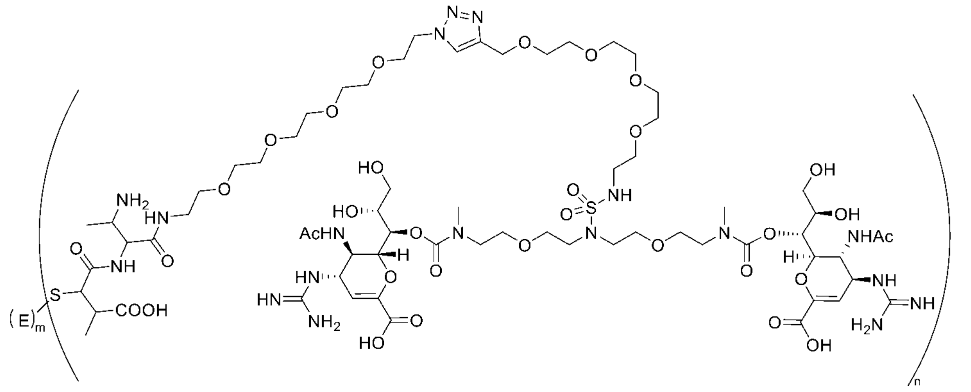 |
| C-18a | 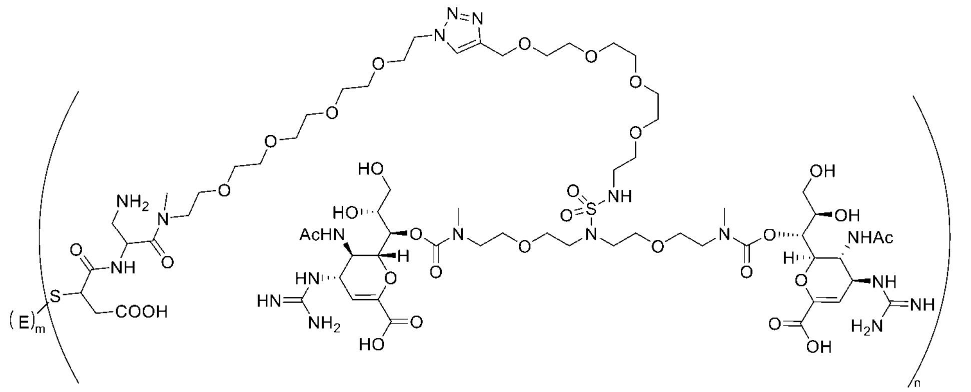 |
| C-19a | 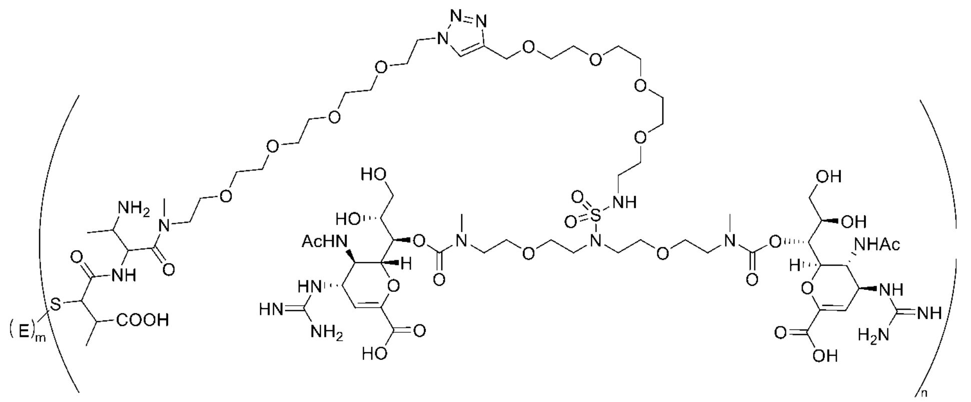 |

(continued)

| No. | The structure of conjugate |
|---|---|
| **C-20a** | 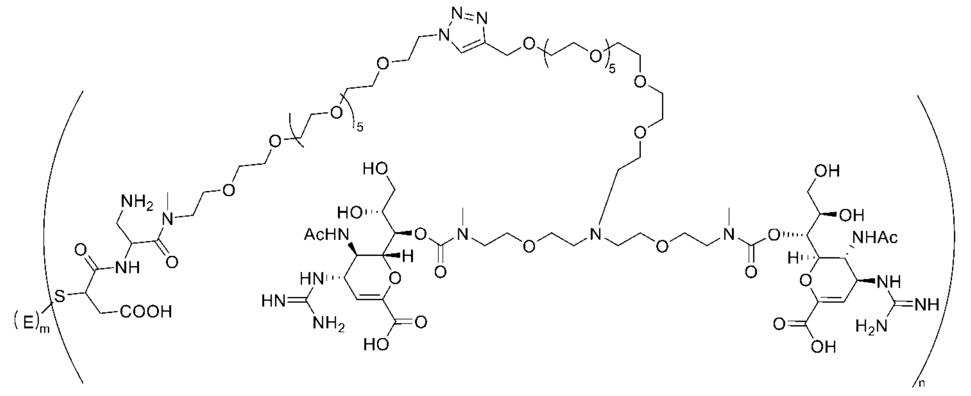 |
| **C-21a** | 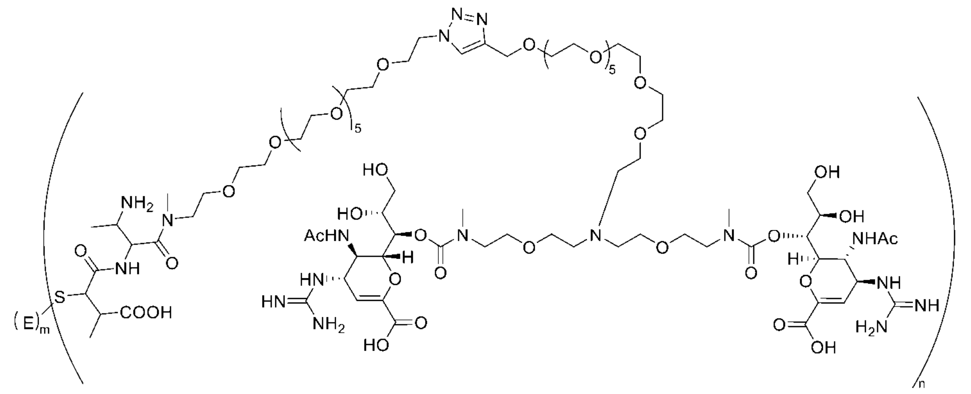 |
| **C-22a** | 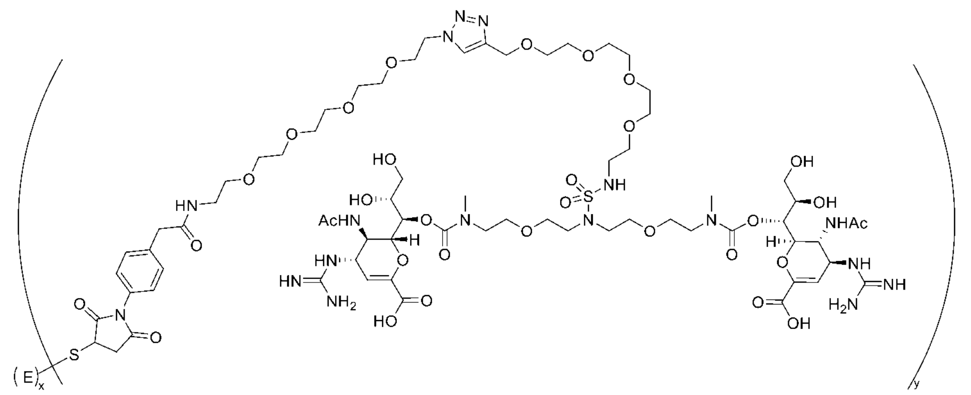 |
| **C-23a** | 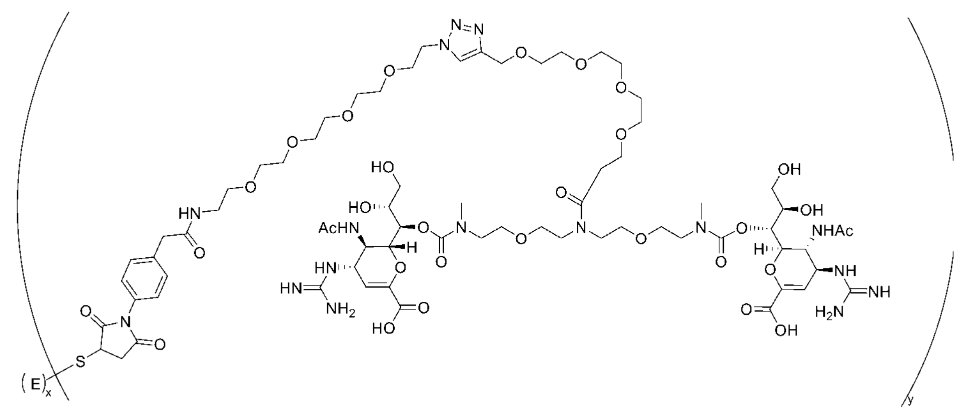 |

(continued)

| No. | The structure of conjugate |
|---|---|
| C-24a | 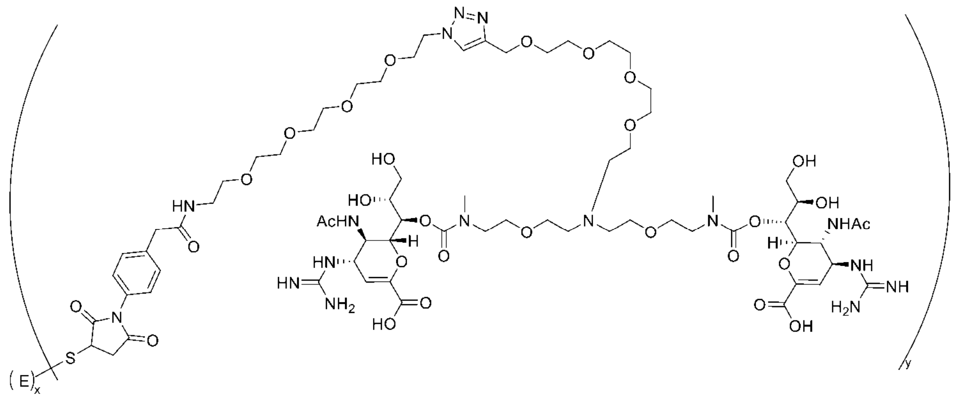 |
| C-25a | 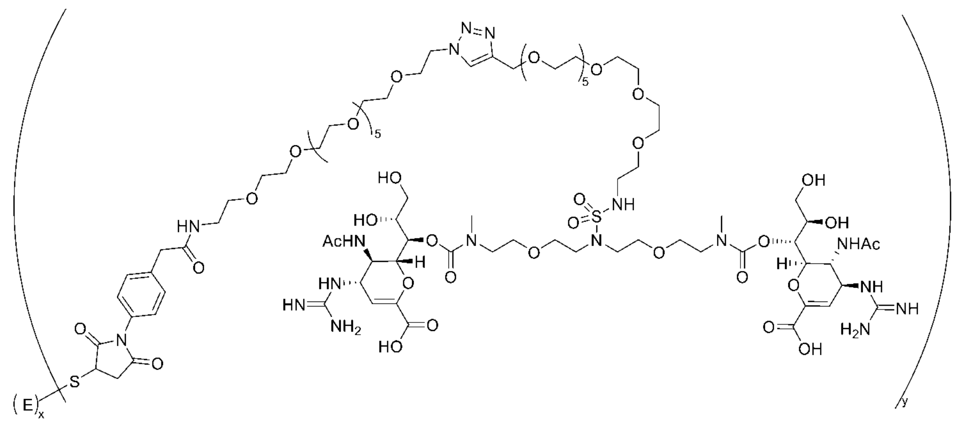 |
| C-26a | 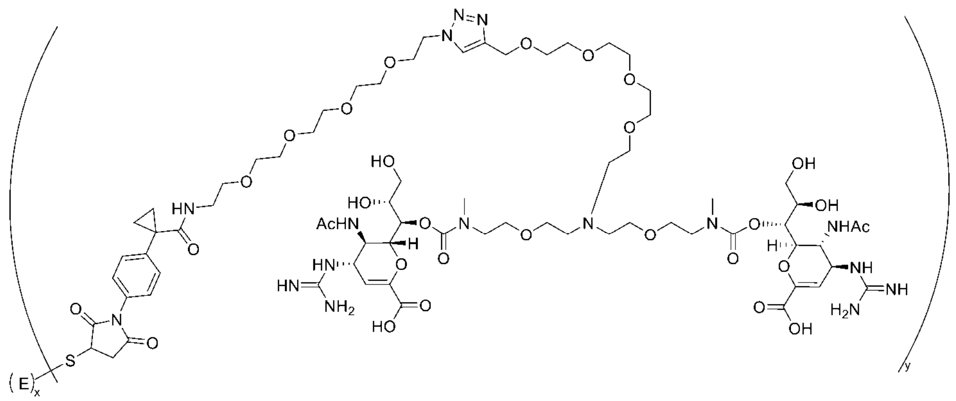 |
| C-27a | 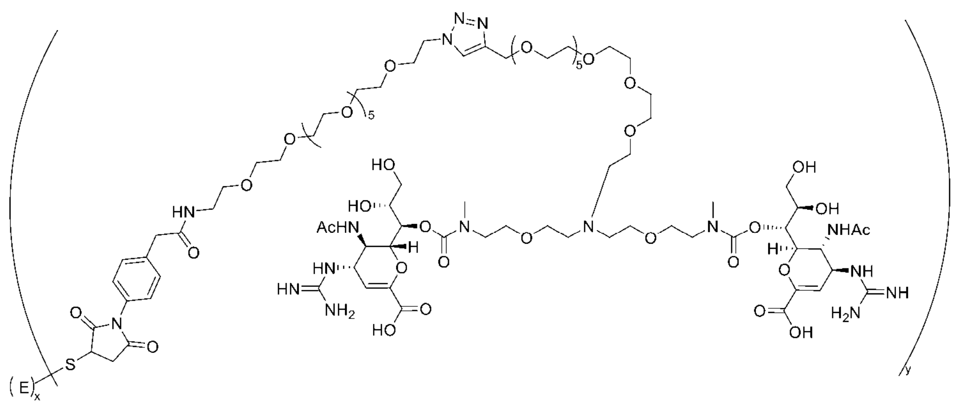 |

wherein m, n, x, y, and E are as defined herein.

In some embodiments, the ratio of n to m ranges from 1 to 20, preferably from 2 to 10, such as 2, 3, 4, 5, 6, 7, 8, 9, or 10.

In some embodiments, the conjugate or a pharmaceutically acceptable salt, ester, isomer, solvate, prodrug, or isotopically labeled compound thereof has an average DAR value between 0.5 and 10.0, such as 2.0-4.5 or 4.8-8.5.

In some embodiments, E comprises an Fc domain monomer or an Fc domain comprising said Fc domain monomer, wherein said Fc domain monomer comprises any one of the amino acid sequences set forth in SEQ ID Nos. 1-81, or an amino acid sequence having at least 95%, 96%, 97%, 98% or 99% identity to it, or consists of said amino acid sequence.

In some embodiments, E is capable of recognizing viral surface antigens, such as CR6261, CR8020, MEDI8897, Palivizumab, SD38, etc.; or

The Fc domain monomer may be an Fc domain monomer of any antibody subtype of immunoglobulin (e.g., IGHG1*01 (such as G1m(za)), IGHG1*07 (such as G1m(zax)), IGHG1*04 (such as G1m(zav)), IGHG1*03 (G1m(f)), IGHG1*08 (such as G1m(fa)), IGHG2*01, IGHG2*02, IGHG2*06, IGHG3*01, IGHG3*04, IGHG3*05, IGHG3*09, IGHG3*10, IGHG3*11, IGHG3*12, IGHG3*06, IGHG3*07, IGHG3*08, IGHG3*13, IGHG3*03, IGHG3*14, IGHG3*15, IGHG3*16, IGHG3*17, IGHG3*18, IGHG3*19, IGHG2*04, IGHG4*01, IGHG4*02, IGHG4*03).

In some embodiments, the conjugate has the following structure:

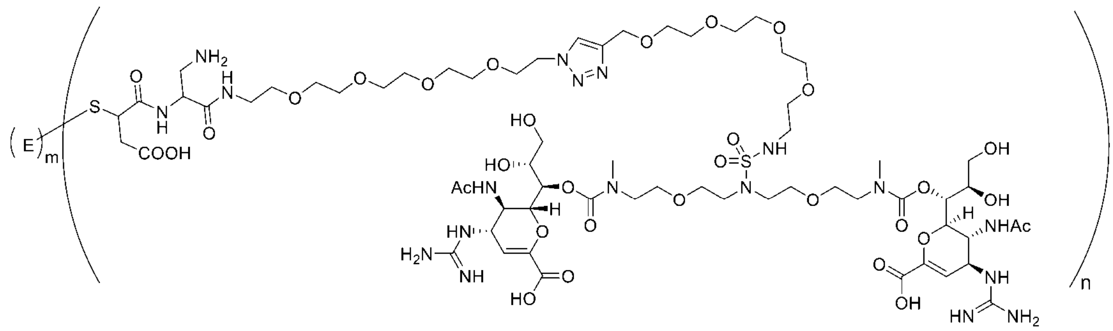

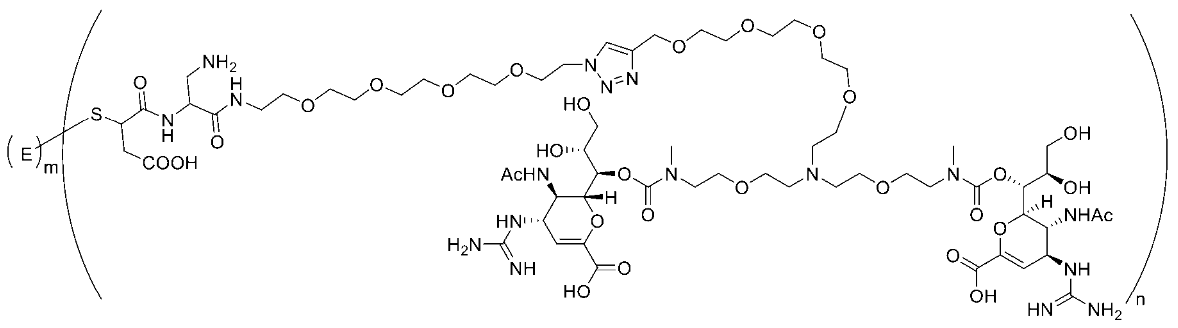

wherein E is as defined herein;

preferably, wherein E is SEQ ID NO: 67, SEQ ID NO: 69 or SEQ ID NO: 71;
and wherein m and n are as defined herein, and m is preferably 1.

In some embodiments, the carbon to which the

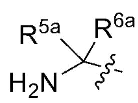

group (or its corresponding group) is attached (for example, in specific compounds containing the

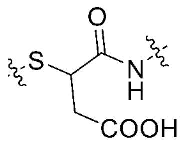

structure, it is the carbon attached to the nitrogen on the right side of

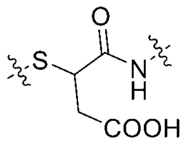

) is in the R or S configuration, and preferably the R configuration.

In some embodiments, the present invention provides a conjugate represented by a structure of Formula I-1, which comprises an anti-influenza small molecule compound conjugated to a protein (also referred to as a "conjugate of protein and anti-influenza compound"), exhibiting significant anti-influenza virus activity.

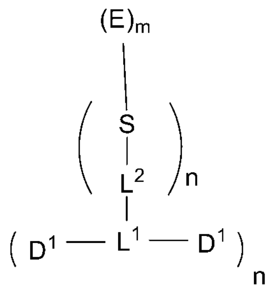

I-1

;

Specifically, the conjugate of protein and anti-influenza compound of the present invention has a small molecule (D1) with anti-influenza virus activity, which is linked to an antibody or its antibody fragment, Fc monomer, Fc domain, Fc binding peptide, albumin, or albumin binding peptide (E) by linkers ($L^1$ and $L^2$). The conjugate of protein and anti-influenza compound of the present invention exhibits significant anti-influenza virus activity, along with excellent *in vitro*/*in vivo* pharmacokinetic properties and safety, a long half-life, and good clinical application prospects.

In some embodiments, the present invention provides a conjugate of formula I-1, or a pharmaceutically acceptable salt, ester, isomer, solvate, prodrug, or isotopically labeled compound thereof,

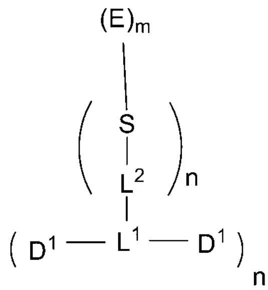

I-1

;

wherein,

m is 1 or 2;

n is an integer selected from 1 to 20, and preferably an integer from 1 to 10, such as 1, 2, 3, 4, 5, 6, 7, 8, 9, or 10; and both n values are the same;

$D^1$ is covalently linked to $L^1$, $L^1$ is linked to $L^2$ via a covalent bond, and $L^2$ is linked to a -S- bond via a covalent bond; E is selected from an antibody or antibody fragment, Fc domain monomer, Fc domain, Fc binding peptide, albumin, or albumin-binding peptide as defined above;

$D^1$ is an anti-influenza virus small molecule drug, and each independently selected from compounds with anti-influenza virus activity represented by formulas D-1-1 to D-1-8:

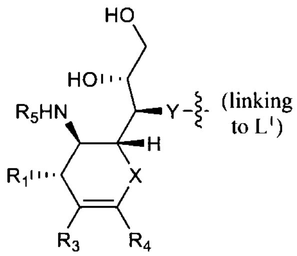
D-1-1

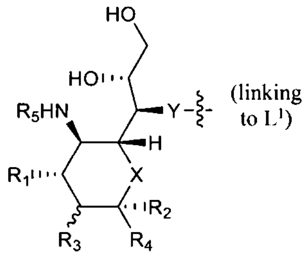
D-1-2

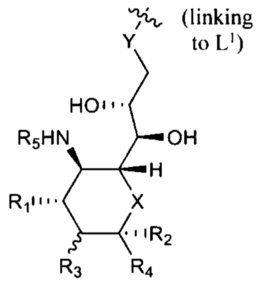
D-1-3

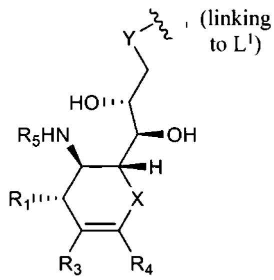
D-1-4

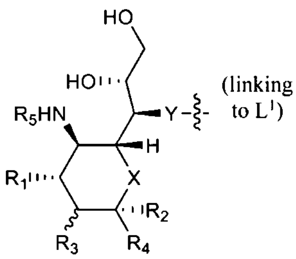
D-1-5

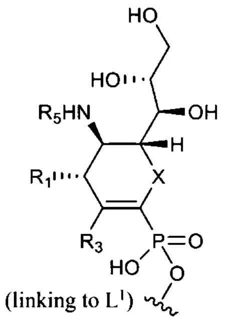
D-1-6

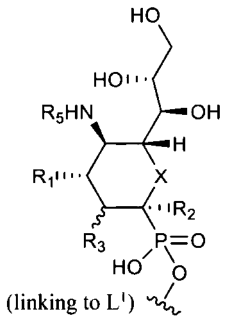
D-1-7

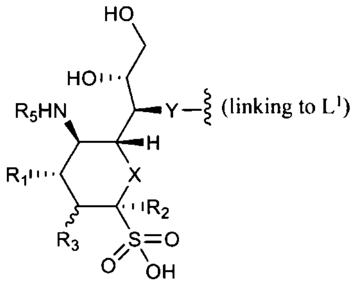
D-1-8

$R_1$ is selected from OH, $NH_2$, -NH(=NH)$NH_2$, and -NHC(=NH)$NHR_6$;
each of $R_2$ and $R_3$ is independently selected from H, OH, F, Cl, and Br;
$R_4$ is selected from -COOH, -P(=O)$(OH)_2$, and -$SO_3$H;
$R_5$ is selected from -$COC_{1-6}$ alkyl, -$COC_{1-6}$ haloalkyl, -$SO_2C_{1-6}$ alkyl and -$SO_2C_{1-6}$ haloalkyl; and is preferably selected from -$COCH_3$, -$COCF_3$, and -$SO_2CH_3$;
X is selected from O or S;
$R_6$ is selected from the following groups:

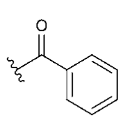 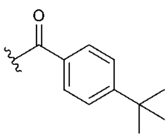 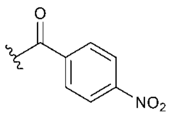 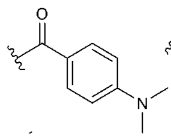 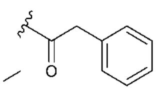

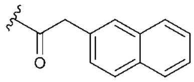 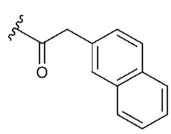 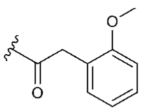 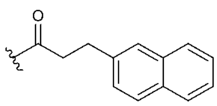

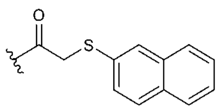 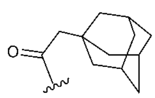 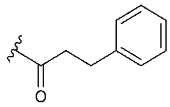 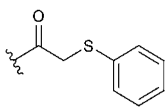

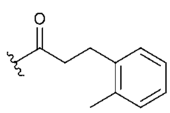 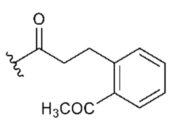 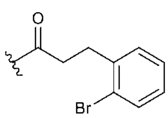 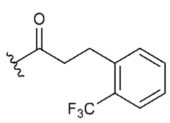

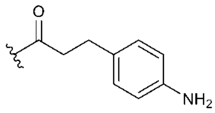 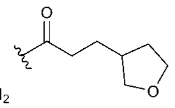 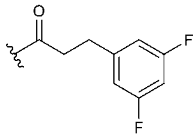 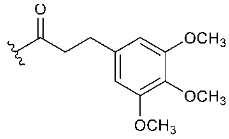

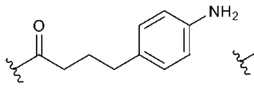 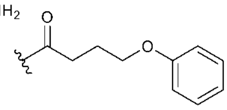 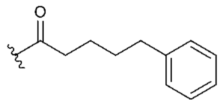

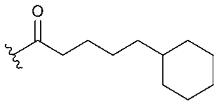 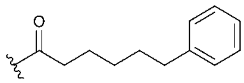 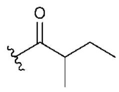 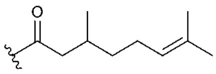

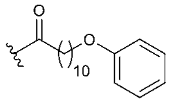 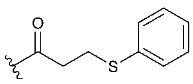 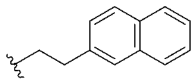 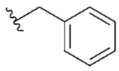

Y is selected from the following groups, wherein "(linking to $L^1$)" indicates that the group Y is linked to the end of $L^1$, and when N is drawn inside the ring, it indicates that N is an atom in the ring:

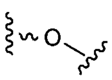 (linking to $L^1$) 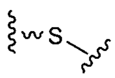 (linking to $L^1$) 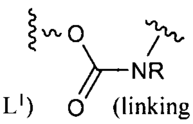 (linking to $L^1$) 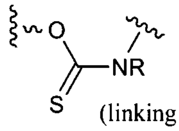 (linking to $L^1$) 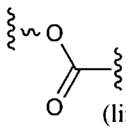 (linking to $L^1$)

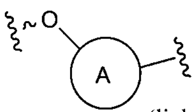 (linking to $L^1$) 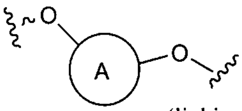 (linking to $L^1$) 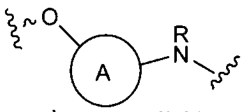 (linking to $L^1$) 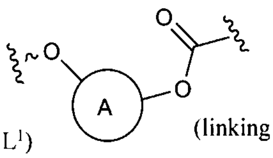 (linking to $L^1$)

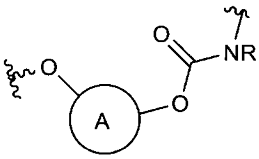 (linking to $L^1$) 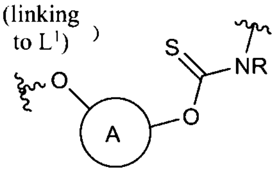 (linking to $L^1$) 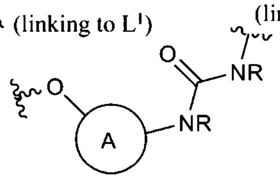 (linking to $L^1$) 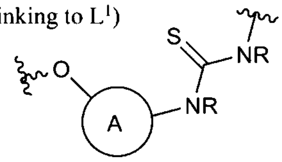 (linking to $L^1$)

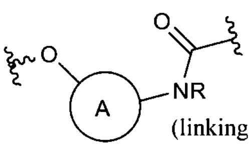 (linking to $L^1$) 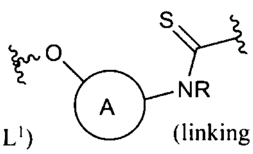 (linking to $L^1$) 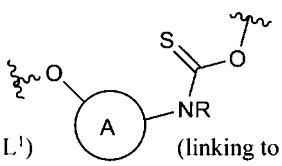 (linking to $L^1$) 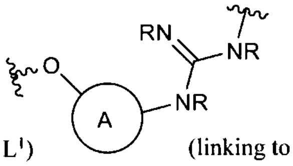 (linking to $L^1$)

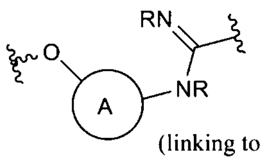 (linking to $L^1$) 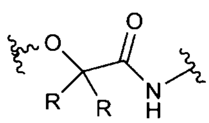 (linking to $L^1$) 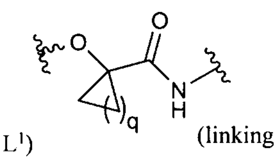 (linking to $L^1$) 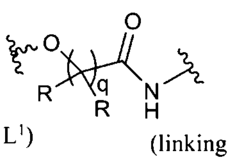 (linking to $L^1$)

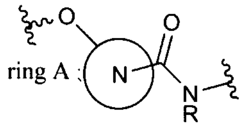
(linking to $L^1$)

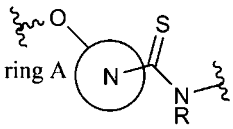
(linking to $L^1$)

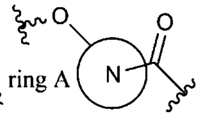
(linking to $L^1$)

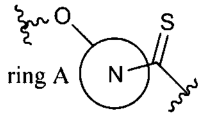
, (linking to $L^1$)

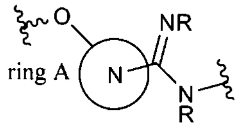
(linking to $L^1$)

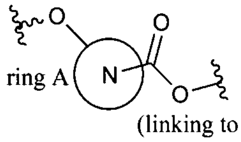
(linking to $L^1$)

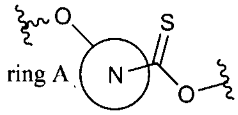
(linking to $L^1$)

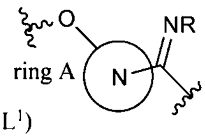
(linking to $L^1$)

wherein, ring A is selected from $C_3$-$C_{20}$ cycloalkyl, substituted $C_3$-$C_{20}$ cycloalkyl, $C_3$-$C_{20}$ cycloalkenyl, substituted $C_3$-$C_{20}$ cycloalkenyl, $C_6$-$C_{15}$ aryl, 3 to 20-membered heterocycloalkyl, substituted 3 to 20-membered heterocycloalkyl, substituted $C_6$-$C_{15}$ aryl, and substituted 5 to 15-membered heteroaryl;

each of R groups is independently selected from H, deuterium, optionally substituted $C_1$-$C_{20}$ alkyl, optionally substituted $C_2$-$C_{20}$ alkenyl, optionally substituted $C_3$-$C_{20}$ cycloalkyl, optionally substituted 3 to 20-membered heterocycloalkyl, optionally substituted $C_6$-$C_{15}$ aryl, and optionally substituted 5 to 15-membered heteroaryl;

q is 0, 1, 2, 3, 4, 5, 6, 7, 8, 9 or 10;

preferably, $D^1$ is an anti-influenza virus small molecule drug as follows:

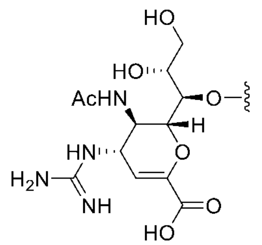

D-1a

$L^1$ is a linker connecting drug ($D^1$) and $L^2$, including but not limited to the following structures:

it should be understood that the bonds at the left and right ends of the following structure are linked to the oxygen atom of the drug ($D^1$), and the bond marked by the wavy line drawn on the intermediate amine group is linked to $L^2$;

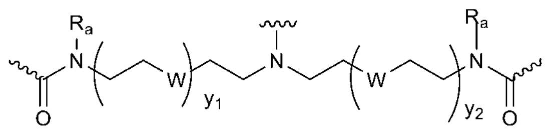

wherein,

W is selected from O, S, $NR^b$, $CH_{2-}$ or absence;

each of $R^a$ and $R^b$ is independently selected from H, optionally substituted $C_1$-$C_{20}$ alkyl, and optionally substituted $C_2$-$C_{20}$ alkenyl; preferably from H and methyl, and is more preferably methyl;

each of $y_1$ and $y_2$ is independently 0, 1, 2, 3, 4, 5 or 6;

$L^2$ is a linker connecting $L^1$ and E, which has a structure of formulas L2-1 to L2-14:

| $L^2$ structure number | Structure |
|---|---|
| Formula L2-1 | 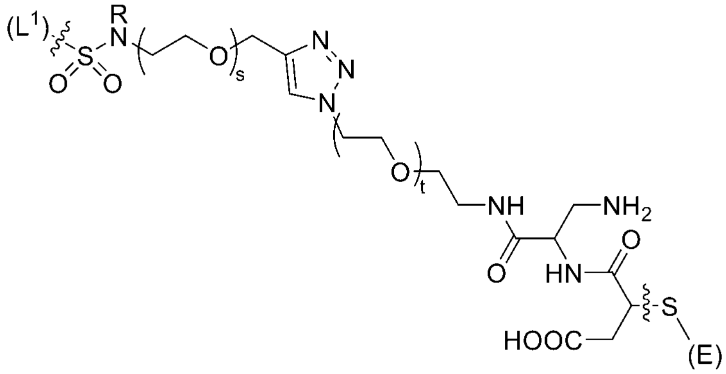 |
| Formula L2-2 | 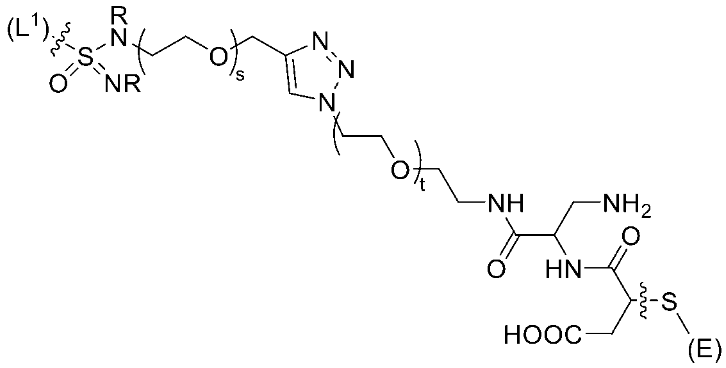 |
| Formula L2-3 | 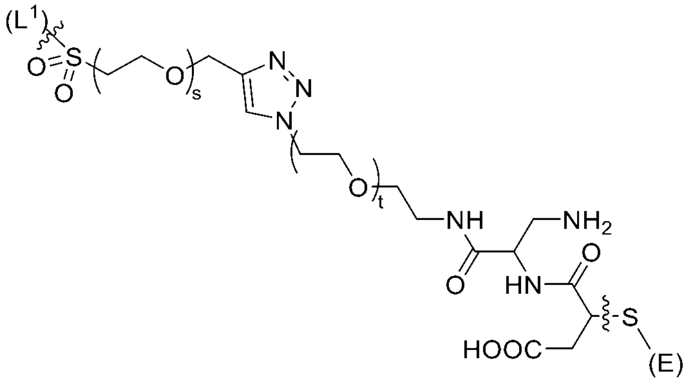 |
| Formula L2-4 | 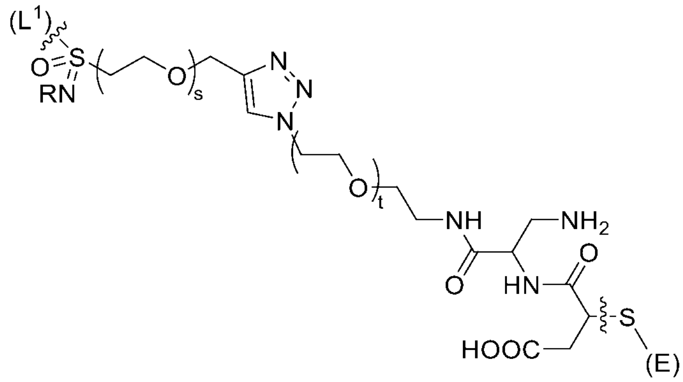 |

(continued)

| L2 structure number | Structure |
| --- | --- |
| Formula L2-5 | 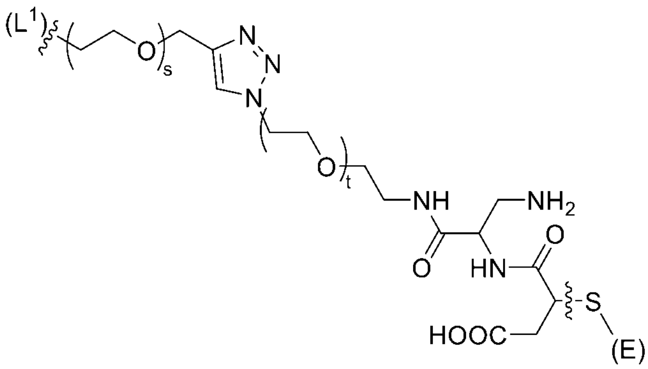 |
| Formula L2-6 | 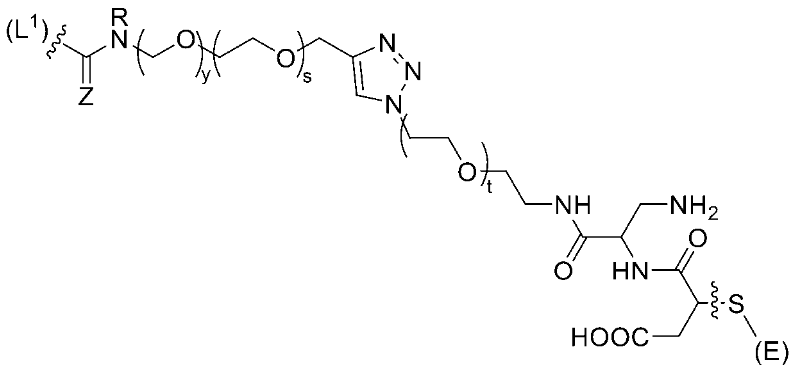 |
| Formula L2-7 | 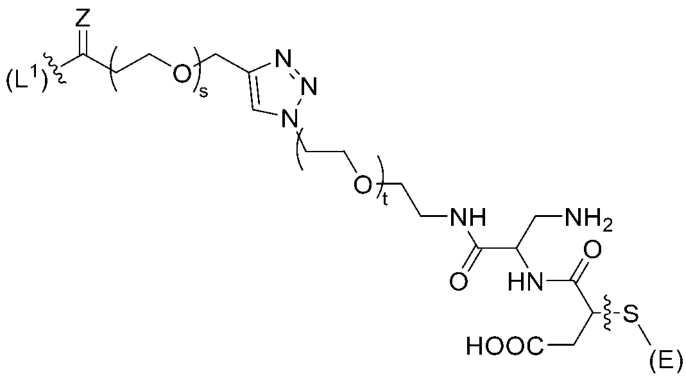 |
| Formula L2-8 | 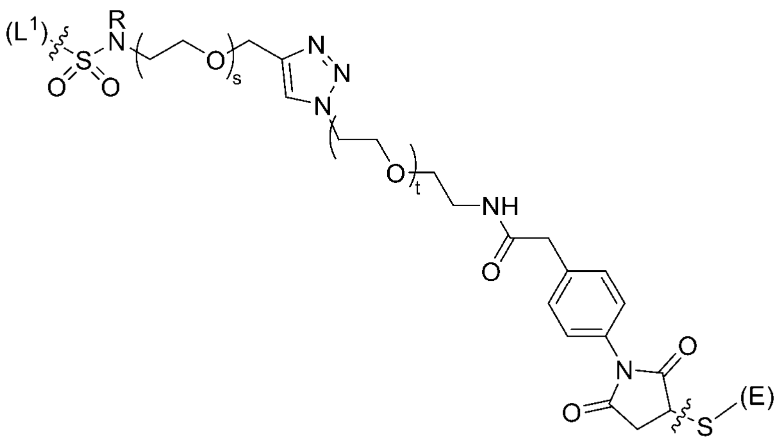 |

(continued)

| $L^2$ structure number | Structure |
|---|---|
| Formula L2-9 | 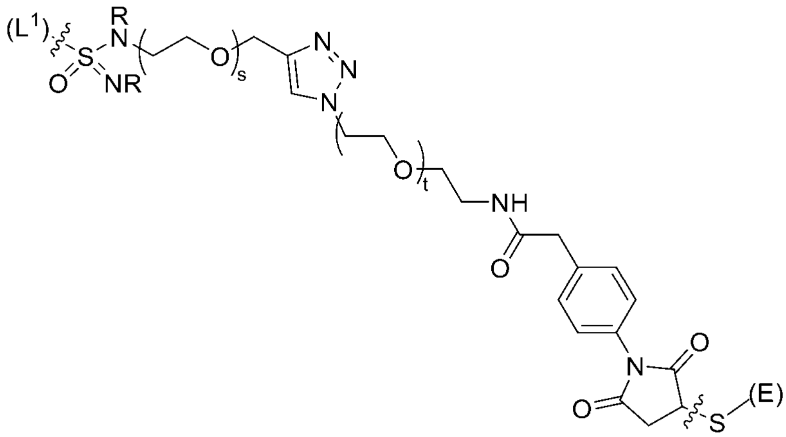 |
| Formula L2-10 | 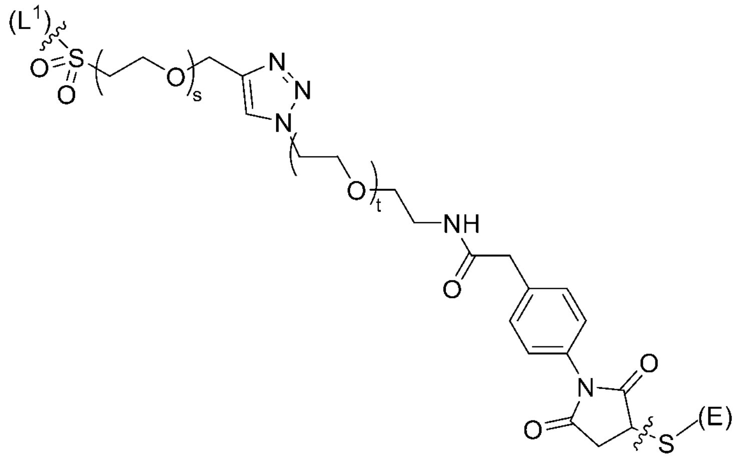 |
| Formula L2-11 | 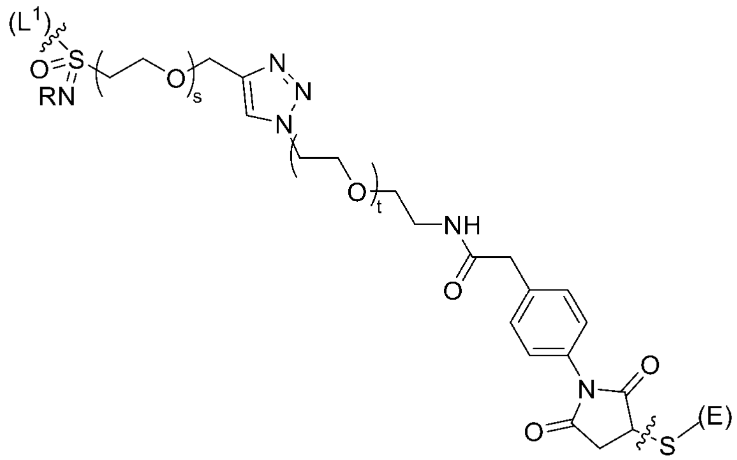 |

(continued)

| $L^2$ structure number | Structure |
|---|---|
| Formula L2-12 | 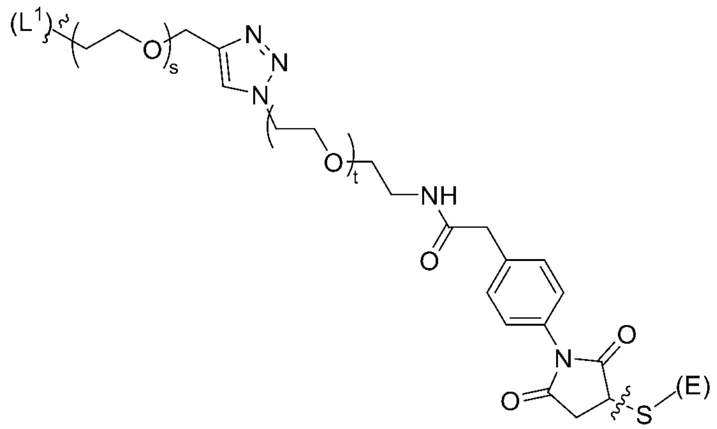 |
| Formula L2-13 | 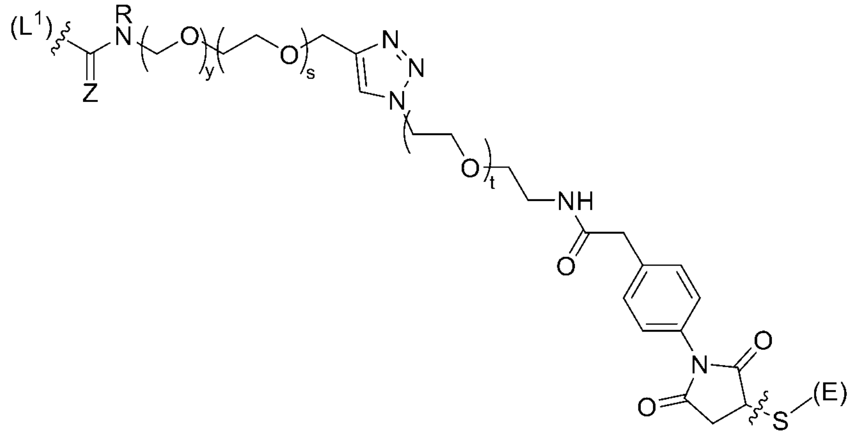 |
| Formula L2-14 | 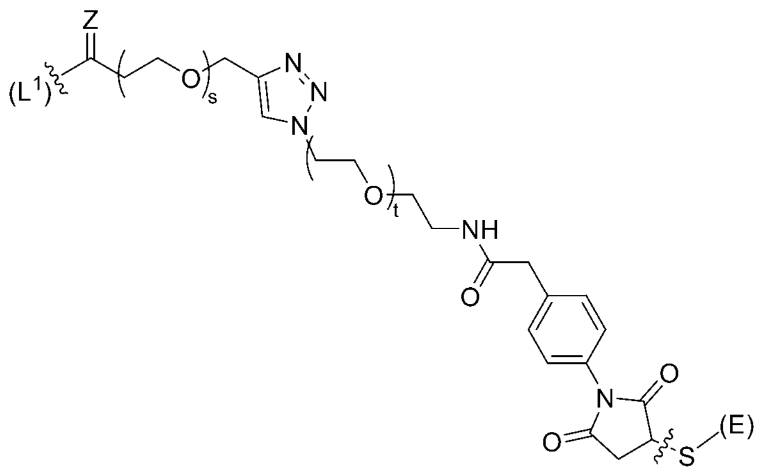 |

wherein,

Z is selected from NR, S and O;

R group is each independently selected from hydrogen, deuterium, optionally substituted $C_1$-$C_{20}$ alkyl, optionally substituted $C_2$-$C_{20}$ alkenyl, optionally substituted $C_3$-$C_{20}$ cycloalkyl, optionally substituted 3 to 20-membered heterocycloalkyl, optionally substituted $C_6$-$C_{15}$ aryl and optionally substituted 5 to 15-membered heteroaryl;

s and t are integers from 1 to 20; for example, 1, 2, 3, 4, 5, 6, 7, 8, 9, 10, 11, or 12;

y is 0 or 1.

It should be understood that in formulas L2-1 to L2-14, L2 refers to the part between the two wavy lines, and moieties ($L^1$) and (E)-S are not a component of $L^2$. They respectively indicate the ends of $L^2$ linked to $L^1$ and E, to more clearly show the connection direction of $L^2$, $L^2$ is linked to the sulfur atom of E via a covalent bond.

It should be understood that the "-S-" in formula I-1 is formed by the reaction between the sulfhydryl group generated by the reduction of the disulfide bond in E and the linker compound.

In some embodiments, $L^1$ is selected from following structures:

| The structure of $L^1$ |
|---|
| 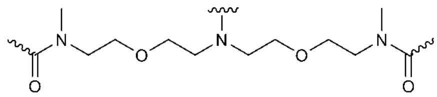 |
| 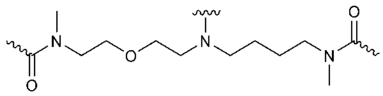 |
| 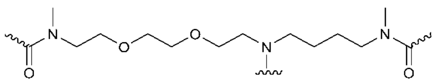 |
| 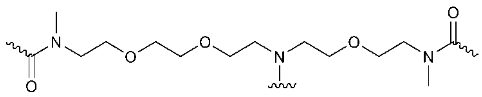 |
| 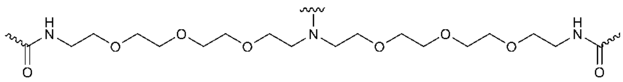 |
| 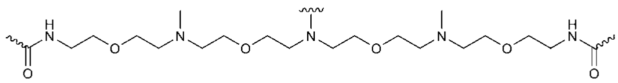 |
| 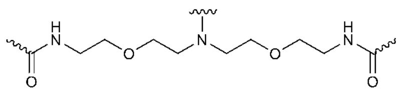 |
| 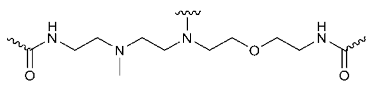 |
| 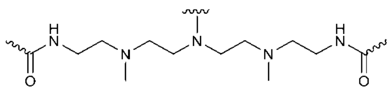 |
| 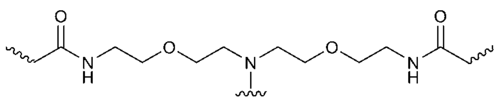 |
| 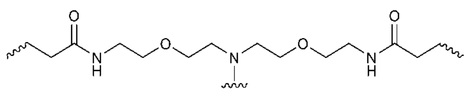 |
| 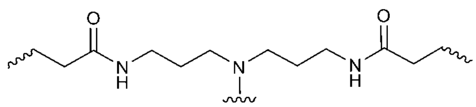 |
| 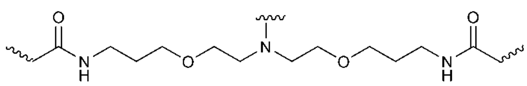 |

(continued)

| The structure of $L^1$ |
|---|
| 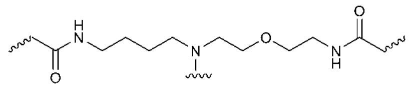 |
| 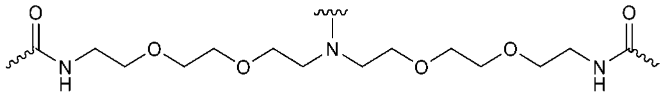 |
| 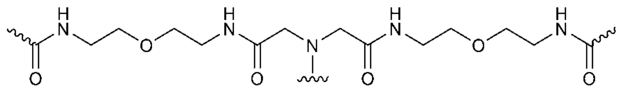 |
| 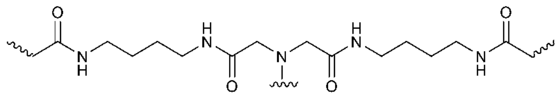 |
| 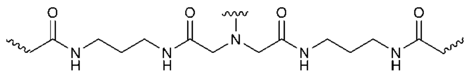 |
| 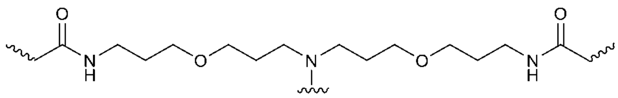 |
| 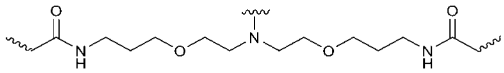 |
| 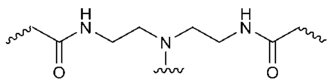 |
| 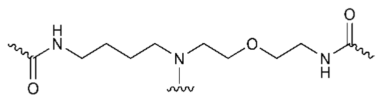 |
| 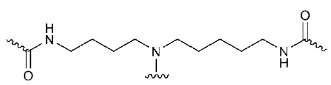 |
| 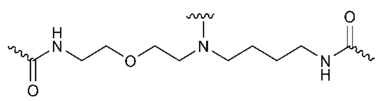 |
| 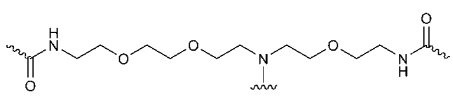 ; |

More preferably, $L^1$ is

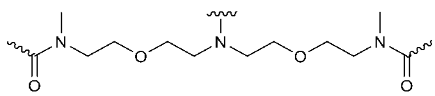 .

In some embodiments, $L^2$ is selected from formulas L2-1, L2-5, L2-7, L2-8, L2-12, and L2-14,

wherein Z is O,
R is H,
s and t are integers selected from 3 to 10, and preferably, s and t are 4 or 8.

In some embodiments, the conjugate of Formula I-1 is represented by a structure of Formula I-1-1:

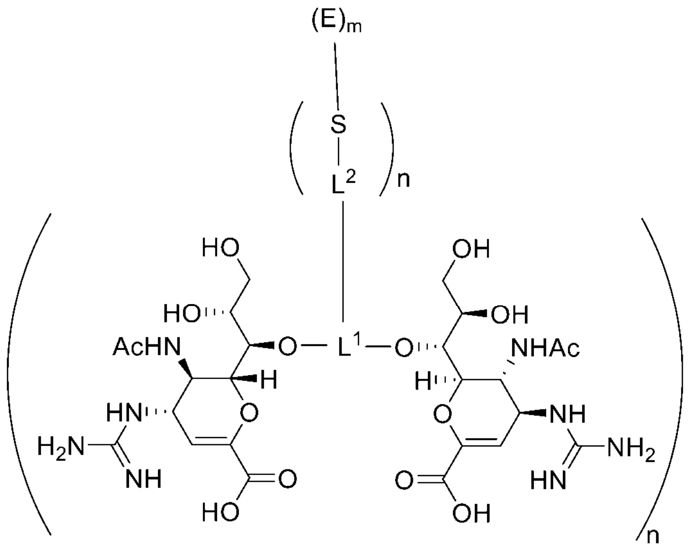

Formula I-1-1

wherein E, $L^1$, $L^2$, m, and n are as defined above.

In some embodiments, the conjugates are preferably selected from conjugates C-1 to C-12:

| Conjugate No. | The structure of conjugate |
| --- | --- |
| **C-1** | 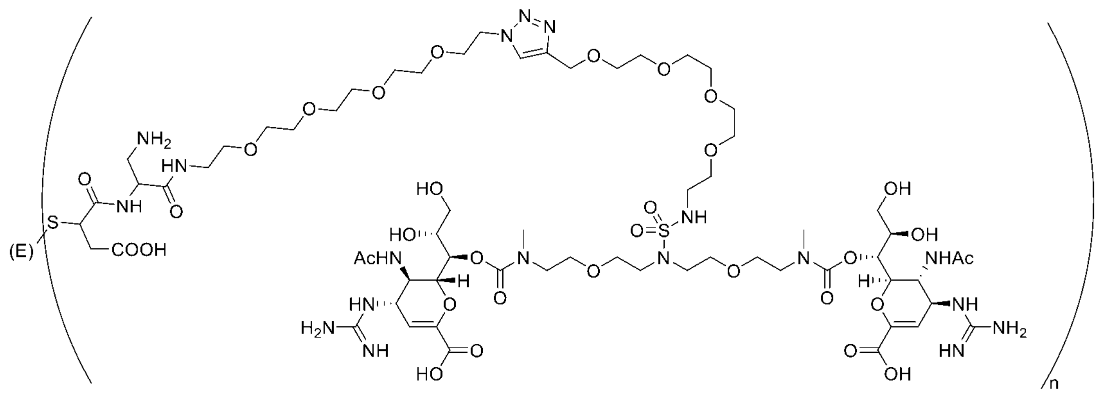 |
| **C-2** | 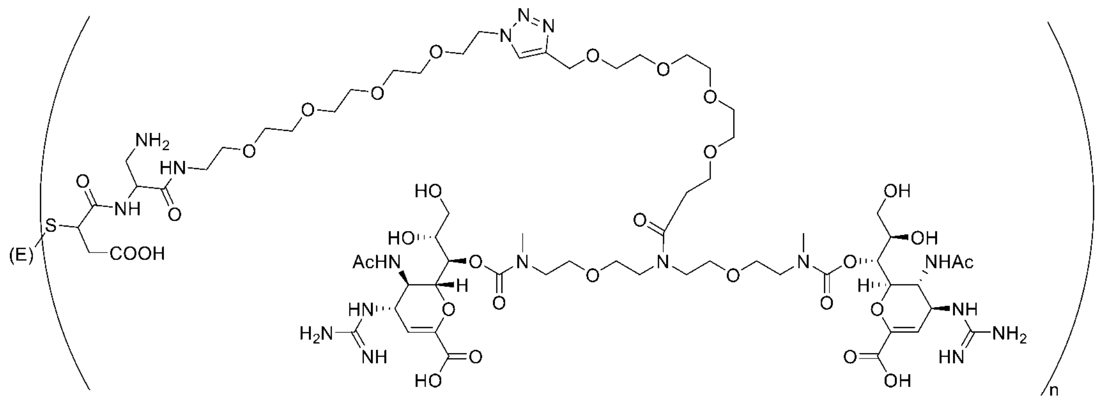 |

(continued)

| Conjugate No. | The structure of conjugate |
|---|---|
| C-3 | 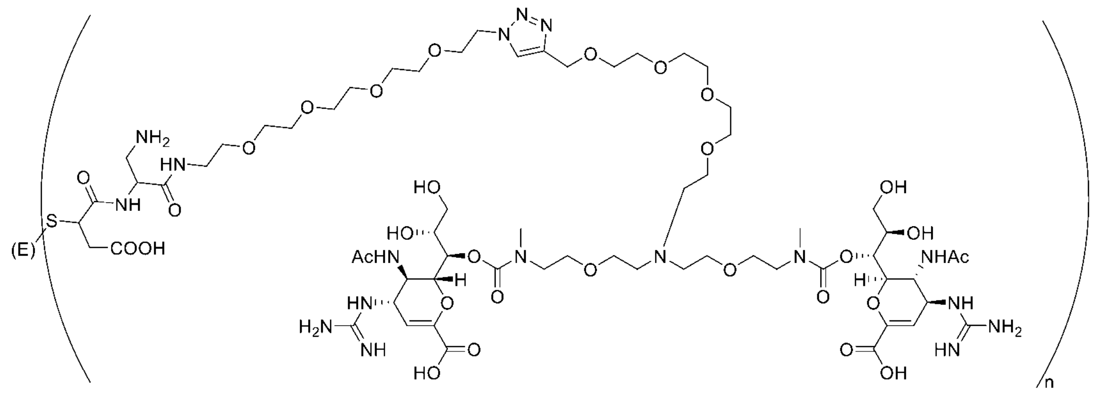 |
| C-4 | 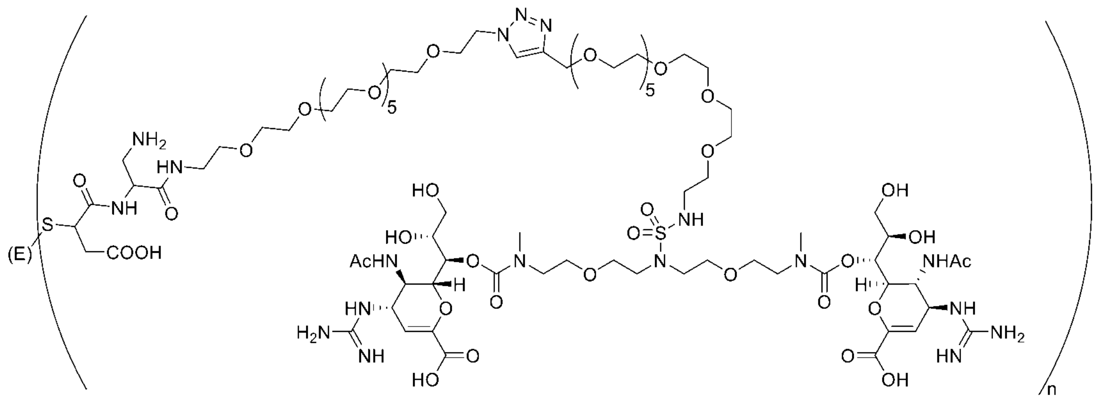 |

(continued)

| Conjugate No. | The structure of conjugate |
| --- | --- |
| C-5 | 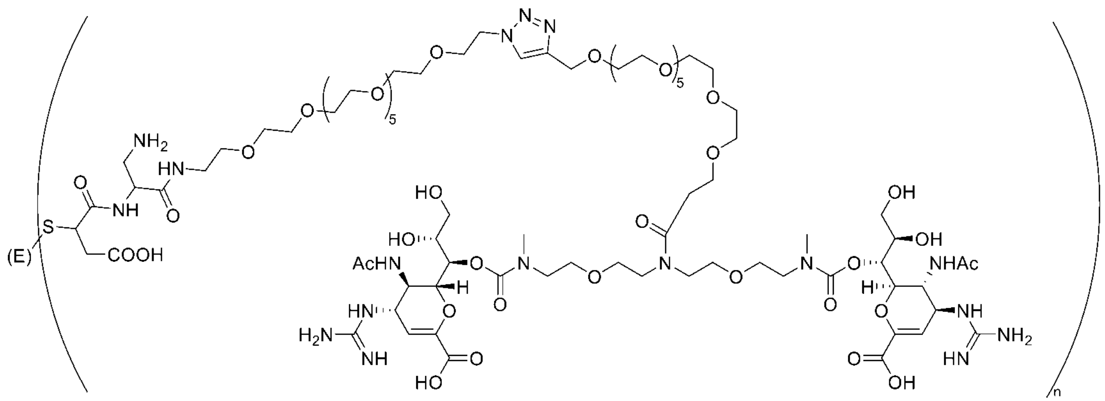 |
| C-6 | 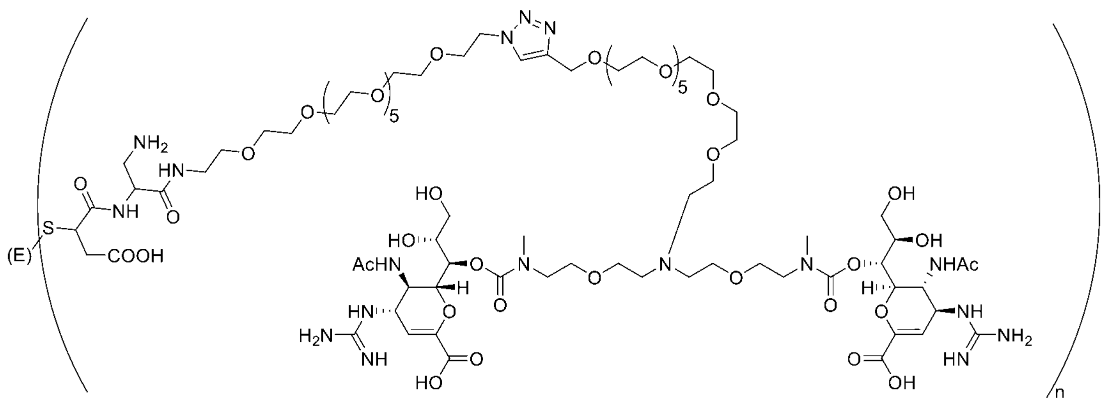 |

(continued)

| Conjugate No. | The structure of conjugate |
|---|---|
| **C-7** | 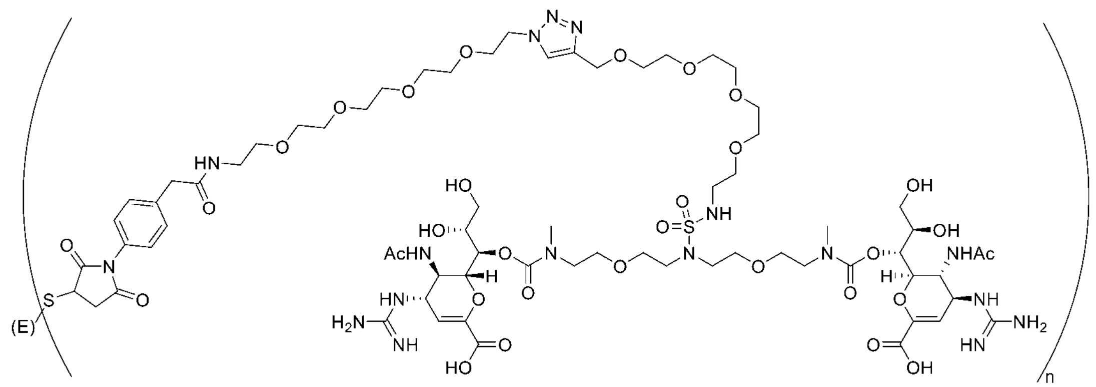 |
| **C-8** | 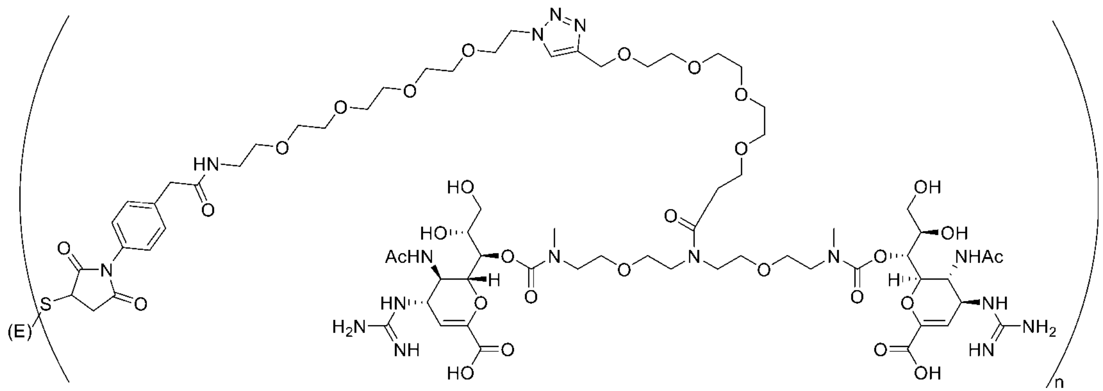 |

(continued)

| Conjugate No. | The structure of conjugate |
| --- | --- |
| C-9 | 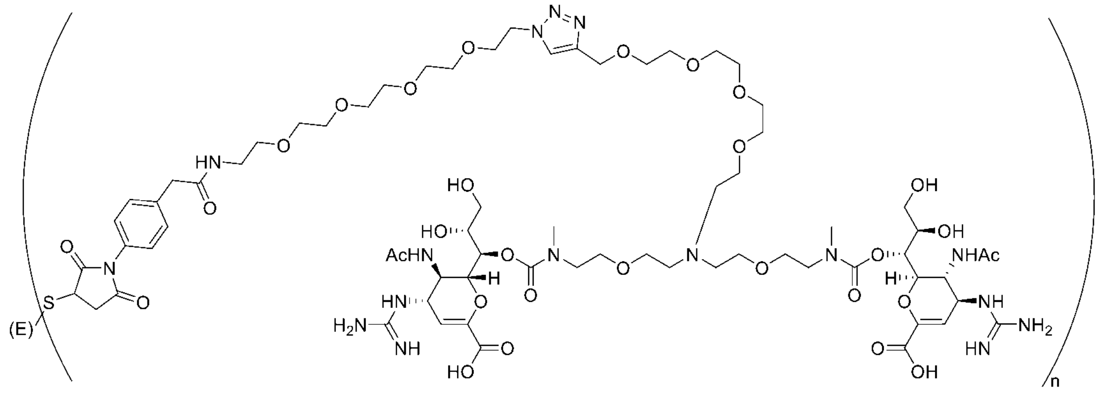 |
| C-10 | 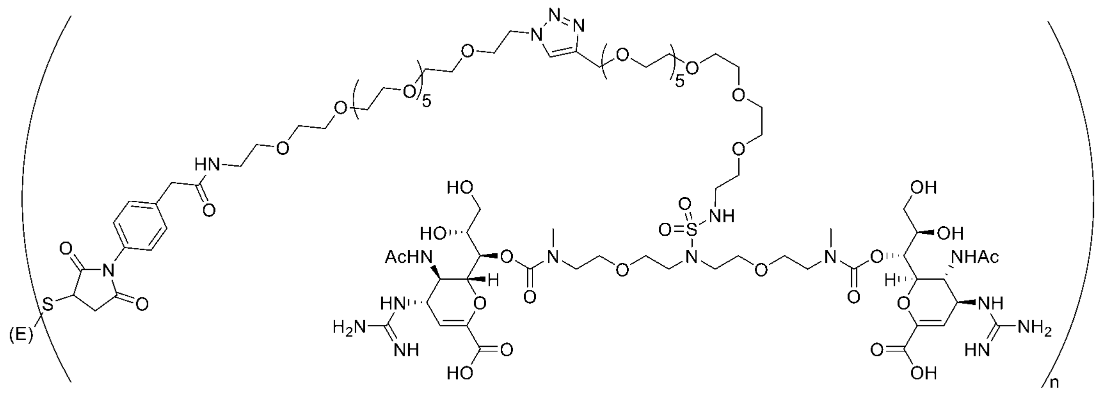 |

(continued)

| Conjugate No. | The structure of conjugate |
|---|---|
| C-11 | 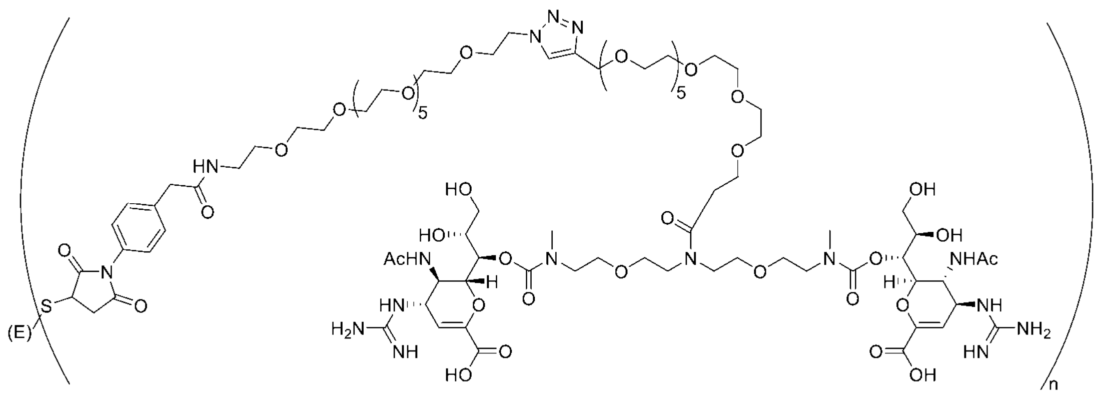 |
| C-12 | 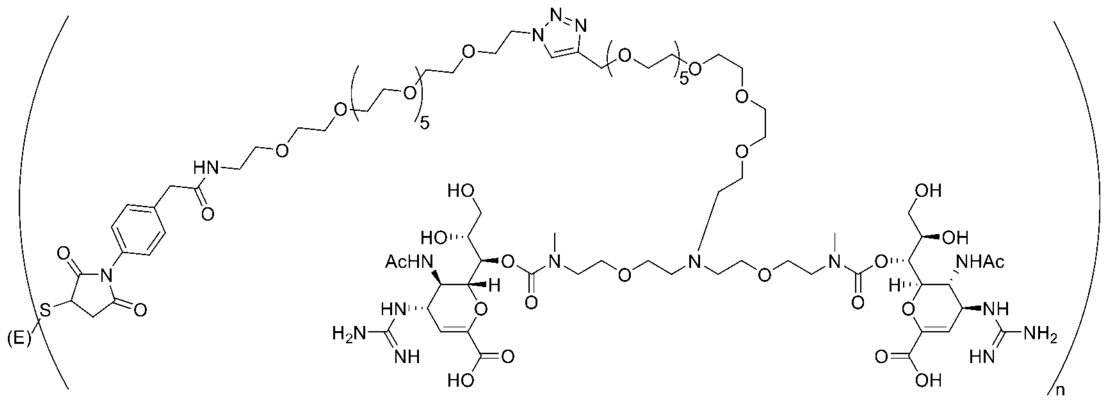 |

wherein n and E are defined as above.

It should be understood that in the above structures C-1 to C-12, (E) indicates the presence of one E in the structure, which is equivalent to the situation where m is 1 in the conjugate of formula I-1. Conjugates with similar structures herein can be similarly understood.

In some embodiments, in C-1 to C-6, the carbon to which the $NH_2$-$CH_2$- on the left side is linked (i.e., the carbon linked to the nitrogen on the right side of

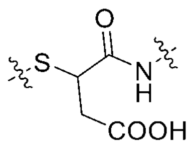

) is in the R or S configuration, and preferably in the R configuration.

In some embodiments, in the structure of formula I-1, the ratio of n to m ranges from 1 to 20, and preferably from 2 to 10, such as 2, 3, 4, 5, 6, 7, 8, 9, or 10.

In some embodiments, m is 1, and n is an integer selected from 1 to 10.

In some embodiments, the conjugate or a pharmaceutically acceptable salt, ester, isomer, solvate, prodrug, or isotopically labeled compound thereof has an average DAR of between 0.5 and 10.0.

In some embodiments, E is an antibody or antibody fragment, a Fc domain monomer, a Fc domain, and/or a Fc binding peptide. In some embodiments, E comprises or consists of a sequence having at least 95% identity to any one of the amino acid sequences set forth in SEQ ID Nos. 1-81.

In an embodiment, in the conjugate including a compound with a structure of Formula I-1, wherein the E monodomain is dimerized to form an Fc domain.

In some embodiments, the antibody fragment is an antigen-binding fragment.

In some embodiments, the antibody or antibody fragment is an antibody or antibody fragment of human, mouse, camelidae, goat, sheep, rabbit, chicken, guinea pig, hamster, horse, or rat.

In some embodiments, the antibody or antibody fragment is IgG, IgA, IgD, IgE, or IgM type of antibody or antibody fragment.

In some embodiments, the antibody fragment includes scFv, sdAb, Fab, Fab', Fab'2, F(ab')2, Fd, Fv, Feb, or SMIP.

In some embodiments, the antibody or antibody fragment is capable of recognizing viral surface antigens, such as CR6261, CR8020, MEDI8897, Palivizumab, SD38, etc.

In some embodiments, the Fc domain monomer may be an Fc domain monomer derived from any antibody subtype of immunoglobulin (e.g., IGHG1*01 (such as G1m(za)), IGHG1*07 (such as G1m(zax)), IGHG1*04 (such as G1m(zav)), IGHG1*03 (G1m(f)), IGHG1*08 (such as G1m(fa)), IGHG2*01, IGHG2*02, IGHG2*06, IGHG3*01, IGHG3*04, IGHG3*05, IGHG3*09, IGHG3*10, IGHG3*11, IGHG3*12, IGHG3*06, IGHG3*07, IGHG3*08, IGHG3*13, IGHG3*03, IGHG3*14, IGHG3*15, IGHG3*16, IGHG3*17, IGHG3*18, IGHG3*19, IGHG2*04, IGHG4*01, IGHG4*02, IGHG4*03) (such as Vidarsson et al., IgG subclasses and allotypes: from structure to effector function. Frontiers in Immunology. 5(520): 1-17 (2014)). The Fc domain monomer may comprise one or more non-natural amino acid sequences, serving as a site for site-specific conjugation of small molecules. The Fc domain monomer may comprise one or more solvent-exposed cysteine or lysine residues that have been site-specifically engineered, providing additional sites for conjugation of small molecules.

In some embodiments, L2 is linked to the S atom on E, wherein the S atom on E derives from cysteine (cys) residues or disulfide bonds in E.

In some embodiments, the Asn amino acid residue in E is substituted with an Ala amino acid residue to prevent conjugation at this site.

In some embodiments, E contains additional Cys amino acid residues to increase the conjugation sites without affecting the spatial three-dimensional structure of the antibody protein.

In some embodiments, the terminus of E comprises an additional amino acid sequence, such as a protein purification tag (e.g., six-histidine tag), or a signal peptide sequence (e.g., signal peptide sequence of human interleukin 2) or MVRS (SEQ ID NO: 82) amino acid sequence or ISAMVRS amino acid sequence (SEQ ID NO: 83).

In some embodiments, the protein purification tag is located at the C-terminus of E. In some embodiments, the signal peptide sequence is located at the N-terminus of E.

In some embodiments, the protein purification tag is selected from a six-histidine tag or a c-Myc tag. In some embodiments, the signal peptide is selected from human IL-2 signal peptide sequence (e.g., MYRMQLLSCIALSLALVTNS (SEQ ID NO: 84)), human serum albumin signal sequence (e.g., MKWVTFISLLFLFSSAYS (SEQ ID NO: 85)), or mouse heavy chain MIgG Vh signal sequence (e.g., MGWSCIILFLVATATGVHS (SEQ ID NO: 86)).

In some embodiments, the N-terminus of E also comprises a hinge region or a portion of hinge region.
In some embodiments, E comprises or consists of the amino acid sequence of any one of SEQ ID NOs: 1-81.
In some embodiments, E comprises or consists of an amino acid sequence that has at least 70%, 75%, 80%, 85%, 90%, 95%, 96%, 97%, 98%, 99% or 100% identity to the amino acid sequence of any one of SEQ ID NO: 1-81.
In some embodiments, E comprises or consists of the following amino acid sequence, the amino acid sequence

(i) has at least 70%, 75%, 80%, 85%, 90%, 95%, 96%, 97%, 98%, or 99% identity to the amino acid sequence of any one of SEQ ID NO: 1-81; and

(ii) comprises the N-terminal addition sequence, mutation, and/or C-terminal addition sequence described for each SEQ ID NO: in the "Sequences SEQ ID NO: 1-81 and their description" section of this disclosure.

In some embodiments, E also comprises a connector. In an embodiment, the connector refers to a short amino acid sequence consisting of amino acids, such as glycine (G) and/or serine (S) and/or threonine (T) residues used alone or in combination. In an embodiment, the connector comprises the amino acid sequence (G4S)n, where n is an integer equal to or greater than 1, for example, n is an integer of 1, 2, 3, 4, 5, 6, or 7. In an embodiment, the connector is GGGGS. In an embodiment, the connector comprises the amino acid sequence TS(G4S)n, wherein n is an integer equal to or greater than 1, for example, n is an integer of 2, 3, 4, 5, 6, or 7. In an embodiment, the connector comprises the amino acid sequence G(G4S)n, wherein n is an integer equal to or greater than 1, for example, n is an integer of 2, 3, 4, 5, 6, or 7.
In some embodiments, E comprises the amino acid sequence set forth in SEQ ID NO: 1. In some embodiments, E comprises an amino acid sequence having at least 70%, 75%, 80%, 85%, 90%, 95%, 96%, 97%, 98%, or 99% identity to the amino acid sequence set forth in SEQ ID NO: 1, and optionally, it has an IL2 signal sequence at its N-terminus.
In some embodiments, E comprises the amino acid sequence set forth in SEQ ID NO: 2. In some embodiments, E comprises an amino acid sequence having at least 70%, 75%, 80%, 85%, 90%, 95%, 96%, 97%, 98% or 99% identity to the amino acid sequence set forth in SEQ ID NO: 2.
In some embodiments, E comprises the amino acid sequence set forth in SEQ ID NO: 3. In some embodiments, E comprises an amino acid sequence having at least 70%, 75%, 80%, 85%, 90%, 95%, 96%, 97%, 98%, or 99% identity to the amino acid sequence set forth in SEQ ID NO: 3, and optionally, it has an IL2 signal sequence at its N-terminus as well as N-terminal MVRS amino acid sequence.
In some embodiments, E comprises the amino acid sequence set forth in SEQ ID NO: 4. In some embodiments, E comprises an amino acid sequence having at least 70%, 75%, 80%, 85%, 90%, 95%, 96%, 97%, 98% or 99% identity to the amino acid sequence set forth in SEQ ID NO: 4, and optionally, has an MVRS amino acid sequence at its N-terminus.
In some embodiments, E comprises the amino acid sequence set forth in SEQ ID NO: 5. In some embodiments, E comprises an amino acid sequence having at least 70%, 75%, 80%, 85%, 90%, 95%, 96%, 97%, 98% or 99% identity to the amino acid sequence set forth in SEQ ID NO: 5, and optionally, it has an IL2 signal sequence at its N-terminus and a six-histidine tag at its C-terminus.
In some embodiments, E comprises the amino acid sequence set forth in SEQ ID NO: 6. In some embodiments, E comprises an amino acid sequence having at least 70%, 75%, 80%, 85%, 90%, 95%, 96%, 97%, 98% or 99% identity to the amino acid sequence set forth in SEQ ID NO: 6, and optionally, it has a six-histidine tag at its C-terminus.
In some embodiments, E comprises the amino acid sequence set forth in SEQ ID NO: 7. In some embodiments, E comprises an amino acid sequence having at least 70%, 75%, 80%, 85%, 90%, 95%, 96%, 97%, 98% or 99% identity to the amino acid sequence set forth in SEQ ID NO: 7, and optionally, it has an IL2 signal sequence and MVRS amino acid sequence at its N-terminus and a six-histidine tag at its C-terminus.
In some embodiments, E comprises the amino acid sequence set forth in SEQ ID NO: 8. In some embodiments, E comprises an amino acid sequence having at least 70%, 75%, 80%, 85%, 90%, 95%, 96%, 97%, 98% or 99% identity to the amino acid sequence set forth in SEQ ID NO: 8, and optionally, it has an MVRS amino acid sequence at its N-terminus and a six-histidine tag at its C-terminus.
In some embodiments, E comprises the amino acid sequence set forth in SEQ ID NO: 9. In some embodiments, E comprises an amino acid sequence having at least 70%, 75%, 80%, 85%, 90%, 95%, 96%, 97%, 98% or 99% identity to the amino acid sequence set forth in SEQ ID NO: 9, and optionally, it has an IL2 signal sequence and MVRS amino acid sequence at its N-terminus, two additional cysteines in the hinge region (at position corresponding to the * position in SEQ ID NO: 9), and a six-histidine tag at its C-terminus.
In some embodiments, E comprises the amino acid sequence set forth in SEQ ID NO: 10. In some embodiments, E comprises an amino acid sequence having at least 70%, 75%, 80%, 85%, 90%, 95%, 96%, 97%, 98% or 99% identity to the amino acid sequence set forth in SEQ ID NO: 10, and optionally, it has an MVRS amino acid sequence at its N-terminus, a hinge region containing two additional cysteines (at position corresponding to the * positions in SEQ ID NO: 10), and a six-histidine tag at its C-terminus.
In some embodiments, E comprises the amino acid sequence set forth in SEQ ID NO: 11. In some embodiments,

E comprises an amino acid sequence having at least 70%, 75%, 80%, 85%, 90%, 95%, 96%, 97%, 98% or 99% identity to the amino acid sequence set forth in SEQ ID NO: 11, and optionally, it has an MVRS amino acid sequence at its N-terminus, and a hinge region containing two additional cysteines (at position corresponding to the * positions in SEQ ID NO: 11).

In some embodiments, E comprises the amino acid sequence set forth in SEQ ID NO: 12. In some embodiments, E comprises an amino acid sequence having at least 70%, 75%, 80%, 85%, 90%, 95%, 96%, 97%, 98% or 99% identity to the amino acid sequence set forth in SEQ ID NO: 12, and optionally, it has an IL2 signal sequence at its N-terminus and a substitution from Asn to Ala (at position corresponding to the * position in SEQ ID NO: 12), and a six-histidine tag at its C-terminus.

In some embodiments, E comprises the amino acid sequence set forth in SEQ ID NO: 13. In some embodiments, E comprises an amino acid sequence having at least 70%, 75%, 80%, 85%, 90%, 95%, 96%, 97%, 98% or 99% identity to the amino acid sequence set forth in SEQ ID NO: 13, and optionally, it contains a substitution from Asn to Ala (at position corresponding to the * position in SEQ ID NO: 13) and has a six-histidine tag at the C-terminus.

In some embodiments, E comprises the amino acid sequence set forth in SEQ ID NO: 14. In some embodiments, E comprises an amino acid sequence having at least 70%, 75%, 80%, 85%, 90%, 95%, 96%, 97%, 98% or 99% identity to the amino acid sequence set forth in SEQ ID NO: 14, and optionally, it has an IL2 signal sequence and MVRS amino acid sequence at its N-terminus, contains a substitution from Asn to Ala (at position corresponding to the * position in SEQ ID NO: 14), and has a six-histidine tag at its C-terminus.

In some embodiments, E comprises the amino acid sequence set forth in SEQ ID NO: 15. In some embodiments, E comprises an amino acid sequence having at least 70%, 75%, 80%, 85%, 90%, 95%, 96%, 97%, 98% or 99% identity to the amino acid sequence set forth in SEQ ID NO: 15, and optionally, it has an MVRS amino acid sequence at its N-terminus, contains a substitution from Asn to Ala (at position corresponding to the * position of SEQ ID NO: 15), and has a six-histidine tag at its C-terminus.

In some embodiments, E comprises the amino acid sequence set forth in SEQ ID NO: 16. In some embodiments, E comprises an amino acid sequence having at least 70%, 75%, 80%, 85%, 90%, 95%, 96%, 97%, 98% or 99% identity to the amino acid sequence set forth in SEQ ID NO: 16, and optionally, it has a human serum albumin signal sequence at its N-terminus and an N-terminal ISAMVRS amino acid sequence.

In some embodiments, E comprises the amino acid sequence set forth in SEQ ID NO: 17. In some embodiments, E comprises an amino acid sequence having at least 70%, 75%, 80%, 85%, 90%, 95%, 96%, 97%, 98% or 99% identity to the amino acid sequence set forth in SEQ ID NO: 17, and optionally, it has a human serum albumin signal sequence at its N-terminus and an N-terminal ISAMVRS amino acid sequence, and contains a C-terminal G4S connector and a C-terminal c-Myc tag.

In some embodiments, E comprises the amino acid sequence set forth in SEQ ID NO: 18. In some embodiments, E comprises an amino acid sequence having at least 70%, 75%, 80%, 85%, 90%, 95%, 96%, 97%, 98% or 99% identity to the amino acid sequence set forth in SEQ ID NO: 18, and optionally, it has an ISAMVRS amino acid sequence at its N-terminus, a G4S connector at its C-terminus, and a c-Myc tag at its C-terminus.

In some embodiments, E comprises the amino acid sequence set forth in SEQ ID NO: 19. In some embodiments, E comprises an amino acid sequence having at least 70%, 75%, 80%, 85%, 90%, 95%, 96%, 97%, 98% or 99% identity to the amino acid sequence of SEQ ID NO: 19, and optionally, it has a human serum albumin signal sequence and an ISAMVRS amino acid sequence at its N-terminus, and contains a lysine-to-serine change (at position corresponding to the * position in SEQ ID NO: 19) to prevent conjugation at this site.

In some embodiments, E comprises the amino acid sequence set forth in SEQ ID NO: 20. In some embodiments, E comprises an amino acid sequence having at least 70%, 75%, 80%, 85%, 90%, 95%, 96%, 97%, 98% or 99% identity to the amino acid sequence set forth in SEQ ID NO: 20, and optionally, it has an ISAMVRS amino acid sequence at its N-terminus and contains a lysine-to-serine change (at position corresponding to the * position in SEQ ID NO: 20) to prevent conjugation at this site.

In some embodiments, E comprises the amino acid sequence set forth in SEQ ID NO: 21. In some embodiments, E comprises an amino acid sequence having at least 70%, 75%, 80%, 85%, 90%, 95%, 96%, 97%, 98% or 99% identity to the amino acid sequence set forth in SEQ ID NO: 21, and optionally, it has a human serum albumin signal sequence and an ISAMVRS amino acid sequence at its N-terminus, contains a lysine-to-serine change (at position corresponding to the * position in SEQ ID NO: 21) to prevent conjugation at this site, and has a G4S connector and a c-Myc tag at its C-terminus.

In some embodiments, E comprises the amino acid sequence set forth in SEQ ID NO: 22. In some embodiments, E comprises an amino acid sequence having at least 70%, 75%, 80%, 85%, 90%, 95%, 96%, 97%, 98% or 99% identity to the amino acid sequence of SEQ ID NO: 22, and optionally, it has an ISAMVRS amino acid sequence at its N-terminus, contains a lysine-to-serine change (at position corresponding to the * position of SEQ ID NO: 22) to prevent conjugation at this site, and has a G4S connector and c-Myc tag at its C-terminus.

In some embodiments, E comprises the amino acid sequence set forth in SEQ ID NO: 23. In some embodiments, E comprises an amino acid sequence having at least 70%, 75%, 80%, 85%, 90%, 95%, 96%, 97%, 98% or 99% identity to the amino acid sequence set forth in SEQ ID NO: 23, and optionally, it has a human serum albumin signal sequence and an

ISAMVRS amino acid sequence at its N-terminus, contains a change from Asn to Ala (at position corresponding to the * position in SEQ ID NO: 23), and has a G4S connector and a c-Myc tag at its C-terminus.

In some embodiments, E comprises the amino acid sequence set forth in SEQ ID NO: 24. In some embodiments, E comprises an amino acid sequence having at least 70%, 75%, 80%, 85%, 90%, 95%, 96%, 97%, 98% or 99% identity to the amino acid sequence set forth in SEQ ID NO: 24, and optionally, it has an ISAMVRS amino acid sequence at its N-terminus, contains a change from Asn to Ala (at position corresponding to the * position in SEQ ID NO: 24), and has a G4S connector and a c-Myc tag at its C-terminus.

In some embodiments, E comprises the amino acid sequence set forth in SEQ ID NO: 25. In some embodiments, E comprises an amino acid sequence having at least 70%, 75%, 80%, 85%, 90%, 95%, 96%, 97%, 98% or 99% identity to the amino acid sequence of SEQ ID NO: 25, and optionally, it has a human serum albumin signal sequence and an ISAMVRS amino acid sequence at its N-terminus, contains H310A and H435A changes to prevent FcRn binding, and has a G4S connector and a c-Myc tag at its C-terminus.

In some embodiments, E comprises the amino acid sequence set forth in SEQ ID NO: 26. In some embodiments, E comprises an amino acid sequence having at least 70%, 75%, 80%, 85%, 90%, 95%, 96%, 97%, 98% or 99% identity to the amino acid sequence set forth in SEQ ID NO: 26, and optionally, it has an ISAMVRS amino acid sequence at its N-terminus, contains H310A and H435A changes to prevent FcRn binding, and has a G4S connector and a c-Myc tag at its C-terminus.

In some embodiments, E comprises the amino acid sequence set forth in SEQ ID NO: 27. In some embodiments, E comprises an amino acid sequence having at least 70%, 75%, 80%, 85%, 90%, 95%, 96%, 97%, 98% or 99% identity to the amino acid sequence set forth in SEQ ID NO: 27, and optionally, it has a human serum albumin signal sequence and ISAMVRS amino acid sequence at its N-terminus, as well as a G4S connector and a mutation (lysine to phenylalanine, at position corresponding to the bold positions in SEQ ID NO: 27) and c-Myc tag at its C-terminus.

In some embodiments, E comprises the amino acid sequence set forth in SEQ ID NO: 28. In some embodiments, E comprises an amino acid sequence having at least 70%, 75%, 80%, 85%, 90%, 95%, 96%, 97%, 98% or 99% identity to the amino acid sequence set forth in SEQ ID NO: 28, and optionally, it has an ISAMVRS amino acid sequence at its N-terminus, as well as a G4S connector and a mutation (lysine to phenylalanine, at position corresponding to the bold position in SEQ ID NO: 28) and c-Myc tag at its C-terminus.

In some embodiments, E comprises the amino acid sequence set forth in SEQ ID NO: 29. In some embodiments, E comprises an amino acid sequence having at least 70%, 75%, 80%, 85%, 90%, 95%, 96%, 97%, 98% or 99% identity to the amino acid sequence set forth in SEQ ID NO: 29, and optionally, it has a human serum albumin signal sequence and an ISAMVRS amino acid sequence at its N-terminus, contains a substitution of Asn with Ala, and has a G4S connector and a mutation (lysine to phenylalanine, at position corresponding to the bold position in SEQ ID NO: 29) and a c-Myc tag at its C-terminus.

In some embodiments, E comprises the amino acid sequence set forth in SEQ ID NO: 30. In some embodiments, E comprises an amino acid sequence having at least 70%, 75%, 80%, 85%, 90%, 95%, 96%, 97%, 98% or 99% identity to the amino acid sequence set forth in SEQ ID NO: 30, and optionally, it has an ISAMVRS amino acid sequence at its N-terminus, contains a substitution of Asn with Ala (at position corresponding to the * position of SEQ ID NO: 30), and has a G4S connector and a mutation (lysine to phenylalanine, at position corresponding to the bold position in SEQ ID NO: 30) and a c-Myc tag at its C-terminus.

In some embodiments, E comprises the amino acid sequence set forth in SEQ ID NO: 31. In some embodiments, E comprises an amino acid sequence having at least 70%, 75%, 80%, 85%, 90%, 95%, 96%, 97%, 98% or 99% identity to the amino acid sequence set forth in SEQ ID NO: 31, and optionally, it has a human serum albumin signal sequence at its N-terminus, an allotype G1m(fa), as well as a G4S connector and a mutation (lysine to phenylalanine, at position corresponding to the bold positions in SEQ ID NO: 31) and a c-Myc tag at its C-terminus.

In some embodiments, E comprises the amino acid sequence set forth in SEQ ID NO: 32. In some embodiments, E comprises an amino acid sequence having at least 70%, 75%, 80%, 85%, 90%, 95%, 96%, 97%, 98% or 99% identity to the amino acid sequence set forth in SEQ ID NO: 32, and optionally, it has a human serum albumin signal sequence at its N-terminus and an allotype G1m(fa).

In some embodiments, E comprises the amino acid sequence set forth in SEQ ID NO: 33. In some embodiments, E comprises an amino acid sequence having at least 70%, 75%, 80%, 85%, 90%, 95%, 96%, 97%, 98% or 99% identity to the amino acid sequence set forth in SEQ ID NO: 33, and optionally, it has an MVRS amino acid sequence at its N-terminus and contains YTE triple mutations.

In some embodiments, E comprises the amino acid sequence set forth in SEQ ID NO: 34. In some embodiments, E comprises an amino acid sequence having at least 70%, 75%, 80%, 85%, 90%, 95%, 96%, 97%, 98% or 99% identity to the amino acid sequence set forth in SEQ ID NO: 34, and optionally, it has a human serum albumin signal sequence at its N-terminus, contains a hinge region EPKSS amino acid sequence of mature human Fc-IgG1, and also comprises a change from cysteine to serine (at position corresponding to the # position in SEQ ID NO: 34) and an allotype G1m(fa).

In some embodiments, E comprises the amino acid sequence set forth in SEQ ID NO: 35. In some embodiments,

E comprises an amino acid sequence having at least 70%, 75%, 80%, 85%, 90%, 95%, 96%, 97%, 98% or 99% identity to the amino acid sequence set forth in SEQ ID NO: 35, and optionally, it has a murine IgG signal sequence at its N-terminus, has the hinge region EPKSSD amino acid sequence of mature human Fc-IgG removed, and has an allotype G1m(fa).

In some embodiments, E comprises the amino acid sequence set forth in SEQ ID NO: 36. In some embodiments, E comprises an amino acid sequence having at least 70%, 75%, 80%, 85%, 90%, 95%, 96%, 97%, 98% or 99% identity to the amino acid sequence set forth in SEQ ID NO: 36, and optionally, it is removed of the hinge region EPKSSD amino acid sequence of mature human Fc-IgG at its N-terminus, and has an allotype G1m(fa), and contains YTE triple mutations.

In some embodiments, E comprises the amino acid sequence set forth in SEQ ID NO: 37. In some embodiments, E comprises an amino acid sequence having at least 70%, 75%, 80%, 85%, 90%, 95%, 96%, 97%, 98% or 99% identity to the amino acid sequence set forth in SEQ ID NO: 37, and optionally, it is removed of the hinge region EPKSSD amino acid sequence of mature human Fc-IgG at N-terminus, and has LS double mutations and allotype G1m(fa).

In some embodiments, E comprises the amino acid sequence set forth in SEQ ID NO: 38. In some embodiments, E comprises an amino acid sequence having at least 70%, 75%, 80%, 85%, 90%, 95%, 96%, 97%, 98% or 99% identity to the amino acid sequence set forth in SEQ ID NO: 38, and optionally, it has a human serum albumin signal sequence at its N-terminus, contains YTE triple mutations, has an allotype G1m(fa), and has a G4S connector and a c-Myc tag at its C-terminus.

In some embodiments, E comprises the amino acid sequence set forth in SEQ ID NO: 39. In some embodiments, E comprises an amino acid sequence having at least 70%, 75%, 80%, 85%, 90%, 95%, 96%, 97%, 98% or 99% identity to the amino acid sequence set forth in SEQ ID NO: 39, and optionally, it has the amino acid defined for the corresponding position by SEQ ID NO: 39 at the position corresponding to X position shown in SEQ ID NO: 39.

In some embodiments, E comprises the amino acid sequence set forth in SEQ ID NO: 40. In some embodiments, E comprises an amino acid sequence having at least 70%, 75%, 80%, 85%, 90%, 95%, 96%, 97%, 98% or 99% identity to the amino acid sequence set forth in SEQ ID NO: 40, and optionally, it has the amino acid defined for the corresponding position by SEQ ID NO: 40 at the position corresponding to X position shown in SEQ ID NO: 40.

In some embodiments, E comprises the amino acid sequence set forth in SEQ ID NO: 41. In some embodiments, E comprises an amino acid sequence having at least 70%, 75%, 80%, 85%, 90%, 95%, 96%, 97%, 98% or 99% identity to the amino acid sequence set forth in SEQ ID NO: 41, and optionally, it has YTE triple mutations, and has the amino acid defined for the corresponding position by SEQ ID NO: 41 at the position corresponding to X position shown in SEQ ID NO: 41.

In some embodiments, E comprises the amino acid sequence set forth in SEQ ID NO: 42. In some embodiments, E comprises an amino acid sequence having at least 70%, 75%, 80%, 85%, 90%, 95%, 96%, 97%, 98% or 99% identity to the amino acid sequence set forth in SEQ ID NO: 42, and optionally, it has YTE triple mutations and has allotype G1m(fa).

In some embodiments, E comprises the amino acid sequence set forth in SEQ ID NO: 43. In some embodiments, E comprises an amino acid sequence having at least 70%, 75%, 80%, 85%, 90%, 95%, 96%, 97%, 98% or 99% identity to the amino acid sequence set forth in SEQ ID NO: 43, and optionally, it has YTE triple mutations and has an allotype G1m(f).

In some embodiments, E comprises the amino acid sequence set forth in SEQ ID NO: 44. In some embodiments, E comprises an amino acid sequence having at least 70%, 75%, 80%, 85%, 90%, 95%, 96%, 97%, 98% or 99% identity to the amino acid sequence set forth in SEQ ID NO: 44, and optionally, it has LS dual mutations and has the amino acid defined for the corresponding position by SEQ ID NO: 44 at the position corresponding to X position shown in SEQ ID NO: 44.

In some embodiments, E comprises the amino acid sequence set forth in SEQ ID NO: 45. In some embodiments, E comprises an amino acid sequence having at least 70%, 75%, 80%, 85%, 90%, 95%, 96%, 97%, 98% or 99% identity to the amino acid sequence set forth in SEQ ID NO: 45, and optionally, it has LS dual mutations and has an allotype G1m(fa).

In some embodiments, E comprises the amino acid sequence set forth in SEQ ID NO: 46. In some embodiments, E comprises an amino acid sequence having at least 70%, 75%, 80%, 85%, 90%, 95%, 96%, 97%, 98% or 99% identity to the amino acid sequence set forth in SEQ ID NO: 46, and optionally, it has LS dual mutations and has an allotype G1m(f).

In some embodiments, E comprises the amino acid sequence set forth in SEQ ID NO: 47. In some embodiments, E comprises an amino acid sequence having at least 70%, 75%, 80%, 85%, 90%, 95%, 96%, 97%, 98% or 99% identity to the amino acid sequence set forth in SEQ ID NO:47, and optionally, it has a mouse heavy chain MIgG Vh signal sequence at its N-terminus, contains a change from cysteine to serine (at position corresponding to # position of SEQ ID NO:47), and has the amino acid defined for the corresponding position by SEQ ID NO:47 at the position corresponding to position X shown in SEQ ID NO:47.

In some embodiments, E comprises the amino acid sequence set forth in SEQ ID NO: 48. In some embodiments, E comprises an amino acid sequence having at least 70%, 75%, 80%, 85%, 90%, 95%, 96%, 97%, 98% or 99% identity to the amino acid sequence set forth in SEQ ID NO: 48, and optionally, it has a mouse heavy chain MIgG Vh signal sequence at its N-terminus, has a change from cysteine to serine (at position corresponding to # position of SEQ ID NO: 48), and has an allotype G1m(fa).

In some embodiments, E comprises the amino acid sequence set forth in SEQ ID NO: 49. In some embodiments,

E comprises an amino acid sequence having at least 70%, 75%, 80%, 85%, 90%, 95%, 96%, 97%, 98% or 99% identity to the amino acid sequence set forth in SEQ ID NO: 49, and optionally, it has a mouse heavy chain MIgG Vh signal sequence at its N-terminus, contains a change from cysteine to serine (at position corresponding to # position of SEQ ID NO: 49), and contains an allotype G1m(f).

In some embodiments, E comprises the amino acid sequence set forth in SEQ ID NO: 50. In some embodiments, E comprises an amino acid sequence having at least 70%, 75%, 80%, 85%, 90%, 95%, 96%, 97%, 98%, or 99% identity to the amino acid sequence set forth in SEQ ID NO: 50, and optionally, it has a mouse heavy chain MIgG Vh signal sequence at its N-terminus, contains a change from cysteine to serine (at position corresponding to # position of SEQ ID NO: 50), comprises M428L and N434S mutations, and has an allotype G1m(fa).

In some embodiments, E comprises the amino acid sequence set forth in SEQ ID NO: 51. In some embodiments, E comprises an amino acid sequence having at least 70%, 75%, 80%, 85%, 90%, 95%, 96%, 97%, 98% or 99% identity to the amino acid sequence set forth in SEQ ID NO: 51, and optionally, it has a mouse heavy chain MIgG Vh signal sequence at its N-terminus, a change from cysteine to serine (at position corresponding to # position of SEQ ID NO: 51), M428L and N434S mutations, and an allotype G1m(f).

In some embodiments, E comprises the amino acid sequence set forth in SEQ ID NO: 52. In some embodiments, E comprises an amino acid sequence having at least 70%, 75%, 80%, 85%, 90%, 95%, 96%, 97%, 98% or 99% identity to the amino acid sequence set forth in SEQ ID NO: 52, and optionally, it has a mouse heavy chain MIgG Vh signal sequence at its N-terminus, a change from cysteine to serine (at position corresponding to # position of SEQ ID NO: 52), YTE triple mutations, and an allotype G1m(fa).

In some embodiments, E comprises the amino acid sequence set forth in SEQ ID NO: 53. In some embodiments, E comprises an amino acid sequence having at least 70%, 75%, 80%, 85%, 90%, 95%, 96%, 97%, 98% or 99% identity to the amino acid sequence set forth in SEQ ID NO: 53, and optionally, it has a mouse heavy chain MIgG Vh signal sequence at its N-terminus, a change from cysteine to serine (at position corresponding to # position of SEQ ID NO: 53), YTE triple mutations, and an allotype G1m(f).

In some embodiments, E comprises the amino acid sequence set forth in SEQ ID NO: 54. In some embodiments, E comprises an amino acid sequence having at least 70%, 75%, 80%, 85%, 90%, 95%, 96%, 97%, 98% or 99% identity to the amino acid sequence set forth in SEQ ID NO: 54, and optionally, it has a mouse heavy chain MIgG Vh signal sequence and ISAMVRS amino acid sequence at its N-terminus, has 428L and N434S mutations, has a G4S connector and c-Myc tag at its C-terminus, and contains an allotype G1m(f).

In some embodiments, E comprises the amino acid sequence set forth in SEQ ID NO: 55. In some embodiments, E comprises an amino acid sequence having at least 70%, 75%, 80%, 85%, 90%, 95%, 96%, 97%, 98% or 99% identity to the amino acid sequence set forth in SEQ ID NO: 55, and optionally, it has a mouse heavy chain MIgG Vh signal sequence and ISAMVRS amino acid sequence at its N-terminus, has M428L and N434S mutations, has a G4S connector and c-Myc tag at its C-terminus, and contains an allotype G1m(fa).

In some embodiments, E comprises the amino acid sequence set forth in SEQ ID NO: 56. In some embodiments, E comprises an amino acid sequence having at least 70%, 75%, 80%, 85%, 90%, 95%, 96%, 97%, 98%, or 99% identity to the amino acid sequence set forth in SEQ ID NO: 56, and optionally, it has a mouse heavy chain MIgG Vh signal sequence and ISAMVRS amino acid sequence at its N-terminus, has YTE triple mutations, has a G4S connector and c-Myc tag at its C-terminus, and contains an allotype G1m(f).

In some embodiments, E comprises the amino acid sequence set forth in SEQ ID NO: 57. In some embodiments, E comprises an amino acid sequence having at least 70%, 75%, 80%, 85%, 90%, 95%, 96%, 97%, 98% or 99% identity to the amino acid sequence set forth in SEQ ID NO: 57, and optionally, it has a mouse heavy chain MIgG Vh signal sequence and ISAMVRS amino acid sequence at its N-terminus, has YTE triple mutations, has a G4S connector and a c-Myc tag at its C-terminus, and contains an allotype G1m(fa).

In some embodiments, E comprises the amino acid sequence set forth in SEQ ID NO: 58. In some embodiments, E comprises an amino acid sequence having at least 70%, 75%, 80%, 85%, 90%, 95%, 96%, 97%, 98% or 99% identity to the amino acid sequence set forth in SEQ ID NO: 58, and optionally, it has a mouse heavy chain MIgG Vh signal sequence at its N-terminus, has a change from cysteine to serine (at position #), has a G4S connector and an IgA peptide tag at its C-terminus, and contains an allotype G1m(fa).

In some embodiments, E comprises the amino acid sequence set forth in SEQ ID NO: 59. In some embodiments, E comprises an amino acid sequence having at least 70%, 75%, 80%, 85%, 90%, 95%, 96%, 97%, 98% or 99% identity to the amino acid sequence set forth in SEQ ID NO: 59, and optionally, it has a mouse heavy chain MIgG Vh signal sequence at its N-terminus, has a change from cysteine to serine (at position corresponding to # position of SEQ ID NO: 59), has M428L and N434S mutations, has a G4S connector and an IgA peptide tag at its C-terminus, and contains an allotype G1m(fa).

In some embodiments, E comprises the amino acid sequence set forth in SEQ ID NO: 60. In some embodiments, E comprises an amino acid sequence having at least 70%, 75%, 80%, 85%, 90%, 95%, 96%, 97%, 98% or 99% identity to the amino acid sequence set forth in SEQ ID NO: 60, and optionally, it has the amino acid defined for the corresponding

position by SEQ ID NO: 60 at the position corresponding to position Z or X as shown in SEQ ID NO: 60.

In some embodiments, E comprises the amino acid sequence set forth in SEQ ID NO: 61. In some embodiments, E comprises an amino acid sequence having at least 70%, 75%, 80%, 85%, 90%, 95%, 96%, 97%, 98% or 99% identity to the amino acid sequence set forth in SEQ ID NO: 61, and optionally, it has a change from cysteine to serine (at position corresponding to # position of SEQ ID NO: 61), and the amino acid defined for the corresponding position by SEQ ID NO: 61 at the position corresponding to position X shown in SEQ ID NO: 61.

In some embodiments, E comprises the amino acid sequence set forth in SEQ ID NO: 62. In some embodiments, E comprises an amino acid sequence having at least 70%, 75%, 80%, 85%, 90%, 95%, 96%, 97%, 98% or 99% identity to the amino acid sequence set forth in SEQ ID NO: 62, and optionally, it has a change from cysteine to serine (at position corresponding to # position of SEQ ID NO: 62), and the amino acid defined for the corresponding position by SEQ ID NO: 62 at the position corresponding to position X shown in SEQ ID NO: 62.

In some embodiments, E comprises the amino acid sequence set forth in SEQ ID NO: 63. In some embodiments, E comprises an amino acid sequence having at least 70%, 75%, 80%, 85%, 90%, 95%, 96%, 97%, 98% or 99% identity to the amino acid sequence set forth in SEQ ID NO: 63, and optionally, it has a change from cysteine to serine (at position corresponding to # position of SEQ ID NO: 63) and has an allotype G1m(f).

In some embodiments, E comprises the amino acid sequence set forth in SEQ ID NO: 64. In some embodiments, E comprises an amino acid sequence having at least 70%, 75%, 80%, 85%, 90%, 95%, 96%, 97%, 98% or 99% identity to the amino acid sequence set forth in SEQ ID NO: 64, and optionally, it has a change from cysteine to serine (at position corresponding to # position of SEQ ID NO: 64) and has an allotype G1m(fa).

In some embodiments, E comprises the amino acid sequence set forth in SEQ ID NO: 65. In some embodiments, E comprises an amino acid sequence having at least 70%, 75%, 80%, 85%, 90%, 95%, 96%, 97%, 98% or 99% identity to the amino acid sequence set forth in SEQ ID NO: 65, and optionally, it has a change from cysteine to serine (at position corresponding to # position of SEQ ID NO: 65), M428L and N343S mutations, and an allotype G1m(fa).

In some embodiments, E comprises the amino acid sequence set forth in SEQ ID NO: 66. In some embodiments, E comprises an amino acid sequence having at least 70%, 75%, 80%, 85%, 90%, 95%, 96%, 97%, 98% or 99% identity to the amino acid sequence set forth in SEQ ID NO: 66, and optionally, it has a change from cysteine to serine (at position corresponding to # position of SEQ ID NO: 66), M428L and N343S mutations, and an allotype G1m(f).

In some embodiments, E comprises the amino acid sequence set forth in SEQ ID NO: 67. In some embodiments, E comprises an amino acid sequence having at least 70%, 75%, 80%, 85%, 90%, 95%, 96%, 97%, 98% or 99% identity to the amino acid sequence set forth in SEQ ID NO: 67, and optionally, it has a change from cysteine to serine (at position corresponding to # position of SEQ ID NO: 67) and YTE triple mutations, and has an allotype G1m(fa).

In some embodiments, E comprises the amino acid sequence set forth in SEQ ID NO: 68. In some embodiments, E comprises an amino acid sequence having at least 70%, 75%, 80%, 85%, 90%, 95%, 96%, 97%, 98% or 99% identity to the amino acid sequence set forth in SEQ ID NO: 68, and optionally, it has a change from cysteine to serine (at position corresponding to # position of SEQ ID NO: 68) and YTE triple mutations, and has an allotype G1m(f).

In some embodiments, E comprises the amino acid sequence set forth in SEQ ID NO: 69. In some embodiments, E comprises an amino acid sequence having at least 70%, 75%, 80%, 85%, 90%, 95%, 96%, 97%, 98% or 99% identity to the amino acid sequence set forth in SEQ ID NO: 69, and optionally, it has YTE triple mutations and has an allotype G1m(fa).

In some embodiments, E comprises the amino acid sequence set forth in SEQ ID NO: 70. In some embodiments, E comprises an amino acid sequence having at least 70%, 75%, 80%, 85%, 90%, 95%, 96%, 97%, 98% or 99% identity to the amino acid sequence set forth in SEQ ID NO: 70, and optionally, it has YTE triple mutations and has an allotype G1m(fa).

In some embodiments, E comprises the amino acid sequence set forth in SEQ ID NO: 71. In some embodiments, E comprises an amino acid sequence having at least 70%, 75%, 80%, 85%, 90%, 95%, 96%, 97%, 98% or 99% identity to the amino acid sequence set forth in SEQ ID NO: 71, and optionally, it has M428L and N343S mutations, and has an allotype G1m(fa).

In some embodiments, E comprises the amino acid sequence set forth in SEQ ID NO: 72. In some embodiments, E comprises an amino acid sequence having at least 70%, 75%, 80%, 85%, 90%, 95%, 96%, 97%, 98% or 99% identity to the amino acid sequence set forth in SEQ ID NO: 72.

In some embodiments, E comprises the amino acid sequence set forth in SEQ ID NO: 73. In some embodiments, E comprises an amino acid sequence having at least 70%, 75%, 80%, 85%, 90%, 95%, 96%, 97%, 98% or 99% identity to the amino acid sequence set forth in SEQ ID NO: 73, and optionally, it has M428L and N343S mutations, and has an allotype G1m(f).

In some embodiments, E comprises the amino acid sequence set forth in SEQ ID NO: 74. In some embodiments, E comprises an amino acid sequence having at least 70%, 75%, 80%, 85%, 90%, 95%, 96%, 97%, 98% or 99% identity to the amino acid sequence set forth in SEQ ID NO: 74, and optionally, it has GAALIE, M428L, N343S mutations, and has an allotype G1m(fa).

In some embodiments, E comprises the amino acid sequence set forth in SEQ ID NO: 75. In some embodiments, E comprises an amino acid sequence having at least 70%, 75%, 80%, 85%, 90%, 95%, 96%, 97%, 98% or 99% identity to the amino acid sequence set forth in SEQ ID NO: 75, and optionally, it has GAALIE, M428L, N343S mutations, and has an allotype G1m(f).

In some embodiments, E comprises the amino acid sequence set forth in SEQ ID NO: 76. In some embodiments, E comprises an amino acid sequence having at least 70%, 75%, 80%, 85%, 90%, 95%, 96%, 97%, 98% or 99% identity to the amino acid sequence set forth in SEQ ID NO: 76, and optionally, it has a change from cysteine to serine (#), GAALIE, M428L, N343S mutations, and has an allotype G1m(fa).

In some embodiments, E comprises the amino acid sequence set forth in SEQ ID NO: 77. In some embodiments, E comprises an amino acid sequence having at least 70%, 75%, 80%, 85%, 90%, 95%, 96%, 97%, 98% or 99% identity to the amino acid sequence set forth in SEQ ID NO: 77, and optionally, it has a change from cysteine to serine (#), GAALIE, M428L, N343S mutations, and has an allotype G1m(f).

In some embodiments, E comprises the amino acid sequence set forth in SEQ ID NO: 78. In some embodiments, E comprises an amino acid sequence having at least 70%, 75%, 80%, 85%, 90%, 95%, 96%, 97%, 98% or 99% identity to the amino acid sequence set forth in SEQ ID NO: 78, and optionally, it has a change from cysteine to serine (#), DHS triple mutations, and has an allotype G1m(fa).

In some embodiments, E comprises the amino acid sequence set forth in SEQ ID NO: 79. In some embodiments, E comprises an amino acid sequence having at least 70%, 75%, 80%, 85%, 90%, 95%, 96%, 97%, 98% or 99% identity to the amino acid sequence of SEQ ID NO: 79, and optionally, it has a change from cysteine to serine (#), DHS triple mutations, and an allotype G1m(f).

In some embodiments, E comprises the amino acid sequence set forth in SEQ ID NO: 80. In some embodiments, E comprises an amino acid sequence having at least 70%, 75%, 80%, 85%, 90%, 95%, 96%, 97%, 98% or 99% identity to the amino acid sequence set forth in SEQ ID NO: 80, and optionally, it has DHS triple mutations and has an allotype G1m(fa).

In some embodiments, E comprises the amino acid sequence set forth in SEQ ID NO: 81. In some embodiments, E comprises an amino acid sequence having at least 70%, 75%, 80%, 85%, 90%, 95%, 96%, 97%, 98% or 99% identity to the amino acid sequence set forth in SEQ ID NO: 81, and optionally, it has DHS triple mutations and has an allotype G1m(f).

In any of the embodiments described herein, wherein E comprises an Fc domain monomer (e.g., having the sequence set forth in any one of SEQ ID NO: 1-81), comprising triple mutations corresponding to M252Y/S254T/T265E (YTE). Those skilled in the art will appreciate that "corresponding to" refers to the specific amino acid sites at the corresponding positions obtained after sequence homology alignment using sequence analysis software (such as, but not limited to, DNA Star, Vector NTI, etc.). For example, any of SEQ ID NO: 1-81 may comprise mutations including YTE.

In any of the embodiments described herein, wherein E comprises an Fc domain monomer (e.g., having the sequence set forth in any one of SEQ ID NO: 1-81), which comprises dual mutations corresponding to M428L/N434S (LS). Those skilled in the art will appreciate that "corresponding to" refers to the specific amino acid sites at the corresponding positions obtained after sequence homology alignment using sequence analysis software (such as, but not limited to, DNA Star, Vector NTI, etc.). For example, any one of SEQ ID NO: 1-81 may comprise mutations including LS.

In any of the embodiments described herein, wherein E comprises an Fc domain monomer (e.g., having the sequence set forth in any of SEQ ID NO: 1-81), it comprises dual mutations corresponding to N434H. Those skilled in the art will appreciate that "corresponding to" refers to the specific amino acid site at the corresponding position obtained after sequence homology alignment using sequence analysis software (such as, but not limited to, DNA Star, Vector NTI, etc.). For example, any one of SEQ ID NO: 1-81 may comprise a mutation including N434H.

In any of the embodiments described herein, where E comprises an Fc domain monomer (e.g., having the sequence set forth in any of SEQ ID NO: 1-81), which comprises dual mutations corresponding to C220S. Those skilled in the art will appreciate that "corresponding to" refers to the specific amino acid site at the corresponding position obtained after sequence homology alignment using sequence analysis software (such as, but not limited to, DNA Star, Vector NTI, etc.). For example, any of SEQ ID NO: 1-81 may comprise a mutation including C220S.

In any of the embodiments described herein, where E comprises a fragment of Fc domain monomer (e.g., a fragment of an Fc domain monomer from any sequence set forth in SEQ ID NO: 1-81), and is a fragment of at least 25 (e.g. 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more), at least 50 (e.g. 51, 52, 53, 54, 55, 56, 57, 58, 59, 60, 61, 62, 63, 64, 65, 66, 67, 68, 69, 70, 71, 72, 73, 74, 75 or more), or at least 75 (e.g. 75, 76, 77, 78, 79, 80, 81, 82, 83, 84, 85, 86, 87, 88, 89, 90, 91, 92, 93, 94, 95, 96, 97, 98, 99, 100 or more) contiguous amino acids in the Fc domain monomer (e.g., an Fc domain monomer from any sequence set forth in SEQ ID NO: 1-81).

In an embodiment, the conjugate has the following structure:

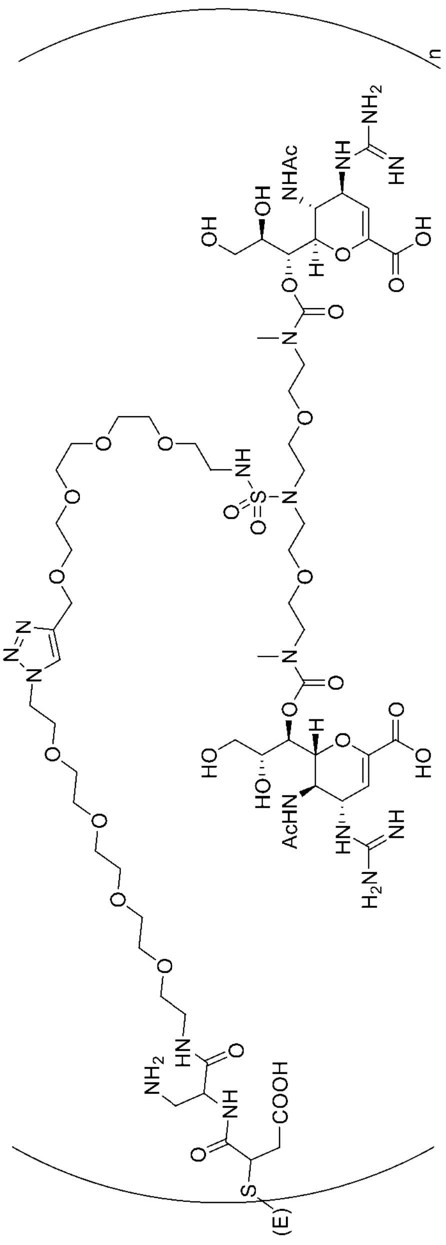

wherein E is SEQ ID NO: 67

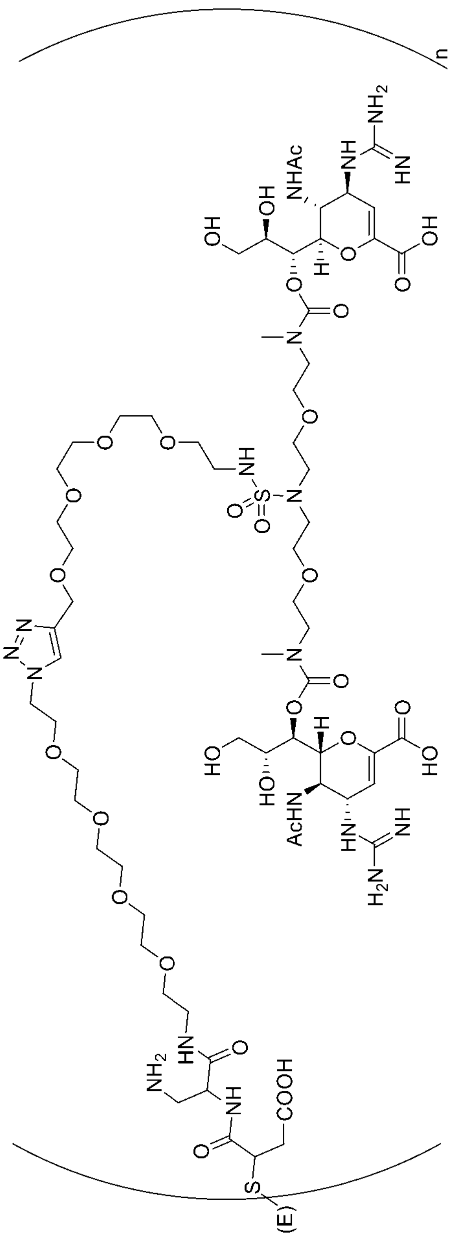

wherein E is SEQ ID NO: 69

(continued)

| |
|---|
| 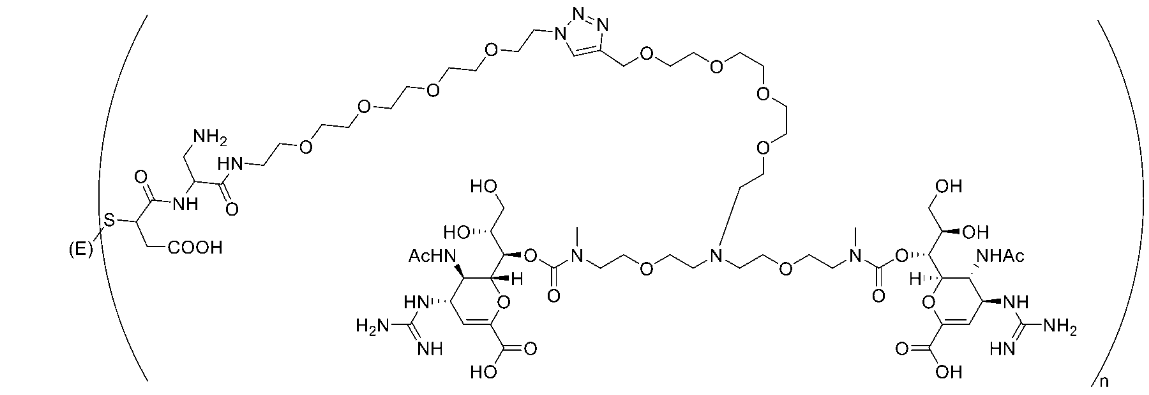<br>wherein E is SEQ ID NO: 71 |
| 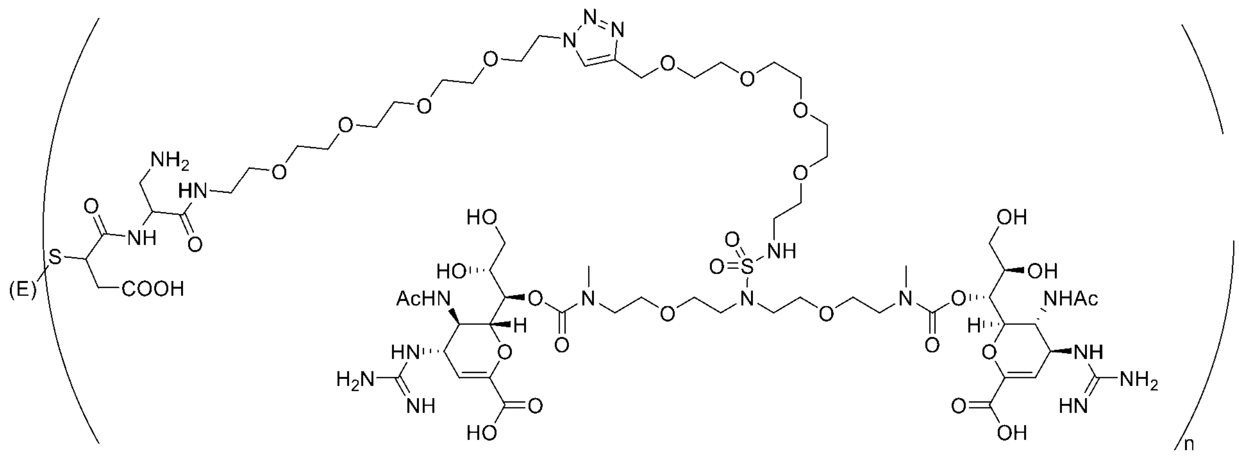<br>wherein E is SEQ ID NO: 71 |

wherein n is defined as above.

In one aspect, the present invention provides a Linker-payload compound, or a pharmaceutically acceptable salt, an ester, an isomer, a solvate, a prodrug, or an isotopically labeled compound thereof.

In some embodiments, the present invention provides compounds represented by a structure of formula I-5a or I-6a, or a pharmaceutically acceptable salt, an ester, an isomer, a solvate, a prodrug, or an isotopically labeled compound thereof,

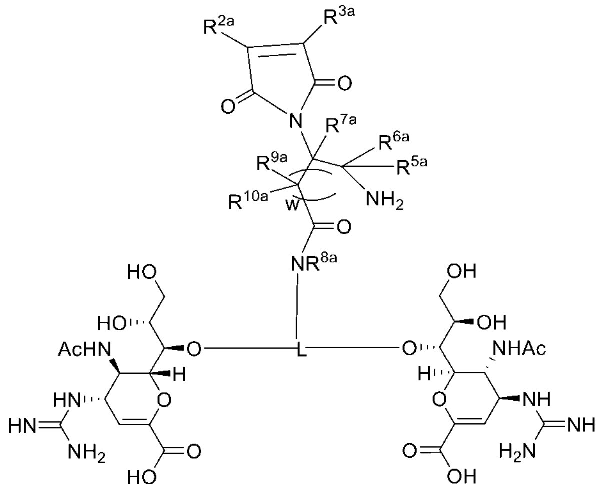

I-5a

wherein each symbol (e.g. L, $R^{2a}$, $R^{3a}$, $R^{5a}$, $R^{6a}$, $R^{7a}$, $R^{8a}$, $R^{9a}$, $R^{10a}$, and w) is as defined herein;

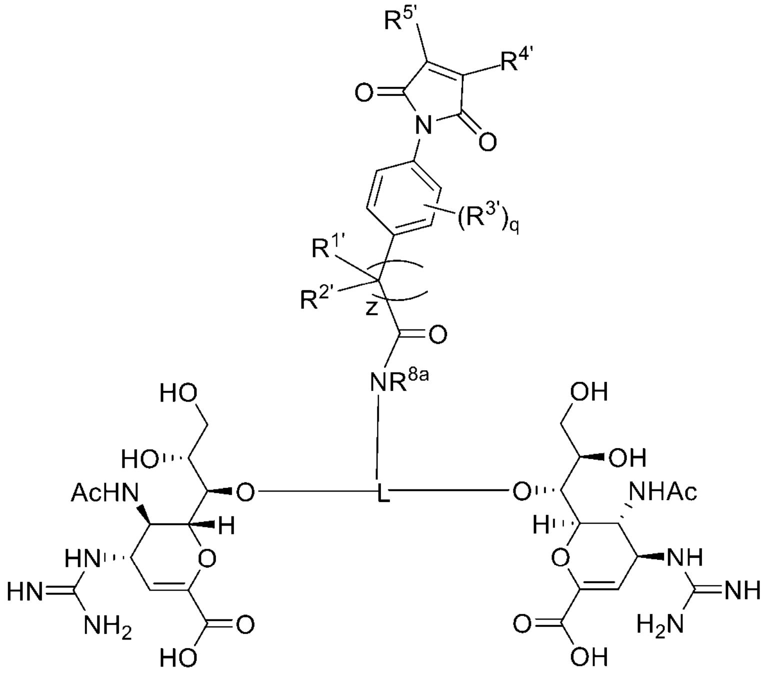

I-6a

wherein each symbol (e.g. L, z, q, $R^{1'}$, $R^{2'}$, $R^{3'}$, $R^{4'}$ and $R^{5'}$) is as defined herein.

In some embodiments, the present invention provides a compound with a structure of formula I-2, or a pharmaceutically acceptable salt, an ester, an isomer, a solvate, a prodrug, or an isotopically labeled compound thereof,

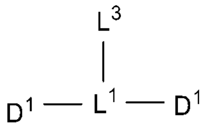

I-2

wherein $L^1$ and $D^1$ are as defined in formula I-1 above;
$L^3$ is selected from the following structures:

| $L^3$ structure No. | Structure |
| --- | --- |
| Formula L3-1 | 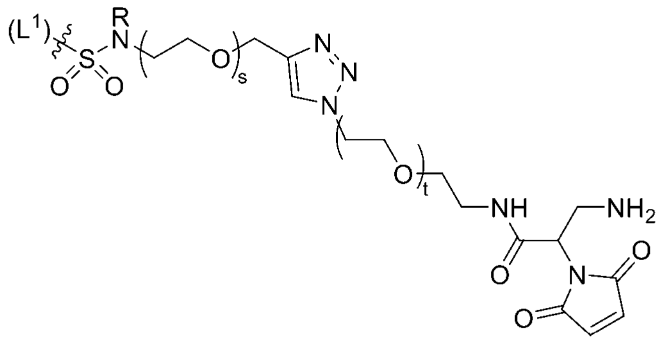 |
| Formula L3-2 | 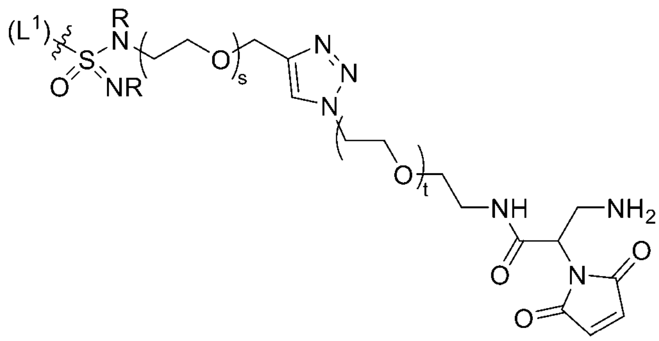 |
| Formula L3-3 | 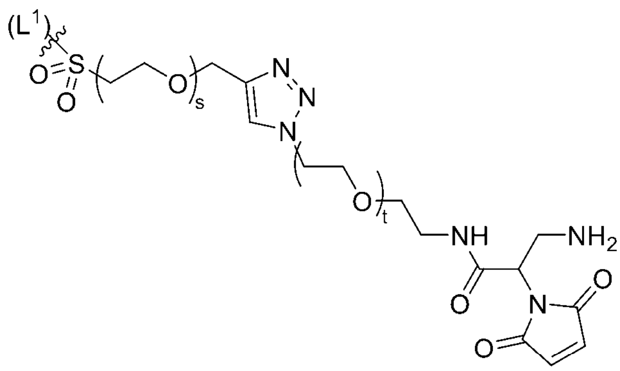 |

(continued)

| $L^3$ structure No. | Structure |
|---|---|
| Formula L3-4 | 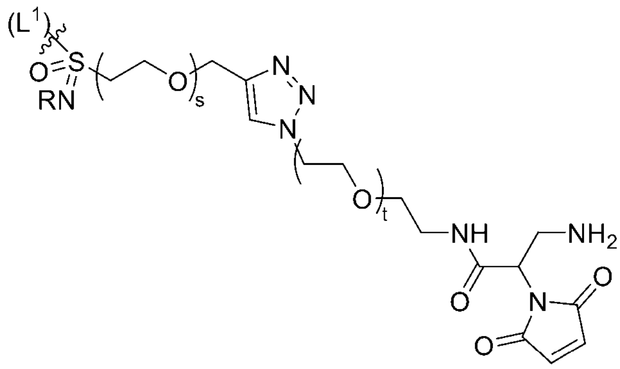 |
| Formula L3-5 | 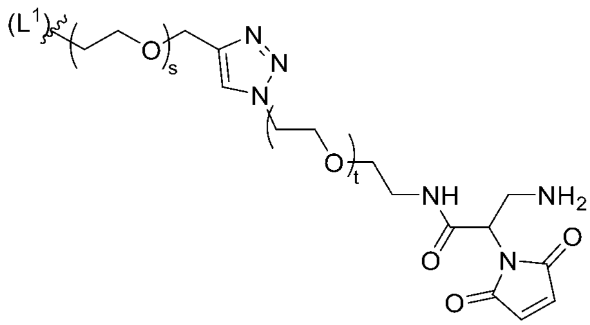 |
| Formula L3-6 | 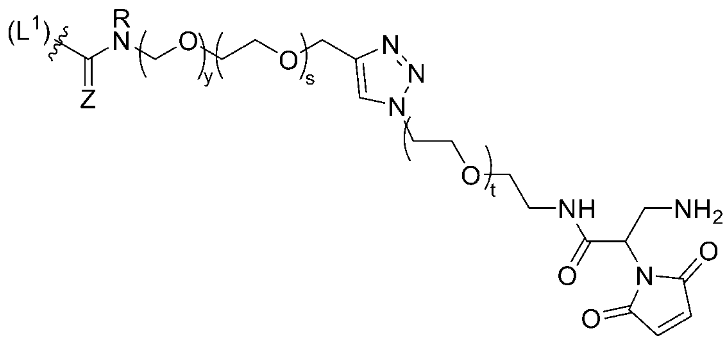 |
| Formula L3-7 | 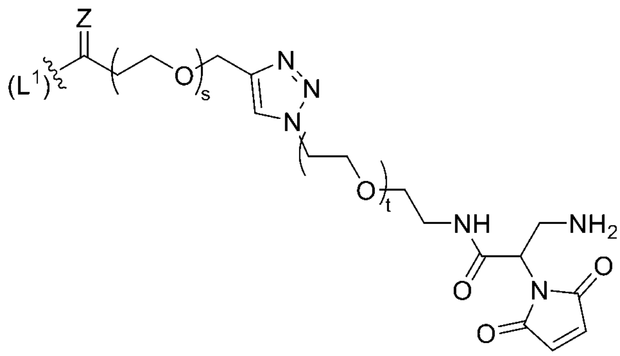 |

(continued)

| $L^3$ structure No. | Structure |
|---|---|
| Formula L3-8 | 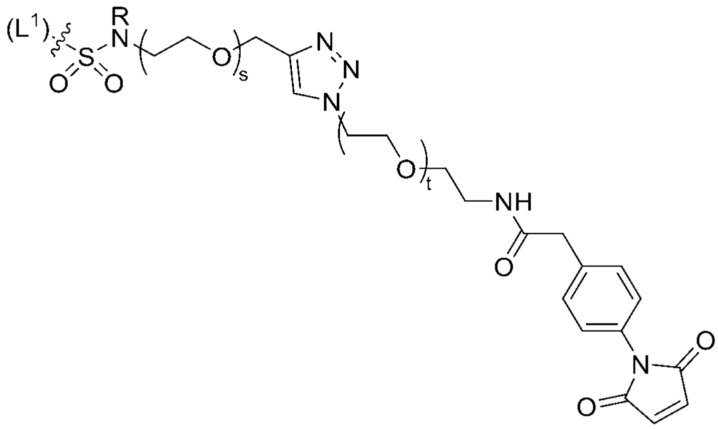 |
| Formula L3-9 | 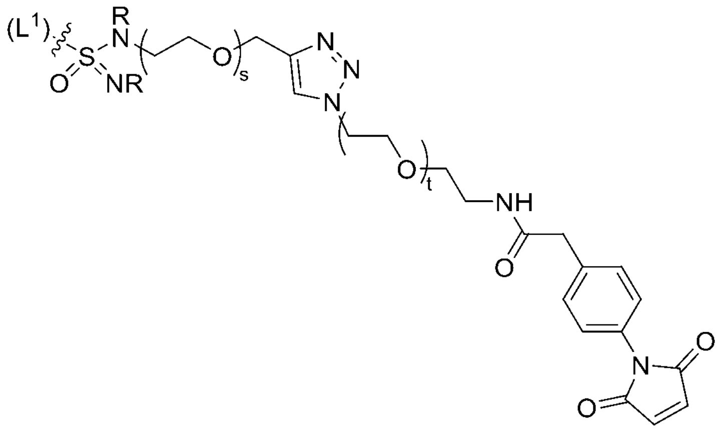 |
| Formula L3-10 | 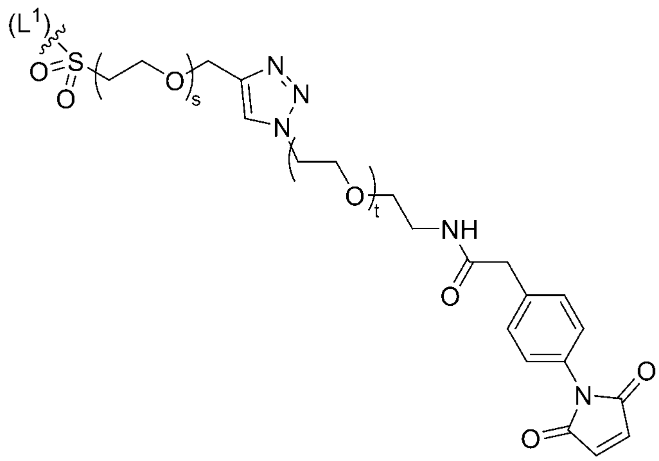 |

(continued)

| $L^3$ structure No. | Structure |
|---|---|
| Formula L3-11 | 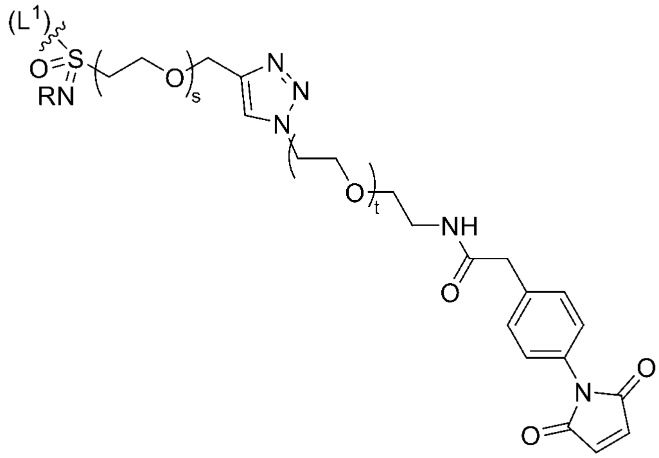 |
| Formula L3-12 | 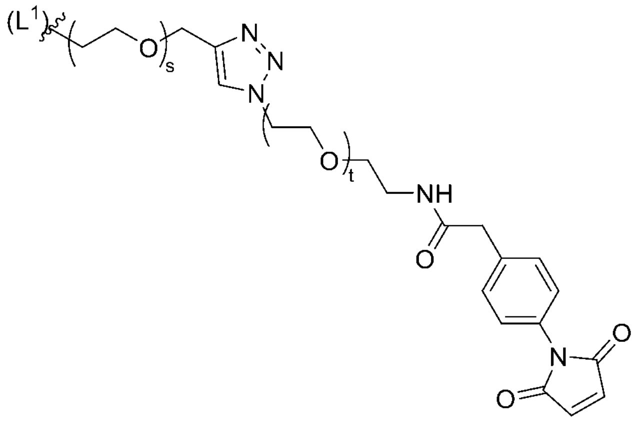 |
| Formula L3-13 | 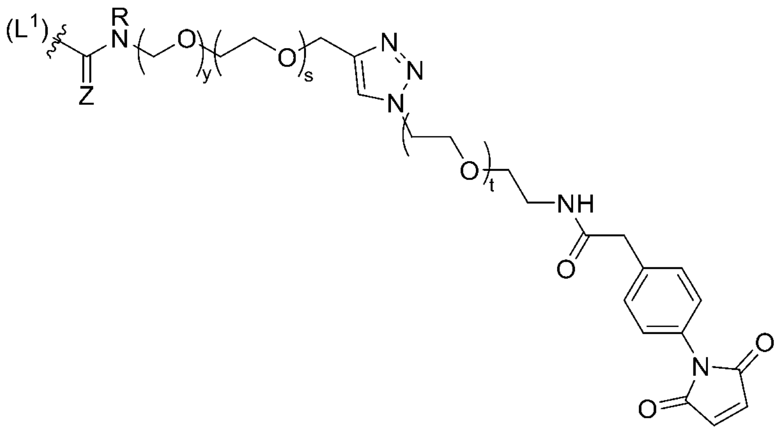 |

(continued)

| $L^3$ structure No. | Structure |
|---|---|
| Formula L3-14 | 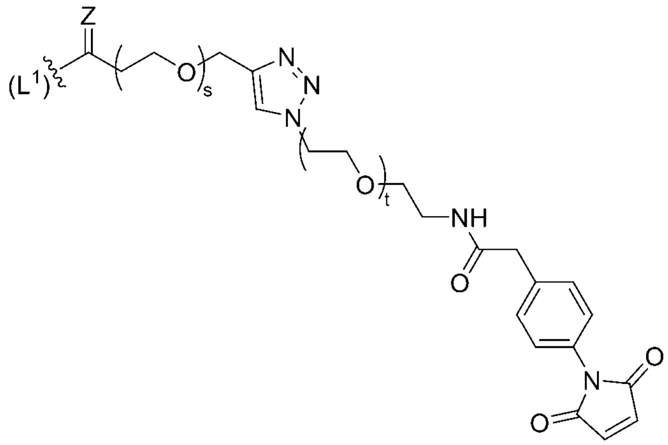 |

wherein each of variables such as Z, R, s, y, and t is as defined for $L^2$ above. It should be understood that in the structures of L3-1 to L3-14, the $L^1$ moiety is not a component of $L^3$, and ($L^1$) is used to indicate the end linked to $L^1$.

In some embodiments, the compounds including compounds of formula I-2, preferably have structures of formula C-inter-1 to formula C-inter-12:

| **Intermediate No.** | **Structure of intermediate** |
|---|---|
| **C-Inter-1** | 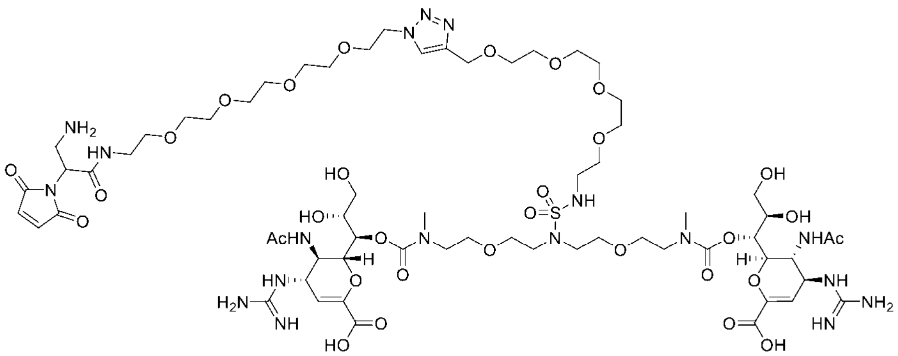 |
| **C-Inter-2** | 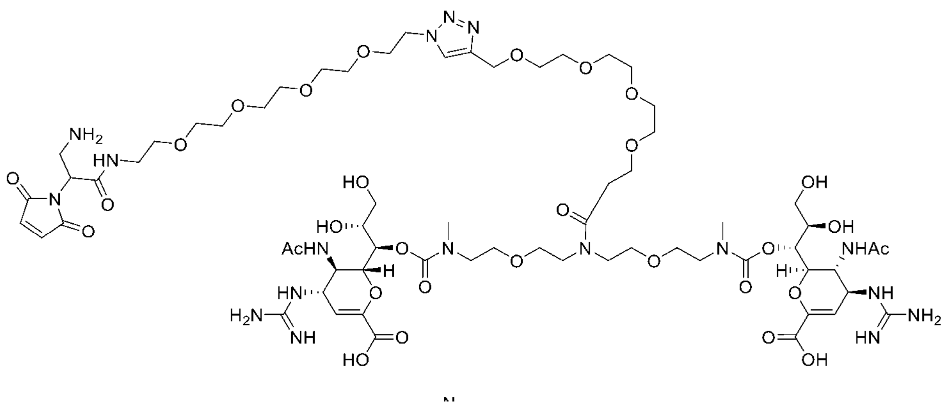 |

(continued)

| Intermediate No. | Structure of intermediate |
|---|---|
| **C-Inter-3** | 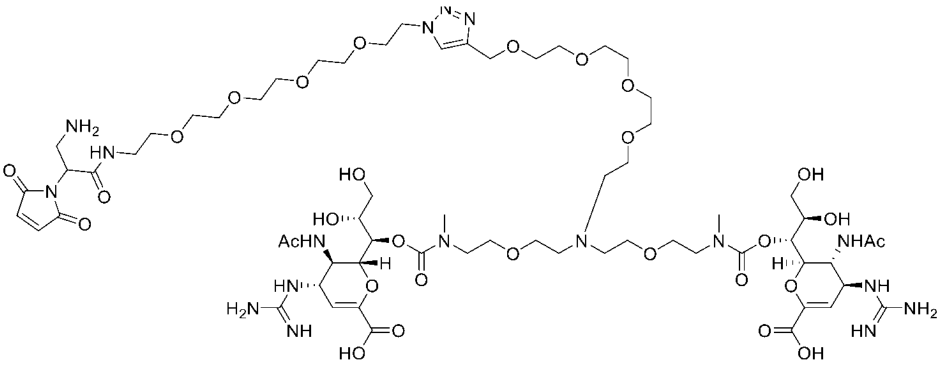 |
| **C-Inter-4** | 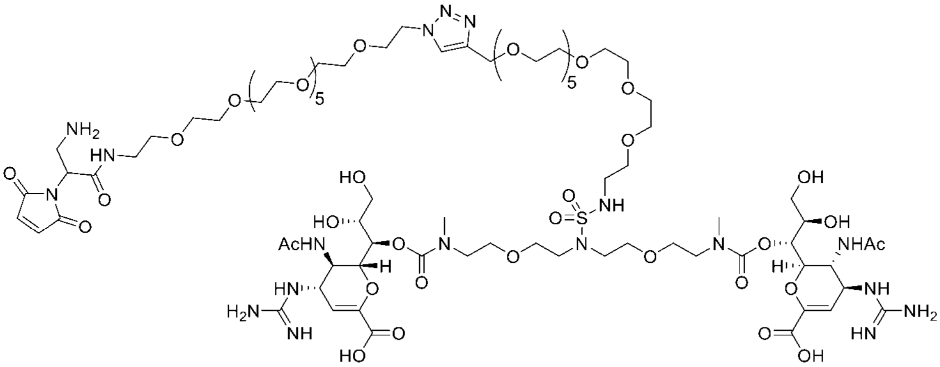 |
| **C-Inter-5** | 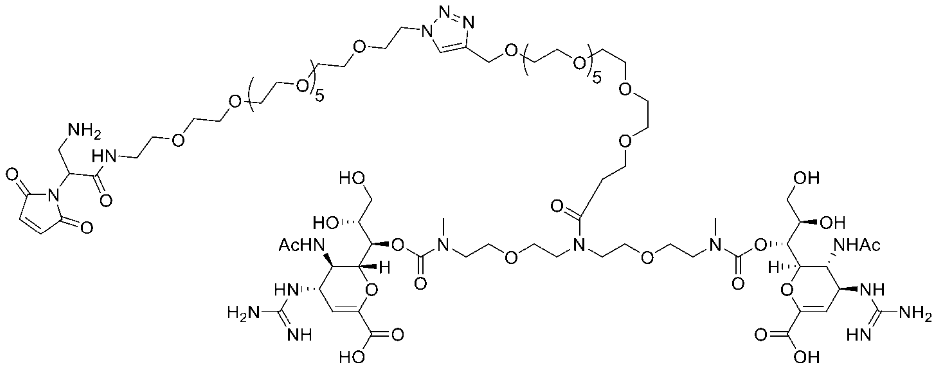 |
| **C-Inter-6** | 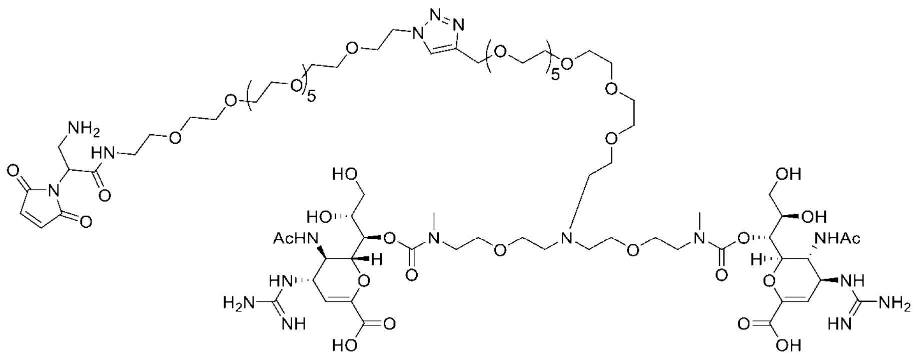 |

(continued)

| Intermediate No. | Structure of intermediate |
|---|---|
| C-Inter-7 | 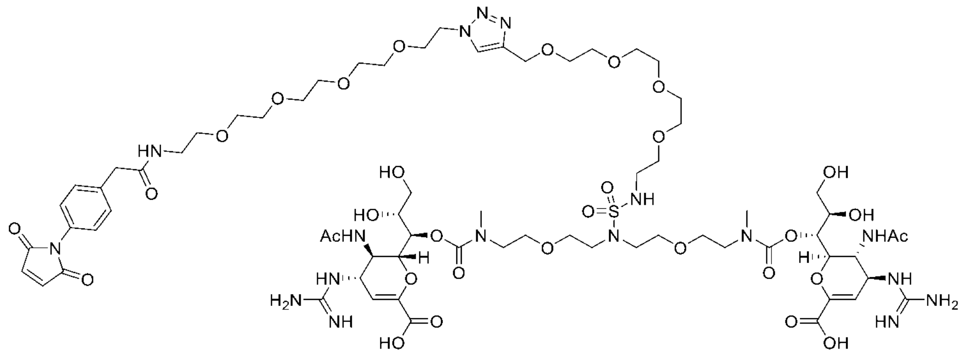 |
| C-Inter-8 | 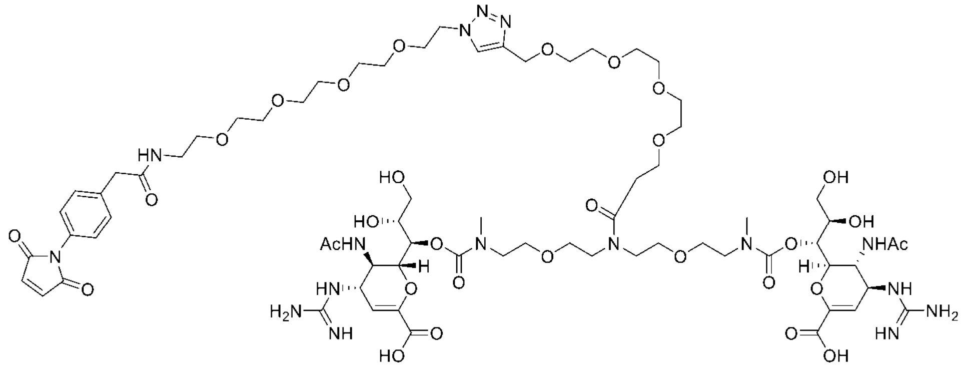 |
| C-Inter-9 | 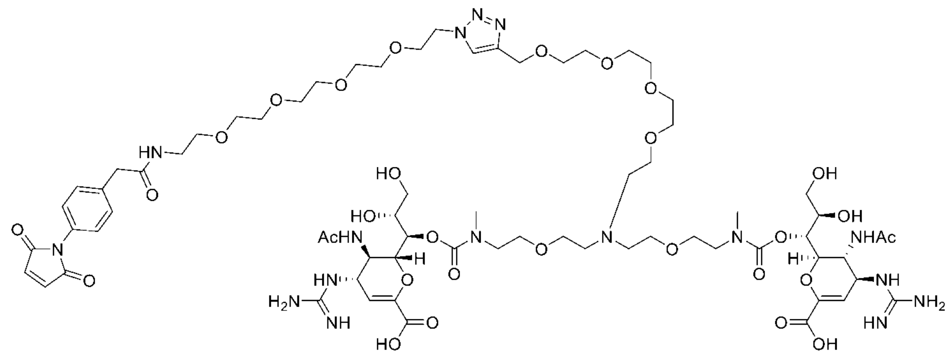 |
| C-Inter-10 | 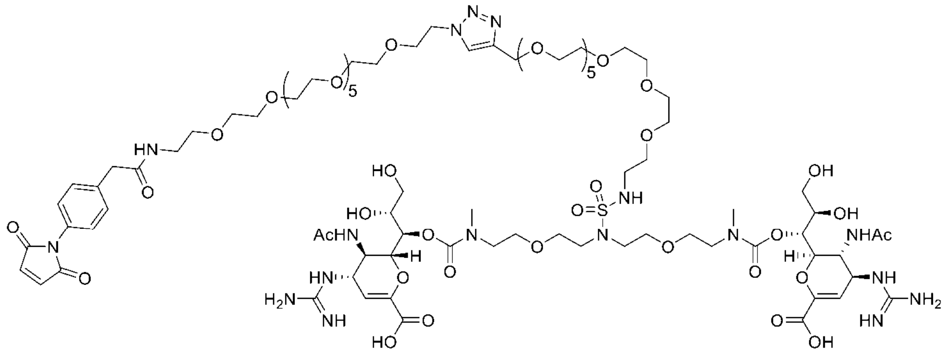 |

(continued)

| Intermediate No. | Structure of intermediate |
|---|---|
| C-Inter-11 | 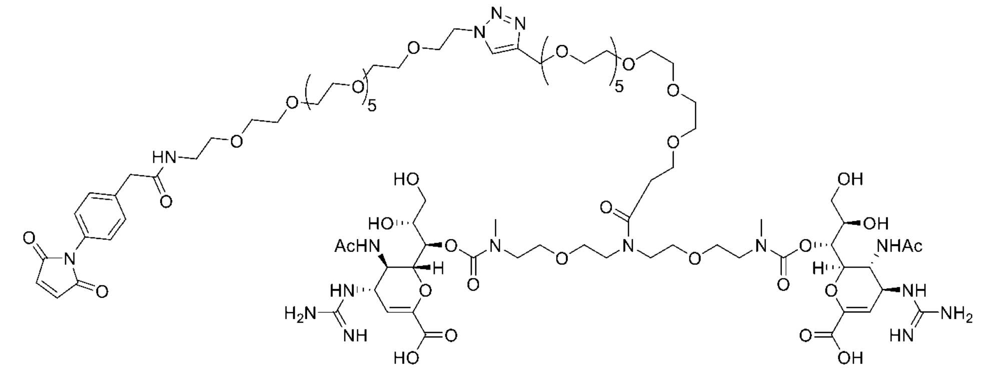 |
| C-Inter-12 | 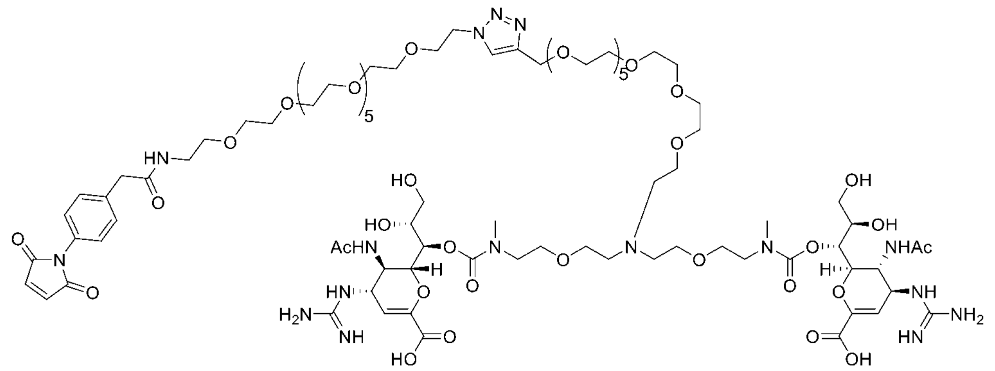 |

In some embodiments, the carbon to which the

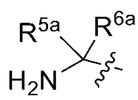

group is linked or the carbon to which the group corresponding to

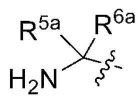

is linked is in R or S configuration, and preferably in R configuration.

In some embodiments, in C-inter-1 to C-inter-6, the carbon to which the $NH_2$-$CH_2$- on the left side is linked is in R or S configuration, and preferably in R configuration.

In another aspect, the present invention provides a pharmaceutical composition, comprising a conjugate of formula I-1, I-A, or formula I-B, a compound of formula I-2, I-5a, or I-6a, or a pharmaceutically acceptable salt, an ester, an isomer, a solvate, a prodrug, or an isotopically labeled compound thereof, as described above, and optionally one or more other therapeutic agents, such as chemotherapeutic agents, angiogenesis inhibitors, cytokines, cytotoxic agents, other antibodies, small molecule drugs, or immunomodulators (e.g., immune checkpoint inhibitors or agonists), as well as optionally a pharmaceutically acceptable excipient.

In another aspect, the present invention provides a pharmaceutical combination, comprising a conjugate of formula I-1, I-A, or formula I-B, a compound of formula I-2, I-5a, or I-6a, or a pharmaceutically acceptable salt, an ester, an isomer, a solvate, a prodrug, or an isotopically labeled compound thereof, as described above, and optionally one or more other therapeutic agents, such as chemotherapeutic agents, angiogenesis inhibitors, cytokines, cytotoxic agents, other antibodies, small molecule drugs, or immunomodulators (e.g., immune checkpoint inhibitors or agonists).

In another aspect, the present invention provides a method for preventing or treating patients infected with viruses or patients who may be at risk of being infected with viruses, which comprises the step of administering to the patients, for example, by injection, an effective dose of any of the conjugates of formula I-1, I-A, or formula I-B, the

compounds of formula 1-2, I-5a, or I-6a, or a pharmaceutically acceptable salt, an ester, an isomer, a solvate, a prodrug, or an isotopically labeled compound thereof.

The use of the conjugates of formula I-1, I-A, or formula I-B, the compounds of formula I-2, I-5a, or I-6a, or the pharmaceutically acceptable salt, ester, isomer, solvate, prodrug, or isotopically labeled compound thereof as described above in the manufacture of a medicament for preventing or treating viral infections in patients infected with viruses or patients who may be at risk of being infected with viruses.

In some embodiments, the viral infection is caused by influenza virus or parainfluenza virus.

In some embodiments, the viral infection is caused by influenza virus A, B, or C, or parainfluenza virus.

In some embodiments, the patient who is infected with the virus or may be at risk of being infected with the virus may be a patient with immune system deficiencies.

In some embodiments, the patient who is infected with the virus or may be at risk of being infected with the virus may be a patient who is receiving or will receive immunosuppressant treatment.

In some embodiments, the patient who is infected with the virus or may be at risk of being infected with the virus may be a patient diagnosed with a disease that causes immunosuppression.

In some embodiments, the patient diagnosed with a disease that causes immunosuppression suffers from cancer or acquired immune deficiency syndrome.

In some embodiments, the patient diagnosed with a cancer disease that cause immunosuppression suffers from leukemia, lymphoma, humoral immunodeficiency, T-cell deficiency, complement deficiency, or multiple myeloma.

In some embodiments, the patient is a patient who is undergoing or will undergo hematopoietic stem cell transplantation.

In some embodiments, the patient is a patient who is undergoing or will undergo organ transplantation.

In some embodiments, the patient may be at risk of secondary infection.

The method for preventing the risk of secondary infection in patients caused by influenza virus infection comprising administering to the patient, for example, by injection, an effective dose of any of the conjugates of formula I-1, I-A, or formula I-B, the compounds of formula 1-2, I-5a, or I-6a, or pharmaceutically acceptable salts, esters, isomers, solvates, prodrugs, or isotopically labeled compounds described above.

In some embodiments, the secondary infection is a respiratory infection.

In some embodiments, the secondary infection is related to pneumonia.

In some embodiments, the secondary infection is a bacterial, viral, or fungal infection.

In some embodiments, the bacterial infection is an infection caused by methicillin-resistant *Staphylococcus aureus.*

In some embodiments, the bacterial infection is an infection caused by *Streptococcus pneumoniae.*

In some embodiments, the conjugate of formula I-1, I-A, or formula I-B, the compound of formula I-2, I-5a, or I-6a, or pharmaceutically acceptable salt, ester, isomer, solvate, prodrug, or isotopically labeled compound may be administered by intramuscular injection, intravenous injection, intradermal injection, intraarterial injection, intraperitoneal injection, intralesional injection, intracranial injection, intra-articular injection, intrapleural injection, endotracheal injection, intraprostatic injection, intranasal injection, intravitreal injection, intravaginal injection, intrarectal injection, topical, intratumoral injection, intraperitoneal injection, subcutaneous injection, subconjunctival injection, intracapsular injection, mucosal injection, pericardial injection, intraumbilical injection, intraocular injection, oral administration, topical inhalation, injection, or infusion.

In some embodiments, the conjugate of formula I-1, I-A, or Formula I-B, compound of formula 1-2, I-5a, or I-6a, or pharmaceutically acceptable salt, ester, isomer, solvate, prodrug, or isotopically labeled compound is administered in combination with an additional therapeutic agent or used to prepare a medicament in combination with an additional therapeutic agent.

In some embodiments, the additional therapeutic agent is an antiviral drug.

In some embodiments, the antiviral drug is baloxavir, pimodivir, oseltamivir, zanamivir, peramivir, laninamivir, amantadine, MEDI8852, or rimantadine.

In some embodiments, the additional drug used by the patient is an antiviral vaccine.

In some embodiments, the antiviral drug and the conjugate of formula I-1, I-A, or formula I-B, the compound of formula 1-2, I-5a, or I-6a, or pharmaceutically acceptable salt, ester, isomer, solvate, prodrug, or isotopically labeled compound are sequentially administered, for example, by injection, to the patient.

In some embodiments, the antiviral drug and the conjugate of formula I-1, I-A, or formula I-B, the compound of formula I-2, I-5a, or I-6a, or pharmaceutically acceptable salt, ester, isomer, solvate, prodrug, or isotopically labeled compound are administered simultaneously, for example, by injection to the patient.

## Effect of the invention

The conjugate of formula I-1, I-A, or formula I-B, the compound of formula 1-2, I-5a, or I-6a, or pharmaceutically

acceptable salt, ester, isomer, solvate, prodrug, or isotopically labeled compound of the present invention has the following advantages:

exhibiting remarkable high antiviral activity,

exhibiting high activity against drug-resistant viral strains; and/or

having excellent *in vitro*/*in vivo* pharmacokinetic properties and safety, such as a long half-life and high systemic exposure, which can reduce the frequency of administration and increase patient compliance, indicating a promising prospect for clinical application.

**Preparation of the conjugate of the present invention**

In one aspect, the present invention provides a method for preparing the conjugate described herein.
In some embodiments, the present invention provides a method for preparing the conjugate of formula I-A described herein,

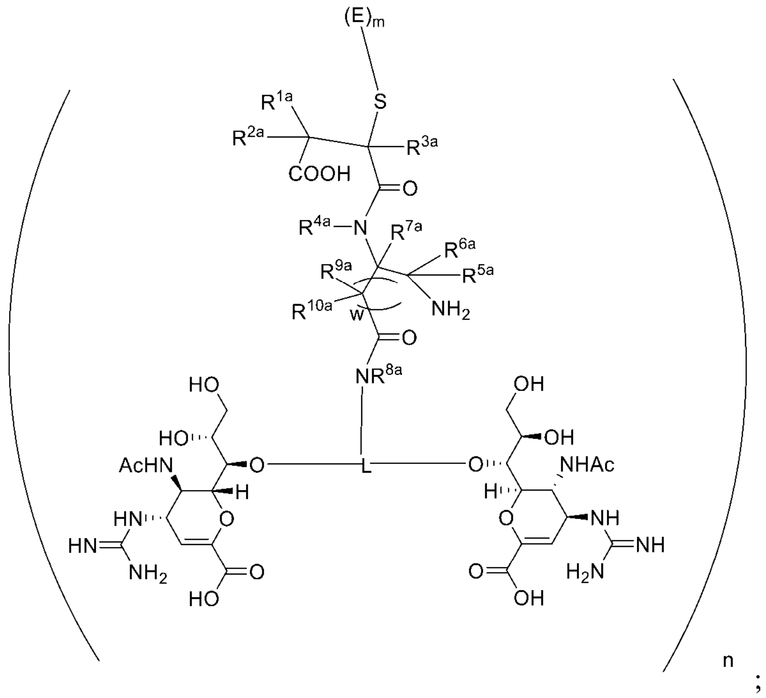

Formula I-A

wherein each symbol (e.g. L, $R^{1a}$, $R^{2a}$, $R^{3a}$, $R^{4a}$, $R^{5a}$, $R^{6a}$, $R^{7a}$, $R^{8a}$, $R^{9a}$, $R^{10a}$, w, n, m and E) is as defined herein; the method comprises the following steps:

Step 1, $(E)_m$ is dissolved in a buffer solution, wherein E and m are as defined herein. Then, a reducing agent (such as TCEP, DTT) is added to reduce the disulfide bond in $(E)_m$, to obtain a compound of formula II-1 with thiol residues,

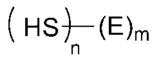

II-1

wherein E, m, and n are as defined herein;

Step 2, a buffer solution containing 6-14 molar equivalents of compound of formula I-5a is added to the compound

of formula II-1.

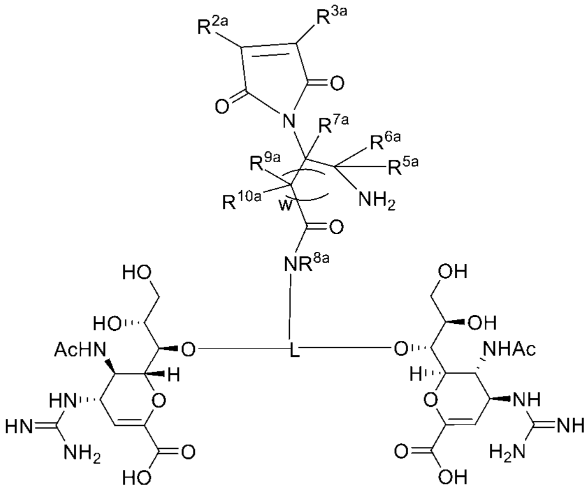

I-5a

wherein each symbol (such as L, $R^{2a}$, $R^{3a}$, $R^{5a}$, $R^{6a}$, $R^{7a}$, $R^{8a}$, $R^{9a}$, $R^{10a}$, and w) is as defined in formula I-A; under the condition of pH 5.5-7.0 (e.g., 5.8-6.8, 6.0-6.6), the Michael addition reaction is carried out for 0.5-10 hours, preferably 0.5-2 hours; then, 3-7 molar equivalents of the buffer solution of compound of formula I-5a are added in batches (e.g., 1-2 batches), and the reaction is continued for 0.5-10 hours (preferably 0.5-2 hours) to obtain the conjugate of formula I-3b.

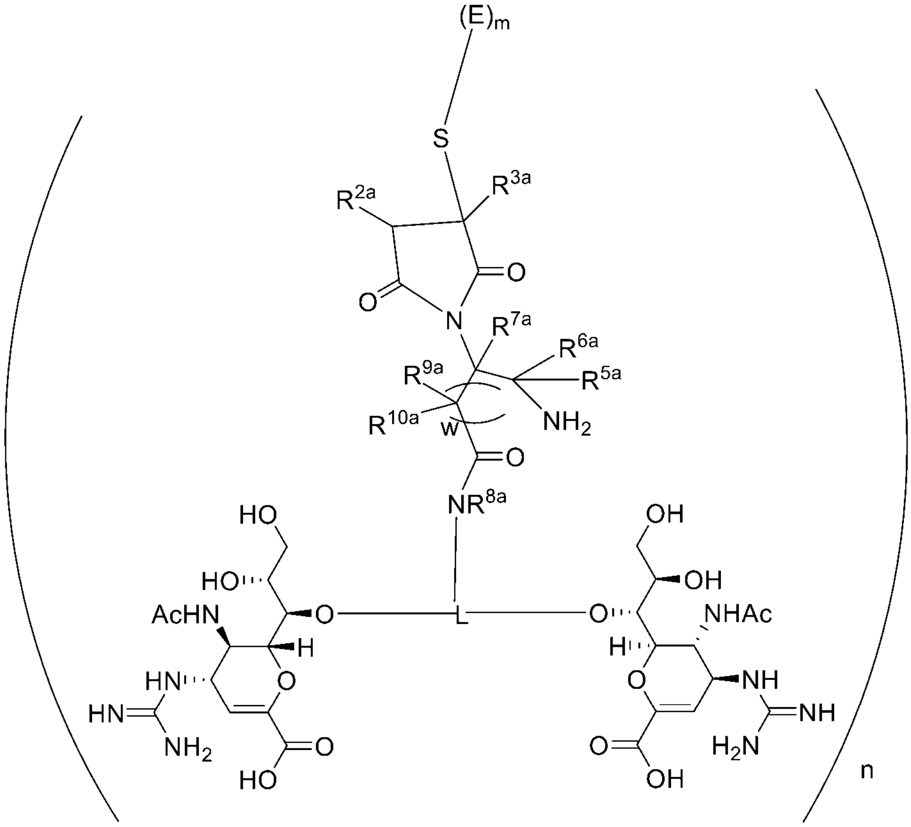

I-3b

wherein each symbol (such as L, $R^{2a}$, $R^{3a}$, $R^{5a}$, $R^{6a}$, $R^{7a}$, $R^{8a}$, $R^{9a}$, $R^{10a}$, w, n, m, and E) is as defined in formula I-A;

Step 3, the pH of the solution containing conjugate of formula I-3b is adjusted to 7-8 (e.g., 7.1-7.9, 7.3-7.7), and then a hydrolysis reaction is performed to obtain the final conjugate of formula I-A;

alternatively,
a method for preparing the conjugate of formula I-B described herein,

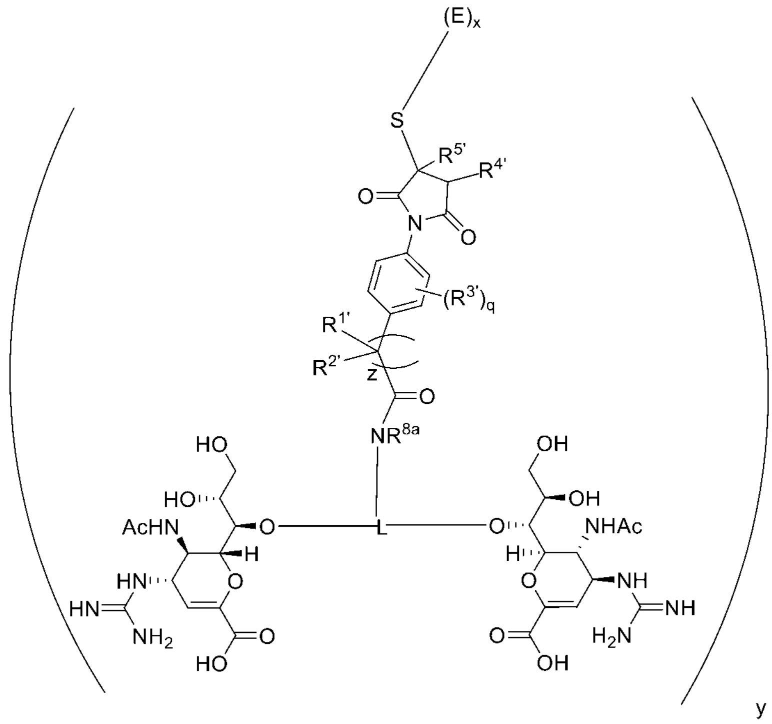

I-B

wherein each symbol (e.g. L, z, q, x, y, $R^{1'}$, $R^{2'}$, $R^{3'}$, $R^{4'}$, $R^{5'}$, $R^{8a}$, and E) is as defined herein;
the method comprises the following steps:

Step 1, $(E)_m$ is dissolved in a buffer solution, wherein E and m are as defined herein, then, a reducing agent (such as TCEP, DTT) is added to reduce the disulfide bond in $(E)_m$, to obtain a compound of formula II-1 with thiol residues,

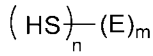

II-1

wherein E, m, and n are as defined herein;

Step 2, a buffer solution containing 6-14 molar equivalents of compound of formula I-6a is added to the compound of formula II-1.

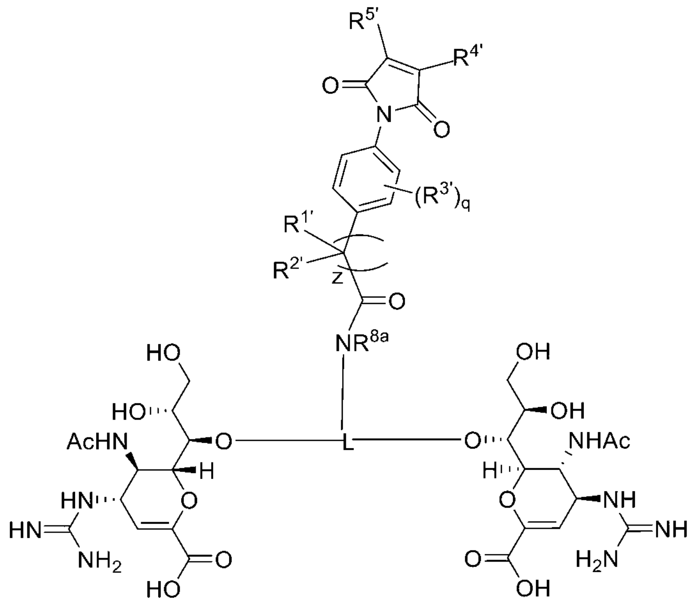

I-6a

wherein each symbol (e.g. L, z, q, $R^{1'}$, $R^{2'}$, $R^{3'}$, $R^{4'}$, $R^{5'}$, and $R^{8a}$) is as defined in formula I-B,

the Michael addition reaction is carried out under the condition of pH 5.5-8 (e.g., 5.8-7.6, 6.0-7.5), to obtain the conjugate of formula I-B.

In some embodiments, the present invention provides two methods for preparing the conjugate of formula I-1, Method 1 comprises the following steps:

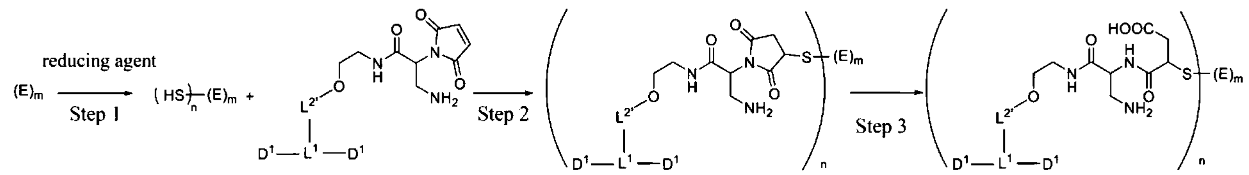


II-1 I-2a I-3 I-1a

wherein,

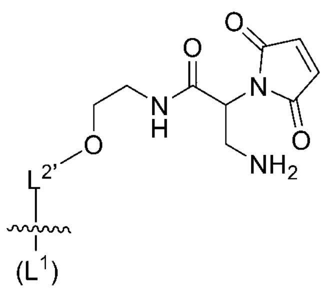

corresponds to $L^3$, wherein ($L^1$) indicates that $L^{2'}$ is linked to $L^1$, and $L^1$ is not part of the above structure;

wherein,

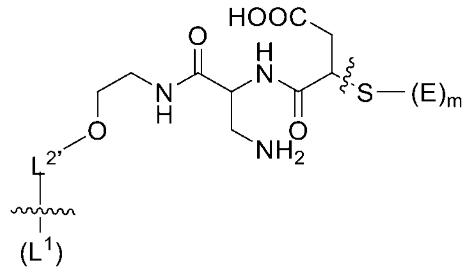

corresponds to $L^2$; as explained above, the structure between the two wavy lines should correspond to $L^2$;

wherein each variable, such as n, m, E, $D^1$, $L^1$, $L^2$, and $L^3$, is as defined above; preferably, $L^3$ is selected from formulas L3-1 to L3-7, and $L^2$ is selected from formulas L2-1 to L2-7;

Step 1, $(E)_m$ is dissolved in a buffer solution, and a reducing agent (such as TCEP, DTT) is added to reduce the disulfide bond in $(E)_m$, to obtain a compound of formula II-1 with thiol residues;

Step 2, a buffer solution containing 6-14 molar equivalents of compound of formula I-2a is added to compound of formula II-1; under the condition of pH 5.5-7.0 (e.g., 5.8-6.8, 6.0-6.6), the Michael addition reaction is carried out for 0.5-10 hours, preferably 0.5-2 hours; then, 3-7 molar equivalents of the buffer solution of compound of formula I-2a are added in batches (e.g., 1-2 batches), and the reaction is continued for 0.5-10 hours preferably 0.5-2 hours to obtain the conjugate of formula I-3;

Step 3, the pH of the solution containing the conjugate of formula I-3 is adjusted to 7-8 (e.g., 7.1-7.9, 7.3-7.7), and then a hydrolysis reaction is performed to obtain the final conjugate of formula I-1a.

In some embodiments, the buffer solution has a pH ranging from 5.0 to 7.5, such as 5.5 to 7.0, 6.0 to 6.5, or 5.5 to 6.5.

In some embodiments, the buffer solution is selected from phosphate buffer solution, citrate buffer solution, acetate buffer solution, and histidine buffer solution.

In some embodiments, the reaction in the above steps is carried out at 10-30 °C, such as 15-28 °C, 20-25 °C, e.g. 22 °C.

In some embodiments, the molar ratio of the reducing agent to $(E)_m$ ranges from 30:1 to 1:1, such as 25:1 to 2:1, 20:1 to 5:1, 15:1 to 8:1, and for instance, 10:1.

In some embodiments, the compound of formula II-1 used in step 2 is present in the reaction solution obtained after the completion of step 1 reaction.

In some embodiments, the solution containing the conjugate of formula I-3 in step 3 is a solution of conjugate of formula I-3 in a buffer (such as the buffer mentioned above).

In some embodiments, the solution containing the conjugate of formula I-3 in step 3 is a reaction solution obtained after the completion of step 2 reaction.

The present inventors had unexpectedly discovered that, in step 2, adding the raw material (compound of formula I-2a) in batches can make the DAR value of the final product more uniform, resulting in a product with improved quality. In addition, adjusting the pH to acidic conditions in step 2, such as 5.5-7.0, 5.8-6.8, or 6.0-6.6, also helped to improve the uniformity of the DAR value of the final product.

Method 2 comprises the following steps:

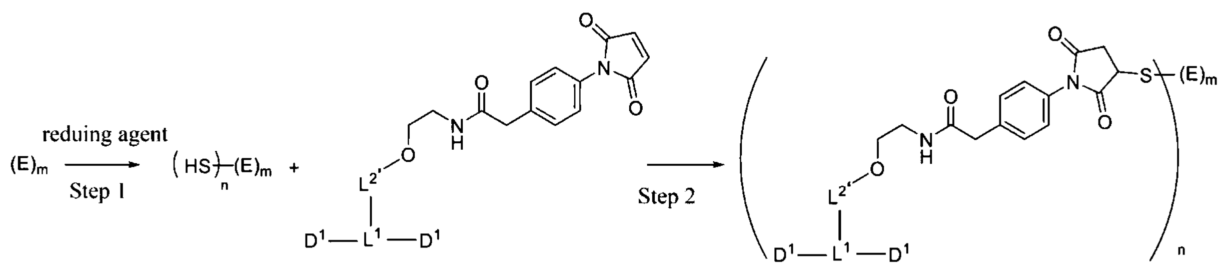


II-1 I-2b I-1b

wherein,

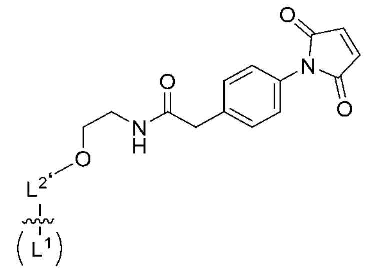

corresponds to $L^3$,

wherein,

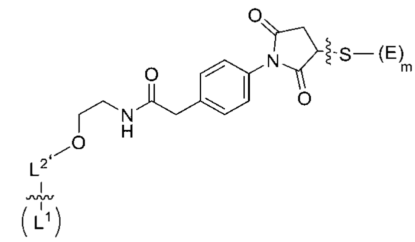

corresponds to $L^2$,

wherein, n, m, E, $D^1$, $L^1$, $L^2$, and $L^3$ are as defined above, preferably, $L^3$ is selected from formulas L3-8 to L3-14, and $L^2$ is selected from formulas L2-8 to L2-14;

wherein, step 1, $(E)_m$ is dissolved in a buffer solution, and a reducing agent (such as TCEP, DTT) is added to reduce the disulfide bond in $(E)_m$, to obtain a compound of formula II-1 with thiol residues;

step 2, a buffer solution containing 6-14 molar equivalents of compound of formula I-2b is added to compound of formula II-1, and under the condition of pH 5.5-8 (e.g., 5.8-7.6, 6.0-7.5), the Michael addition reaction is carried out, to obtain conjugate of formula I-1b.

In some embodiments, the buffer solution, reaction temperature, the molar ratio of reducing agent to $(E)_m$ is as defined in Method 1.

In some embodiments, the compound of formula II-1 used in step 2 is present in a reaction solution obtained after completion of step 1 reaction.

**Pharmaceutical compositions**

In some embodiments, the present invention provides a pharmaceutical composition comprising the conjugate of Formula I-1, I-A, or Formula I-B, the compound of Formula I-2, I-5a, or I-6a, or a conjugate or compound of a subformula thereof, or a pharmaceutically acceptable salt, ester, isomer, solvate, prodrug, or isotopically labeled compound thereof. For the sake of simplicity, the conjugate of Formula I-1, I-A, or Formula I-B, the compound of Formula I-2, I-5a, or I-6a, or the conjugate or compound of subformula thereof, or the pharmaceutically acceptable salt, ester, isomer, solvate, prodrug, or isotopically labeled compound thereof may be referred to simply as "the compound of the invention". In an embodiment, the composition further comprises a pharmaceutically acceptable excipient. In an embodiment, the pharmaceutical composition comprises the compound of the present invention in combination with one or more other therapeutic agents.

"Pharmaceutically acceptable excipients" as used herein includes any and all physiologically compatible solvents, disperse media, isotonic agents, absorption delaying agents, and the like.

For the use and application of pharmaceutically acceptable excipients, please also refer to "Handbook of Pharmaceutical Excipients", the 8th edition, R.C. Rowe, P.J. Seskey, and S.C. Owen, Pharmaceutical Press, London, Chicago.

The pharmaceutical composition of the present invention can exist in various forms. These forms include, for example, liquid, semi-solid, and solid dosage forms, such as liquid solutions (e.g., injectable solutions and infusion solutions), powders or suspensions, liposomal formulations, and suppositories. The preferred form depends on the intended mode of administration and therapeutic use.

The pharmaceutical comprising the compound of the present invention may be prepared by mixing the compound of the present invention with one or more optional pharmaceutically acceptable excipients, preferably in the form of a lyophilized formulation or an aqueous solution.

## Definitions

The following provides definitions for the terms involved in the present invention. In addition, those skilled in the art can also understand these terms in conjunction with the prior art. For undefined terms, they have their common meaning as understood by one of ordinary skill in the art to which this invention pertains.

The term "inhibiting neuraminidase activity" used herein refers to an $IC_{50}$ value of less than or equal to 1000 nM. For instance, it is determined according to the neuraminidase inhibition experimental method in the examples herein. In some embodiments, an $IC_{50}$ value of less than or equal to 100 nM or less than or equal to 10 nM for inhibiting neuraminidase activity is used to indicate a compound inhibits neuraminidase activity.

The term "inhibiting virus growth" used herein refers to an $EC_{50}$ value of less than or equal to 1000 nM. For example, it is determined by the experimental method for the influenza virus-mediated cytopathic inhibition activity in the examples herein. In some embodiments, an $EC_{50}$ value of less than or equal to 100 nM or less than or equal to 10 nM for inhibiting virus growth activity is used to indicate a compound inhibitis virus growth activity.

The term "Fc domain monomer" used herein refers to a polypeptide chain or its functional fragment (such as a fragment capable of forming a dimer with another Fc domain monomer and binding to the Fc receptor) that possesses the following characteristics: the polypeptide chain comprises the second and third antibody constant regions (CH2 and CH3), as well as, optionally, the fourth antibody constant region. In some cases, the Fc domain monomer also comprises at least one hinge region or a portion of the hinge region. This Fc domain monomer may be any immunoglobulin antibody type, including IgG, IgE, IgM, IgA, and IgD. Additionally, the Fc domain monomer may be any IgG subtype (such as IgG1, IgG2a, IgG2b, IgG3, or IgG4). For example, in natural antibodies, the immunoglobulin Fc domain comprises the second and third constant domains (CH2 and CH3 domains) derived from the two heavy chains of IgG, IgA, and IgD antibodies; or comprises the second, third, and fourth constant domains (CH2, CH3, and CH4 domains) derived from the two heavy chains of IgM and IgE antibodies. Unless otherwise specified herein, the amino acid numbering in this IgG or Fc domain monomer follows the EC numbering system for antibodies (also known as the Kabat EU, or EU numbering system, described in Kabat et al., Sequences of Proteins of Immunological Interest, the 5th Edition, Public Health Services, National Institute of Health, Bethesda, MD, 1991). The Fc domain monomer may be derived from any species, such as synthetic, human, mouse, rat, llama, etc. The Fc domain monomer may comprise one or more non-natural amino acid sequences, serving as site-specific conjugation sites for small molecule drugs. The Fc domain monomer may comprise one or more solvent-exposed cysteine or lysine residues that have been site-specifically engineered, providing additional conjugation sites for small molecule drugs.

The term "Fc domain" used herein refers to a dimer formed by the interaction between two Fc domain monomers by the hinge region, and/or CH2, and/or CH3 antibody constant regions. In some embodiments, the monomers of the dimer have one or more disulfide bonds between them. The Fc domain may be derived from any species, such as synthetic, human, mouse, rat, llama, etc. The Fc domain can comprise one or more non-natural amino acid sequences, serving as site-specific conjugation sites for small molecule drugs. The Fc domain may comprise one or more solvent-exposed cysteine or lysine residues that have been site-specifically engineered to provide additional conjugation sites for small molecule drugs.

The term "covalent linking" used herein refers to two parts of a conjugate are linked to each other by a covalent bond, which is formed between two atoms in the two parts of the conjugate.

The term "Fc binding peptide" used herein refers to a polypeptide consisting of a continuous amino acid sequence of 5 to 50 amino acids (e.g., 5 to 40, 5 to 30, 5 to 20, 5 to 15, 5 to 10, 10 to 50, 10 to 40, 10 to 30, or 10 to 20), which has affinity for the Fc domain (e.g., any Fc domain described herein) and the function to bind to the Fc domain. The Fc binding peptide may be derived from any species, such as synthetic, human, mouse, rat, llama, etc. The Fc binding peptide may comprise one or more non-natural amino acid sequences, serving as site-specific conjugation sites for small molecule drugs. The Fc binding peptide may comprise one or more solvent-exposed cysteine or lysine residues that have been site-specifically engineered to provide additional conjugation sites for small molecule drugs.

The term "solvent exposed" used herein refers to amino acid residues surrounded by solvent molecules that surround the protein or polypeptide. Solvent-exposed amino acid molecules may be naturally occurring or artificially modified (including both natural and non-natural amino acids). In some embodiments, the modification of this site does not affect the three-dimensional structure of the protein.

The term "% identity" used herein refers to the percentage of amino acid residues in a protein or polypeptide sequence that are identical to the amino acid residues in a reference sequence after gaps are introduced into the alignment sequence as necessary to achieve maximum percentage identity (i.e., gaps may be introduced into one or both of the candidate and reference sequence for optimal alignment, and nonhomologous sequences may be ignored for comparison purposes). Alignments for the purpose of achieving percentage identity in sequence alignment can be achieved in various ways within the skill of the art, such as but not limited to publicly available computer software, such as software BLAST, ALIGN, MegAlign (DNA Star), etc.

The term "treatment" or "for treatment" used herein refers to therapeutic treatment of an individual after infection

with a virus. In some embodiments, therapeutic treatment may slow down the progression of viral infection, alleviate symptoms in the individual, and/or eliminate viral infection.

The term "effective amount" or "effective dose" refers to the amount or dose of the antibody, fragment, composition, or combination of the present invention that, when administered to a patient in a single or multiple doses, produces the desired effect in the patient in need of treatment or prevention.

The term "$C_1$-$C_{20}$ alkyl" alone or in combination (e.g., $C_1$-$C_{20}$ alkylaryl) refers to a saturated, straightchain or branched alkyl containing 1-20 carbons, such as 1-15, 1-10 (e.g., 1, 2, 3, 4, 5, 6, 7, 8, 9, or 10 carbons), particularly 1-6 carbons, including methyl, ethyl, propyl, isopropyl, butyl, sec-butyl, isobutyl, tert-butyl, n-pentyl, 2-pentyl, 3-pentyl, 2-methyl-2-butyl, 3-methyl-2-butyl, 3-methyl-1-butyl, 2-methyl-1-butyl, n-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, etc. Preferably, "$C_{1-20}$ alkyl" is any one of methyl, ethyl, isopropyl, and tert-butyl.

The term "$C_2$-$C_{20}$ alkenyl" refers to a straight or branched alkyl group as defined above, but it contains one or more double bonds, for example, 1 to 10 double bonds, such as 1, 2, 3, 4, or 5 double bonds, but it does not include an alkynyl. Exemplary alkenyls include vinyl, propenyl, isopropenyl, butenyl, sec-butenyl, isobutenyl, n-pentenyl, 2-pentenyl, 3-pentenyl, 2-methyl-2-butenyl, 3-methyl-2-butenyl, 3-methyl-1-butenyl, 2-methyl-1-butenyl, n-hexenyl, 2-hexenyl, 3-hexenyl, 2-methyl-2-pentenyl, etc.

The term "$C_{3-20}$ cycloalkyl" alone or in combination (e.g., $C_3$-$C_{20}$ cycloalkylaryl) refers to a saturated cycloalkyl having 3-20, such as 3-15, 3-10 (e.g., 3, 4, 5, 6, 7, 8, 9, or 10 carbon atoms), particularly 3-6 carbon atoms, including cyclopropyl, cyclobutyl, cyclopentyl, cyclohexyl, cycloheptyl, etc. Particular "$C_{3-7}$ cycloalkyl" refers to cyclopropyl, cyclopentyl, cyclohexyl, etc.

The term "$C_3$-$C_{20}$ cycloalkenyl" refers to a cycloalky containing 3-20 carbons, such as 3-15 or 3-10 carbons (e.g., 3, 4, 5, 6, 7, 8, 9, or 10 carbons), particularly 3-6 carbons, it contains one or more double bonds, such as 1-10 double bonds, for example, 1, 2, 3, 4, or 5 double bonds, but it does not contain an alkynyl and is not an aromatic group. Exemplary cycloalkenyls include cyclopropenyl, cyclobutenyl, cyclopentenyl, cyclohexenyl, cycloheptenyl, etc. Particular "$C_{3-7}$ cycloalkenyl" refers to cyclopropenyl, cyclopentenyl, cyclohexenyl, etc.

The term "halogen" alone or in combination refers to fluorine, chlorine, bromine, or iodine, and particularly fluorine, chlorine, or bromine.

The term "haloalkyl" alone or in combination refers to an alkyl as defined above that is substituted with one or more (e.g., 1, 2, 3, 4, or 5) halogens.

The term "amino" alone or in combination refers to primary amino ($-NH_2$), secondary amino (-NH-), or tertiary amino.

The term "carbonyl ", also known as "-C(=O)-", refers to a divalent radical consisting of only one carbon and one oxygen, with the carbon and oxygen atoms linked by a double bond, and the carbon in its own structure is also linked to two other fragments via single bonds.

The term "heterocycloalkyl", also known as "heterocyclic group", refers to a saturated or partially unsaturated (containing 1 or 2 double bonds) non-aromatic cyclic group composed of carbons and heteroatoms such as nitrogen, oxygen, or sulfur. This cyclic group may be a monocyclic or bicyclic group. In the present invention, the heterocycloalkyl may be a 3-20 membered heterocycloalkyl, such as a 3-15 membered, 3-10 membered, 3-7 membered, 3-6 membered, 5-7 membered, or 4-6 membered heterocycloalkyl. The number of carbons in the heterocycloalkyl may range from 2 to 16, such as 2 to 11, and the number of heteroatoms may be one or more, preferably 1, 2, 3, or 4. The nitrogen, carbon, or sulfur atoms in the heterocycloalkyl may be optionally oxidized. The hydrogens in the "heterocycloalkyl" may be independently and optionally substituted with one or more substituents described in the present invention. The "heterocycloalkyl" may be linked to the parent molecule by any ring atom in the ring. The terms "3-6 membered heterocycloalkyl" and "3-7 membered heterocycloalkyl" refer to saturated or partially unsaturated monocyclic or polycyclic heterocycloalkyls containing 3-6 and 3-7 ring members (which are selected from carbons and heteroatoms or heteroatom groups), respectively, with the heteroatoms or heteroatom groups being selected from N, O, $S(O)_m$ (wherein m is any integer from 0 to 2); e.g. aziridinyl, azetidinyl, oxetanyl, tetrahydropyrrolyl, oxopyrrolidinyl, tetrahydrofuranyl, tetrahydrothienyl, piperidinyl, morpholinyl, piperazinyl, thiomorpholinyl, tetrahydropyranyl, 1,1-dioxothiomorpholinyl, etc.

The term "aryl" refers to any stable 6-15 membered, for example 6-10 membered, monocyclic or bicyclic aromatic carbocyclic hydrocarbon group, including phenyl, naphthyl, tetrahydronaphthyl, 2,3-dihydroindenyl, or biphenyl, etc. The hydrogens in the "aryl" are independently and optionally substituted with one or more substituents described in this invention.

The term "heteroaryl" or "heterocyclic aryl" refers to an aromatic ring group formed by the substitution of a carbon in the ring with at least one heteroatom selected from sulfur, oxygen, or nitrogen. This aromatic ring group may be a 5-15 membered ring, such as a 5-7 membered or 5-6 membered monocyclic ring or a 7-12 membered bicyclic ring, including but not limited to 5, 6, 7, 8, 9, or 12 membered heteroaryls. In the present invention, the number of heteroatoms in the heteroaryl is preferably 1, 2, 3, or 4. Exemplary heteroaryls are selected from thienyl, pyridyl, pyrimidinyl, pyrazinyl, pyridazinyl, pyridin-2(1H)-one group, pyridin-4(1H)-one group, pyrrolyl, pyrazolyl, thiazolyl, 1,2,3-triazolyl, 1,2,4-triazolyl,

imidazolyl, tetrazolyl, isothiazolyl, oxazolyl, isoxazolyl, thiadiazolyl, oxadiazolyl, benzothienyl, indolyl, benzimidazolyl, benzothiazolyl, benzofuranyl, quinolinyl, isoquinolinyl, quinazolinyl, etc. The hydrogens in the "heteroaryl" are independently and optionally substituted with one or more substituents described in the present invention.

The term "$C_{6-15}$ aryl" refers to an aryl containing 6-15 carbons, where "aryl" is as defined above.

The term "5 to 15-membered heteroaryl" refers to an aromatic heterocycle group containing 5-15 ring atoms, where "heteroaryl" is as defined above.

The term "cyano" refers to the group -CN.

The term "carboxyl" refers to the group -COOH.

The term "hydroxyl" refers to the group -OH.

The term "substituted" refers to having one or more substituents, such as 1-25, 1-20, 1-10, or 1-5 substituents, for example, 1, 2, 3, 4, 5 substituents. Substituents include, but are not limited to, alkyl, cycloalkyl, alkenyl, alkynyl, aryl, alkaryl, acyl, heteroaryl, heteroalkyl, heterocycloalkyl, heteroalkenyl, heteroalkynyl, heteroalkaryl, halogen, oxo, cyano, nitro, amino, alkylamino, hydroxyl, alkoxy, alkanoyl, carbonyl, carbamoyl, guanidino, ureido, amidino, and combinations of the above groups or moieties. Substituents include but are not limited to F, Cl, methyl, phenyl, and benzyl, OR, $NR_2$, SR, SOR, $SO_2R$, OCOR, NRCOR, $NRCONR_2$, NRCOOR, $OCONR_2$, RCO, COOR, alkyl-OOCR, $SO_3R$, $CONR_2$, $SO_2NR_2$, $NRSO_2NR_2$, CN, $CF_3$, $OCF_3$, $SiR_3$, and $NO_2$, wherein each R is independently selected from H, alkyl, alkenyl, aryl, heteroalkyl, heteroalkenyl, or heteroaryl, and wherein two optionally present substituents in the same or adjacent atoms may be joined to form a fused, optionally substituted, aromatic or non-aromatic, saturated or unsaturated ring containing 3-8 members, or two optionally present substituents in the same atom may be joined to form an optionally substituted, aromatic or non-aromatic, saturated or unsaturated ring containing 3-8 members.

The term "optionally" indicates that the subsequent event or situation and the same may occur/exist or not occur/exist. For example, "optionally substituted" indicates that the involved group may be substituted or not substituted.

The term "comprising" or "including" also encompasses situations consisting of or essentially consisting of the features or elements involved.

The term "stereoisomer" encompasses all forms of isomeric forms, including enantiomers, diastereomers, and geometric isomers (cis/trans isomers). Therefore, individual stereochemical isomers of the compounds designed in the present invention, or mixtures of their enantiomers, diastereomers, or geometric isomers (or cis/trans isomers), all fall within the scope of the present invention.

The term "pharmaceutically acceptable salt" refers to the compounds of the present invention in the form of their pharmaceutically useful salts, including acid addition salts and base addition salts. In the present invention, pharmaceutically acceptable non-toxic acid addition salts refer to salts formed between the compounds of the present invention and organic or inorganic acids, including but not limited to hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, etc. Pharmaceutically acceptable non-toxic base addition salts refer to salts formed between the compounds of the present invention and organic or inorganic bases, including but not limited to alkali metal salts such as lithium, sodium, or potassium salts; alkaline earth metal salts such as calcium or magnesium salts; and organic base salts such as ammonium salts or $N^+(C_{1-6}$ alkyl$)_4$ salts formed by reacting with organic bases containing N groups.

The term "solvate" refers to a complex formed by one or more solvent molecules and the compound of the present invention. Solvents that form solvates include, but are not limited to, water, methanol, ethanol, isopropanol, ethyl acetate, tetrahydrofuran, N,N-dimethylformamide, dimethylsulfoxide, etc.

The term "hydrate" refers to a complex formed by water and the compound of the present invention.

The term "prodrug" refers to a chemical derivative of the compound of the present invention, which is converted into the compound represented by general formula I-1 or I-2 by a chemical reaction *in vivo.*

The term "isotopically labeled compound" refers to isotopically labeled compound obtained by substituting one or more hydrogens, such as 1, 2, 3, 4, or 5 hydrogens, in general formula I-1 and formula I-2 with deuterium, and isotopically labeled compound obtained by substituting carbons with one or more (such as 1-3) carbon-14 atoms ($^{14}C$).

The term "pharmaceutical combination" refers to non-fixed combination products or fixed combination products, including but not limited to drug kits and pharmaceutical compositions. The term "non-fixed combination" means that the active ingredients (such as (i) compound of the present invention (including conjugate of Formula I-1, I-A, or Formula I-B, compound of Formula I-2, I-5a, or I-6a, or pharmaceutically acceptable salt, ester, isomer, solvate, prodrug, or isotopically labeled compound), and (ii) other therapeutic agents) are administered to patients simultaneously, without specific time limitation, or sequentially at the same or different time intervals, as separate entities, wherein such administration provides two or more active agents at a prophylactically or therapeutically effective level in the patient. In some embodiments, the compound of the present invention and other therapeutic agents used in the pharmaceutical combination are administered at the doses that do not exceed those when they are used individually. The term "fixed combination" refers to simultaneous administration of two or more active agents to a patient as a single entity. It is preferred to select the doses and/or time

intervals of the two or more active agents so that the combined use of each component can produce a greater effect in treating a disease or condition than that of any one component alone. Each component may be in the form of a separate formulation, which may be the same or different.

The term "drug : antibody ratio" or "DAR" refers to the ratio of the small molecule drug moieties ($D^1$ and $D^2$) conjugated to the E moiety (such as antibody, Fc domain, or albumin) described herein to the E moiety. In some embodiments described herein, DAR is represented by n:m in formula I-1 and ranges from 1 to 20, preferably from 2 to 10, such as 2, 3, 4, 5, 6, 7, 8, 9, or 10. DAR can also be calculated as the average DAR of the molecular population in the product, which is the overall ratio of the small molecule drug moieties ($D^1$ and $D^2$) conjugated to the E moiety (such as antibody, Fc domain, or albumin) described herein to the E moiety in the product determined by a detection method (such as MS). This DAR is referred to as average DAR in the text. In some embodiments, the average DAR value of the conjugates of the present invention is between 0.5 and 10.0, such as 1.0-8.0, for example, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0, and ranges having two of these values as endpoints.

In the context, when variables such as n and y are integers within or selected from a certain range, it implies that all integers within that range are considered in the present invention, as if they were listed one by one herein. For instance, when describing n as an integer from 1 to 8, it is equivalent to having described n as an integer of 1, 2, 3, 4, 5, 6, 7, or 8 herein.

**Sequences SEQ ID NO: 1-81 and their description**

SEQ ID NO: 1: Murine Fc-IgG2a with an IL2 signal sequence (in bold) at the N-terminus (in bold)

**MYRMQLLSCIALSLALVTNS**PRGPTIKPC(!)PPC(!)KCPAPNLLGGPSVFIFPPKIKDVLMISLSPIVTC
VVVDVSEDDPDVQISWFVNNVEVHTAQTQTHREDYNSTLRVVSALPIQHQDWMSGKEFKCKVNN
KDLPAPIERTISKPKGSVRAPQVYVLPPPEEEMTKKQVTLTCMVTDFMPEDIYVEWTNNGKTELNYK
NTEPVLDSDGSYFMYSKLRVEKKNWVERNSYSCSVVHEGLHNHHTTKSFSRTPGK

SEQ ID NO: 2: Mature murine Fc-IgG2a

PRGPTIKPC(!)PPC(!)KCPAPNLLGGPSVFIFPPKIKDVLMISLSPIVTCVVVDVSEDDPDVQISWFVNN
VEVHTAQTQTHREDYNSTLRVVSALPIQHQDWMSGKEFKCKVNNKDLPAPIERTISKPKGSVRAPQ
VYVLPPPEEEMTKKQVTLTCMVTDFMPEDIYVEWTNNGKTELNYKNTEPVLDSDGSYFMYSKLRV
EKKNWVERNSYSCSVVHEGLHNHHTTKSFSRTPGK

SEQ ID NO: 3: Human Fc-IgG1 with an N-terminal (in bold) IL2 signal sequence and an N-terminal MVRS amino acid sequence (underlined)

**MYRMQLLSCIALSLALVTNS**MVRSDKTHTC(!)PPC(!)PAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVS
NKALPAPIEKTISKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK

SEQ ID NO: 4: Mature human Fc-IgG1 with MVRS amino acid sequence (underlined) added at the N-terminus

MVRSDKTHTC(!)PPC(!)PAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

SEQ ID NO: 5: Murine Fc-IgG2a with an IL2 signal sequence (in bold) added at the N-terminus and six-histidine tag (in italic) added at the C-terminus

**MYRMQLLSCIALSLALVTNS**PRGPTIKPC(!)PPC(!)KCPAPNLLGGPSVFIFPPKIKDVLMISLSPIVTCVVVDVSEDDPDVQISWFVNNVEVHTAQTQTHREDYNSTLRVVSALPIQHQDWMSGKEFKCKVNNKDLPAPIERTISKPKGSVRAPQVYVLPPPEEEMTKKQVTLTCMVTDFMPEDIYVEWTNNGKTELNYKNTEPVLDSDGSYFMYSKLRVEKKNWVERNSYSCSVVHEGLHNHHTTKSFSRTPGK*HHHHHH*

SEQ ID NO: 6: Mature murine Fc-IgG2a with six-histidine tag (in italics) added at the C-terminus

PRGPTIKPC(!)PPC(!)KCPAPNLLGGPSVFIFPPKIKDVLMISLSPIVTCVVVDVSEDDPDVQISWFVNNVEVHTAQTQTHREDYNSTLRVVSALPIQHQDWMSGKEFKCKVNNKDLPAPIERTISKPKGSVRAPQVYVLPPPEEEMTKKQVTLTCMVTDFMPEDIYVEWTNNGKTELNYKNTEPVLDSDGSYFMYSKLRVEKKNWVERNSYSCSVVHEGLHNHHTTKSFSRTPGK*HHHHHH*

SEQ ID NO: 7: Human Fc-IgG1 with an IL2 signal sequence (in bold) and MVRS amino acid sequence (underlined) added at the N-terminus, and six-histidine tag (in italic) added at the C-terminus

**MYRMQLLSCIALSLALVTNS**<u>MVRS</u>DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK*HHHHHH*

SEQ ID NO: 8: Mature human Fc-IgG1 with an MVRS amino acid sequence (underlined) added at the N-terminus and six-histidine tag (in italic) added at the C-terminus

<u>MVRS</u>DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK*HHHHHH*

SEQ ID NO: 9: Human Fc-IgG1 with an IL2 signal sequence (in bold) and MVRS amino acid sequence (underlined) added at the N-terminus, two additional cysteines (*) in the hinge region, and six-histidine tag (in italic) added at the C-terminus

**MYRMQLLSCIALSLALVTNS**<u>MVRS</u>DKTHTC(!)PPC(!)PPC*KC*PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGKHHHHHH

SEQ ID NO:10: Mature human Fc-IgG1 with MVRS amino acid sequence (underlined) added at the N-terminus, two additional cysteines (*) in the hinge region, and six-histidine tag (in italic) added at the C-terminus

<u>MVRS</u>DKTHTC(!)PPC(!)PPC*KC*PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK*HHHHHH*

SEQ ID NO: 11: Mature human Fc-IgG1 with MVRS amino acid sequence (underlined) added at the N-terminus, and two additional cysteines (*) in the hinge region

<u>MVRS</u>DKTHTC(!)PPC(!)PPC*KC*PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQMQGNVFSCSVMHEALHNHYTQKSLSLSPGK

SEQ ID NO: 12: Murine Fc-IgG2a with an IL2 signal sequence (in bold) and an Asn-to-Ala substitution (*) at the N-terminus, and six-histidine tag (in italic) added at the C-terminus

**MYRMQLLSCIALSLALVTNS**PRGPTIKPC(!)PPC(!)KCPAPNLLGGPSVFIFPPKIKDVLMISLSPIVTCVVVDVSEDDPDVQISWFVNNVEVHTAQTQTHREDYA*STLRVVSALPIQHQDWMSGKEFKCKVNNKDLPAPIERTISKPKGSVRAPQVYVLPPPEEEMTKKQVTLTCMVTDFMPEDIYVEWTNNGKTELNYKNTEPVLDSDGSYFMYSKLRVEKKNWVERNSYSCSVVHEGLHNHHTTKSFSRTPGK*HHHHHH*

SEQ ID NO: 13: Murine Fc-IgG2a with an Asn-to-Ala substitution (*) and six-histidine tag (in italic) added at the C-terminus

PRGPTIKPC(!)PPC(!)KCPAPNLLGGPSVFIFPPKIKDVLMISLSPIVTCVVVDVSEDDPDVQISWFVNNVEVHTAQTQTHREDYA*STLRVVSALPIQHQDWMSGKEFKCKVNNKDLPAPIERTISKPKGSVRAPQVYVLPPPEEEMTKKQVTLTCMVTDFMPEDIYVEWTNNGKTELNYKNTEPVLDSDGSYFMYSKLRVEKKNWVERNSYSCSVVHEGLHNHHTTKSFSRTPGK*HHHHHH*

SEQ ID NO: 14: Human Fc-IgG1 with an IL2 signal sequence (in bold) and MVRS amino acid sequence (underlined) added at the N-terminus, containing an Asn-to-Ala substitutions (*), and with six-histidine tag (in italic) added at the C-terminus

**MYRMQLLSCIALSLALVTNS**<u>MVRS</u>DKTHTC(!)PPC(!)PAPELLGGPSVFLFPPKPKDTLMISRTPEVTCVVVDVSHEDPEVKFNWYVDGVEVHNAKTKPREEQYA*STYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK*HHHHHH*

SEQ ID NO: 15: Human Fc-IgG1 with MVRS amino acid sequence (underlined) added at the N-terminus, containing an Asn-to-Ala substitutions (*), and with six-histidine tag (in italic) added at the C-terminus

<u>MVRS</u>DKTHTC(!)PPC(!)PAPELLGGPSVFLFPPKPKDTLMISRTPEVTCVVVDVSHEDPEVKFNWYVDGVEVHNAKTKPREEQYA*STYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK*HHHHHH*

SEQ ID NO: 16: Human Fc-IgG1 with a human serum albumin signal sequence (in bold) added at the N-terminus and an N-terminal ISAMVRS amino acid sequence (underlined)

**MKWVTFISLLFLFSSAYS**<u>ISAMVRS</u>DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 17: Human Fc-IgG1 with a human serum albumin signal sequence (in bold) added at the N-terminus, an N-terminal ISAMVRS amino acid sequence (underlined), a C-terminal G4S connector (in italic), and a C-terminal c-Myc tag (underlined and in italic)

**MKWVTFISLLFLFSSAYS**<u>ISAMVRS</u>DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG*GGGGS*<u>*EQKLISEEDL*</u>

SEQ ID NO: 18: Mature human Fc-IgG1 with an ISAMVRS amino acid sequence (underlined) added at the N-terminus, a G4S connector (in italic) added at the C-terminus, and a c-Myc tag (underlined and italicized) added at the C-terminus

<u>ISAMVRS</u>DKTHTC(!)PPC(!)PAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAKGQ PREPQVYTLPPSREEMTKNQVSLTCLVKGFYPSDIAVEWESNGQPENNYKTTPPVLDSDGSFFLYSKL TVDKSRWQQGNVFSCSVMHEALHNHYTQKSLSLSPG*GGGGS<u>EQKLISEEDL</u>*

SEQ ID NO: 19: Human Fc-IgG1 with a human serum albumin signal sequence (in bold) and the ISAMVRS amino acid sequence (underlined) added at the N-terminus, and containing a lysine-to-serine change (*) to prevent conjugation of at this site

**MKWVTFISLLFLFSSAYS**<u>ISAMVRS</u>DKTHTC(!)PPC(!)PAPELLGGPSVFLFPPKPS*DTLMISRTPEVT CVVVDVSHEDPEVKFNWYVDGVEVHNAKTKPREEQYNSTYRVVSVLTVLHQDWLNGKEYKCKVS

NKALPAPIEKTISKAKGQPREPQVYTLPPSREEMTKNQVSLTCLVKGFYPSDIAVEWESNGQPENNYK TTPPVLDSDGSFFLYSKLTVDKSRWQQGNVFSCSVMHEALHNHYTQKSLSLSPGK

SEQ ID NO: 20: Mature human Fc-IgG1 with the ISAMVRS amino acid sequence (underlined) at the N-terminus, and containing a lysine-to-serine change (*) to prevent conjugation at this site

<u>ISAMVRS</u>DKTHTC(!)PPC(!)PAPELLGGPSVFLFPPKPS*DTLMISRTPEVTCVVVDVSHEDPEVKFNW YVDGVEVHNAKTKPREEQYNSTYRVVSVLTVLHQDWLNGKEYKCKVSNKALPAPIEKTISKAKGQ PREPQVYTLPPSREEMTKNQVSLTCLVKGFYPSDIAVEWESNGQPENNYKTTPPVLDSDGSFFLYSKL TVDKSRWQQGNVFSCSVMHEALHNHYTQKSLSLSPGK

SEQ ID NO: 21: Human Fc-IgG1 with a human serum albumin signal sequence (in bold) and the ISAMVRS amino acid sequence (underlined) added at the N-terminus, containing a lysine-to-serine change (*) to prevent conjugation at this site, and with a G4S connector (in italic) and a c-Myc tag (underlined and in italic) added at C-terminus

**MKWVTFISLLFLFSSAYS**<u>ISAMVRS</u>DKTHTC(!)PPC(!)PAPELLGGPSVFLFPPKPS(*)DTLMISRTPEV TCVVVDVSHEDPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLSLSPG*GGGGS<u>EQKLIS EEDL</u>*

SEQ ID NO: 22: Mature human Fc-IgG1 with an ISAMVRS amino acid sequence (underlined) added at the N-terminus, containing a lysine-to-serine change (*) to prevent conjugation at this site, and with a G4S connector (in italic) and c-Myc tag (underlined, in italic) added at the C-terminus

<u>ISAMVRS</u>DKTHTC(!)PPC(!)PAPELLGGPSVFLFPPKPS(*)DTLMISRTPEVTCVVVDVSHEDPEVKFN WYVDGVEVHNAKTKPREEQYNSTYRVVSVLTVLHQDWLNGKEYKCKVSNKALPAPIEKTISKAKG QPREPQVYTLPPSREEMTKNQVSLTCLVKGFYPSDIAVEWESNGQPENNYKTTPPVLDSDGSFFLYSK LTVDKSRWQQGNVFSCSVMHEALHNHYTQKSLSLSPG*GGGGS<u>EQKLISEEDL</u>*

SEQ ID NO: 23: Human Fc-IgG1 with a human serum albumin signal sequence (in bold) and the ISAMVRS amino acid sequence (underlined) added at the N-terminus, containing a change from Asn to Ala (*), and with a G4S connector (in italic) and a c-Myc tag (underlined and in italic) added at the C-terminus

**MKWVTFISLLFLFSSAYS**<u>ISAMVRS</u>DKTHTC(!)PPC(!)PAPELLGGPSVFLFPPKPKDTLMISRTPEVT CVVVDVSHEDPEVKFNWYVDGVEVHNAKTKPREEQYA(*)STYRVVSVLTVLHQDWLNGKEYKCK VSNKALPAPIEKTISKAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLSLSPG*GGGGS<u>EQKLIS EEDL</u>*

SEQ ID NO: 24: Mature human Fc-IgG1 with an ISAMVRS amino acid sequence (underlined) added at the N-terminus, containing a change from Asn to Ala (*), and with a G4S connector (in italic) and c-Myc tag (underlined, in

italic) added at the C-terminus

ISAMVRSDKTHTC(!)PPC(!)PAPELLGGPSVFLFPPKPKDTLMISRTPEVTCVVVDVSHEDPEVKFNWYVDGVEVHNAKTKPREEQYA(*)STYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQKLISEEDL*

SEQ ID NO: 25: Human Fc-IgG1 with a human serum albumin signal sequence (in bold) and ISAMVRS amino acid sequence (underlined) added at the N-terminus, containing H310A (*) and H435A (*) changes to prevent FcRn binding, and with a G4S connector (in italic) and c-Myc tag (underlined, in italic) added at the C-terminus

**MKWVTFISLLFLFSSAYS**ISAMVRSDKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLA(*)QDWLNGKEYKCKVSNKALPAPIEKTISKA(*)KGQPREPQVYTLPPSREEMTKNQVSLTCLVKGFYPSDIAVEWESNGQPENNYKTTPPVLDSDGSFFLYSKLTVDKSRWQQGNVFSCSVMHEALHNAYTQKSLSLSPG*GGGGSEQKLISEEDL*

SEQ ID NO: 26: Human Fc-IgG1 with an ISAMVRS amino acid sequence (underlined) added at the N-terminus, containing H310A (*) and H435A (*) changes to prevent FcRn binding, and with a G4S connector (in italic) and c-Myc tag (underlined, in italic) added at the C-terminus

ISAMVRSDKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLA(*)QDWLNGKEYKCKVSNKALPAPIEKTISKA(*)KGQPREPQVYTLPPSREEMTKNQVSLTCLVKGFYPSDIAVEWESNGQPENNYKTTPPVLDSDGSFFLYSKLTVDKSRWQQGNVFSCSVMHEALHNAYTQKSLSLSPG*GGGGSEQKLISEEDL*

SEQ ID NO: 27: Human Fc-IgG1 with a human serum albumin signal sequence (in bold) and ISAMVRS amino acid sequence (underlined) added at the N-terminus, and with a G4S connector (in italic) and a mutant (lysine to phenylalanine, in bold) c-Myc tag (underlined, in italic) added at the C-terminus

**MKWVTFISLLFLFSSAYS**ISAMVRSDKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQ***F***LISEEDL*

SEQ ID NO: 28: Mature human Fc-IgG1 with an ISAMVRS amino acid sequence (underlined) added at the N-terminus and with a G4S connector (in italic) and a mutant (lysine to phenylalanine, in bold) c-Myc tag (underlined, in italic) added at the C-terminus

ISAMVRSDKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQ***F***LISEEDL*

SEQ ID NO: 29: Human Fc-IgG1 with a human serum albumin signal sequence (in bold) and an ISAMVRS amino acid sequence (underlined) added at the N-terminus, containing a substitution from Asn to Ala (*), and with a G4S connector (in italic) and a mutant (lysine to phenylalanine, in bold) c-Myc tag (underlined, in italic) added at the C-terminus

**MKWVTFISLLFLFSSAYS**ISAMVRSDKTHTC(!)PPC(!)PAPELLGGPSVFLFPPKPKDTLMISRTPEVTCVVVDVSHEDPEVKFNWYVDGVEVHNAKTKPREEQYA(*)STYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQ**F**LISEEDL*

SEQ ID NO: 30: Mature human Fc-IgG1 with an ISAMVRS amino acid sequence (underlined) added at the N-terminus, containing a substitution from Asn to Ala (*), and with a G4S connector (in italic) and a mutant (lysine to phenylalanine, in bold) c-Myc tag (underlined, in italic) added at the C-terminus

ISAMVRSDKTHTC(!)PPC(!)PAPELLGGPSVFLFPPKPKDTLMISRTPEVTCVVVDVSHEDPEVKFNWYVDGVEVHNAKTKPREEQYA(*)STYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQ**F**LISEEDL*

SEQ ID NO: 31: Human Fc-IgG1 with a human serum albumin signal sequence (in bold) added at the N-terminus, with an allotype G1m(fa) (in bold and italic), and with a G4S connector (in italic) and a mutant (lysine to phenylalanine, in bold) c-Myc tag (underlined, in italic) added at the C-terminus

**MKWVTFISLLFLFSSAYS**DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQ**F**LISEEDL*

SEQ ID NO: 32: Human Fc-IgG1 with a human serum albumin signal sequence (in bold) added at the N-terminus, containing an allotype G1m(fa) (in bold and italic)

**MKWVTFISLLFLFSSAYS**DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 33: Mature human Fc-IgG1 with an MVRS amino acid sequence (underlined) added at the N-terminus and containing the YTE triple mutations (in bold, underlined)

MVRSDKTHTC(!)PPC(!)PAPELLGGPSVFLFPPKPKDTL**Y**I**T**R**E**PEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 34: Human Fc-IgG1 with a human serum albumin signal sequence (in bold) at the N-terminus, containing the EPKSS amino acid sequence (underlined) of the hinge region of mature human Fc-IgG1, and containing a change from cysteine to serine (#) and allotype G1m(fa) (in bold and italic)

**MKWVTFISLLFLFSSAYS**EPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 35: Human Fc-IgG1 with a murine IgG signal sequence (in bold) added at the N-terminus, and having the hinge region EPKSSD amino acid sequence of Mature human Fc-IgG removed, and having an allotype G1m(fa) (in bold and italic)

**MGWSCIILFLVATATGVHS**KTHTC(!)PPC(!)PAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPI
EKTISKAKGQPREPQVYTLPPSR***D***E***L***TKNQVSLTCLVKGFYPSDIAVEWESNGQPENNYKTTPPVLDS
DGSFFLYSKLTVDKSRWQQGNVFSCSVMHEALHNHYTQKSLSLSPGK

SEQ ID NO: 36: Mature human Fc-IgG1 which has the hinge region EPKSSD amino acid sequence of mature human Fc-IgG1 removed at the N-terminus, has allotype G1m(fa) (in bold and italic), and contains YTE triple mutations (in bold, underlined)

KTHTC(!)PPC(!)PAPELLGGPSVFLFPPKPKDTL**Y**I**T**R**E**PEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTL
PPSR***D***E***L***TKNQVSLTCLVKGFYPSDIAVEWESNGQPENNYKTTPPVLDSDGSFFLYSKLTVDKSRWQ
QGNVFSCSVMHEALHNHYTQKSLSLSPGK

SEQ ID NO: 37: Mature human Fc-IgG1 which has the hinge region EPKSSD amino acid sequence of mature human Fc-IgG1 removed at the N-terminus, has LS double mutations (in bold, underlined), and possesses allotype G1m(fa) (in italic)

KTHTC(!)PPC(!)PAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTL
PPSR***D***E***L***TKNQVSLTCLVKGFYPSDIAVEWESNGQPENNYKTTPPVLDSDGSFFLYSKLTVDKSRWQ
QGNVFSCSV**L**HEALH**S**HYTQKSLSLSPGK

SEQ ID NO: 38: Human Fc-IgG1 with a human serum albumin signal sequence (in bold) added at the N-terminus, containing YTE triple mutations (in bold, underlined), having allotype G1m(fa) (in bold and italic), and with a G4S connector (in italic) and c-Myc tag (underlined) added at the C-terminus

**MKWVTFISLLFLFSSAYS**DKTHTC(!)PPC(!)PAPELLGGPSVFLFPPKPKDTL**Y**I**T**R**E**PEVTCVVVDVS
HEDPEVKFNWYVDGVEVHNAKTKPREEQYNSTYRVVSVLTVLHQDWLNGKEYKCKVSNKALPAPI
EKTISKAKGQPREPQVYTLPPSR***D***E***L***TKNQVSLTCLVKGFYPSDIAVEWESNGQPENNYKTTPPVLDS
DGSFFLYSKLTVDKSRWQQGNVFSCSVMHEALHNHYTQKSLSLSPG*GGGGSEQKLISEEDL*

SEQ ID NO: 39: Mature human Fc-IgG1, wherein $X_1$ is Met or Trp, $X_2$ is Ser or Thr, $X_3$ is Thr or Glu, $X_4$ is Asp or Glu, and $X_5$ is Leu or Met, $X_6$ is Met or Leu, and $X_7$ is Asn or Ser

DKTHTC(!)PPC(!)PAPELLGGPSVFLFPPKPKDTL$X_1$I$X_2$R$X_3$PEVTCVVVDVSHEDPEVKFNWYVDGV
EVHNAKTKPREEQYNSTYRVVSVLTVLHQDWLNGKEYKCKVSNKALPAPIEKTISKAKGQPREPQV
YTLPPSR$X_4$E$X_5$TKNQVSLTCLVKGFYPSDIAVEWESNGQPENNYKTTPPVLDSDGSFFLYSKLTVDKS
RWQQGNVFSCSV$X_6$HEALH$X_7$HYTQKSLSLSPG

SEQ ID NO: 40: Mature human Fc-IgG1, wherein $X_4$ is Asp or Glu, and $X_5$ is Leu or Met

DKTHTC(!)PPC(!)PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR$X_4$E$X_5$TKNQVSLTCLVKGFYPSDIAVEWESNGQPENNYKTTPPVLDSDGSFFLYSKLTVDKSR
WQQGNVFSCSVMHEALHNHYTQKSLSLSPG

SEQ ID NO: 41: Mature human Fc-IgG1 with YTE triple mutations (in bold, underlined), wherein $X_4$ is Asp or Glu, and $X_5$ is Leu or Met

DKTHTC(!)PPC(!)PAPELLGGPSVFLFPPKPKDTL**Y**I**T**R**E**PEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR$X_4$E$X_5$TKNQVSLTCLVKGFYPSDIAVEWESNGQPENNYKTTPPVLDSDGSFFLYSKLTVDKSR
WQQGNVFSCSVMHEALHNHYTQKSLSLSPG

SEQ ID NO: 42: Mature human Fc-IgG1 with YTE triple mutations (in bold, underlined) and with allotype G1m(fa) (in bold and italic)

DKTHTC(!)PPC(!)PAPELLGGPSVFLFPPKPKDTL**Y**I**T**R**E**PEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR***D***E***L***TKNQVSLTCLVKGFYPSDIAVEWESNGQPENNYKTTPPVLDSDGSFFLYSKLTVDKSRW
QQGNVFSCSVMHEALHNHYTQKSLSLSPG

SEQ ID NO: 43: Mature human Fc-IgG1 with YTE triple mutations (in bold, underlined) and with allotype G1m(f) (in bold and italic)

DKTHTC(!)PPC(!)PAPELLGGPSVFLFPPKPKDTL**Y**I**T**R**E**PEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR***E***E***M***TKNQVSLTCLVKGFYPSDIAVEWESNGQPENNYKTTPPVLDSDGSFFLYSKLTVDKSRW
QQGNVFSCSVMHEALHNHYTQKSLSLSPG

SEQ ID NO: 44: Mature human Fc-IgG1 with LS double mutations (in bold, underlined), wherein $X_4$ is Asp or Glu, and $X_5$ is Leu or Met

DKTHTC(!)PPC(!)PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR$X_4$E$X_5$TKNQVSLTCLVKGFYPSDIAVEWESNGQPENNYKTTPPVLDSDGSFFLYSKLTVDKSR
WQQGNVFSCSV**L**HEALH**S**HYTQKSLSLSPG

SEQ ID NO: 45: Mature human Fc-IgG1 with LS double mutations (in bold, underlined) and with allotype G1m(fa) (in bold and italic)

DKTHTC(!)PPC(!)PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR***D***E***L***TKNQVSLTCLVKGFYPSDIAVEWESNGQPENNYKTTPPVLDSDGSFFLYSKLTVDKSRW
QQGNVFSCSV**L**HEALH**S**HYTQKSLSLSPG

SEQ ID NO: 46: Mature human Fc-IgG1 with LS double mutations (in bold, underlined) and with allotype G1m(f) (in bold and italic)

DKTHTC(!)PPC(!)PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYT
LPPSR***E***E***M***TKNQVSLTCLVKGFYPSDIAVEWESNGQPENNYKTTPPVLDSDGSFFLYSKLTVDKSRW
QQGNVFSCSV**L**HEALH**S**HYTQKSLSLSPG

SEQ ID NO: 47: Mature human Fc-IgG1 with a mouse heavy chain MIgG Vh signal sequence (in bold) added at the N-terminus, and having a change from cysteine to serine (#), wherein $X_1$ is Met or Trp, $X_2$ is Ser or Thr, $X_3$ is Thr or Glu, $X_4$ is Asp or Glu, and $X_5$ is Leu or Met, $X_6$ is Met or Leu, and $X_7$ is Asn or Ser

**MGWSCIILFLVATATGVHS**NVNHKPSNTKVDKKVEPKSS(#)DKTHTC(!)PPC(!)PAPELLGGPSVFLF
PPKPKDTL$X_1$I$X_2$R$X_3$PEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSR$X_4$E$X_5$TKNQVSLTCLVKGF
YPSDIAVEWESNGQPENNYKTTPPVLDSDGSFFLYSKLTVDKSRWQQGNVFSCSV$X_6$HEALH$X_7$HYT
QKSLSLSPG

SEQ ID NO: 48: Mature human Fc-IgG1 with a mouse heavy chain MIgG Vh signal sequence (in bold) added at the N-terminus, having a change from cysteine to serine (#), and having allotype G1m(fa) (in bold and italic)

**MGWSCIILFLVATATGVHS**NVNHKPSNTKVDKKVEPKSS(#)DKTHTC(!)PPC(!)PAPELLGGPSVFLF
PPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSR***D***E***L***TKNQVSLTCLVKGFYPSD
IAVEWESNGQPENNYKTTPPVLDSDGSFFLYSKLTVDKSRWQQGNVFSCSVMHEALHNHYTQKSLS
LSPGK

SEQ ID NO: 49: Mature human Fc-IgG1 with a mouse heavy chain MIgG Vh signal sequence (in bold) added at the N-terminus, having a change from cysteine to serine (#), and having allotype G1m(f) (in bold and italic)

**MGWSCIILFLVATATGVHS**NVNHKPSNTKVDKKVEPKSS(#)DKTHTC(!)PPC(!)PAPELLGGPSVFLF
PPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSR***E***E***M***TKNQVSLTCLVKGFYPS
DIAVEWESNGQPENNYKTTPPVLDSDGSFFLYSKLTVDKSRWQQGNVFSCSVMHEALHNHYTQKSL
SLSPGK

SEQ ID NO: 50: Mature human Fc-IgG1 with a mouse heavy chain MIgG Vh signal sequence (in bold) added at the N-terminus, having a change from cysteine to serine (#), having M428L and N434S mutations (in bold, underlined), and having allotype G1m(fa) (in bold and italic)

**MGWSCIILFLVATATGVHS**NVNHKPSNTKVDKKVEPKSS(#)DKTHTC(!)PPC(!)PAPELLGGPSVFLF
PPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSR***D***E***L***TKNQVSLTCLVKGFYPSD
IAVEWESNGQPENNYKTTPPVLDSDGSFFLYSKLTVDKSRWQQGNVFSCSV**L**HEALH**S**HYTQKSLSL
SPGK

SEQ ID NO: 51: Mature human Fc-IgG1 with a mouse heavy chain MIgG Vh signal sequence (in bold) added at the N-terminus, having a change from cysteine to serine (#), having M428L and N434S mutations (in bold, underlined), and having allotype G1m(f) (in bold and italic)

**MGWSCIILFLVATATGVHS**NVNHKPSNTKVDKKVEPKSS(#)DKTHTC(!)PPC(!)PAPELLGGPSVFLF
PPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSR***E***E***M***TKNQVSLTCLVKGFYPS
DIAVEWESNGQPENNYKTTPPVLDSDGSFFLYSKLTVDKSRWQQGNVFSCSV**L**HEALH**S**HYTQKSLS
LSPGK

SEQ ID NO: 52: Mature human Fc-IgG1 with a mouse heavy chain MIgG Vh signal sequence (in bold) added at the N-terminus, having a change from cysteine to serine (#), having YTE triple mutations (in bold, underlined), and having allotype G1m(fa) (in bold and italic)

**MGWSCIILFLVATATGVHS**NVNHKPSNTKVDKKVEPKSS(#)DKTHTC(!)PPC(!)PAPELLGGPSVFLF
PPKPKDTL**Y**I**T**R**E**PEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSR***D***E***L***TKNQVSLTCLVKGFYPSD
IAVEWESNGQPENNYKTTPPVLDSDGSFFLYSKLTVDKSRWQQGNVFSCSVMHEALHNHYTQKSLS
LSPGK

SEQ ID NO: 53: Mature human Fc-IgG1 with a mouse heavy chain MIgG Vh signal sequence (in bold) added at the N-terminus, having a change from cysteine to serine (#), having YTE triple mutations (in bold, underlined), and having allotype G1m(f) (in bold and italic)

**MGWSCIILFLVATATGVHS**NVNHKPSNTKVDKKVEPKSS(#)DKTHTC(!)PPC(!)PAPELLGGPSVFLF
PPKPKDTL**Y**I**T**R**E**PEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSR***E***E***M***TKNQVSLTCLVKGFYPS
DIAVEWESNGQPENNYKTTPPVLDSDGSFFLYSKLTVDKSRWQQGNVFSCSVMHEALHNHYTQKSL
SLSPGK

SEQ ID NO: 54: Mature human Fc-IgG1 with a mouse heavy chain MIgG Vh signal sequence (in bold) and ISAMVRS amino acid sequence (in italic) added at the N-terminus, having M428L and N434S mutations (in bold, underlined), with a G4S connector (in italic) and a c-Myc tag (underlined) added at the C-terminus, and containing allotype G1m(f) (in bold and italic)

**MGWSCIILFLVATATGVHS***ISAMVRS*DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***E***E***M***TKNQVSLTCLVKGFYPSDIAVEWESNGQPENNYKTTPPVLDSDGSFFLYSKLTVDKSRWQQGNVFSCSV**L**HEALH**S**HYTQKSLSLSPG*GGGGSEQKLISEEDL*

SEQ ID NO: 55: Mature human Fc-IgG1 with a mouse heavy chain MIgG Vh signal sequence (in bold) and ISAMVRS amino acid sequence (in italic) added at the N-terminus, having M428L and N434S mutations (in bold, underlined), with a G4S connector (in italic) and a c-Myc tag (underlined) added at the C-terminus, and containing allotype G1m(fa) (in bold and italic)

**MGWSCIILFLVATATGVHS***ISAMVRS*DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSV**L**HEALH**S**HYTQKSLSLSPG*GGGGSEQKLISEEDL*

SEQ ID NO: 56: Mature human Fc-IgG1 with a mouse heavy chain MIgG Vh signal sequence (in bold) and ISAMVRS amino acid sequence (in italic) added at the N-terminus, having YTE triple mutations (in bold, underlined), with a G4S connector (in italic) and a c-Myc tag (underlined) added at the C-terminus, and containing allotype G1m(f) (in bold and italic)

**MGWSCIILFLVATATGVHS***ISAMVRS*DKTHTC(!)PPC(!)PAPELLGGPSVFLFPPKPKDTL**Y**I**T**R**E**PEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***E***E***M***TKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQKLISEEDL*

SEQ ID NO: 57: Mature human Fc-IgG1 with a mouse heavy chain MIgG Vh signal sequence (in bold) and ISAMVRS amino acid sequence (in italic) added at the N-terminus, having YTE triple mutations (in bold, underlined), with a G4S connector (in italic) and a c-Myc tag (underlined) added at the C-terminus, and containing allotype G1m(fa) (in bold and italic)

**MGWSCIILFLVATATGVHS***ISAMVRS*DKTHTC(!)PPC(!)PAPELLGGPSVFLFPPKPKDTL**Y**I**T**R**E**PEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSEQKLISEEDL*

SEQ ID NO: 58: Mature human Fc-IgG1 with a mouse heavy chain MIgG Vh signal sequence (in bold) added at the N-terminus, having a change from cysteine to serine (#), with a G4S connector (in italic) and a IgA peptide tag (underlined) added at the C-terminus, and containing allotype G1m(fa) (in bold and italic)

**MGWSCIILFLVATATGVHS**EPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALHNHYTQKSLSLSPG*GGGGSQRNPRLRLIRRHPTLRIPPI*

SEQ ID NO: 59: Mature human Fc-IgG1 with a mouse heavy chain MIgG Vh signal sequence (in bold) added at the N-terminus, having a change from cysteine to serine (#), having M428L and N434S mutations (in bold, underlined), with a G4S connector (in italic) and a IgA peptide tag (underlined) added at the C-terminus, and containing allotype G1m(fa) (in bold and italic)

**MGWSCIILFLVATATGVHS**EPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSV**L**HEALH**S**HYTQKSLSLSPG*GGGGSQRNPRLRLIRRHPTLRIPPI*

SEQ ID NO: 60: Mature human Fc-IgG1, $Z_1$ is Cys or Ser, wherein $X_1$ is Met or Trp, $X_2$ is Ser or Thr, $X_3$ is Thr or Glu, $X_4$ is Asp or Glu, and $X_5$ is Leu or Met, $X_6$ is Met or Leu, and $X_7$ is Asn or Ser

NVNHKPSNTKVDKKVEPKS$Z_1$DKTHTC(!)PPC(!)PAPELLGGPSVFLFPPKPKDTL$X_1$I$X_2$R$X_3$PEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR$X_4$E$X_5$TKNQVSLTCLVKGFYPSDIAVEWESNGQPENNYKTTPPVLDSDGSFFLYSKLTVDKSRWQQGNVFSCSV$X_6$HEALH$X_7$HYTQKSLSLSPGK

SEQ ID NO: 61: Mature human Fc-IgG1, having a change from cysteine to serine (#), wherein $X_1$ is Met or Trp, $X_2$ is Ser or Thr, $X_3$ is Thr or Glu, $X_4$ is Asp or Glu, and $X_5$ is Leu or Met, $X_6$ is Met or Leu, and $X_7$ is Asn or Ser

NVNHKPSNTKVDKKVEPKSS(#)DKTHTC(!)PPC(!)PAPELLGGPSVFLFPPKPKDTL$X_1$I$X_2$R$X_3$PEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR$X_4$E$X_5$TKNQVSLTCLVKGFYPSDIAVEWESNGQPENNYKTTPPVLDSDGSFFLYSKLTVDKSRWQQGNVFSCSV$X_6$HEALH$X_7$HYTQKSLSLSPGK

SEQ ID NO: 62: Mature human Fc-IgG1, having a change from cysteine to serine (#), wherein $X_4$ is Asp or Glu, and $X_5$ is Leu or Met

NVNHKPSNTKVDKKVEPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR$X_4$E$X_5$TKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 63: Mature human Fc-IgG1, having a change from cysteine to serine (#) and allotype G1m(f) (in bold and italic)

NVNHKPSNTKVDKKVEPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNK

ALPAPIEKTISKAKGQPREPQVYTLPPSR***E***E***M***TKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 64: Mature human Fc-IgG1, having a change from cysteine to serine (#) and allotype G1m(fa) (in bold and italic)

NVNHKPSNTKVDKKVEPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 65: Mature human Fc-IgG1, having a change from cysteine to serine (#), M428L and N434S mutations (in bold, underlined), and allotype G1m(fa) (in bold and italic)

NVNHKPSNTKVDKKVEPKSS(#)DKTHTC(!)PPC(!)PAPELLGGPSVFLFPPKPKDTLMISRTPEVTCVV
VDVSHEDPEVKFNWYVDGVEVHNAKTKPREEQYNSTYRVVSVLTVLHQDWLNGKEYKCKVSNK
ALPAPIEKTISKAKGQPREPQVYTLPPSR***D***E***L***TKNQVSLTCLVKGFYPSDIAVEWESNGQPENNYKTT
PPVLDSDGSFFLYSKLTVDKSRWQQGNVFSCSV**L**HEALH**S**HYTQKSLSLSPGK

SEQ ID NO: 66: Mature human Fc-IgG1, having a change from cysteine to serine (#), M428L and N434S mutations (in bold, underlined), and allotype G1m(f) (in bold and italic)

NVNHKPSNTKVDKKVEPKSS(#)DKTHTC(!)PPC(!)PAPELLGGPSVFLFPPKPKDTLMISRTPEVTCVV
VDVSHEDPEVKFNWYVDGVEVHNAKTKPREEQYNSTYRVVSVLTVLHQDWLNGKEYKCKVSNK
ALPAPIEKTISKAKGQPREPQVYTLPPSR***E***E***M***TKNQVSLTCLVKGFYPSDIAVEWESNGQPENNYKTT
PPVLDSDGSFFLYSKLTVDKSRWQQGNVFSCSV**L**HEALH**S**HYTQKSLSLSPGK

SEQ ID NO: 67: Mature human Fc-IgG1, having a change from cysteine to serine (#), YTE triple mutations (in bold, underlined), and allotype G1m(fa) (in bold and italic)

NVNHKPSNTKVDKKVEPKSS(#)DKTHTC(!)PPC(!)PAPELLGGPSVFLFPPKPKDTL**Y**I**T**R**E**PEVTCVV
VDVSHEDPEVKFNWYVDGVEVHNAKTKPREEQYNSTYRVVSVLTVLHQDWLNGKEYKCKVSNK
ALPAPIEKTISKAKGQPREPQVYTLPPSR***D***E***L***TKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID NO: 68: Mature human Fc-IgG1, having a change from cysteine to serine (#), YTE triple mutations (in bold, underlined), and allotype G1m(f) (in bold and italic)

NVNHKPSNTKVDKKVEPKSS(#)DKTHTC(!)PPC(!)PAPELLGGPSVFLFPPKPKDTL**Y**I**T**R**E**PEVTCVV
VDVSHEDPEVKFNWYVDGVEVHNAKTKPREEQYNSTYRVVSVLTVLHQDWLNGKEYKCKVSNK
ALPAPIEKTISKAKGQPREPQVYTLPPSR***E***E***M***TKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID NO: 69: Mature human Fc-IgG1, having YTE triple mutations (in bold, underlined), and allotype G1m(fa) (in bold and italic)

NVNHKPSNTKVDKKVEPKSC(!)DKTHTC(!)PPC(!)PAPELLGGPSVFLFPPKPKDTL**Y**I**T**R**E**PEVTCVV
VDVSHEDPEVKFNWYVDGVEVHNAKTKPREEQYNSTYRVVSVLTVLHQDWLNGKEYKCKVSNK
ALPAPIEKTISKAKGQPREPQVYTLPPSR***D***E***L***TKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID NO: 70: Mature human Fc-IgG1, having a glycine-to-alanine change (#), an alanine-to-leucine change ($), an isoleucine-to-glutamic acid change (*), YTE triple mutations (in bold, underlined), and allotype G1m(fa) (in bold and italic)

NVNHKPSNTKVDKKVEPKSSDKTHTC(!)PPC(!)PAPELLA(#)GPSVFLFPPKPKDTL**Y**I**T**R**E**PEVTCVV
VDVSHEDPEVKFNWYVDGVEVHNAKTKPREEQYNSTYRVVSVLTVLHQDWLNGKEYKCKVSNK
ALPL($)PE(*)EKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

SEQ ID NO: 71: Mature human Fc-IgG1, having M428L and N434S mutations (in bold, underlined), and allotype G1m(fa) (in bold and italic)

NVNHKPSNTKVDKKVEPKSC(!)DKTHTC(!)PPC(!)PAPELLGGPSVFLFPPKPKDTLMISRTPEVTCVV
VDVSHEDPEVKFNWYVDGVEVHNAKTKPREEQYNSTYRVVSVLTVLHQDWLNGKEYKCKVSNK
ALPAPIEKTISKAKGQPREPQVYTLPPSR***D***E***L***TKNQVSLTCLVKGFYPSDIAVEWESNGQPENNYKTT
PPVLDSDGSFFLYSKLTVDKSRWQQGNVFSCSV**L**HEALH**S**HYTQKSLSLSPGK

SEQ ID NO: 72: (wild type)

NVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 73: Mature human Fc-IgG1, having M428L and N434S mutations (in bold, underlined), and allotype G1m(f) (in bold and italic)

NVNHKPSNTKVDKKVEPKSC(!)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSR***E***E***M***TKNQVSLTCLVKGFYPSDIAVEWESNGQPENNYKTTPPVLDSDGSFFLYSKLTVDKSRWQQGNVFSCSV**L**HEALH**S**HYTQKSLSLSPGK

SEQ ID NO: 74: Mature human Fc-IgG1, having GAALIE, M428L and N434S mutations (in bold, underlined), and allotype G1m(fa) (in bold and italic)

NVNHKPSNTKVDKKVEPKSC(!)DKTHTC(!)PPC(!)PAPELL**A**GPSVFLFPPKPKDTLMISRTPEVTCVVVDVSHEDPEVKFNWYVDGVEVHNAKTKPREEQYNSTYRVVSVLTVLHQDWLNGKEYKCKVSNKALP**L**P**E**EKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSV**L**HEALH**S**HYTQKSLSLSPGK

SEQ ID NO: 75: Mature human Fc-IgG1, having GAALIE, M428L and N434S mutations (in bold, underlined), and allotype G1m(f) (in bold and italic)

NVNHKPSNTKVDKKVEPKSC(!)DKTHTC(!)PPC(!)PAPELL**A**GPSVFLFPPKPKDTLMISRTPEVTCVVVDVSHEDPEVKFNWYVDGVEVHNAKTKPREEQYNSTYRVVSVLTVLHQDWLNGKEYKCKVSNKALP**L**P**E**EKTISKAKGQPREPQVYTLPPSR***E***E***M***TKNQVSLTCLVKGFYPSDIAVEWESNGQPENNYKTTPPVLDSDGSFFLYSKLTVDKSRWQQGNVFSCSV**L**HEALH**S**HYTQKSLSLSPGK

SEQ ID NO: 76: Mature human Fc-IgG1, having a change from cysteine to serine (#), GAALIE, M428L and N434S mutations (in bold, underlined), and allotype G1m(fa) (in bold and italic)

NVNHKPSNTKVDKKVEPKSS(#)DKTHTC(!)PPC(!)PAPELL**A**GPSVFLFPPKPKDTLMISRTPEVTCVVVDVSHEDPEVKFNWYVDGVEVHNAKTKPREEQYNSTYRVVSVLTVLHQDWLNGKEYKCKVSNKALP**L**P**E**EKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSV**L**HEALH**S**HYTQKSLSLSPGK

SEQ ID NO: 77: Mature human Fc-IgG1, having a change from cysteine to serine (#), GAALIE, M428L and N434S mutations (in bold, underlined), and allotype G1m(f) (in bold and italic)

NVNHKPSNTKVDKKVEPKSS(#)DKTHTC(!)PPC(!)PAPELL**A**GPSVFLFPPKPKDTLMISRTPEVTCVVVDVSHEDPEVKFNWYVDGVEVHNAKTKPREEQYNSTYRVVSVLTVLHQDWLNGKEYKCKVSNKALP**L**P**E**EKTISKAKGQPREPQVYTLPPSR***E***E***M***TKNQVSLTCLVKGFYPSDIAVEWESNGQPENNYKTTPPVLDSDGSFFLYSKLTVDKSRWQQGNVFSCSV**L**HEALH**S**HYTQKSLSLSPGK

SEQ ID NO: 78: Mature human Fc-IgG1, having a change from cysteine to serine (#), DHS triple mutations (in bold, underlined), and allotype G1m(fa) (in bold and italic)

NVNHKPSNTKVDKKVEPKSS(#)DKTHTC(!)PPC(!)PAPELLGGPSVFLFPPKPKDTLMISRTPEVTCVVVDVSHEDPEVKFNWYVDGVEVHNAKTKPREEQYNSTYRVVSVLTV**D**H**H**DWLNGKEYKCKVSNKALPAPIEKTISKAKGQPREPQVYTLPPSR***D***E***L***TKNQVSLTCLVKGFYPSDIAVEWESNGQPENNYKTTPPVLDSDGSFFLYSKLTVDKSRWQQGNVFSCSVMHEALH**S**HYTQKSLSLSPGK

SEQ ID NO: 79: Mature human Fc-IgG1, having a change from cysteine to serine (#), DHS triple mutations (in bold, underlined), and allotype G1m(f) (in bold and italic)

NVNHKPSNTKVDKKVEPKSS(#)DKTHTC(!)PPC(!)PAPELLGGPSVFLFPPKPKDTLMISRTPEVTCVV
VDVSHEDPEVKFNWYVDGVEVHNAKTKPREEQYNSTYRVVSVLTV**D**H**H**DWLNGKEYKCKVSNK
ALPAPIEKTISKAKGQPREPQVYTLPPSR***E***E***M***TKNQVSLTCLVKGFYPSDIAVEWESNGQPENNYKT
TPPVLDSDGSFFLYSKLTVDKSRWQQGNVFSCSVMHEALH**S**HYTQKSLSLSPGK

SEQ ID NO: 80: Mature human Fc-IgG1, having DHS triple mutations (in bold, underlined), and allotype G1m(fa) (in bold and italic)

NVNHKPSNTKVDKKVEPKSC(!)DKTHTC(!)PPC(!)PAPELLGGPSVFLFPPKPKDTLMISRTPEVTCVV
VDVSHEDPEVKFNWYVDGVEVHNAKTKPREEQYNSTYRVVSVLTV**D**H**H**DWLNGKEYKCKVSNK
ALPAPIEKTISKAKGQPREPQVYTLPPSR***D***E***L***TKNQVSLTCLVKGFYPSDIAVEWESNGQPENNYKTT
PPVLDSDGSFFLYSKLTVDKSRWQQGNVFSCSVMHEALH**S**HYTQKSLSLSPGK

SEQ ID NO: 81: Mature human Fc-IgG1, having DHS triple mutations (in bold, underlined), and allotype G1m(f) (in bold and italic)

NVNHKPSNTKVDKKVEPKSC(!)DKTHTC(!)PPC(!)PAPELLGGPSVFLFPPKPKDTLMISRTPEVTCVV
VDVSHEDPEVKFNWYVDGVEVHNAKTKPREEQYNSTYRVVSVLTV**D**H**H**DWLNGKEYKCKVSNK
ALPAPIEKTISKAKGQPREPQVYTLPPSR***E***E***M***TKNQVSLTCLVKGFYPSDIAVEWESNGQPENNYKT
TPPVLDSDGSFFLYSKLTVDKSRWQQGNVFSCSVMHEALH**S**HYTQKSLSLSPGK

C(!) in the sequence set forth in SEQ ID NO:1~81 is cysteine which may be linked to $L^2$.

**Description of Figures**

**Figure 1:** MS detection spectrum of conjugate 1.
**Figure 2:** SEC detection chromatogram of conjugate 1.
**Figure 3:** Detection chromatogram of residual small molecules in conjugate 1.
**Figure 4:** MS detection spectrum of conjugate 2.
**Figure 5:** SEC detection chromatogram of conjugate 2.
**Figure 6:** Detection chromatogram of residual small molecules in conjugate 2.
**Figure 7:** MS detection results of product conjugate 1 in screening experiment.
**Figure 8:** MS detection spectrum of conjugate 3.
**Figure 9:** SEC detection chromatogram of conjugate 3.
**Figure 10:** Detection chromatogram of residual small molecules in conjugate 3.
**Figure 11:** MS detection spectrum of conjugate 4.
**Figure 12:** SEC detection chromatogram of conjugate 4.
**Figure 13:** Detection chromatogram of residual small molecules in conjugate 4.

**Examples**

The above and other aspects and embodiments of the present invention are exemplified in the following examples. Any or all of the features discussed above and throughout this application may be combined in various embodiments of the present invention. The following examples further illustrate the present invention. However, it should be understood that the examples are described in an illustrative and non-limiting manner, and various modifications may be made by those skilled in the art.

**Example 1:** Preparation of Fc moiety

The protein amino acid sequence (SEQ ID No:1~68) was translated in reverse, and the corresponding nucleotide sequence was synthesized. The nucleotide sequence was added with appropriate restriction enzyme cutting sites (XbaI +

SalI) at two ends thereof and then cloned into pWX4.1 expression vector (WuXi Biologics, Shanghai). Each constructed vector carried the signal peptide sequence of human interleukin 2 or the signal peptide sequence of human serum albumin. The pWX4.1 plasmids were transfected into *E. coli* Top10 strains (Life Technologies) for DNA amplification and then purified using the PURELINK® HiPURE Plasmid Filter Maxiprep Kit (Life Technologies). Then, the plasmids were transfected into CHO cells (ATCC) by the EXPIFECTAMINE™ 293 Transfection Kit (Life Technologies). The transfected cells were cultured for 7 days, followed by centrifugation, filtration, and collection of the supernatant. Then purification was carried out using MabSelect Sure Resin (GE Healthcare, Chicago, IL, USA) to obtain purified h-IgG Fc. The purified samples were subjected to 4-12% Bis Tris SDS-PAGE electrophoresis, with 1-2 μg of sample loaded, and then stained with Coomassie Brilliant Blue. Each sample was subjected to reducing (R) and non-reducing (NR) treatment.

**Example 2:** Preparation of Fc moiety

The protein amino acid sequence (SEQ ID No: 69-81) was translated in reverse, and the corresponding nucleotide sequence was synthesized. The sequence was then homologously recombined into the pCDNA3.4 vector, with each constructed vector carrying the signal peptide sequence of human interleukin 10. The constructed plasmids were electroporated into CHO cells, and the transfected cells were cultured for 7 days, then were centrifuged to precipitate, and the supernatant was collected by filtration. Purification was performed using a protein A affinity chromatography column to obtain purified h-IgG Fc. The purified samples were subjected to Bis Tris SDS-PAGE electrophoresis, followed by staining with Coomassie Brilliant Blue. Each sample was subjected to reducing (R) and non-reducing (NR) treatment.

**Example 3:** General synthesis method of h-IgG1 Fc-SH

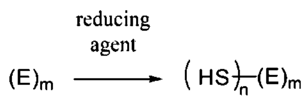


To a centrifuge tube, were successively added 50 mM PB, pH 6.5 buffer, antibody ($(E)_m$) solution (30 mg), and 10 mM reducing agent (TCEP) solution (reducing agent/$(E)_m$ molar equivalent ratio being 10). The reaction concentration of $(E)_m$ was controlled to be 5 mg/mL, and the reaction was placed in a metal bath to control the reaction temperature at 22 °C for 2 h. $(HS)_n$-$(E)_m$ with partially opened disulfide bonds was obtained, wherein E, m, and n are as defined herein.

**Example 4:** General method I for preparing conjugates

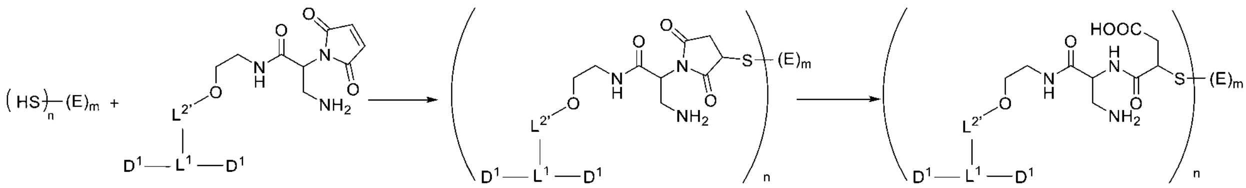

II-1 I-2a I-3 I-1a

wherein each variable is as defined herein.

To the reaction solution containing II-1, were added 50 mM PB, pH 6.5 buffer solution, and a solution of compound of formula I-2a (I-2/II-1 = 7~12). The mixture was placed in a metal bath to control the reaction temperature at 22 °C and allowed to react for 1 h. After reaching the reaction time, additional compound of formula I-2a (I-2/II-1 = 3.5~6) was added to the reaction solution. The mixture was placed in the metal bath, and the reaction temperature was controlled at 22 °C for additional 0.5 h. After reaching the reaction time, additional compound of formula I-2a (I-2/II-1 = 3.5~6) was added to the reaction solution. The mixture was placed in the metal bath, and the reaction temperature was controlled at 22 °C for additional 1.0 h, to obtain I-3.

The reaction solution was adjusted to pH = 7.5 with 0.5 M NaOH, and then placed in a metal bath to control the reaction temperature at 22 °C and allowed to react for 2 h, until all I-3 was hydrolyzed to I-1a. After the reaction solution was subjected to ultrafiltration and centrifugation for concentration, and ultrafiltration for solution exchange, the purified target conjugated compound was prepared. MS analysis was used to determine the average DAR (drug/antibody ratio) of the conjugate.

**Example 5:** Synthesis of drug intermediate (INT-DRUG)

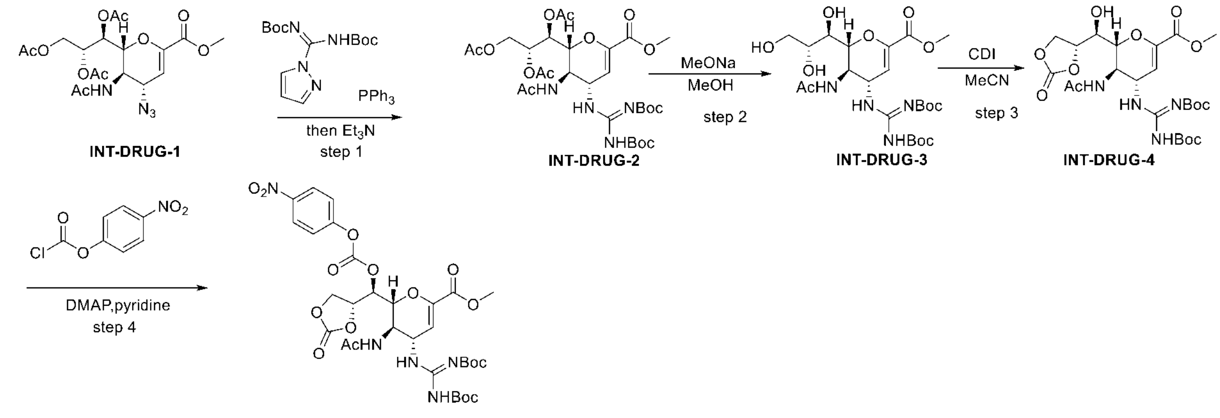

INT-DRUG-1 INT-DRUG-2 INT-DRUG-3 INT-DRUG-4 INT-DRUG

Step 1: Synthesis of INT-DRUG-2

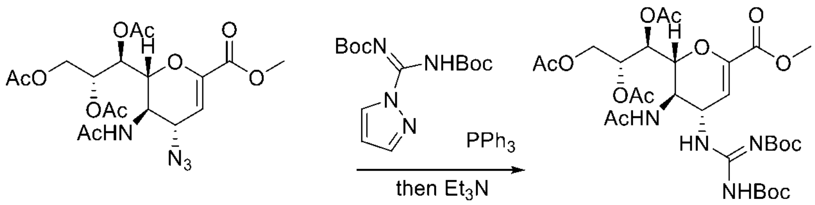

INT-DRUG-1 INT-DRUG-2

To a reaction flask, was added INT-DRUG-1 (3.0 g, 6.57 mmol), followed by addition of tetrahydrofuran (30 mL) and triphenylphosphine (1.9 g, 7.23 mmol), and then the mixture was allowed to react under stirring at 30 °C for 2 h. After completion of the reaction, lithium hydroxide monohydrate (28 mg) and water (1.5 mL) were added, and the resultant solution was stirred at 30 °C for additional 16 h. The reaction was complete by monitoring with TLC, and then N,N'-di-Boc-1H-1-guanidinylpyrazole (2.1 g, 6.91 mmol) was added, followed by stirring at 30 °C for 48 h. After completion of the reaction, the reaction solution was concentrated and purified by silica gel column chromatography (ethyl acetate/petroleum ether = 1:1). After purification, INT-DRUG-2 (2.2 g) was obtained as a colorless oil. MS: $[M+1]^+$ found 673.40.

Step 2: Synthesis of INT-DRUG-3

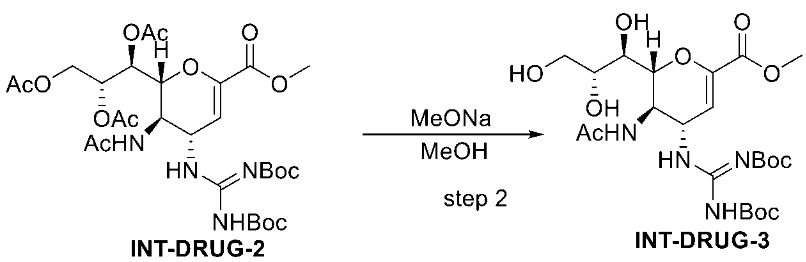

INT-DRUG-2 INT-DRUG-3

To a reaction flask, was added INT-DRUG-2 (2.19 g, 3.25 mmol), and then was protected with nitrogen, to which was then added a solution of dry sodium methoxide (0.65 mmol) in methanol (8 mL). The reaction was stirred at room temperature for 10 min, and quenched with HCl (0.4 N, 1,4-dioxane solution, 2.5 mL). The reaction solution was concentrated, and purified by silica gel column chromatography (ethyl acetate/petroleum ether, 70% to 100%), to obtain INT-DRUG-3 (1.15 g) as a white solid. MS: $[M+1]^+$ found 547.33.

Step 3: Synthesis of INT-DRUG-4

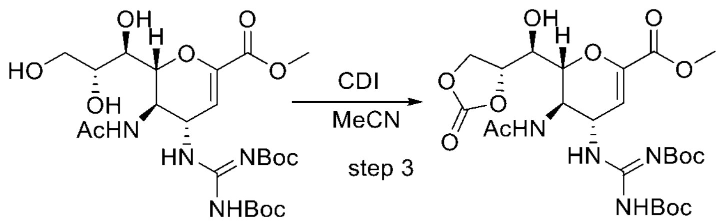


INT-DRUG-3 INT-DRUG-4

To a reaction flask, were added INT-DRUG-3 (386.9 mg, 0.71 mmol), acetonitrile (10 mL), CDI (160.8 mg, 0.99 mmol), and DMAP (302.7 mg, 2.47 mmol). The reaction was stirred at room temperature for 16 h. After completion of the reaction, the reaction solution was concentrated, and the residue was isolated and purified by silica gel column chromatography (ethyl acetate/petroleum ether, 0% to 45%) to obtain INT-DRUG-4 (242.8 mg) as a white solid. MS: $[M+1]^+$ found 573.35.

Step 4: Synthesis of INT-DRUG

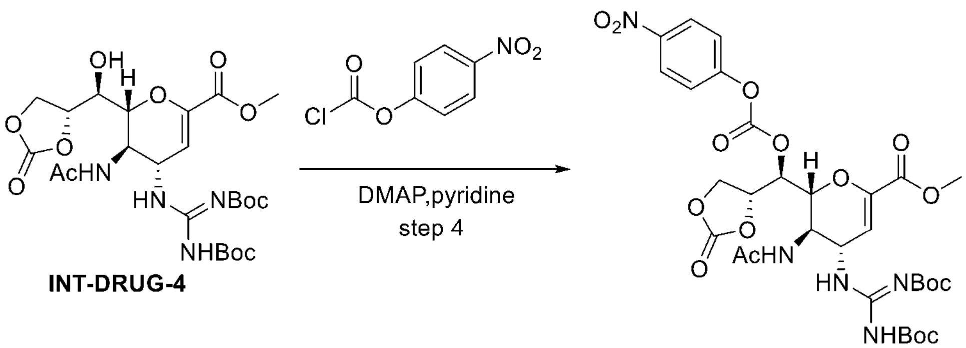


INT-DRUG-4

INT-DRUG

To a reaction flask, were added INT-DRUG-4 (403 mg, 0.7 mmol), DMAP (1.37g, 11.2 mmol), pyridine (60 mL), and p-nitrophenyl chloroformate (2.11 g, 10.5 mmol). After nitrogen purging, the reaction was stirred at 40 °C for 4 h. After LCMS detection showed >90% conversion of the reaction, the reaction was quenched by adding water (20 mL) (it cannot be placed for long). The reaction was separated with a reversed-phase column (acetonitrile/water 0%-90%) and lyophilized, to obtain INT-DRUG (173 mg) as a white solid. MS: $[M+1]^+$ found 738.33.

**Example 6:** Synthesis of intermediate A (inter-A)

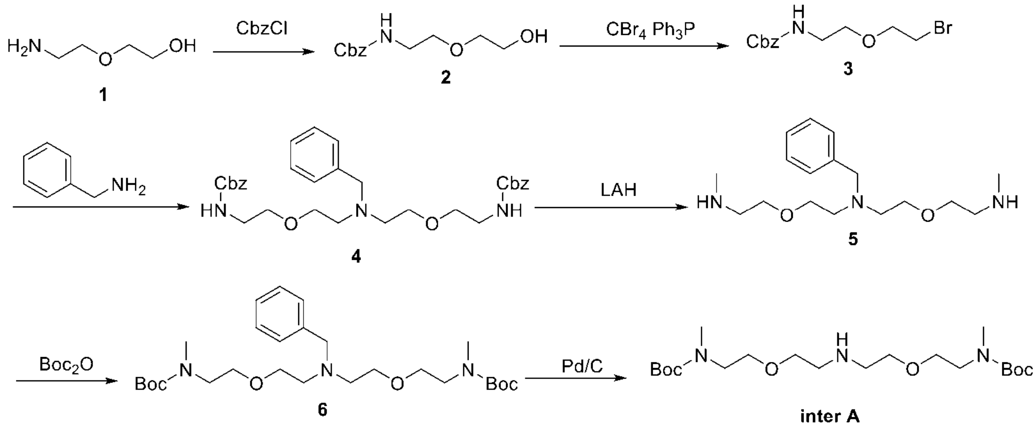


1 2 3 4 5 6 inter A

Step 1: Synthesis of intermediate 2

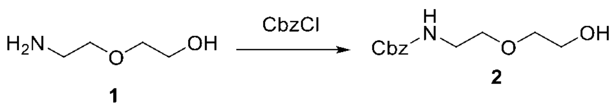


1 2

Compound 1 (150 g, 1.43 mol) and triethylamine (289 g, 2.85 mol) were added to a reaction flask, followed by addition of tetrahydrofuran (1 L). After nitrogen purging, the reaction was stirred at 0 °C. CbzCl (243 g, 1.43 mol) was dissolved in tetrahydrofuran (500 mL) and then added into the reaction solution dropwise. The reaction solution was stirred at room temperature overnight. After completion of the reaction, the reaction was quenched by adding water (3 L) and then extracted with ethyl acetate (300 mL x 2). The organic phases were combined, washed with saturated saline solution, dried over anhydrous sodium sulfate, and concentrated to obtain the crude product. The crude product was purified by silica gel column chromatography (dichloromethane/methanol, 20/1) to obtain compound 2 (255 g) as a yellow oil.

Step 2: Synthesis of intermediate 3

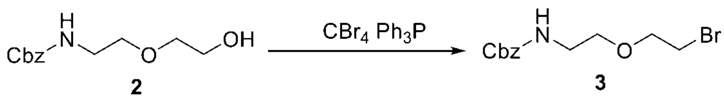


2 3

Compound 2 (245 g, 1.02 mol) and carbon tetrabromide (509 g, 1.54 mol) were added to a reaction flask, followed by addition of dichloromethane (2 L). After nitrogen purging, the mixture was stirred at 0 °C. Triphenylphosphine (403 g, 1.54 mol) was dissolved in dichloromethane (500 mL) and then added dropwise to the reaction solution. The reaction solution was stirred at room temperature for 1.5 h. After completion of the reaction, the reaction solution was concentrated to obtain a crude product. The crude product was purified by silica gel column chromatography (dichloromethane/methanol, 20/1) to yield compound 3 (200 g, crude product) as a yellow oil.

Step 3: Synthesis of intermediate 4

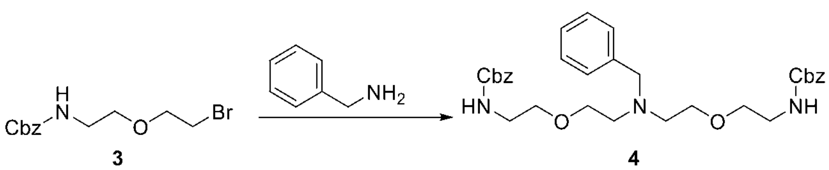

3 4

Compound 3 (200 g, crude product) was added to a reaction flask, followed by addition of N,N-dimethylformamide (2 L). Benzylamine (28.3 g, 264 mmol) and sodium carbonate (73.0 g, 528 mmol) were added to the reaction solution. The reaction solution was stirred at 60 °C overnight. After completion of the reaction, the reaction was cooled to room temperature, to which was added water (3 L), and then the resultant solution was extracted with ethyl acetate (300 mL x 2). The organic phase was combined, washed with saturated saline solution, dried over anhydrous sodium sulfate, and concentrated to obtain the crude product. The crude product was purified by silica gel column chromatography (dichloromethane/methanol, 20/1) to obtain compound 4 (45 g) as a yellow oil.

Steps 4-5: Synthesis of intermediate 6

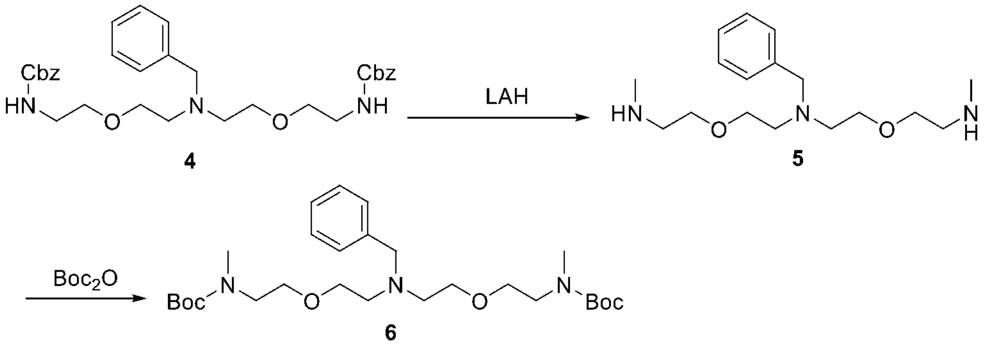


Compound 4 (43.0 g, 78.2 mmol) and tetrahydrofuran (450 mL) were added to a reaction flask, and the mixture was stirred at 0 °C after nitrogen purging. $LiAlH_4$ (23.7 g, 62.6 mmol) was added to the reaction solution. The reaction solution was stirred at room temperature for 1.5 h, and then heated to reflux and stirred for additional 6 h. After completion of the reaction, the solution was cooled to 0 °C, and sodium sulfate decahydrate (90 g) was added to quench the reaction. The reaction solution was stirred at room temperature for 1 h. Triethylamine (31.7 g, 31.3 mmol) and Boc anhydride (68.3 g, 31.3 mmol) were added to the reaction solution, and the resultant solution was stirred overnight at room temperature. After completion of the reaction, water (2 L) was added, followed by extraction with ethyl acetate (300 mL x 2). The combined organic phases were washed with saturated saline solution, dried over anhydrous sodium sulfate, and concentrated to obtain a crude product. The crude product was purified by silica gel column chromatography (dichloromethane/methanol, 30/1) to yield compound 6 (20 g) as a yellow oil. MS: $[M+1]^+$ found 510.2。

Step 6: Synthesis of inter A

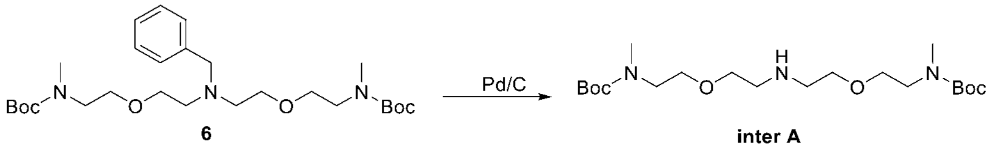


Under nitrogen atmosphere, compound 6 (19 g, 3.73 mmol), Pd/C (2 g), and methanol (400 mL) were added to a reaction flask. Replacement with hydrogen gas was performed for several times. The reaction solution was stirred at room temperature overnight. After completion of the reaction, the reaction solution was filtered, and the filter cake was washed with methanol (50 mL). The filtrate was concentrated to obtain a crude product. The crude product was purified by silica gel column chromatography (dichloromethane/methanol, 15/1) to obtain intermediate A (14 g) as a yellow oil. MS: $[M+1]^+$ found 420.2.

**Example 7:** Synthesis of intermediate B (inter B)

Step 1: Synthesis of intermediate 9

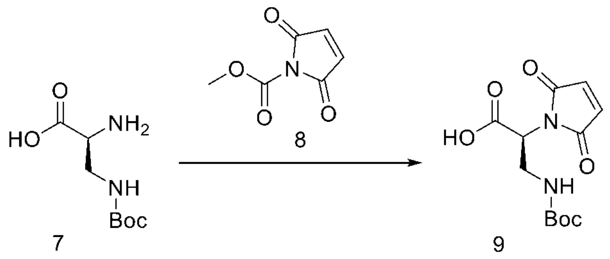

At 0 °C, compound 8 (8.4 g, 54 mmol) was gradually added to a solution of compound 7 (5.5 g, 27 mmol) in saturated aqueous sodium bicarbonate (110 ml). The mixture was then stirred at room temperature for 18 h. After completion of the reaction, the pH of the reaction solution was adjusted to weakly acidic using a saturated aqueous solution of potassium bisulfate. After the acid adjustment, the resultant solution was extracted with DCM, and the extract was then rotary evaporated to dryness. Purification was carried out using a reversed-phase column (water/acetonitrile), and then the product 9 (470 mg) was obtained after lyophilization. MS $[M+1]^+$: 285.1

Step 2: Synthesis of Inter B

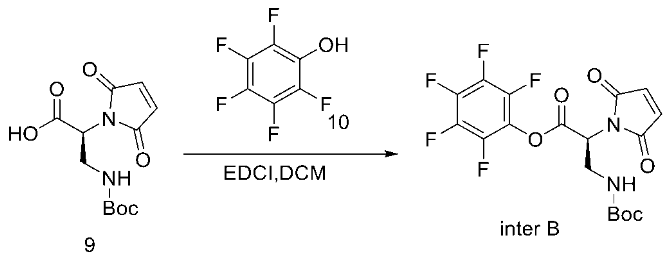


At 0 °C, EDCI (201 mg, 1.05 mmol) was added to a solution of 9 (150 mg, 0.53 mmol) and 10 (149 mg, 0.79 mmol) in DCM (20 ml). The reaction solution was stirred at room temperature for 4 h. After completion of the reaction, the solvent was removed by rotary evaporation, and purification was performed by silica gel column chromatography (PE:EA=3: 1) to obtain Inter B (110 mg) as a white solid. MS $[M+1]^+$: 451.32

**Example 8:** Synthesis of Inter C

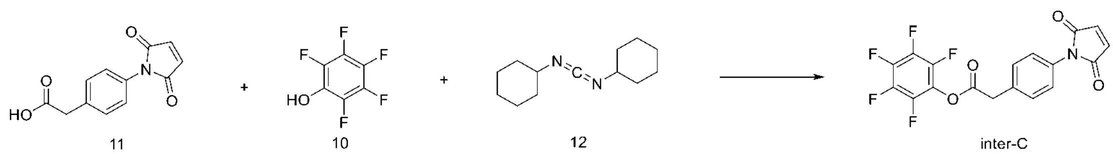

11 (1 g, 4.33 mmol), 10 (796 mg, 4.33 mmol), and 12 (892 mg, 4.33 mmol) were dissolved in DMF (40 mL), and stirred at room temperature for 2 h. After completion of the reaction, the reaction solution was filtered, and the filtrate was concentrated to obtain the crude product, which was then purified by silica gel column chromatography (ethyl acetate/-petroleum ether), to obtain Inter C (1.34 g) as a colorless oil.

**Example 9:** Synthesis of Linker 1

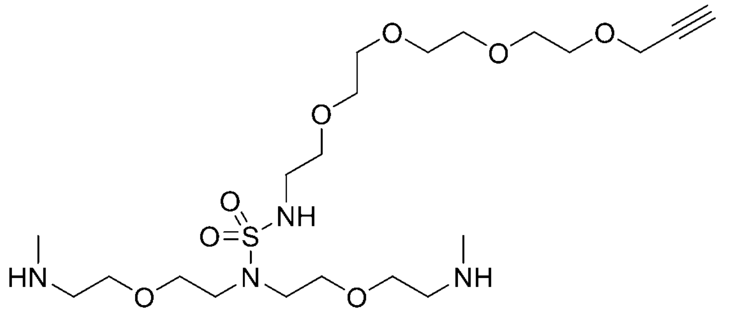

Linker 1

Step 1: Synthesis of L1-2

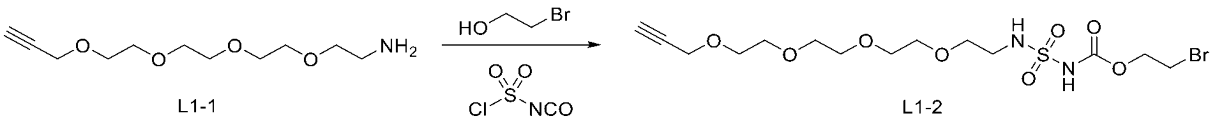


Chlorosulfonyl isocyanate (400 mg, 2.83 mmol) was dissolved in dichloromethane (10 mL), and then protected with nitrogen, followed by stirring at 0 °C. The solution of bromoethanol (353 mg, 2.83 mmol) in dichloromethane (5 mL) was added dropwise, and then the resultant solution was stirred at 0 °C for 1 h, to which was then added dropwise a solution of L1-1 (2.83 mmol) in dichloromethane (5 mL). The reaction was stirred at 0 °C for 1 h. After completion of the reaction, dichloromethane (10 mL) was added, and then the reaction solution was washed with dilute hydrochloric acid (1M, 20 mL). The organic phase was dried over anhydrous sodium sulfate. The solvent was removed by rotary evaporation, and the residue was purified by silica gel column chromatography (PE:EA=1:1), to obtain crude L1-2 (800 mg) as a yellow oil.

Step 2: Synthesis of L1-3

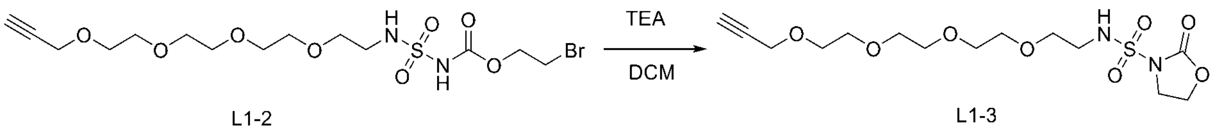


Crude L1-2 (800 mg) was dissolved in dichloromethane (10 mL), to which was added TEA (2 mL), and then the reaction was stirred at room temperature for 16 h. After completion of the reaction, dichloromethane (10 mL) was added, and then the resultant mixture was washed with dilute hydrochloric acid (1M, 20 mL). The organic phase was dried over anhydrous sodium sulfate. The solvent was removed by rotary evaporation, and the residue was purified by silica gel column chromatography (PE:EA=1:3), to obtain L1-3 (670 mg) as a yellow oil.

Step 3: Synthesis of L1-4

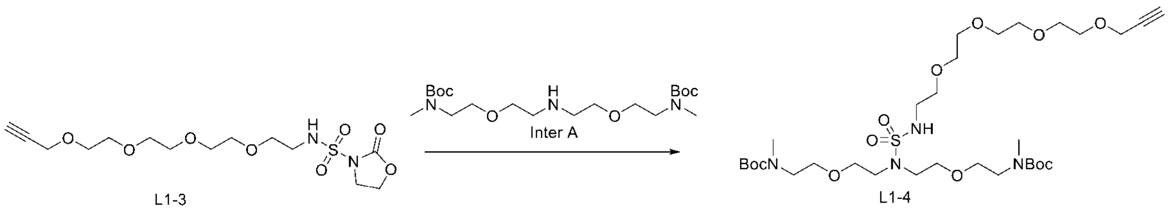


L1-3 (300 mg, 0.79 mmol), Inter-A (330 mg, 0.79 mmol), and TEA (160 mg, 1.60 mmol) were dissolved in

acetonitrile (10 mL) and stirred at 80 °C for 10 h. After completion of the reaction, the solvent was removed by rotary evaporation, and the residue was purified by reversed-phase column chromatography (acetonitrile/water) to obtain L1-4 (190 mg) as yellow oil. MS $[M+1]^+$: 713.43

Step 4: Synthesis of Linker 1

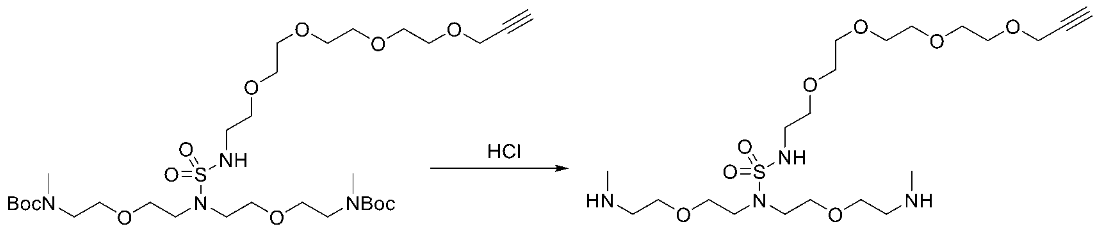


L1-4 Linker 1

L1-4 (107 mg, 0.15 mmol) was placed in a solution of hydrochloric acid in 1,4-dioxane (5 ml) and stirred at room temperature for 1 h. After completion of the reaction, the reaction solution was concentrated to obtain crude Linker 1, which was directly used in the next step. MS $[M+1]^+$: 513.51.

**Example 10:** Synthesis of Linker 2

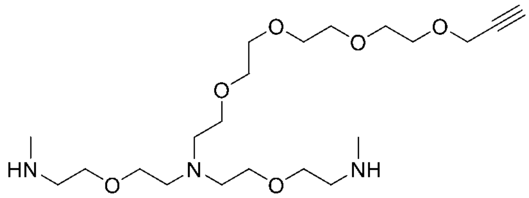

Linker 2

Step 1: Synthesis of L2-2

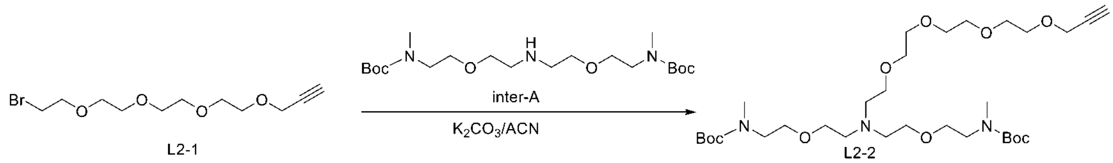


L2-1 L2-2

L2-1 (464 mg, 1.57 mmol), Inter A (600 mg, 4.06 mmol), potassium carbonate (395 mg, 2.86 mmol), and acetonitrile (10 mL) were added to a reaction flask and stirred at 85 °C for 18 h. After completion of the reaction, the reaction solution was concentrated, and the crude product was purified by a reversed-phase column to obtain L2-2 (320 mg) as a colorless oil. MS $[M+1]^+$: 634.31

Step 2: Synthesis of Linker 2

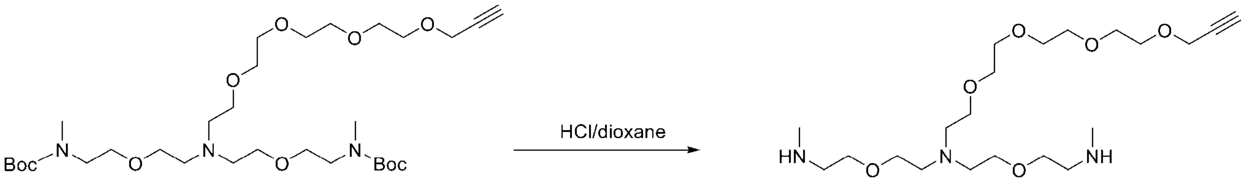

L2-2 Linker-2

L2-2 (320 mg, 0.51 mmol) was dissolved in dichloromethane (10 mL), to which was then added HCl (4 mL, 4 M in 1,4-dioxane). The reaction was stirred at room temperature for 4 h, and after completion of the reaction, the mixture was concentrated to obtain Linker 2 (219 mg) as a colorless liquid. MS $[M+1]^+$: 434.53

**Example 11:** Synthesis of Linker 3

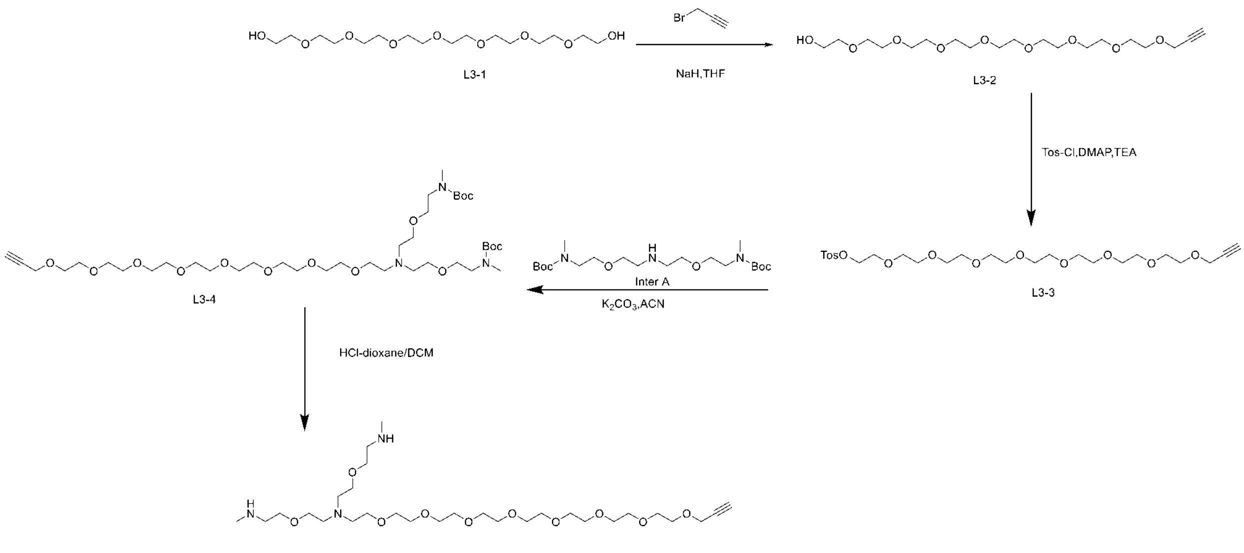

Linker 3

Step 1: Synthesis of L3-2

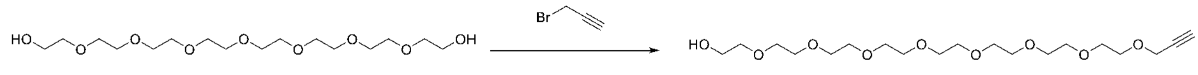

L3-1 L3-2

L3-1 (1 g, 2.7 mmol) and NaH (216 mg, 5.4 mmol) were added to a reaction flask, followed by addition of tetrahydrofuran (20 mL). After nitrogen purging, the reaction was stirred at 25 °C for 3 h. After completion of the reaction, the reaction was quenched by adding water (5 mL), and the resultant mixture was concentrated to obtain a crude product, which was purified by silica gel column chromatography (dichloromethane/methanol, 20/1), to obtain L3-2 (400 mg) as a yellow oil.

Step 2: Synthesis of L3-3

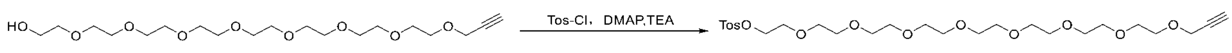

L3-2 L3-3

L3-2 (1.7 g, 4.16 mmol) and DMAP (1.01 g, 8.32 mmol) were added to a reaction flask, followed by addition of

dichloromethane (20 mL). After nitrogen purging, the reaction was stirred at 25 °C. Tos-Cl (1.18 g, 6.24 mmol) and TEA (840 mg, 8.32 mmol) were added to the above system. The reaction solution was stirred at room temperature for 15 h. After completion of the reaction, the crude product was obtained by concentration, which was purified by silica gel column chromatography (dichloromethane/methanol, 20/1), to obtain L3-3 (1.1 g, crude product) as a yellow oil.

Step 3: Synthesis of L3-4

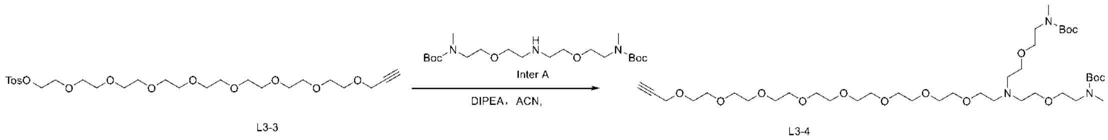


L3-3 (440 mg, 0.78 mmol) was dissolved in acetonitrile (10 mL), and then stirred. Inter A (326 mg, 0.78 mmol) and DIPEA (201 mg, 1.56 mmol) were added to the reaction solution, and then stirred at 80 °C for 10 h. After completion of the reaction, the solvent was removed by rotary evaporation. Purification was performed by reversed-phase column chromatography (acetonitrile/water), to obtain L3-4 (310 mg) as a yellow oil.

Step 4: Synthesis of Linker 3

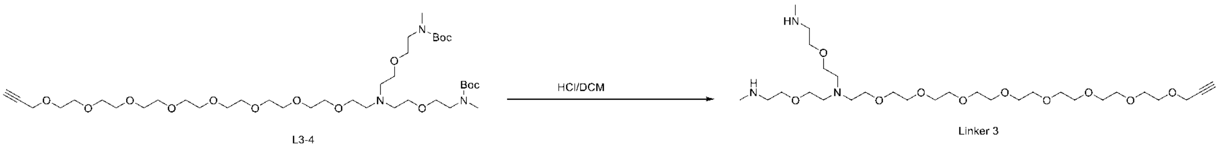


Compound 4 (247 mg, 0.3 mmol) was dissolved in dichloromethane (10 mL), to which was added a solution of hydrochloric acid in 1,4-dioxane (3 mL), and then the reaction was stirred at room temperature for 1 h. After completion of the reaction, the reaction was concentrated to obtain Linker 3 (182 mg) as a colorless oil. MS $[M+1]^+$: 610.52

**Example 12:** Synthesis of C-Inter-1

;

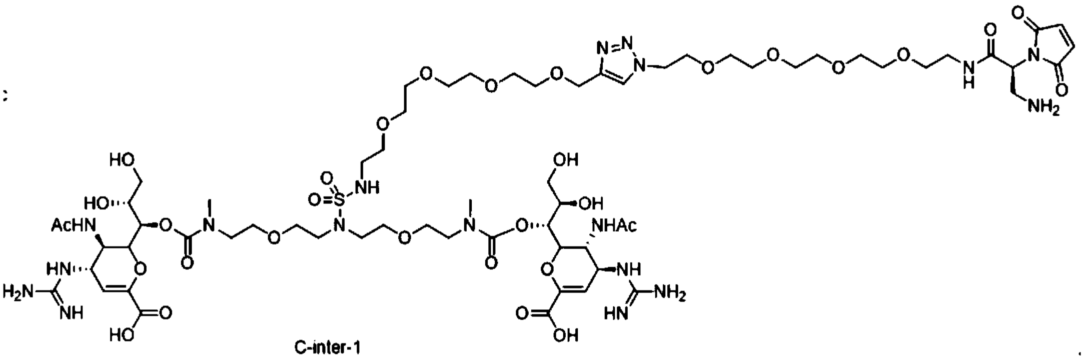

C-inter-1

.

Step 1: Synthesis of C-Inter-1-1

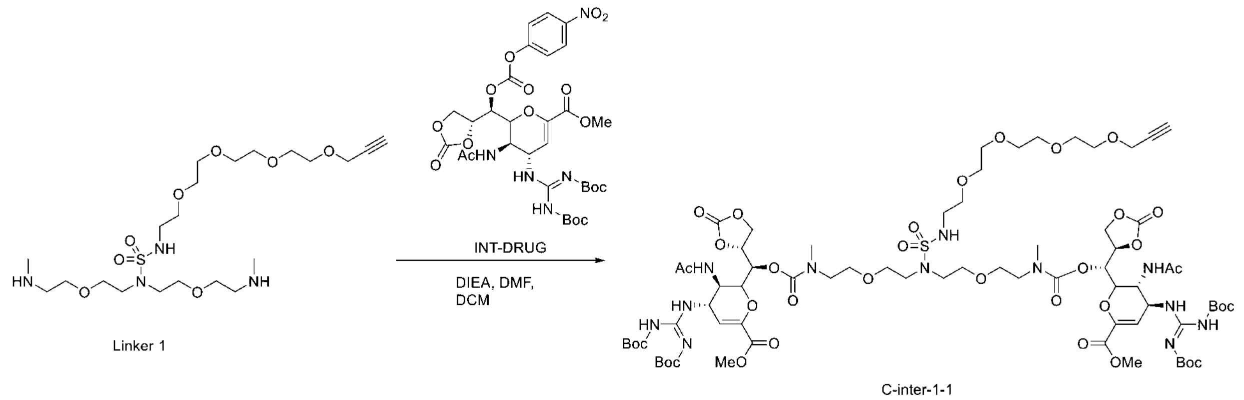


Linker 1 (0.2 mmol) was dissolved in DCM (2 ml), to which was added DIEA (0.6 g, 4.5 mmol). INT-DRUG (280 mg, 0.4 mmol) was dissolved in DCM (2 ml) and then added into the mixed solution dropwise, and stirred at room temperature for 16 h. A crude product was obtained by concentrating under reduced pressure, which was purified by reversed-phase column chromatography (acetonitrile/water) and lyophilized, to obtain C-Inter-1-1 (240 mg) as a white solid. MS $[M/2+1]^+$: 855.5

Step 2: Synthesis of C-Inter-1-2

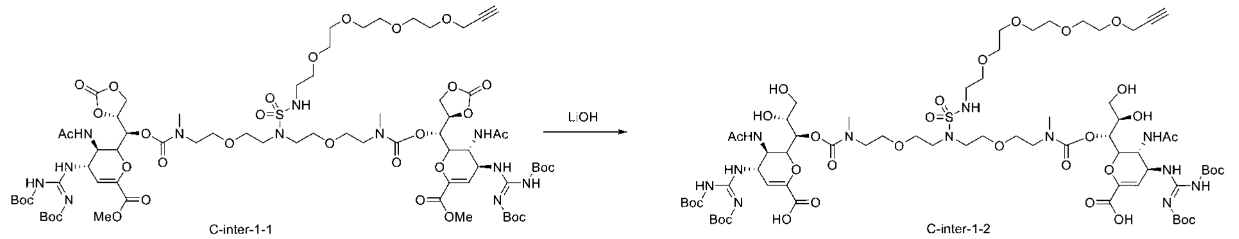


C-Inter-1-1 (240 mg, 0.146 mmol) was dissolved in methanol (5 ml), to which was added lithium hydroxide aqueous solution (0.55 mmol, 1 ml). The reaction was stirred at room temperature for 1 h, followed by addition of acetic acid (0.3 mL). After low-temperature concentration, purification was performed by reversed-phase column chromatography (acetonitrile/water) to obtain C-Inter-1-2 (213 mg, 0.131 mmol) as a white solid. MS $[M/2+1]^+$: 815.4

Step 3: Synthesis of C-Inter-1-3

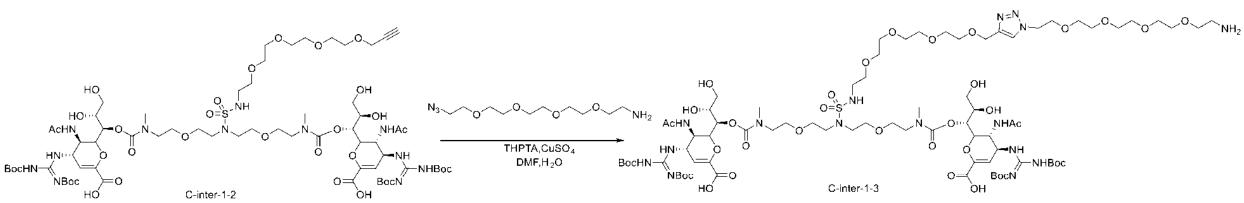


At 0 °C, to a solution of C-Inter-1-2 (213 mg, 0.131 mmol) and azide-tetraethylene glycol-amine (137 mg, 0.523 mmol) in MeCN (2 ml), was added a solution of THPTA (45.5 mg, 0.105 mmol), sodium ascorbate (51.8 mg, 0.262 mmol), and $CuSO_4$ (51.8 mg, 0.262 mmol) in clarified water (2 ml). Under nitrogen protection, the reaction solution was stirred at room temperature for 1 h. After completion of the reaction, C-Inter-1-3 (187 mg) was obtained by purification using preparative high-pressure liquid chromatography. MS $[M/2+1]^+$: 946.4

Step 4: Synthesis of C-Inter-1-4

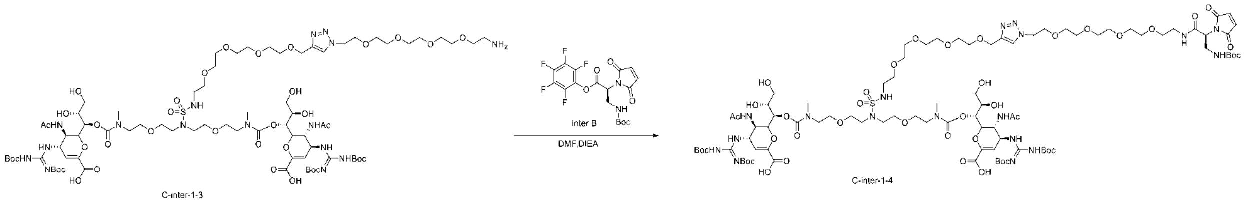


At 0 °C, to a solution of C-Inter-1-3 (270 mg, 0.143 mmol) in DMF (3 ml), were added Inter B (77.3 mg, 0.171 mmol) and DIEA (57.56 mg, 0.445 mmol), and then the reaction solution was stirred at room temperature for 1 h. After completion of the reaction, 1.5 ml of trifluoroacetic acid was added to the reaction solution, and then lyophilized, to obtain crude C-Inter-1-4. MS $[M/2+1]^{+}$: 1079.5

Step 5: Synthesis of C-Inter-1

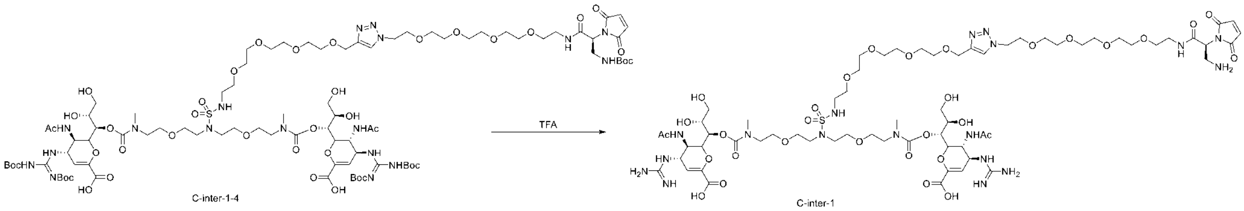


The crude product of C-Inter-1-4 was dissolved in a 10% aqueous trifluoroacetic acid solution (30 ml), stirred at room temperature for 15 min, and then rotatory evaporated to dryness. Purification was performed by preparative high-pressure liquid chromatography to obtain C-Inter-1 (85 mg). MS $[M/2+1]^{+}$: 829.4

**Example 13:** Synthesis of C-Inter-3

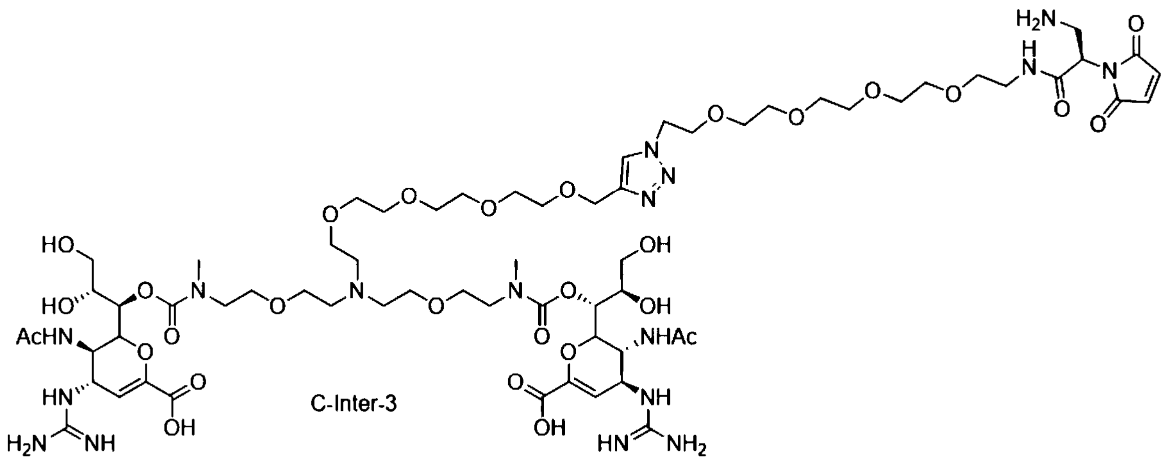


C-Inter-3

Step 1: Synthesis of C-Inter-3-1

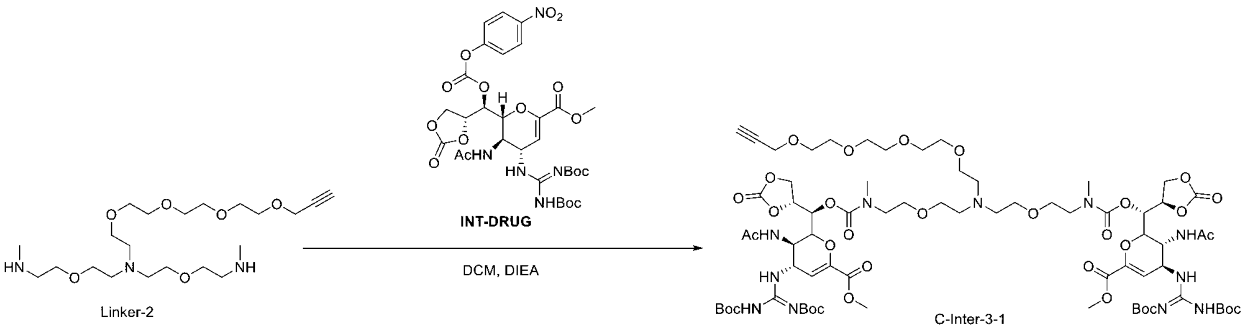


Linker 2 (219 mg, 0.5 mmol) and DIEA (396 mg, 3 mmol) were dissolved in dichloromethane (10 mL). Then, INT-DRUG (885 mg, 1.2 mmol) was dissolved in dichloromethane (5 mL), and added dropwise to the reaction solution. The reaction solution was stirred at room temperature for 16 h. After completion of the reaction, the reaction solution was concentrated, and purified by reversed-phase column, to obtain C-Inter-3-1 (730 mg) as a white solid. MS $[M/2+1]^+$: 815.8

Step 2: Synthesis of C-Inter-3-2

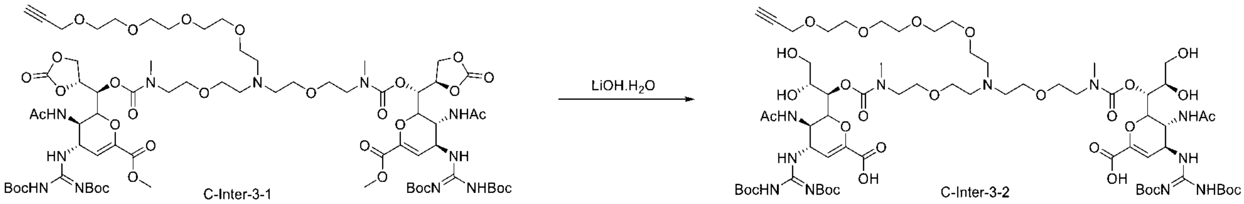


C-Inter-3-1 (730 mg, 0.45 mmol) was dissolved in methanol (10 mL). Then, lithium hydroxide monohydrate (112.7 mg, 2.69 mmol) was dissolved in water (3 mL) and added dropwise into the reaction solution. The reaction solution was stirred at room temperature for 4 h. After completion of the reaction, acetic acid (1 mL) was added. The reaction solution was concentrated, and purified by reversed-phase column, to obtain C-Inter-3-2 (290 mg) as a white solid. MS $[M/2+1]^+$: 775.8

Step 3: C-Inter-3-3

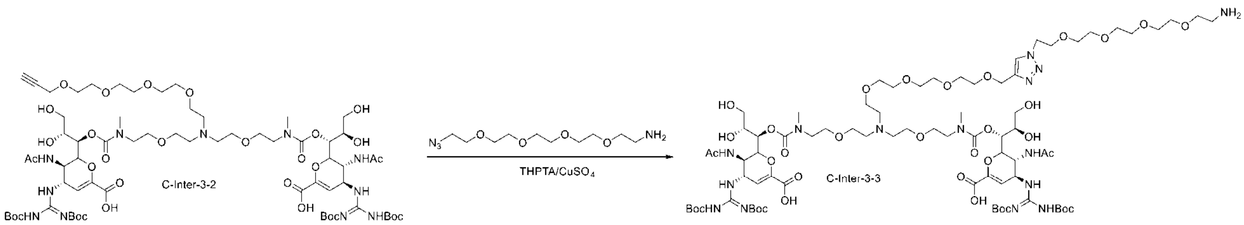


C-Inter-3-2 (290 mg) and azide-tetraethylene glycol-amine (196 mg, 0.75 mmol) were dissolved in acetonitrile. Then, THPTA (65.1 mg, 0.15 mmol), sodium ascorbate (74 mg, 0.374 mmol), and copper sulfate (14.9 mg, 0.093 mmol) were dissolved in water (5 mL), and added dropwise into the reaction solution. After nitrogen purging, the reaction was stirred at room temperature for 2 h. The reaction mixture was purified by reversed-phase column, to obtain C-Inter-3-3 (123 mg) as a white solid. MS $[M/2+1]^+$: 907.0

Step 4: Synthesis of C-Inter-3-4

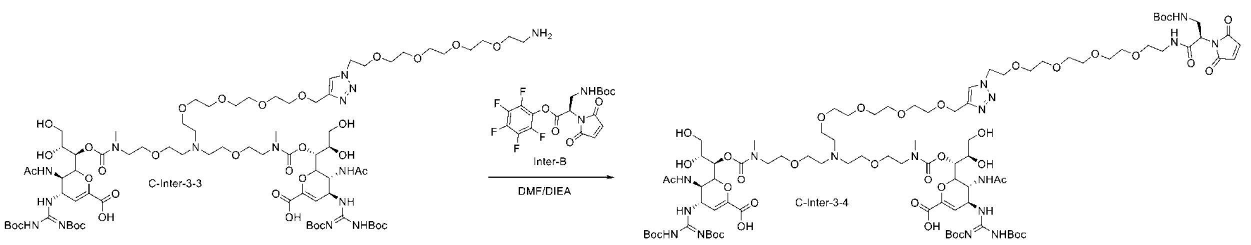


C-Inter-3-3 (123 mg) and intermediate B (36.6 mg, 0.081 mmol) were dissolved in DMF (7 mL), followed by addition of DIEA (27.36 mg). The reaction was stirred at room temperature for 2 h. After completion of the reaction, the reaction mixture was lyophilized to obtain the crude product of C-Inter-3-4. MS $[M+1]^+$: 1040.0

Step 5: Synthesis of C-Inter-3

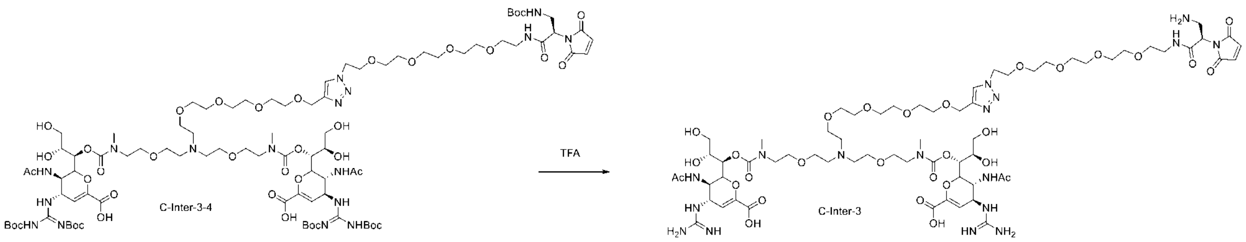


C-Inter-3-4 (crude product), TFA (10 mL), and water (0.5 mL) were added to a reaction flask, and then stirred at room temperature for 30 min. The reaction solution was concentrated at low temperature to remove the solvent. The crude product was purified by preparative high-pressure liquid chromatography to obtain C-Inter-3 (65 mg). MS $[M/2+1]^+$: 789.8

**Example 14:** Synthesis of C-Inter-6

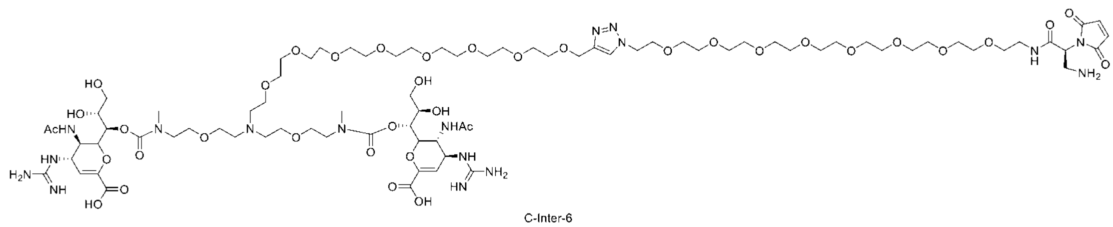

C-Inter-6

Step 1: Synthesis of C-Inter-6-1

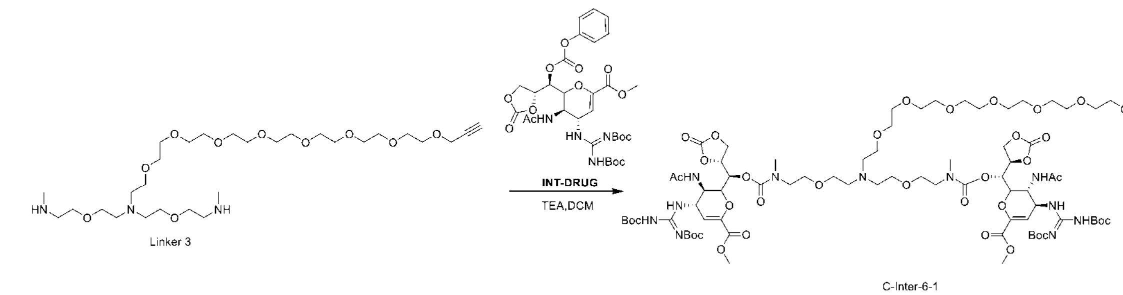


C-Inter-6-1

Linker 3 (182 mg, 0.3 mmol) was dissolved in DCM (5 ml), to which was added TEA (60.6 mg, 0.6 mmol). INT-DRUG (220 mg, 0.3 mmol) was dissolved in DCM (3 ml), and then added dropwise into the mixed solution. The resultant

solution was stirred at room temperature for 16 h, and then concentrated under reduced pressure to obtain the crude product, which was purified by reversed-phase column chromatography (acetonitrile/water) and lyophilized to obtain C-Inter-6-1 (397 mg) as white solid. MS [M/2+1]$^+$: 903.9

Step 2: Synthesis of C-Inter-6-2

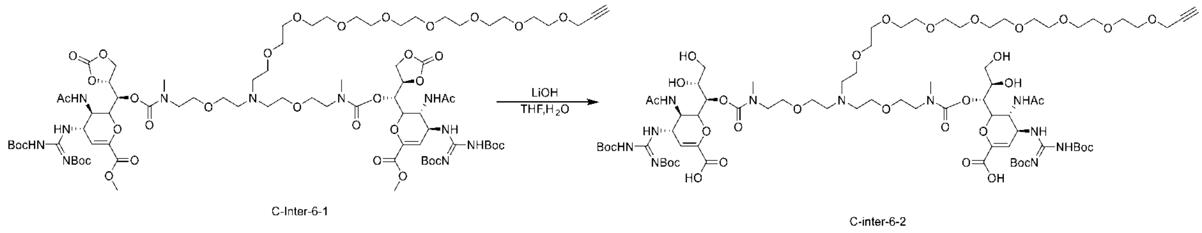


C-Inter-6-1

C-inter-6-2

C-Inter-6-1 (440 mg, 0.24 mmol) was dissolved in methanol (6 ml), to which was added aqueous lithium hydroxide solution (0.48 mmol, 1 ml). The reaction was stirred at room temperature for 1 hour, followed by adding acetic acid (0.2 mL). After low-temperature concentration, the reaction mixture was purified by reversed-phase column chromatography (acetonitrile/water) to obtain C-Inter-6-2 (310 mg) as a white solid. MS [M/2+1]$^+$: 863.9

Step 3: Synthesis of C-Inter-6-3

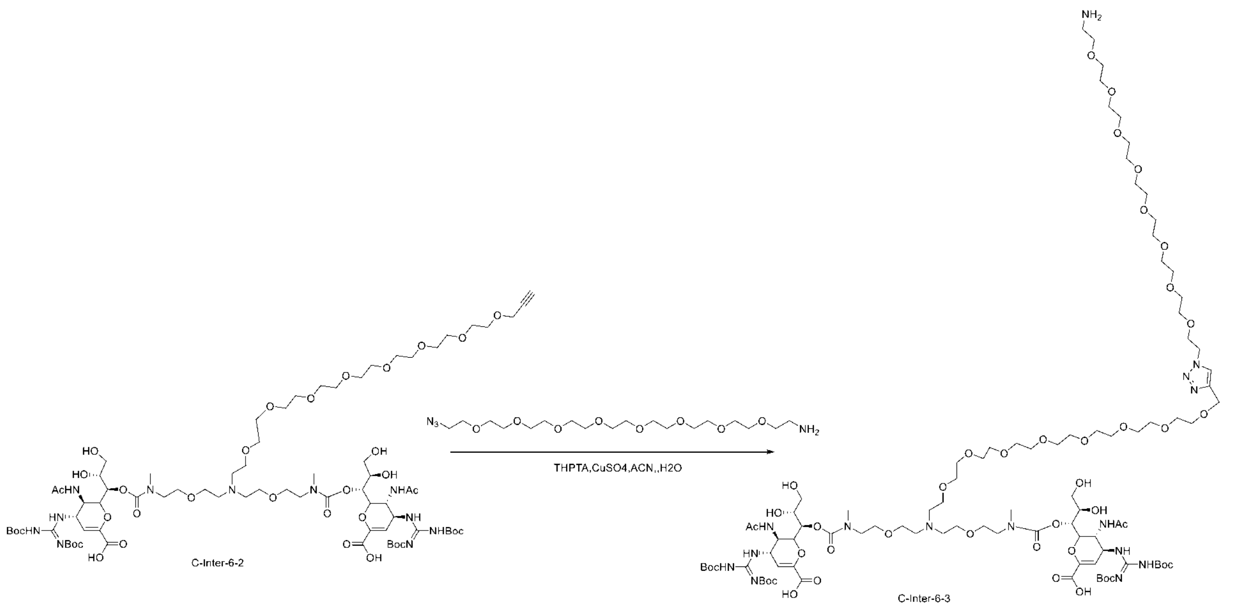


C-Inter-6-2

C-Inter-6-3

At 0 °C, to a solution of C-Inter-6-2 (310 mg, 0.18 mmol) and azide-octaethylene glycol-amine (188 mg, 0.72 mmol) in MeCN (2 ml), was added a solution of THPTA (62.6 mg, 0.14 mmol), sodium ascorbate (71.28 mg, 0.36 mmol), and $CuSO_4$ (57.2 mg, 0.36 mmol) in clarified water (2 ml). Under nitrogen protection, the reaction solution was stirred at room temperature for 1 h. After completion of the reaction, C-Inter-6-3 (176 mg) was obtained by purification using preparative high-pressure liquid chromatography. MS [M/2+1]$^+$: 1083.0

Step 4: Synthesis of C-Inter-6-4

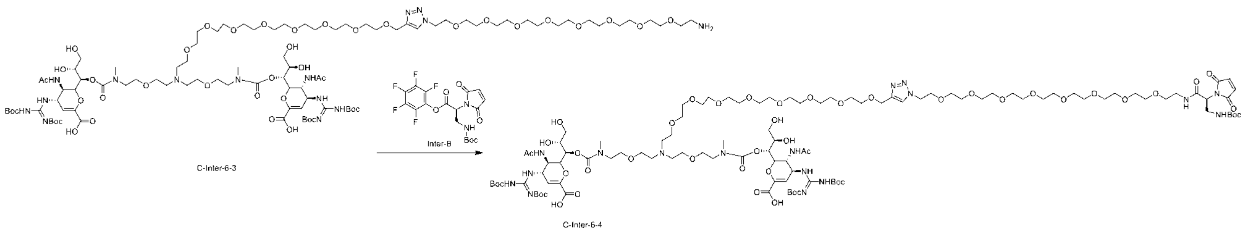


At 0 °C, to a solution of C-Inter-6-3 (176 mg, 0.08 mmol) in DMF (3 ml), were added Inter B (43.04 mg, 0.09 mmol) and DIEA (30.96 mg, 0.24 mmol), and then the reaction solution was stirred at room temperature for 1 h. After completion of the reaction, 1.5 ml of trifluoroacetic acid was added to the reaction solution, and then lyophilized, to obtain crude C-Inter-6-4. MS $[M/2+1]^+$: 1216.1

Step 5: Synthesis of C-Inter-6

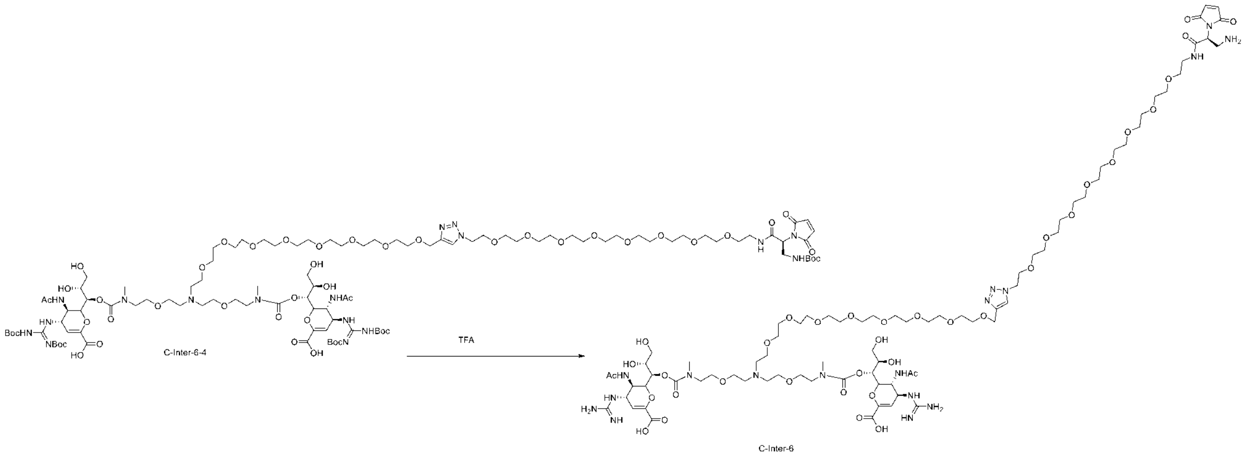


C-Inter-6-4 was dissolved in a 5% aqueous trifluoroacetic acid solution (10 ml), stirred at room temperature for 15 min, and then rotatory evaporated to dryness. Purification was performed by preparative high-pressure liquid chromatography to obtain C-Inter-6 (25 mg). MS $[M/2+1]^+$: 967.4

**Example 15:** Synthesis of C-Inter-9

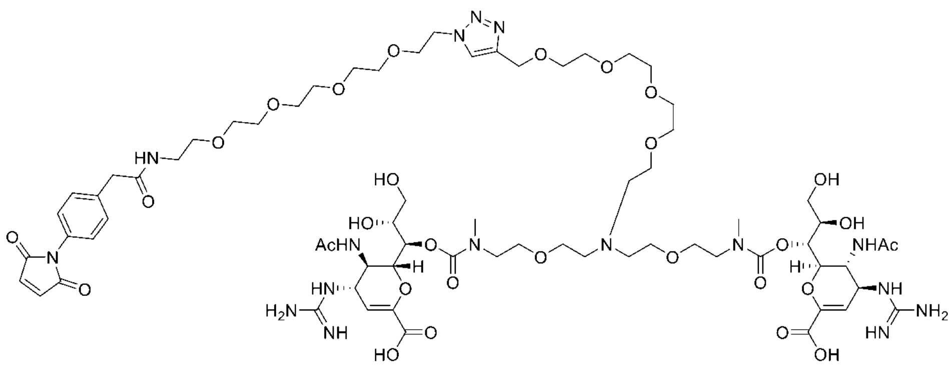

C-Inter-9

Step 1: Synthesis of C-Inter-9-1

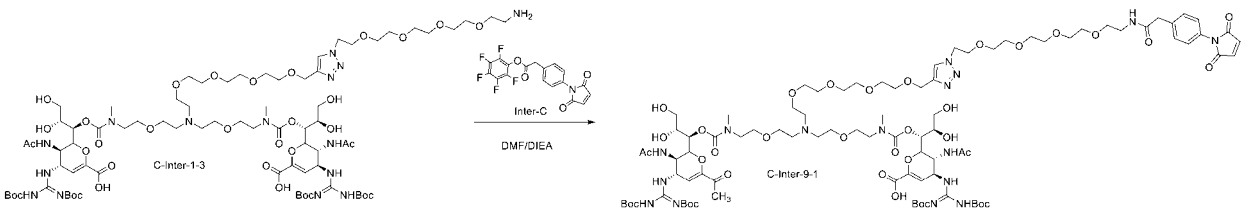


C-Inter-3-3 (155 mg), Inter-C (40.7 mg), and DIEA (34.89 mg) were dissolved in DMF, and then stirred at room temperature for 2 h. After completion of the reaction, the solution was lyophilized to obtain crude C-Inter-9-1. MS $[M/2+1]^+$: 1013.5

Step 2: Synthesis of C-Inter-9

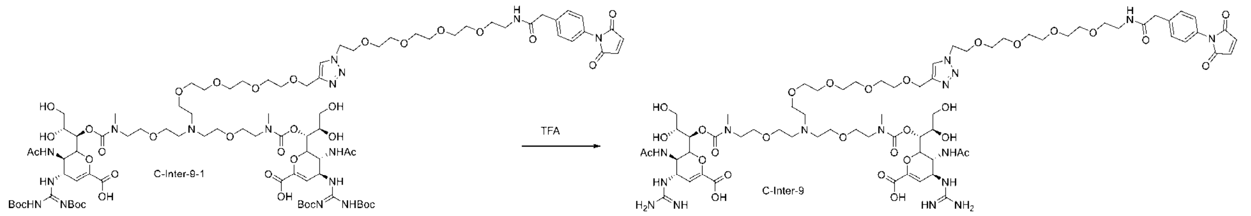


The crude product of C-Inter-9-1, TFA (10 mL), and water (0.5 mL) were added to a reaction flask, and then stirred at room temperature for 30 min. After completion of the reaction, the reaction solution was concentrated at low temperature to remove the solvent, and purification was performed by preparative high-pressure liquid chromatography to obtain C-Inter-9 (26 mg). MS $[M/2+1]^+$: 813.5

**Example 16: Expression and purification of hIgG1-Fc**

**1. Experimental materials**

| Name | Manufacturer | Cat. No. |
|---|---|---|
| ExpiCHO-S cells | Thermo | A29127 |
| ExpiFectamine™ CHO transfection kit | Thermo | A29130 |
| OptiPRO™ SFM reduced serum medium | Thermo | 12309019 |

**2. Expression of hIgG1-Fe**

1) One day before transfection (Day -1), the cells were diluted to a final density of 3-4 $\times$ $10^6$ viable cells/mL and allowed to grow overnight.

2) On the day of transfection, the cell density should be 5-6 $\times$ $10^6$ cells/mL. The cell survival rate must be > 95%.

3) According to the protocol, 0.8 $\mu$g of pcDNA3.1 plasmid expressing hIgG1-Fc was transfected per millilitre of cells. 0.8 $\mu$g of the plasmid was diluted in 40 $\mu$L of OptiPRO™ SFM medium (at 4°C) and mixed gently.

4) 3.2 μL of ExpiFectamine™CHO was diluted with 37 μL of OptiPRO™ SFM medium (4 °C), and mixed gently.

5) The diluted ExpiFectamine™ CHO reagent was added to the diluted DNA, gently mixed, and incubated at room temperature for 1-5 min.

6) The above complex was slowly added to each shake flask.

7) The cells were placed in a shaker at 37 °C under 8% $CO_2$, and the rotational speed was set to 125 rpm.

8) After incubating the cells for 20 h, 10 μL of ExpiCHO transfection enhancer and 400 μL of ExpiCHO feed were added to each milliliter of cells, and mixed homogeneously, and then added to each culture flask.

9) The cell supernatant was harvested after cultivating for eight days.

**3. Purification of hIgG1-Fe**

**1) Collection of supernatant**

The Fc protein fermentation broth that had been transfected and reached the harvesting condition was transferred into 50 ml centrifuge tubes that had been labeled with the order and name, and then centrifuged at 10000 rpm/min for 5 min. After centrifugation, the supernatant was poured into new 50 ml centrifuge tubes that had been labeled with the name, until all the supernatant from the same Fc fermentation broth had been centrifuged and collected.

**2) Preprocessing**

The gravity column was soaked in 0.5 M NaOH solution. The Pro A packing solution was added to the empty gravity column. Once the solution in the gravity column had dripped out, 0.1M NaOH solution was added to the column. After the solution in the gravity column had dripped out, pure water was added to the column.

**3) Equilibration and sample loading**

The Pro A column was equilibrated by adding 5-10 column volumes of Pro A equilibration buffer to the column. The supernatant treated by centrifugation and to be purified was loaded to the Pro A column. The supernatant that flew through the column was collected into a 250 ml clean shake flask. After all the supernatant had passed through the column, 5-10 column volumes of Pro-A equilibration buffer was added until all the equilibration buffer flew out from the bottom.

**4) Protein elution**

The bottom of the column that had undergone washing was sealed with a clean plug. 2-3 column volumes of Pro-A/G elution buffer was added to the column that was washed and sealed the bottom. A pipette was used to fully suspend the packing material in the column filled with elution buffer and let it stand for 5 min. As for making a ready for elution, a new collection tube was taken out, and a certain proportion of Tris neutralizing solution was added to prevent protein denaturation. The plug was removed, and the liquid was collected into the collection tube. The protein concentration was measured, and then the liquid was stored at -80 °C in the refrigerator.

**4. Characterization of hIgG1 Fc**

Based on the Fc sequence information described herein, the target Fc was obtained following the expression and purification steps provided in this example.

| SEQ ID NO | Expression volume (L) | Final yield (mg) | Final concent ration (mg/L) | Final volume (mL) | Medium (PBS) | Purity (SEC_ HPLC) (%) | Average molecular weight (Da) | Isoelectric point |
|---|---|---|---|---|---|---|---|---|
| 64 | 5 | 3919.5 | 26.13 | 150.0 | PH7.4 | 99.66 | 55266.6 | 8.93 |
| 67 | 5 | 2810.8 | 23.62 | 119.0 | PH7.4 | 99.68 | 55176.4 | 8.93 |

**Example 17: Expression and purification of hIgG1-Fc**

hIgG1-Fc (SEQ ID NO:69) was prepared and expressed as described in Example 16.

**2. Purification of hIgG1-Fc**

Experimental method: Purification of Protein A using affinity chromatography column

1) Equilibrium of chromatography column: 1xPBS, flow rate 1 ml/min, 20 ml
2) Sample loading: Flow rate 1 ml/min
3) Column washing: 1xPBS, flow rate 1 ml/min, 20 ml
4) Elution: Sodium acetate buffer (pH 3.2), 1 ml/min, collecting in separate tubes, approximately 500 μl per tube. A total of 10 tubes were collected, and the absorbance value at 280 nm was measured using a NanoDrop instrument.
5) Dialysis: The high-concentration protein was transferred into a dialysis bag and placed in a beaker filled with 1X PBS for dialysis.

**3. Characterization of hIgG1 Fc**

Based on the Fc sequence information described herein, the target Fc was obtained following the expression and purification steps mentioned in this example.

| SEQ ID NO | Expression volume (L) | Final yield (mg) | Final concent ration (mg/mL) | Final volume (mL) | Medium (PBS) | Purity (SEC_ HPLC) (%) | Average molecular weight (KDa) | Isoelectric point |
|---|---|---|---|---|---|---|---|---|
| 69 | 0.2 | 40 mg | 12.03 | 3.32 mL | PH7.4 | 99.38 | 56 | 7.72 |

**Example 18: Synthesis and characterization of conjugate 1**

**1. Experimental materials and methods**

1.1 Instrument/equipment

| No. | Name of instrument/equipment | Equipment model | Equipment number |
|---|---|---|---|
| 1 | pH meter | S470K | RD-080 |
| 2 | Centrifuge | Sorvall SI 16R | BP-0317 |
| 3 | Oscillating metal bath | SD2-100 | BP-0379 |
| 4 | Nanodrop lite | NanoDrop One | BP-0318 |
| 5 | Clean bench | SW-CJ-1D | QC-085 |
| | Super Clean bench | NA | QC-438 |
| | Thermostatic test tube device | NA | QC-324 |

1.2 Reagents/solutions

| No. | Reagent/solution name | Reagent/solution Lot. No. /Cat. No. | Remarks |
|---|---|---|---|
| 1 | 50 mM PB, pH 6.5 buffer | SO-014-087 | NA |

(continued)

| No. | Reagent/solution name | Reagent/solution Lot. No. /Cat. No. | Remarks |
|---|---|---|---|
| 2 | 10 mM His-HCl, pH 5.5 buffer | SO-014-088 | NA |
| 3 | 10 mM TCEP solution | SO-014-089 | NA |
| 4 | 10 mg/mL C-Inter-1 aqueous | SO-014-090 | NA |
| | solution | | |
| 5 | Endotoxin standard | 150601-202292 | Manufacturer: National Institute for Food and Drug Control, 90 EU/piece |
| 6 | Limulus reagent | 21061034 | Manufacturer: Xiamen Bioendo Technology Co., Ltd., 0.06 EU/mL |
| 7 | Water for endotoxin testing | 2008130 | Manufacturer: Zhanjiang Andus Biological Co., Ltd, < 0.003 EU/mL |
| 8 | Tris buffer | 2109240 | Manufacturer: Zhanjiang Andus Biological Co., Ltd, < 0.03 EU/mL |

1.3 Experimental methods

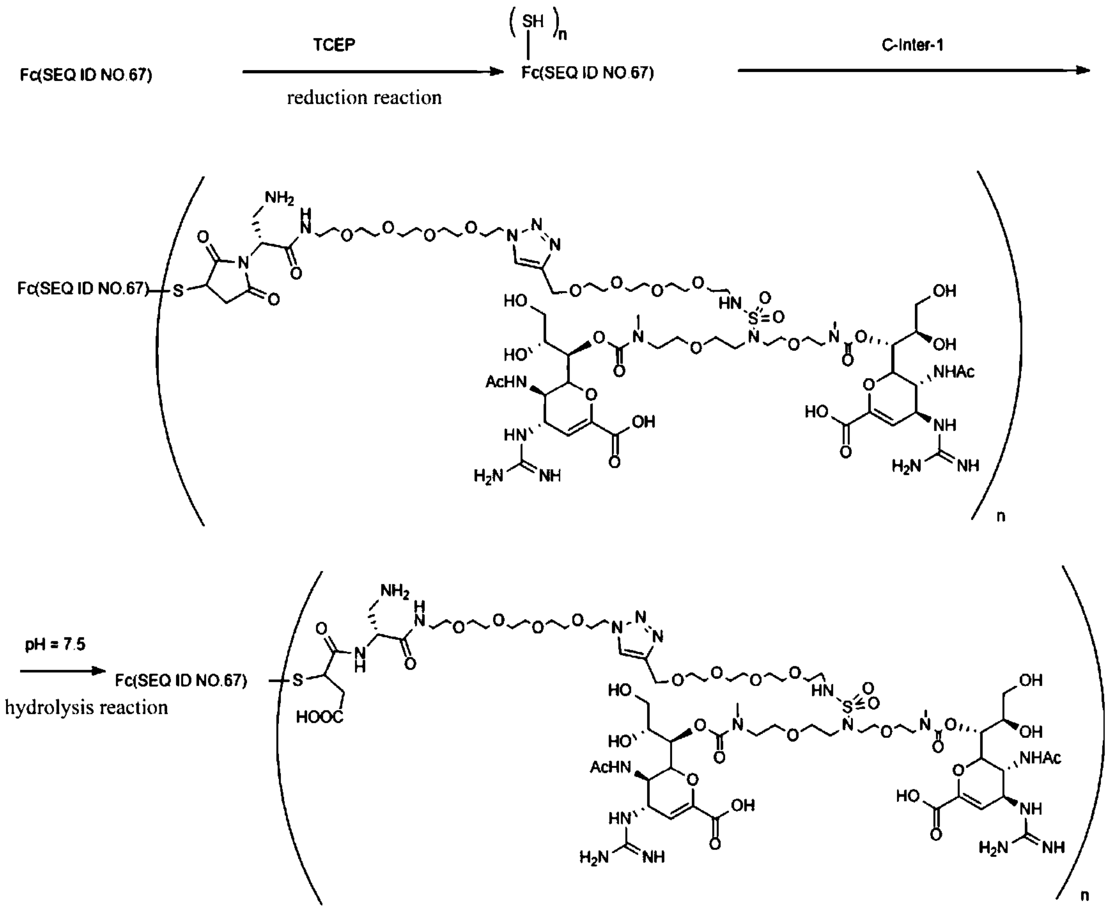


Conjugate 1

1.3.1 Ultrafiltration-1

1) Pre-treatment of ultrafiltration centrifuge tubes

To two 15 mL 10KDa ultrafiltration centrifuge tubes, was added 0.1 M NaOH solution, and then the tubes were centrifuged at 4000 G for 10 min, repeating three times.

Ultrapure water was added to the ultrafiltration centrifuge tube, and then centrifuged at 4000 G for 10 min, repeating twice.

50 mM PB, pH 6.5 buffer solution was added to the ultrafiltration centrifuge tube, and then centrifuged at 4000 G for 10 min. This was repeated until the pH of the permeate was around 6.5.

2) Antibody ultrafiltration and fluid exchange

To the processed ultrafiltration centrifuge tube, was added Fc (SEQ ID NO:67) antibody. The solution volume in the ultrafiltration tubes was filled to 12 mL using 50 mM PB, pH 6.5 buffer, and then the tube was centrifuged at 4000 G for 10 min, repeating 4 times. After fluid exchange, the samples were collected to detect the antibody concentration.

3) Antibody concentration detection

The detection was performed using the Protein A280 method in the NanoDrop One, with the sample category selected as "Other protein E 1%". The extinction coefficient E 1%L/gm-cm was set to 13.5, and the baseline correction wavelength was 340 nm.

Using 50 mM PB, pH 6.5 buffer as the blank, 2.5 μL of the sample was taken for detection, and the average of three detection results was taken as the sample concentration.

1.3.2 Reduction reaction

50 mM PB, pH 6.5 buffer, Fc(SEQ ID NO:67) antibody solution (30 mg), and 10 mM TCEP solution (TCEP/mAb molar equivalent ratio being 10) were successively added to the centrifuge tube, in which the antibody reaction concentration was controlled at 5 mg/mL. The reaction was placed in a metal bath to control the reaction temperature at 22 °C, and allowed to react for 2 h.

1.3.3 Coupling reaction

After the reduction reaction had proceeded for 2 h, 50 mM PBS, pH 6.5 buffer solution, and 10 mg/mL **C-Inter-1** solution (with a C-Inter/Fc molar equivalent ratio of 7) were added to the reaction solution. The final concentration of the reaction was controlled to be 2 mg/mL, and then the reaction was placed in a metal bath to control the reaction temperature at 22 °C for 1 h.

After reaching the reaction time, 10 mg/mL C-Inter-1 solution at a C-Inter-1/Fc molar equivalent ratio of 3.5 was further added to the reaction solution. The reaction was placed in a metal bath to control the reaction temperature at 22 °C for additional 0.5 h.

After reaching the reaction time, an additional 10 mg/mL **C-Inter-1** solution at a C-Inter-1/mAb molar equivalent ratio of 3.5 was added to the reaction solution, and then the reaction was placed in a metal bath to control the reaction temperature at 22 °C for additional 1.0 h.

1.3.4 Hydrolysis reaction

0.5 M NaOH solution was added to the reaction solution, to adjust the pH to around 7.5. Then, the reaction was placed in a metal bath to control the reaction temperature at 22 °C for additional 2 h, to obtain **Conjugate 1.**

1.3.5 Ultrafiltration-2

1) Pre-treatment of ultrafiltration centrifuge tubes

To two 15 mL 10KDa ultrafiltration centrifuge tubes, was added 0.1 M NaOH solution, and then the tubes were centrifuged at 4000 G for 10 min, repeating three times.

Ultrapure water was added to the ultrafiltration centrifuge tube, and then centrifuged at 4000 G for 10 min, repeating twice.

10 mM His-HCl, pH 5.5 buffer solution was added to the ultrafiltration centrifuge tube, and then centrifuged at 4000 G for 10 min. This was repeated until the pH of the permeate was about 5.6.

2) Antibody ultrafiltration and fluid exchange

To the processed ultrafiltration centrifuge tube, was added **Conjugate 1.** The solution volume in the ultrafiltration tubes was filled to 12 mL using 10 mM His-HCl, pH 5.5 buffer, and then the tube was centrifuged at 4000 G for 10 min, repeating 6 times. The solution after ultrafiltration was filtered with 0.22 μm PES filter membrane. Then, the antibody concentration was detected after fluid exchange.

3) Antibody concentration detection

The detection was performed using the Protein A280 method in the NanoDrop One, with the sample category selected as "Other protein E 1%". The extinction coefficient E 1%L/gm-cm was set to 13.5, and the baseline correction wavelength was 340 nm.

Using 10 mM His-HCl, pH 5.5 buffer as the blank, 2.5 μL of the sample was taken for detection, and the average of three detection results was taken as the sample concentration.

Based on the detected sample concentration and sample volume, the sample was diluted to a concentration of approximately 10 mg/mL using 10 mM His-HCl, pH 5.5 buffer solution, and then the concentration of the diluted sample was re-detected.

**2. Experimental results and analysis**

2.1 Detection of LC-MS DAR value

The DAR value of the sample was detected by LC-MS, and the relevant method conditions are shown in the table below:

LC method conditions

| Name | Parameter settings |
|---|---|
| Instrument | SCIEX ExionLC |
| Chromatographic column | ACQUITY UPLC BEH 200 Å, SEC 1.7 μm, 4.6×300 mm |
| Mobile phase | A: 0.1% FA, 0.05%TFA, 25% ACN in $H_2O$ |
| Injection volume | 10 μL |
| Column temperature | 25 °C |
| Flow rate | 200 μL/min |
| Gradient | Isocratic |

MS method conditions

| Name | Parameter settings |
|---|---|
| Instrument | SCIEX 5600 |
| Scanning range | 900-5000 |
| Polarity | Positive |
| Atomized gas (GS1) | 50 |
| Auxiliary gas (GS2) | 50 |
| Curtain gas (CUR) | 25 |
| Ion transport tube temperature (TEM) | 450 |

(continued)

| Name | Parameter settings |
|---|---|
| Spray voltage (ISVF) | 5500 |
| Declustering voltage (DP) | 250 |
| Collision energy (CE) | 10 |
| Accumulated time (AT) | 1 s |

The detection results for the MS-DAR value of the sample are shown in the table below. The MS detection results indicated that the MS-DAR value of the Conjugate 1 sample was 4.0. After sugar cleavage and reduction treatment, the sample peak appeared as a single chain peak. C0, C1, C2, and C3 represented the molecular weights of the conjugates with 0, 1, 2, and 3 small molecules attached, respectively.

| Sample name | Payload distribution | | | | MS-DAR |
|---|---|---|---|---|---|
| | C0 | C1 | C2 | C3 | |
| Conjugate 1 | 0.28% | 0.62% | 99.10% | 0.00% | 4.0 |

The MS detection spectrum of the sample is shown in Figure 1:

2.2 SEC-HPLC purity testing

The purity of the sample was tested by SEC-HPLC, and the relevant method conditions are shown in the table below:

<table>
<tr><th>Name</th><th>Parameter settings</th></tr>
<tr><td>Instrument</td><td>Thermo scuentific Vanquish</td></tr>
<tr><td>Chromatographic column</td><td>TSKgel guardcolumn SWxl 6.0 mm x 4 cm<br>TSKgel G3000SWxl 7.8 mm x 30 cm</td></tr>
<tr><td>Column temperature</td><td>25 °C</td></tr>
<tr><td>Mobile phase</td><td>0.2M $KH_2PO_4$,0.25M KCl, 15%ACN</td></tr>
<tr><td>Flow rate</td><td>0.5 mL/min</td></tr>
<tr><td>Injection volume</td><td>25 µg</td></tr>
<tr><td>Detection wavelength</td><td>280 nm</td></tr>
<tr><td>Gradient</td><td><table>
<tr><th>Flow (mL/min)</th><th>Time</th><th>A (%)</th><th>B (%)</th></tr>
<tr><td>0.500</td><td>0.00</td><td>100</td><td>0</td></tr>
<tr><td>0.500</td><td>30.00</td><td>100</td><td>0</td></tr>
</table></td></tr>
</table>

The small molecule residual detection results of the sample are shown in Figure 2. The SEC detection results indicated that the purity of conjugate 1 was 98.21%.

2.3 Small molecule residual detection by RP-HPLC

The sample was tested for residual free drug levels (mol/mol, free drug/bound drug) by RP-HPLC. Initially, two standards containing free drug and Fc (2% & 5%) were prepared. The percentage of free drug was determined by comparing the EIC signal peak area with that of the residual free drug in the conjugate sample. The relevant method conditions are outlined in the table below:

<table>
<tr><th>Name</th><th>Parameter settings</th></tr>
<tr><td>Instrument</td><td>Agilent 1260 Infinity ll</td></tr>
<tr><td>Chromatographic column</td><td>Agilent PLRP-S 1000A, 8 μm, 250 x 4.6 mm</td></tr>
<tr><td>Column temperature</td><td>80 °C</td></tr>
<tr><td>Mobile phase A</td><td>0.1% TFA in $H_2O$</td></tr>
<tr><td>Mobile phase B</td><td>0.1% in acetonitrile</td></tr>
<tr><td>Flow rate</td><td>0.4 mL/min</td></tr>
<tr><td>Injection volume</td><td>10 μg</td></tr>
<tr><td>Detection wavelength</td><td>250 nm, 236 nm, 214 nm</td></tr>
<tr><td>Gradient</td><td><table>
<tr><th>Flow (mL/min)</th><th>Time</th><th>A (%)</th><th>B (%)</th></tr>
<tr><td>0.400</td><td>0.00</td><td>90</td><td>10</td></tr>
<tr><td>0.400</td><td>2.00</td><td>90</td><td>10</td></tr>
<tr><td>0.400</td><td>8.00</td><td>75</td><td>25</td></tr>
<tr><td>0.400</td><td>15.00</td><td>10</td><td>90</td></tr>
<tr><td>0.400</td><td>17.00</td><td>10</td><td>90</td></tr>
<tr><td>0.400</td><td>17.10</td><td>90</td><td>10</td></tr>
<tr><td>0.400</td><td>25.00</td><td>90</td><td>10</td></tr>
</table></td></tr>
</table>

Preparation of the standard solution:

$C_{Fc(SEQ ID NO:67)}$ =1 mg/mL, $C_{Fe(SEQ ID NO:69)}$ = 1 mg/mL; the DAR values of the samples were 4.0 and 6.0, respectively;

$$MW_{Fc(SEQ\ ID\ NO:67)} = 55414\ g/mol,\ MW_{Fc(SEQ\ ID\ NO:69)} = 56000\ g/mol,\ MW_{C\text{-}Inter\text{-}1} = 1656\ g/mol;$$

$$M_{mAb} = 1/\ MW_{mAb},\ M_{Total\ drug} = DAR/MW_{mAb};$$

The molarity of a 2% standard solution = DAR × $M_{mAb}$ × 2%;

The molarity of a 5% standard solution = DAR × $M_{mAb}$ × 5%;

The weight of the small molecule added to 100 μL of 2% standard solution (2%$m_{C\text{-}Inter\text{-}1}$) = The moles of 2% standard solution × 100 μL × $MW_{C\text{-}Inter\text{-}1}$;

The weight of the small molecule added to 100 μL of 5% standard solution (5%$m_{C\text{-}Inter\text{-}1}$) = The moles of 5% standard solution × 100 μL × $MW_{C\text{-}Inter\text{-}1}$;

The calculated weight of the small molecule was added to 100 μL of 1 mg/mL antibody solution to obtain the standard solution.

The small molecule residual detection results of the samples are shown in Figure 3, wherein AKSZ-2627-0.01mg/mL sample is the AKSZ2627 small molecule sample, AKSZ-2627-2% and AKSZ-2627-5% are samples obtained by adding 2% and 5% mol/mol small molecules to the corresponding Fc, respectively, and C060-7607B-230424 is the sample of Conjugate 1. The RP detection results indicated that no small molecule peaks were detected in both samples by RP-HPLC. Compared with the antibody samples with 2% mol/mol and 5% mol/mol small molecules added, the residuals in the samples are both less than 2% mol/mol.

2.4 Endotoxin residue detection

The endotoxin residue of the sample was detected using the following steps:

1) A vial of endotoxin working standard was added to 1 mL of BET water, vortexed for 15 min, and gradually diluted to 4λ($E_{4\lambda}$) and 2λ($E_{2\lambda}$) for future use.

2) Preparation of test solution: The sample solution was diluted stepwise with Tris buffer in a ratio of 1:80 ($S_{80}$) and 1:160 ($S_{160}$) for use.

3) Preparation of solutions for each group

Test group A: $S_{160}$ solution;

Positive control group B of the test sample: one volume of $S_{80}$ solution was added to one volume of $E_{4\lambda}$ solution, and mixed well, to prepare $S_{160}E_{2\lambda}$;

Positive control group C: $E_{2\lambda}$ solution;

Negative control group D: BET water.

4) Reconstitution of limulus reagent: 0.1 mL of BET water was added to each vial of the limulus reagent for reconstitution.

5) After the limulus reagent was reconstituted, 0.1 mL of each solution for four groups prepared in step 3) was added to the limulus reagent. Each group received two limulus reagents in parallel.

6) Incubation: The mixed ampoule was placed into a test-tube thermostat preheated to 37 °C, and incubated for 60 min.

Test results:

The concentration of Conjugate 1 was 10.13 mg/mL, and the endotoxin residue in the sample was less than 1 EU/mg.

**Example 19: Synthesis and characterization of Conjugate 2**

**1. Experimental materials and methods**

1.1 Instrument/equipment

Same as Example 18.

1.2 Reagents/Test Solutions

Same as Example 18.

1.3 Experimental methods

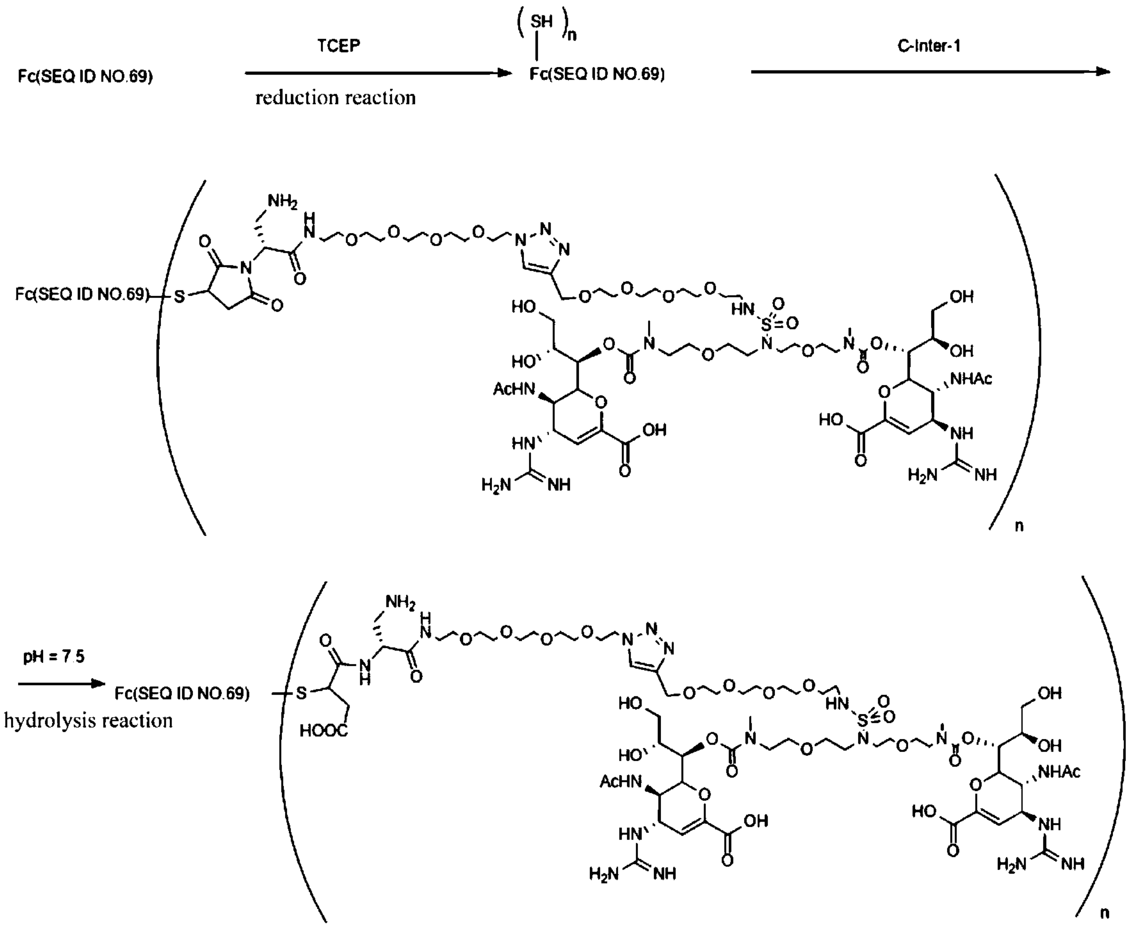


Conjugate 2

1.3.1 Ultrafiltration-1

1) Pre-treatment of ultrafiltration centrifuge tubes

To two 15 mL 10KDa ultrafiltration centrifuge tubes, was added 0.1 M NaOH solution, and then the tubes were centrifuged at 4000 G for 10 min, repeating three times.

Ultrapure water was added to the ultrafiltration centrifuge tube, and then centrifuged at 4000 G for 10 min, repeating twice.

50 mM PB, pH 6.5 buffer solution was added to the ultrafiltration centrifuge tube, and then centrifuged at 4000 G for 10 min. This was repeated until the pH of the permeate was around 6.5.

2) Antibody ultrafiltration and fluid exchange

To the processed ultrafiltration centrifuge tube, was added Fc (SEQ ID NO:67) antibody. The solution volume in the ultrafiltration tubes was filled to 12 mL using 50 mM PB, pH 6.5 buffer, and then the tube was centrifuged at 4000 G for 10 min, repeating 4 times. After fluid exchange, the samples were collected to detect the antibody concentration.

3) Antibody concentration detection

The detection was performed using the Protein A280 method in the NanoDrop One, with the sample category selected as "Other protein E 1%". The extinction coefficient E 1%L/gm-cm was set to 13.44, and the baseline correction wavelength was 340 nm.

Using 50 mM PB, pH 6.5 buffer as the blank, 2.5 μL of the sample was taken for detection, and the average of three detection results was taken as the sample concentration.

1.3.2 Reduction reaction

50 mM PBS, pH 6.5 buffer, Fc (SEQ ID NO:67) antibody solution (30 mg), and 10 mM TCEP solution (TCEP/Fc molar equivalent ratio being 10) were successively added to the centrifuge tube, in which the antibody reaction concentration was controlled at 5 mg/mL. The reaction was placed in a metal bath to control the reaction temperature at 22 °C, and allowed to react for 2 h.

1.3.3 Coupling reaction

After the reduction reaction had proceeded for 2 h, 50 mM PBS, pH 6.5 buffer solution, and 10 mg/mL C-Inter-1 solution (with a C-Inter-1/Fc molar equivalent ratio of 12) were added to the reaction solution. The final concentration of the reaction was controlled to be 2 mg/mL, and then the reaction was placed in a metal bath to control the reaction temperature at 22 °C, followed by reacting for 1 h.

After reaching the reaction time, 10 mg/mL C-Inter-1 solution at C-Inter-1/Fc molar equivalent ratio of 6.0 was further added to the reaction solution. The reaction was placed in a metal bath to control the reaction temperature at 22 °C, and allowed to further react for 0.5 h.

After reaching the reaction time, an additional 10 mg/mL C-Inter-1 solution at C-Inter-1/Fc molar equivalent ratio of 6.0 was added to the reaction solution, and then the reaction was placed in a metal bath to control the reaction temperature at 22 °C for additional 1.0 h.

1.3.4 Hydrolysis reaction

0.5 M NaOH solution was added to the reaction solution, to adjust the pH to around 7.5. Then, the reaction was placed in a metal bath to control the reaction temperature at 22 °C, followed by reacting for additional 2 h, to obtain **Conjugate 2.**

1.3.5 Ultrafiltration-2

1) Pre-treatment of ultrafiltration centrifuge tubes

To two 15 mL 10KDa ultrafiltration centrifuge tubes, was added 0.1 M NaOH solution, and then the tubes were centrifuged at 4000 G for 10 min, repeating three times.

Ultrapure water was added to the ultrafiltration centrifuge tube, and then centrifuged at 4000 G for 10 min, repeating twice.

10 mM His-HCl, pH 5.5 buffer solution was added to the ultrafiltration centrifuge tube, and then centrifuged at 4000 G for 10 min. This was repeated until the pH of the permeate was about 5.6.

2) Antibody ultrafiltration and fluid exchange

To the processed ultrafiltration centrifuge tube, was added **Conjugate 2.** The solution volume in the ultrafiltration tubes was filled to 12 mL with 10 mM His-HCl, pH 5.5 buffer, and then the tube was centrifuged at 4000 G for 10 min, repeating 6 times. The ultrafiltered solution was filtered with 0.22 μm PES filter membrane. Then, the antibody concentration was detected after fluid exchange.

3) Antibody concentration detection

The detection was performed using the Protein A280 method in the NanoDrop One, with the sample category selected as "Other protein E 1%". The extinction coefficient E 1%L/gm-cm was set to 13.44, and the baseline correction wavelength was 340 nm.

Using 10 mM His-HCl, pH 5.5 buffer as the blank, 2.5 μL of the sample was taken for detection, and the average of

three detection results was taken as the sample concentration.

Based on the detected sample concentration and sample volume, the sample was diluted to a concentration of approximately 10 mg/mL with 10 mM His-HCl, pH 5.5 buffer solution, and then the concentration of the diluted sample was re-detected.

2. **Experimental results and analysis**

2.1 Detection of LC-MS DAR value

The method was the same as in **Example 18,** and the MS-DAR value of the **Conjugate 2** sample was 6.0.

The MS detection spectrum of the sample is shown in Figure 4. After undergoing sugar removal and reduction treatment, the sample peak appeared as a single chain peak. C0, C1, C2, and C3 represent the molecular weights obtained by attaching 0, 1, 2, and 3 small molecules, respectively.

| **Sample name** | **Payload distribution** | | | | **MS-DAR** |
|---|---|---|---|---|---|
| | **C0** | **C1** | **C2** | **C3** | |
| Conjugate 2 | 0.00% | 0.53% | 0.31% | 99.16% | 6.0 |

2.2 SEC-HPLC purity detection

The method was the same as that in **Example 18.** The SEC detection results are shown in Figure 5, with the purity of Conjugate 2 being 94.47%.

2.3 RP-HPLC detection of small molecule residues

The method was the same as that in Example 18, with small molecule residues <2%, as shown in Figure 6.

2.4 Endotoxin residue detection

The method was the same as that in Example 18. The concentration of Conjugate 2 was 10.26 mg/mL, and the endotoxin residue of the sample was less than 1 EU/mg.

**Example 20: Preparation of Conjugate 3**

1. Experimental materials and methods

1) Experimental instrument

| Instrument name | Manufacturer | Model/Specification | Instrument number |
|---|---|---|---|
| Oscillating metal bath | Titan Scientific Co., Ltd. | SD2-100 | BP-0379 |
| pH meter | Mettler Toledo | S470K | RD-080 |
| Electronic balance | Mettler Toledo | XSR205DU/A | RD-079 |
| Electronic balance | Mettler Toledo | Me802E | BP-0308 |
| Vortex mixer | IKA | IKA MS3 Basic | BP-113 |
| Nanodrop lite | Thermo | NanoDrop One | BP-0318 |
| Medical lowtemperature refrigerator | Haier | DW-25L262 | BP-291 |
| Ultra-low temperature freezer | Thermo | 902-ULTS | BP-290 |

**2)** Reagent/solution

| Name | Lot. No. |
|---|---|
| 50 mM PB, pH 6.5 buffer | SO-007-096 |
| 10 mM His-HCl, pH 5.5 buffer | SO-038-032 |
| 10 mM TCEP solution | SO-038-037 |
| 10 mg/mL C-Inter-3 aqueous solution | S0-883-098 (N) |

2. Experimental methods

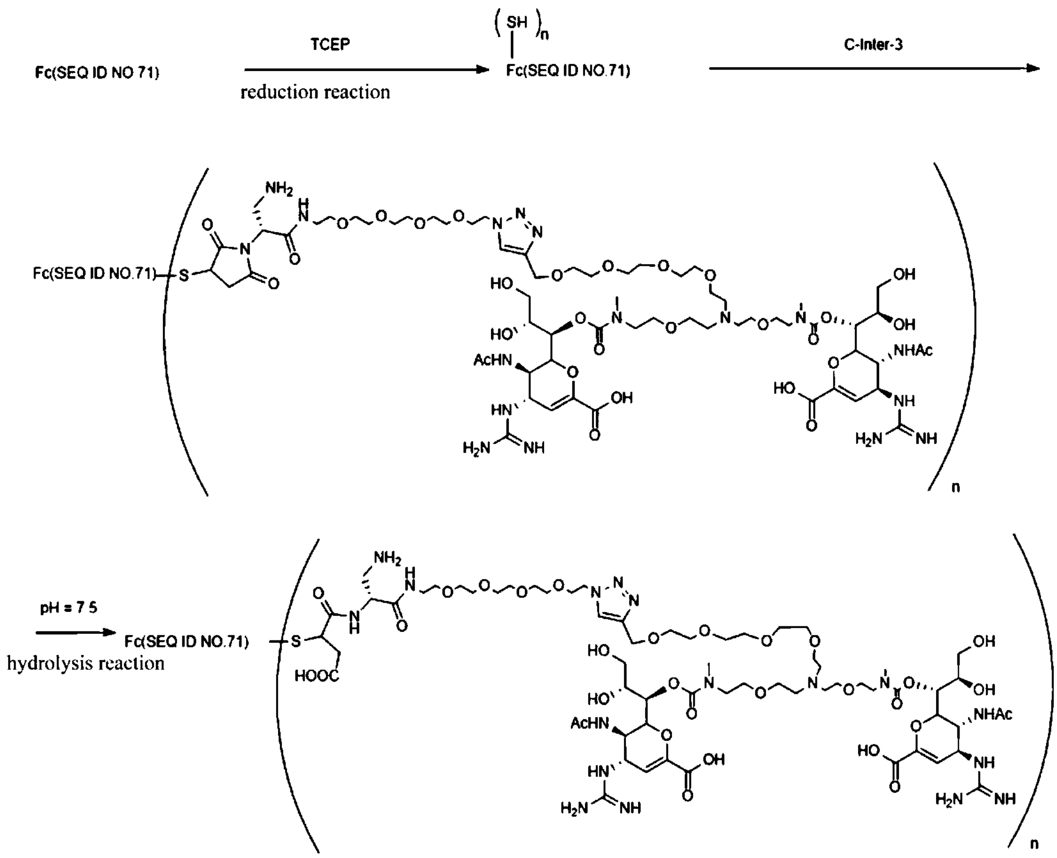


Conjugate 3

1) Ultrafiltration-1

(1) Antibody ultrafiltration and fluid exchange

To the ultrafiltration centrifuge tube, was added Fc (SEQ ID NO:71). The solution volume in the ultrafiltration tubes was filled to 12 mL using 50 mM PB, pH 6.5 buffer, and then the tube was centrifuged at 4500 G for 10 min, repeating 4 times. The samples were collected to detect the antibody concentration.

(2) Antibody concentration detection

The detection was performed using the Protein A280 method in the NanoDrop One, with the sample category

selected as "Other protein E 1%". The extinction coefficient E 1%L/gm-cm was set to 13.44 (7607H), and the baseline correction wavelength was 340 nm. Using 50 mM PB, pH 6.5 buffer as the blank, 2.5 μL of the sample was taken for detection, and the average of three detection results was taken as the sample concentration.

2) Reduction reaction

50 mM PB, pH 6.5 buffer, corresponding antibody solution (28 mg), and 10 mM TCEP solution (TCEP/mAb molar equivalent ratio being 20) were successively added to the centrifuge tube, in which the antibody reaction concentration was controlled at 5 mg/mL. The tube was placed in a metal bath to control the reaction temperature at 25 °C, and allowed to react for 2 h. The term "equivalent" used herein refers to molar equivalent, unless otherwise specified or contradicted by the context.

3) Coupling reaction

1. After the reduction reaction had proceeded for 2 h, 10 mg/mL C-Inter-3 solution (with a Drug/mAb equivalent ratio of 10) was added to the reaction solution. The reaction was placed in a metal bath to control the reaction temperature at 25 °C for 1 h. At one-hour intervals, 10 mg/mL C-Inter-3 aqueous solution (AKSZ2665 aqueous solution) was repeatedly added (with a Drug/mAb equivalent ratio of 10), until the total reaction equivalent reached 50 (adding 0.5 M NaOH to adjust the pH to around 6.5). After completion of the reaction, the pH of the reaction solution was adjusted to 7.0-7.5 with 0.5 M NaOH, and then the reaction was placed in a metal bath and allowed to further react at 25 °C for 2 h.

4) Ultrafiltration

(1) Antibody ultrafiltration and fluid exchange

3. To the processed ultrafiltration centrifuge tube, was added the **conjugate** sample. The solution volume in the ultrafiltration tubes was filled to 12 mL with 10 mM His-HCl, pH 5.5 buffer, and then the tube was centrifuged at 4000 G for 10 min, repeating 12 times. The ultrafiltered solution was filtered with 0.22 μm PES filter membrane. Then, the antibody concentration was detected after fluid exchange.

(2) Antibody concentration detection

The detection was performed using the Protein A280 method in the NanoDrop One, with the sample category selected as "Other protein E 1%". The extinction coefficient E 1%L/gm-cm was set to 13.44, and the baseline correction wavelength was 340 nm.

Using 10 mM His-HCl, pH 5.5 buffer as the blank, 2.5 μL of the sample was taken for detection, and the average of three detection results was taken as the sample concentration.

5) Experimental results and analysis

(1) Detection of LC-MS DAR value

The DAR value of the sample was detected by LC-MS, and the relevant method conditions are shown in the table below:

LC method conditions

| Name | Parameter settings |
|---|---|
| Instrument | SCIEX ExionLC |
| Chromatographic column | ACQUITY UPLC BEH 200Å, SEC 1.7 μm, 4.6×300 mm |
| Mobile phase | A: 0.1% FA, 0.05%TFA, 25% ACN in $H_2O$ |
| Injection volume | 10 μL |

(continued)

| Name | Parameter settings |
|---|---|
| Column temperature | 25 °C |
| Flow rate | 200 μL/min |
| Gradient | Isocratic |

MS method conditions

| Name | Parameter settings |
|---|---|
| Instrument | SCIEX 5600 |
| Scanning range | 900-5000 |
| Polarity | Positive |
| Atomized gas (GS1) | 50 |
| Auxiliary gas (GS2) | 50 |
| Curtain gas (CUR) | 25 |
| Ion transport tube temperature (TEM) | 450 |
| Spray voltage (ISVF) | 5500 |
| Declustering voltage (DP) | 250 |
| Collision energy (CE) | 10 |
| Accumulated time (AT) | 1 s |

The MS-DAR value detection results of the sample are shown in the table (figure) below. The MS detection results indicated that the MS-DAR value of the C060-7607H-2665-240125 (Conjugate 3) sample was 5.9.

| Sample name | Payload distribution | | | | MS-DAR |
|---|---|---|---|---|---|
| | C0 | C1 | C2 | C3 | |
| Conjugate 3 | 0.00% | 0.00% | 6.50% | 93.50% | 5.9 |

Note:

Equation for drug load distribution:

Drug load distribution (%) = (Area of single-chain peak with drug load n / Sum of areas of two single-chain peaks) × 100 (%);

wherein n represents the drug loading capacity, and a single chain consists of heavy chain constant domain 2, heavy chain constant domain 3, and ½ hinge region (CH2+CH3+½Hi);

DAR value calculation equation:

Single-chain weighted peak area = (Drug load n × Drug load distribution %)

$$\text{Drug-to-antibody ratio} = 2 \times \sum(\text{weighted peak area of single chain})$$

The MS detection spectrum of the sample is shown in Figure 8:

(2) SEC-HPLC purity testing

The purity of the sample was tested by SEC-HPLC, and the relevant method conditions are shown in the table below:

<table>
<tr><th>Name</th><th>Parameter settings</th></tr>
<tr><td>Instrument</td><td>Thermo scientific Vanquish</td></tr>
<tr><td>Chromatographic column</td><td>TSKgel guardcolumn SWxl 6.0 mm x 4 cm</td></tr>
<tr><td></td><td>TSKgel G3000SWxl 7.8 mm x 30 cm</td></tr>
<tr><td>Column temperature</td><td>25 °C</td></tr>
<tr><td>Mobile phase</td><td>0.2 M $KH_2PO_4$, 0.25 M KCl, 15% ACN</td></tr>
<tr><td>Flow rate</td><td>0.5 mL/min</td></tr>
<tr><td>Injection volume</td><td>25 μg</td></tr>
<tr><td>Detection wavelength</td><td>280 nm</td></tr>
<tr><td>Gradient</td><td>Isocratic<br><table>
<tr><td>Flow(mL/min)</td><td>Time</td><td>A(%)</td><td>B(%)</td></tr>
<tr><td>0.500</td><td>0.00</td><td>100</td><td>0</td></tr>
<tr><td>0.500</td><td>30.00</td><td>100</td><td>0</td></tr>
</table></td></tr>
</table>

The purity test results of the sample are shown in Figure 9. The SEC test results indicated that the purity of C060-7607H-2665-240125 (Conjugate 3) is 97.50%.

(3) Detection of small molecule residues by RP-HPLC

The sample was tested for residual free drug levels (mol/mol, free drug/bound drug) by RP-HPLC. Initially, two standards containing free drug and Fc (2% & 5%) were prepared. The percentage of free drug was determined by comparing the EIC signal peak area with that of the residual free drug in the conjugate sample. The relevant method conditions are outlined in the table below:

| Name | Parameter settings | | | |
|---|---|---|---|---|
| Instrument | Agilent 1260 Infinity ll | | | |
| Chromatographic column | Agilent, PLRP-S 1000Å 4.6 mm*250 mm 8 μm | | | |
| Column temperature | 80 °C | | | |
| Mobile phase A | 0.1% TFA in $H_2O$ | | | |
| Mobile phase B | 0.1% TFA in acetonitrile | | | |
| Flow rate | 0.6 mL/min | | | |
| Injection volume | 40 μg | | | |
| Detection wavelength | 252 nm | | | |
| Gradient | Flow(mL/min) | Time | A(%) | B(%) |
| | 0.600 | 0.00 | 90 | 10 |
| | 0.600 | 2.00 | 90 | 10 |
| | 0.600 | 7.00 | 75 | 25 |
| | 0.600 | 15.00 | 25 | 75 |
| | 0.600 | 17.00 | 10 | 90 |
| | 0.600 | 17.10 | 90 | 10 |
| | 0.600 | 22.00 | 90 | 10 |

Preparation of the standard solution:

$C_{7607H}$=1 mg/mL; the DAR value of the sample is 5.9;

$$MW_{7607H} = 56000 \text{ g/mol}, MW_{drug} = 1578.63 \text{ g/mol};$$

$$M_{mAb} = 1/MW_{mAb}, M_{Total\ drug} = DAR/MW_{mAb};$$

$$\text{The moles of 2\% standard solution} = DAR \times MmAb \times 2\%;$$

$$\text{The moles of 5\% standard solution} = DAR \times MmAb \times 5\%;$$

The weight of the small molecule added to 100 μL of 2% standard solution ($2\%m_{drug}$) = The moles of 2% standard solution × 100 μL × $MW_{drug}$;

The weight of the small molecule added to 100 μL of 5% standard solution ($5\%m_{drug}$) = The moles of 5% standard solution × 100 μL × $MW_{drug}$;

The calculated weight of the small molecule was added to 100 μL of 1 mg/mL antibody solution to obtain the

standard solution.

The detection results for the small molecule residues in the sample are shown in Figure 10. The RP detection results indicated that the sample residue was less than 2% mol/mol compared with the antibody sample with 2% mol/mol and 5% mol/mol small molecules added by RP-HPLC.

### Example 21: Preparation of Conjugate 4

#### 1. Experimental overview

In this experiment, the reducing agent TCEP was used to break the disulfide bonds between antibody chains. Then, a Michael addition reaction was carried out between -SH and the maleimide on the Linkerpayload, resulting in the target coupling product DAR6. The DAR value of the coupling product was detected by LC-MS, the purity of the coupling product was tested by SEC-HPLC, and the small molecule residues in the coupling product were detected by RP-HPLC.

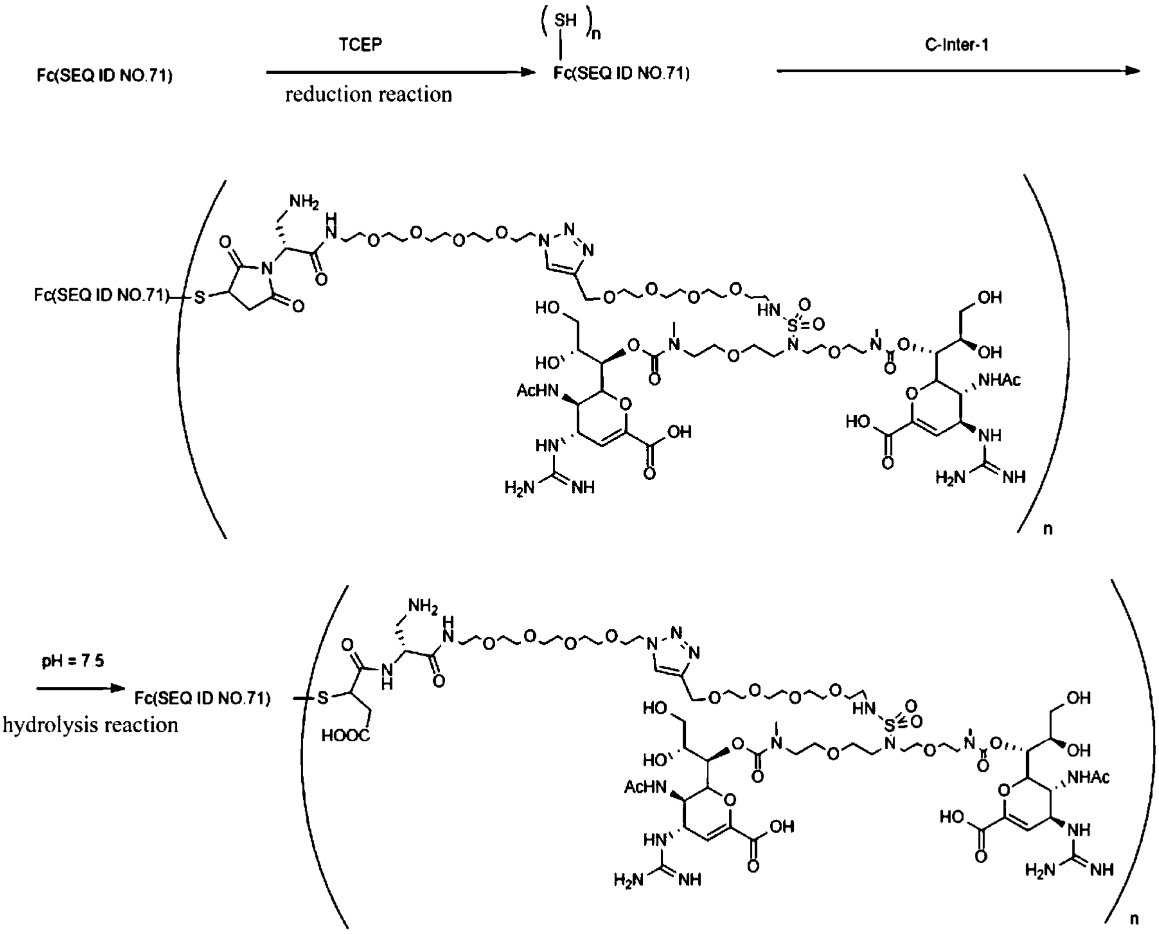


Conjugate 4

#### 2. Summary of experimental results

| No. | Experimental topic | Experimental results/conclusion |
|---|---|---|
| 1 | Preparation of thiol-coupled sample | The sample concentration of Conjugate 4 (internal number: C060-7607H-231008) was 6.22 mg/mL, the sample volume was 5.77 mL, and the total sample amount was 35.9 mg. The MS-DAR was 5.9, the SEC purity was 98.57%, the Free drug was less than 2% (no small molecule peak detected), and the endotoxin was less than 1 EU/mg. |

**3. Experimental materials and methods**

3.1 Instrument /Equipment

| No. | Name of instrument/equipment | Equipment model | Equipment number |
|---|---|---|---|
| 1 | pH meter | S470K | RD-080 |
| 2 | Centrifuge | Sorvall SI 16R | BP-0317 |
| 3 | Oscillating metal bath | SD2-100 | BP-0379 |
| 4 | Nanodrop lite | NanoDrop One | BP-0318 |
| 5 | Clean bench | SW-CJ-1D | QC-085 |

3.2 Reagents/solutions

| No. | Reagent/solution name | Reagent/solution Lot. No. | Remarks |
|---|---|---|---|
| 1 | 50 mM PB, pH 6.5 buffer | SO-007-076 | NA |
| 2 | 10 mM His-HCl, pH 5.5 buffer | SO-007-077 | NA |
| 3 | 10 mM TCEP solution | SO-014-089 | NA |
| 4 | 10 mg/mL C-Inter-1 aqueous solution | SO-007-045 | NA |

3.3 Experimental methods

3.3.1 Ultrafiltration-1

1) Pre-treatment of ultrafiltration centrifuge tubes

To a 15 mL 10KDa ultrafiltration centrifuge tube, was added 0.1 M NaOH solution, and then the tubes were centrifuged at 4500 G for 10 min, repeating three times. Ultrapure water was added to the ultrafiltration centrifuge tube, and then centrifuged at 4500 G for 10 min, repeating twice. 50 mM PB, pH 6.5 buffer solution was added to the ultrafiltration centrifuge tube, and then centrifuged at 4500 G for 10 min. This was repeated until the pH of the permeate was around 6.5.

2) Antibody ultrafiltration and fluid exchange

To the processed ultrafiltration centrifuge tube, was added 7607H antibody (SEQ ID NO: 71). The solution volume in the ultrafiltration tubes was filled to 12 mL with 50 mM PB, pH 6.5 buffer, and then the tube was centrifuged at 4500 G for 10 min, repeating 4 times, followed by filtrating with 0.22 μm filter membrane. The samples were collected to detect the antibody concentration.

3) Antibody concentration detection

The detection was performed using the Protein A280 method in the NanoDrop One, with the sample category selected as "Other protein E 1%". The extinction coefficient E 1%L/gm-cm was set to 13.44 (7607H), and the baseline correction wavelength was 340 nm. Using 50 mM PB, pH 6.5 buffer as the blank, 2.5 μL of the sample was taken for detection, and the average of three detection results was taken as the sample concentration.

3.3.2 Reduction reaction

50 mM PB, pH 6.5 buffer, corresponding antibody solution (45 mg), and 10 mM TCEP solution (TCEP/mAb equivalent ratio being 12) were successively added to the centrifuge tube, in which the antibody reaction concentration

was 5 mg/mL. The reaction was placed in a metal bath to control the reaction temperature at 22 °C for 2 h.

3.3.3 Coupling reaction and hydrolysis reaction

After the reduction reaction had proceeded for 2 h, 50 mM PB, pH 6.5 buffer solution, and 10 mg/mL C-**Inter-1** aqueous solution (AKSZ2627 aqueous solution) at a small molecule/mAb equivalent ratio of 10 (Drug/mAb equivalent ratio of 10) were added to the reaction solution. The final concentration of the reaction was controlled to be 2 mg/mL, and then the reaction was placed in a metal bath to control the reaction temperature at 22 °C, followed by reacting for 1 h. After reaching the reaction time, 10 mg/mL **C-Inter-1 aqueous solution** (AKSZ2627 solution) at a Drug/mAb equivalent ratio of 5 was further added to the 7607H antibody reaction solution. The reaction was placed in a metal bath to control the reaction temperature at 22 °C, and allowed to further react for 2 h.

3.3.4 Ultrafiltration-2

1) Pre-treatment of ultrafiltration centrifuge tubes

To a 15 mL 10KDa ultrafiltration centrifuge tube, was added 0.1 M NaOH solution, and then the tubes were centrifuged at 4500 G for 10 min, repeating three times. Ultrapure water was added to the ultrafiltration centrifuge tube, and then centrifuged at 4500 G for 10 min, repeating twice. 10 mM His-HCl, pH 5.5 buffer solution was added to the ultrafiltration centrifuge tube, and then centrifuged at 4500 G for 10 min. This was repeated until the pH of the permeate was about 5.5.

2) Antibody ultrafiltration and fluid exchange

To the processed ultrafiltration centrifuge tube, was added the conjugate sample. The solution volume in the ultrafiltration tubes was filled to 12 mL with 10 mM His-HCl, pH 5.5 buffer, and then the tube was centrifuged at 4500 G for 10 min, repeating 8 times. The ultrafiltered solution was filtered with 0.22 μm PES filter membrane. Then, the antibody concentration was detected after fluid exchange.

3) Antibody concentration detection

The detection was performed using the Protein A280 method in the NanoDrop One, with the sample category selected as "Other protein E 1%". The extinction coefficient E 1%L/gm-cm was set to 13.44, and the baseline correction wavelength was 340 nm. Using 10 mM His-HCl, pH 5.5 buffer as the blank, 2.5 μL of the sample was taken for detection, and the average of three detection results was taken as the sample concentration. Based on the detected sample concentration and sample volume, the sample was diluted to a concentration of approximately 10 mg/mL using 10 mM His-HCl, pH 5.5 buffer solution, and then the concentration of the diluted sample was re-detected.

**4. Experimental results and analysis**

4.1 Detection of LC-MS DAR value

The DAR value of the sample was detected by LC-MS, and the relevant method conditions are shown in the table below:

LC method conditions

| Name | Parameter settings |
| --- | --- |
| Instrument | SCIEX ExionLC |
| Chromatographic column | ACQUITY UPLC BEH 200 Å, SEC 1.7 μm, 4.6 × 300 mm |
| Mobile phase | A: 0.1% FA, 0.05% TFA, 25% ACN in $H_2O$ |
| Injection volume | 10 μL |
| Column temperature | 25 °C |
| Flow rate | 200 μL/min |

(continued)

| Name | Parameter settings |
|---|---|
| Gradient | Isocratic |

MS method conditions

| Name | Parameter settings |
|---|---|
| Instrument | SCIEX 5600 |
| Scanning range | 900-5000 |
| Polarity | Positive |
| Atomized gas (GS1) | 50 |
| Auxiliary gas (GS2) | 50 |
| Curtain gas (CUR) | 25 |
| Ion transport tube temperature (TEM) | 450 |
| Spray voltage (ISVF) | 5500 |
| Declustering voltage (DP) | 250 |
| Collision energy (CE) | 10 |
| Accumulated time (AT) | 1 s |

The detection results for the MS-DAR value of the sample are shown in the table below. The MS detection results indicated that the MS-DAR value of the Conjugate 4 sample (Internal number C060-7607H-231008) was 5.9.

| Sample name | Payload distribution | | | | MS-DAR |
|---|---|---|---|---|---|
| | C0 | C1 | C2 | C3 | |
| C060-7607H-231008 | 0.00% | 0.34% | 3.96% | 95.70% | 5.9 |

Note:

Equation for drug load distribution: Drug load distribution (%) = (Area of single-chain peak with drug load n / Sum of areas of two single-chain peaks) $\times$ 100 (%); Equation for drug load distribution:

wherein n represents the drug loading capacity, and a single chain consists of heavy chain constant domain 2, heavy chain constant domain 3, and ½ hinge region (CH2+CH3+½Hi);

DAR value calculation equation: Single-chain weighted peak area = (Drug load n × Drug load distribution %)

$$\text{Drug-to-antibody ratio} = 2 \times \sum(\text{weighted peak area of single chain})$$

The MS detection spectrum of the sample is shown in Figure 11:

4.2 SEC-HPLC purity testing

The purity of the sample was tested by SEC-HPLC, and the relevant method conditions are shown in the table

below:

| Name | Parameter settings |
|---|---|
| Instrument | Thermo scientific Vanquish |
| Chromatographic column | TSKgel guardcolumn SWxl 6.0 mm x 4 cm |
| | TSKgel G3000SWxl 7.8 mm x 30 cm |
| Column temperature | 25 °C |
| Mobile phase | 0.2 M $KH_2PO_4$, 0.25 M KCl, 15% ACN |
| Flow rate | 0.5 mL/min |
| Injection volume | 25 µg |
| Detection wavelength | 280 nm |
| Gradient | Isocratic<br>Flow(mL/min) / Time / A(%) / B(%)<br>0.500 / 0.00 / 100 / 0<br>0.500 / 30.00 / 100 / 0 |

The purity test results of the sample are shown in Figure 12. The SEC test results indicated that the purity of Conjugate 4 (C060-7607H-231008) is 98.57%.

4.3 Detection of small molecule residues by RP-HPLC

The sample was tested for residual free drug levels (mol/mol, free drug/bound drug) by RP-HPLC. Initially, two standards containing free drug and Fc (2% & 5%) were prepared. The percentage of free drug was determined by comparing the EIC signal peak area with that of the residual free drug in the conjugate sample. The relevant method conditions are outlined in the table below:

| Name | Parameter settings | | | |
|---|---|---|---|---|
| Instrument | Agilent 1260 Infinity ll | | | |
| Chromatographic column | Agilent, PLRP-S 1000 Å<br>4.6 mm*250 mm 8 μm | | | |
| Column temperature | 80 °C | | | |
| Mobile phase A | 0.1% TFA in $H_2O$ | | | |
| Mobile phase B | 0.1% TFA in Acetonitrile | | | |
| Flow rate | 0.6 mL/min | | | |
| Injection volume | 40 μg | | | |
| Detection wavelength | 252 nm | | | |
| Gradient | Flow(mL/min) | Time | A(%) | B(%) |
| | 0.600 | 0.00 | 90 | 10 |
| | 0.600 | 2.00 | 90 | 10 |
| | 0.600 | 8.00 | 75 | 25 |
| | 0.600 | 15.00 | 10 | 90 |
| | 0.600 | 17.00 | 10 | 90 |
| | 0.600 | 17.10 | 90 | 10 |
| | 0.600 | 25.00 | 90 | 10 |

Preparation of the standard solution:

$C_{7607H}$=1 mg/mL; the DAR value of the sample is 5.9;

$$MW_{7607H} = 56000 \text{ g/mol}, MW_{drug} = 1590.79 \text{ g/mol};$$

$$M_{mAb} = 1/MW_{mAb}, M_{Total\ drug} = DAR/MW_{mAb};$$

$$\text{The moles of 2\% standard solution} = DAR \times M_{mAb} \times 2\%;$$

$$\text{The moles of 5\% standard solution} = DAR \times M_{mAb} \times 5\%;$$

The weight of the small molecule added to 100 μL of 2% standard solution (2%$m_{drug}$) = The moles of 2% standard solution × 100 μL × $MW_{drug}$;

The weight of the small molecule added to 100 μL of 5% standard solution (5%$m_{drug}$) = The moles of 5% standard solution × 100 μL × $MW_{drug}$;

The calculated weight of the small molecule was added to 100 μL of 1 mg/mL antibody solution to obtain the

standard solution.

The detection results for the small molecule residues in the sample are shown in Figure 13. The RP detection results indicated that no small molecule peak was detected, and that the sample residue was less than 2% mol/mol compared with the antibody sample with 2% mol/mol and 5% mol/mol small molecules added by RP-HPLC.

4.4 Endotoxin residue detection

Test results:

Based on the comparison with a 1 EU/mg positive control, the endotoxin residue in the sample was less than 1 EU/mg.

**Example 22: *In vitro* anti-influenza virus activity of conjugates (Inhibition experiment of cell lesion mediated by influenza virus H1N1)**

1. Experimental method

By an *in vitro* anti-IAV/IBV activity assay based on cytopathic effects, the MDCK cell lines were used to quantitatively detect the activities of compounds on IAV/IBV-induced cytopathic effects.

2. Experimental materials

| **Materials** | **Source** | **Lot. No.** |
|---|---|---|
| H1N1 (A/PR/8/34) virus | ATCC | VR1469 |
| MDCK cells | ATCC | CCL-34 |
| 96-well plate | Corning | 3917 |
| Fetal Bovine Serum (FBS) | Hyclone | SH30406.05 |
| 100x Pen-Strep solution (P/S) | Hyclone | SV30010 |
| DMEM | Hyclone | SH30243.01 |
| TPCK treated trypsin | Sigma | T1426 |
| Cell-titer Glo | Promega | G7571 |
| Zanamivir | Adamas | P1695633 |

3. Experimental procedures

1) MDCK cells were digested and diluted with DMEM medium containing 2% FBS and 1% P/S to a concentration of $2\times 10^5$/mL. Then, the cells were inoculated into a 96-well plate at 50 μL/well, and incubated overnight in an incubator.

2) First, the compound was diluted to 2 μM, and then three-fold dilutions were performed from 2 μM, 8 dilutions in total. 5 μL of the diluted compound was added to each well and placed in an incubator for incubating 1 h.

3) The influenza viruses were diluted to 2222 pfu/mL with DMEM medium containing 2% FBS, 1% P/S, and 4 μg/mL TPCK-treated trypsin, and then added to each well at 45 μL/well. Simultaneously, a control group without compounds but with viruses was set up, together with a cell control group without compounds and viruses. The plate was placed in an incubator and incubated for 4 days.

4) 50 μL of Cell-titer Glo was added to each well, and the chemiluminescence was detected using a microplate reader.

4. Data analysis: The virus inhibition rate of the compound was calculated.

Inhibition rate = (the measured value of sample- the average value of virus control group) / (the average value of cell control group - the average value of virus control group) * 100%

Using the calculated inhibition rates, the inhibition curve was fitted using the log(inhibitor) vs. response -- Variable slope (four parameters) in the Nonlinear regression (curve fit) function of GraphPad Prism 8 software, and the $EC_{50}$ value was calculated.

Reference molecule A and reference molecule B were prepared according to the specific procedures described in Example 156 (Conjugate 45b) and Example 188 (Conjugate 46) of the patent WO2021046549A1, respectively.

The purity of reference molecule A was 99.2% (SEC-HPLC), with a DAR value of 4.26.

The purity of reference molecule B is 98.8% (SEC-HPLC), with a DAR value of 5.87.

Result Summary:

**Summary of results**

| Compound | DAR value | $EC_{50}$ (nM) |
|---|---|---|
| Conjugate 1 | 4 | 0.068 |
| Conjugate 2 | 6 | 0.038 |
| Conjugate 3 | 5.9 | 0.060 |
| Conjugate 4 | 5.9 | 0.060 |
| Reference molecule A | 4.26 | 0.60 |
| Reference molecule B | 5.87 | 0.40 |

The data revealed that the tested conjugate molecules exhibited excellent *in vitro* anti-H1N1 virus activity. Compared with reference molecules A and B, Conjugate 1 showed an activity increase of 8.8 times and 5.9 times, respectively. Conjugate 2 showed an activity increase of 15.8 times and 10.5 times, respectively. Conjugates 3 and 4 showed activity increase of 10.0 times and 6.7 times, respectively.

The high *in vitro* antiviral activity indicated that the conjugate molecules of the present invention possess superior antiviral activity in animals and in clinical human trials.

**Example 23: Screening experiment of coupling conditions for Conjugate 1**

Except for the different coupling conditions, the experimental materials, experimental methods, and detection methods were all the same as those in Example 18.

The coupling reaction for adding C-Inter-1 in one portion: After the reduction reaction had reached 2 h, 50 mM PBS, pH 6.5 buffer solution, and 10 mg/mL C-Inter-1 solution (C-Inter-1/Fc molar equivalent ratio of 7) were added to the reaction solution in one portion. The final concentration of the reaction was controlled to be 1 mg/mL. The reaction was placed in a metal bath to control the reaction temperature at 22 °C for 2 h.

The DAR value detected by LC-MS was as follows:

| Sample name | Payload distribution | | | | MS-DAR |
|---|---|---|---|---|---|
| | C0 | C1 | C2 | C3 | |
| Conjugate 1-1 | 31.41% | 11.65% | 56.94% | 0.00% | 2.51 |

The MS detection results are shown in Figure 7.

**Example 24: Pharmacokinetic study of Conjugate 1 in mice**

**1. Experimental information**

Pharmacokinetic studies were performed in male CD-1(ICR) mice. Blood samples were analyzed by the ELISA method, and the specific experimental information and design are detailed in the table below.

| **Test compound** | **Conjugate 1** |
|---|---|
| **Route of administration** | Intravenous administration (IV) |
| **Dosage (mg/kg)** | 5.0 |
| **Vehicle** | Dissolving in 10 mM histidine solution (pH 5.5) at 1 mg/mL |
| **Analytical method** | ELISA |
| **Design of blood sampling time points** | 0, 0.083, 1, 3, 5, 24, 72, and 168 h |
| **Number of animals** | 15 |

**2. Key experimental materials**

| Category | Name | Lot No./Cat No. | Supplier |
|---|---|---|---|
| Capturing agent | Conjugate capture agent | 40017-VNAHC | Sino Biological |
| HRP | anti-human-IgG-HRP | 151886 | Jackson |
| Washing Buffer | 0.1 %PBST | WB-20220314-GKS | WuXi AppTec |
| Coating buffer | CBS | CB-20220314-GKS | WuXi AppTec |
| blocking buffer (BSA) | 5% BSA in 0.1% PBST | BB-20220316-GKS | WuXi AppTec |
| Assay Buffer | 1% BSA in 0.1% PBST | AB-20220314-SWZ | WuXi AppTec |
| TMB | TMB Substrate Kit | 10537557 | SeraCare™ |
| Stop solution | 2N $H_2SO_4$ | SS-20220219-YJC | WuXi AppTec |

**3. Detection steps**

Method introduction: The concentration of the conjugate in the plasma of CD-1 mice was determined using the ELISA method. The lower limit of quantification (LLOQ) for the conjugate was 50.0 ng/mL, while the upper limit of quantification (ULOQ) was 3000 ng/mL. The specific steps were as follows:

1) Blood was collected at the pre-determined time points, and using EDTA-K2 as anticoagulant, corresponding test samples were prepared.

2) 100 μL of coating solution was added to a 96-well microplate, and then the plate was sealed, and incubated overnight at 2-8°C before use.

3) The plate was washed five times with washing buffer (300 μL/well).

4) The plate was blocked by adding 300 μL of blocking buffer to each well. The plate was sealed and incubated at room temperature for 2 h, without shaking.

5) Step 2 was repeated.

6) 100 μL of standard solution and test sample were added to each well. The plate was sealed and incubated at room temperature with shaking at 450RPM for 2 h ± 10 min.

7) Step 2 was repeated.

8) 100 μL of HRP solution was added to each well. The plate was seal and incubated with shaking at 450RPM at room temperature for 1 h ± 10 min.

9) Step 2 was repeated.

10) 100 μL of TMB was added to each well. The plate was incubated at room temperature for 5-20 min.

11) 100 μL of stop solution was added to each well.

12) The plate was read within 30 minutes using SpectraMax M5e /M5/Plus 384 microplate reader at wavelengths of 450 nm and 630 nm.

13) The readings of the standard solution were converted to the corresponding concentrations of the conjugate.

14) Data processing was carried out using Watson LIMS V7.6 or SoftMax Pro GxP 7.0.3, along with Microsoft Excel 2007 or later versions.

**4. Summary of results**

| PK parameters of Conjugate 1 | |
|---|---|
| **PK parameters** | **IV** |
| | **Plasma average** |
| **$C_0$ (ng/mL)** | 105000 |
| **$T_{max}$ (h)** | 0.0830 |
| **$T_{1/2}$ (h)** | 165 |
| **Cl(mL/min/kg)** | -- |
| **$T_{last}$ (h)** | 168 |
| **$AUC_{0\text{-}last}$ (ng.h/mL)** | 3648250 |
| **$AUC_{0\text{-}inf}$ (ng.h/mL)** | 6927391 |
| **$MRT_{0\text{-}last}$ (h)** | 68.8 |
| **$MRT_{0\text{-}int}$ (h)** | 229 |
| **$AUC_{Extra}$ (%)** | 47.3 |
| **$AUMC_{Extra}$ (%)** | 84.1 |

From the pharmacokinetic data of mice, it could be observed that Conjugate 1 had a relatively long half-life, reaching 165 hours. At the same time, Conjugate 1 exhibited good exposure *in vivo.* The above data suggested that Conjugate 1 had a relatively long half-life and high exposure in clinical settings, potentially exhibiting extremely excellent antiviral activity.

**Example 25: Pharmacokinetic study of Conjugate 2 in mice**

The pharmacokinetic study of Conjugate 2 was performed according to the method described in Example 22, and the results are summarized as follows:

| PK parameters of Conjugate 2 | |
|---|---|
| **PK parameter** | **IV** |
| | **Plasma average** |
| **$C_0$ (ng/mL)** | 109000 |
| **$T_{max}$ (h)** | 0.0830 |

(continued)

| PK parameters of Conjugate 2 | |
|---|---|
| PK parameter | IV |
| | Plasma average |
| $T_{1/2}$ (h) | 140 |
| Cl(mL/min/kg) | -- |
| $T_{last}$ (h) | 168 |
| $AUC_{0\text{-}last}$ (ng.h/mL) | 4377223 |
| $AUC_{0\text{-}inf}$ (ng.h/mL) | 7503571 |
| $MRT_{0\text{-}last}$ (h) | 68.1 |
| $MRT_{0\text{-}inf}$ (h) | 194 |
| $AUC_{Extra}$ (%) | 41.7 |
| $AUMC_{Extra}$ (%) | 79.5 |

From the pharmacokinetic data of mice, it could be observed that Conjugate 2 had a relatively long half-life, reaching 140 hours. Meanwhile, Conjugate 1 exhibited good exposure *in vivo.* The above data suggested that Conjugate 1 had a relatively long half-life and high exposure in clinical settings, potentially exhibiting extremely excellent antiviral activity.

**Example 26: Pharmacokinetic study of Conjugate 4 in mice**

**1. Experimental information**

Pharmacokinetic studies were performed in male CD-1(ICR) mice. Blood samples were analyzed by the ELISA method, and the specific experimental information and design are detailed in the table below.

| Test compound | Conjugate 4 |
|---|---|
| Route of administration | Intravenous administration (IV); subcutaneous injection (SC); intraperitoneal injection (IM) |
| Dosage (mg/kg) | 5.0 |
| Vehicle | IV: Dissolving in 10 mM histidine solution (pH 5.5) at 2 mg/mL<br>SC: Dissolving in 10 mM histidine solution (pH 5.5) at 2 mg/mL<br>IM: Dissolving in 10 mM histidine solution (pH 5.5) at 2 mg/mL |
| Analytical method | ELISA |
| Design of blood sampling time points | IV (hour): 0, 0.083, 1, 4, 24, 72, 120, 168, 336, 504, 576, 672<br>SC (hour): 0, 0.25, 1, 4, 24, 72, 120, 168, 336, 504, 576, 672<br>IM (hour): 0, 0.25, 1, 4, 24, 72, 120, 168, 336, 504, 576, 672 |
| Number of animals | 27 mice, with 9 mice for each administration mode. |

**2. Testing the concentration of the conjugate drug (ADC concentration) in animal plasma by the ELISA method**

**1) Key reagents**

| Category | Name | Concentration | Lot No. /Supplier | Storage temperature |
|---|---|---|---|---|
| Test compound | Conjugate 4 | 16.71 mg/ml | 20220721001/ ArkBio | ≤-60°C |

(continued)

| Category | Name | Concentration | Lot No. /Supplier | Storage temperature |
|---|---|---|---|---|
| Coating agent | ADC-capturing antibody | 800 U/mL | 40017-VNAHC /Sino Biological# | ≤-60°C |
| Testing/HRP | anti-human IgG-HRP | 0.8 mg/mL | 709-036-098/ Jackson Immuno Research | ≤-60°C |
| Medium | Mixed mouse plasma | NA | RAT-EDTA2K-PPL-20220808-GKS | ≤-60°C |

**2) Key experimental parameters**

| | |
|---|---|
| **Blocking buffer** | 3% BSA in 0.1% PBST |
| **Test buffer solution** | 1% BSA in 0.1% PBST |
| **Washing buffer** | 0.1% PBST |
| **MRD** | 1:50 in the test buffer solution |
| **Coating working concentration** | 1U/mL in carbonate-bicarbonate buffer solution |
| **Detection/HRP dilution factor** | 1:4000 in the test buffer solution |
| **Content determination range** | 50.0 ng/mL (LLOQ) to 3000 ng/mL (ULOQ) |
| **Regression and weighting factors** | Logic with a weighting factor of $1/Y^2$ (automatically estimated) (applicable to Watson LIMS) or four-parameter logic with a weighting factor of $1/1Y^2$ (applicable to SoftMax) |
| **Data processing and computation** | Watson LIMS V7.6 or SoftMax Pro GxP 7.0.3 and Microsoft Excel 2007 or later version |
| **Plate reader** | SpectraMax M5e/M5/Plus 384 from Molecular Devices |

**3. Experimental procedures**

(1) 100 µL of coating solution (Solarbio) was added to a 96-well microplate, and then the plate was sealed, and incubated overnight at 2-8°C before use.

(2) The plate was washed three times with washing buffer (300 µL/well).

(3) The plate was sealed by adding 300 µL of blocking solution to each well. The plate was sealed and incubated at room temperature for 2 h without shaking.

(4) Repeat step (2).

(5) 100 µL of standard/quality control product and sample were added to each well. The plate was sealed and incubated at room temperature with shaking (450 rpm) for 1 hour ± 10 minutes.

(6) Repeat step 5.2.

(7) 100 µL of HRP solution was added to each well. The plate was sealed and incubated at room temperature with shaking (450 rpm) for 1 hour ± 10 minutes.

(8) Repeat step 5.2.

(9) 100 μL of TMB was added to each well. The plate was incubated at room temperature for 5-20 min.

(10) 100 μL of 2N $H_2SO_4$ was added to each well.

(11) The absorbance was read at 450 nm using a plate reader within 30 min.

**3. Determining the concentration of the conjugate drug (Total drug concentration) in animal plasma using the ELISA method**

**1) Key reagents**

| Category | Name | Concentration | Lot No. /Batch No./Supplier | Storage temperature |
|---|---|---|---|---|
| Test compound 1 | Conjugate 4 | 16.71mg/ml | 20220721001/ArkBio | ≤-60°C |
| Test compound 2 | Fc (SEQ ID NO:67) | 28.11mg/ml | 20210926-7607B/ArkBio | ≤-60°C |
| Coating agent | Goat anti-human IgG, monkey ads antibody | 1mg/mL | C2822-PH02 | 2-8°C |
| Detection/HRP | anti-human-IgG-HRP | 0.8 mg/mL | 709-036-098/ Jackson Immuno Research (Jackson) | ≤-60°C |
| Medium | Pooled mouse plasma | NA | RAT-EDTA2K-PPL-20220808-GKS | ≤-60°C |

**2) Key experimental parameters**

| | |
|---|---|
| **Blocking solution** | SuperBlock™ blocking buffer in PBS |
| **Test buffer solution** | 1% BSA in 0.1% PBST |
| **Washing buffer** | 0.1% PBST |
| **MRD** | 1:50 in the test buffer solution |
| **Coating working concentration** | 2 μg/mL in carbonate-bicarbonate buffer solution |
| **Detection/HRP dilution factor** | 1:10000 in the test buffer solution |
| **Content determination range** | 10.0 ng/mL (LLOQ) to 600 ng/mL (ULOQ) |
| **Regression and weighting factors** | Logic with a weighting factor of $1/Y^2$ (automatically estimated) (applicable to Watson LIMS) or four-parameter logic with a weighting factor of $1/Y^2$ (applicable to SoftMax) |
| **Data processing and computation** | Watson LIMS V7.6 or SoftMax Pro GxP 7.0.3 and Microsoft Excel 2007 or later version |
| **Plate reader** | SpectraMax M5e/M5/Plus 384 from Molecular Devices |

**3. Experimental procedures**

(1) 100 μL of coating solution was added to a 96-well microplate, and then the plate was sealed, and incubated overnight at 2-8°C before use.

(2) The plate was washed five times with washing buffer (300 μL/well).

(3) The plate was sealed by adding 300 μL of blocking solution to each well. The plate was sealed and incubated at room temperature for 2 h without shaking.

(4) Repeat step (2).

(5) 100 μL of standard/quality control product and sample were added to each well. The plate was sealed and incubated at room temperature with shaking (450 rpm) for 1 hour ± 10 minutes.

(6) Repeat step 5.2.

(7) 100 μL of HRP solution was added to each well. The plate was sealed and incubated at room temperature with shaking (450 rpm) for 1 hour ± 10 minutes.

(8) Repeat step 5.2.

(9) 100 μL of TMB was added to each well. The plate was incubated at room temperature for 5-20 min.

(10) 100 μL of 2N $H_2SO_4$ was added to each well.

(11) The absorbance was read at 450 nm and 630 nm using a plate reader within 30 min.

**4. Experimental results**

| Conjugate 4 (Total) | | | |
|---|---|---|---|
| **PK parameters** | **IV** | **SC** | **IM** |
| | **Plasma average** | **Plasma average** | **Plasma average** |
| **Rsq_adj** | 0.924 | 0.971 | 0.908 |
| **No. points used for $T_{1/2}$** | 8.00 | 7.00 | 7.00 |
| **$C_0$ or $C_{max}$ (ng/mL)** | 123604 | 33567 | 44033 |
| **$T_{max}$ (h)** | -- | 24.0 | 24.0 |
| **$T_{1/2}$ (h)** | 182 | 195 | 177 |
| **$Vd_{ss}$ (L/kg)** | 0.113 | -- | -- |
| **Cl (mL/min/kg)** | 0.00731 | -- | -- |
| **$T_{last}$ (h)** | 672 | 672 | 672 |
| **$AUC_{0-last}$ (ng.h/mL)** | 10416070 | 8416855 | 11020340 |
| **$AUC_{0-inf}$ (ng.h/mL)** | 11395300 | 9434079 | 11712340 |
| **$MRT_{0-last}$ (h)** | 195 | 216 | 212 |
| **$MRT_{0-inf}$ (h)** | 258 | 295 | 254 |
| **$AUC_{Extra}$ (%)** | 8.59 | 10.8 | 5.91 |
| **$AUMC_{Extra}$ (%)** | 31.1 | 34.8 | 21.6 |
| **Bioavailability (%)** | -- | 82.8 | 103 |

| Conjugate 4 (ADC) | | | |
|---|---|---|---|
| PK Parameters | IV bolus1 | SC2 | IM3 |
| | Mean Plasma | Mean Plasma | Mean Plasma |
| Rsq_adj | 0.910 | 0.995 | 0.889 |
| No. points used for $T_{1/2}$ | 8.00 | 7.00 | 7.00 |
| $C_0$ or $C_{max}$ (ng/mL) | 127492 | 36600 | 35967 |
| $T_{max}$ (h) | -- | 24.0 | 24.0 |
| $T_{1/2}$ (h) | 201 | 190 | 196 |
| $Vd_{ss}$ (L/kg) | 0.112 | -- | -- |
| Cl(mL/min/kg) | 0.00640 | -- | -- |
| $T_{last}$ (h) | 672 | 672 | 672 |
| $AUC_{0-last}$ (ng.h/mL) | 11497170 | 9949327 | 10649680 |
| $AUC_{0-inf}$ (ng.h/mL) | 13014400 | 10990350 | 11596360 |
| $MRT_{0-last}$ (h) | 204 | 219 | 224 |
| $MRT_{0-inf}$ (h) | 292 | 288 | 283 |
| $AUC_{Extra}$ (%) | 11.7 | 9.47 | 8.16 |
| $AUMC_{Extra}$ (%) | 38.4 | 31.1 | 27.5 |
| Bioavailability (%) | -- | 84.4 | 89.1 |

From the pharmacokinetic data of mice, it could be observed that Conjugate 4 had a relatively long half-life, reaching 190 hours and above for all three administration routes (intravenous injection (IV), subcutaneous injection (SC), and intraperitoneal injection (IM)). Conjugate 4 also exhibited a high exposure level *in vivo.* These data suggested that Conjugate 4 had a relatively long half-life and high exposure in clinical settings, potentially exhibiting extremely excellent antiviral activity.

**Example 27: Pharmacokinetic study of Conjugate 4 in rats**

**1. Experimental information**

Pharmacokinetic studies were performed using male SD rats. Blood samples were analyzed by ELISA method, and the specific experimental information and design are detailed in the table below.

| **Test compound** | **Conjugate 4** |
|---|---|
| **Route of administration** | Intravenous administration (IV) |
| **Dosage (mg/kg)** | 15.0 |
| **Vehicle** | Dissolving in 10 mM histidine solution (pH 5.5) at 3 mg/mL |
| **Analytical method** | ELISA |
| **Design of blood sampling time points** (hour) | IV (hour): 0, 0.083, 1, 3, 5, 24, 72, 168, 336, 504 |
| **Number of animals** | 3 |

**2. Determining the concentration of conjugate drug in animal plasma by ELISA method**

Referring to the testing method of conjugates in animal plasma in Example 26, the drug concentrations of ADC and the Total drug concentration in rat plasma were tested.

**3. Experimental results**

| Conjugate 4 (Total) (ng/mL) | | | | | | | |
|---|---|---|---|---|---|---|---|
| IV | | | | | | | |
| **Time (h)** | **R01** | **R02** | **R03** | **Mean** | ± | **SD** | **CV (%)** |
| **0** | BQL | BQL | BQL | ND | ± | ND | ND |
| **0.083** | 346000 | 310000 | 332000 | 329333 | ± | 18148 | 5.51 |
| **1** | 271000 | 252000 | 261000 | 261333 | ± | 9504 | 3.64 |
| **3** | 209000 | 221000 | 205000 | 211667 | ± | 8327 | 3.93 |
| **5** | 178000 | 151000 | 178000 | 169000 | ± | 15588 | 9.22 |
| **24** | 96300 | 95400 | 96000 | 95900 | ± | 458 | 0.478 |
| **72** | 71100 | 68500 | 64000 | 67867 | ± | 3592 | 5.29 |
| **168** | 41300 | 44000 | 43700 | 43000 | ± | 1480 | 3.44 |
| **336** | 25900 | 23600 | 23600 | 24367 | ± | 1328 | 5.45 |
| **504** | 13900 | 13700 | 13700 | 13767 | ± | 115 | 0.839 |
| **PK parameter** | **R01** | **R02** | **R03** | **Mean** | ± | **SD** | **CV (%)** |
| **Rsq_adj** | 0.986 | 0.997 | 0.997 | -- | ± | -- | -- |
| **No. points used for $T_{1/2}$** | 3.00 | 3.00 | 4.00 | ND | ± | -- | -- |
| **$C_0$ (ng/mL)** | 353737 | 315867 | 339310 | 336305 | ± | 19113 | 5.68 |
| **$T_{1/2}$ (h)** | 214 | 200 | 194 | 203 | ± | 10.1 | 4.97 |
| **$Vd_{ss}$ (L/kg)** | 0.155 | 0.156 | 0.155 | 0.155 | ± | 0.000470 | 0.303 |
| **Cl (mL/min/kg)** | 0.00961 | 0.00994 | 0.0100 | 0.00986 | ± | 0.000221 | 2.25 |
| **$T_{last}$ (h)** | 504 | 504 | 504 | 504 | ± | -- | -- |
| **$AUC_{0-last}$ (ng.h/mL)** | 21738170 | 21194500 | 21103040 | 21345237 | ± | 343349 | 1.61 |
| **$AUC_{0-inf}$ (ng.h/mL)** | 26027000 | 25139700 | 24945500 | 25370733 | ± | 576579 | 2.27 |
| **$MRT_{0-last}$ (h)** | 162 | 162 | 161 | 162 | ± | 0.454 | 0.280 |
| **$MRT_{0-inf}$ (h)** | 269 | 261 | 257 | 263 | ± | 6.18 | 2.35 |
| **$AUC_{Extra}$ (%)** | 16.5 | 15.7 | 15.4 | 15.9 | ± | 0.556 | 3.51 |
| **$AUMC_{Extra}$ (%)** | 49.7 | 47.6 | 47.0 | 48.1 | ± | 1.43 | 2.98 |

| Conjugate 4 (ADC) (ng/mL) | | | | | | | |
|---|---|---|---|---|---|---|---|
| IV | | | | | | | |
| **Time (h)** | **R01** | **R02** | **R03** | **Mean** | ± | **SD** | **CV (%)** |
| **0** | BQL | BQL | BQL | ND | ± | ND | ND |
| **0.083** | 299000 | 278000 | 272000 | 283000 | ± | 14177 | 5.01 |
| **1** | 264000 | 226000 | 212000 | 234000 | ± | 26907 | 11.5 |
| **3** | 190000 | 179000 | 167000 | 178667 | ± | 11504 | 6.44 |
| **5** | 162000 | 129000 | 140000 | 143667 | ± | 16803 | 11.7 |
| **24** | 85300 | 93800 | 80700 | 86600 | ± | 6646 | 7.67 |
| **72** | 54200 | 51800 | 46500 | 50833 | ± | 3940 | 7.75 |

(continued)

| Conjugate 4 (ADC) (ng/mL) | | | | | | | |
|---|---|---|---|---|---|---|---|
| IV | | | | | | | |
| **Time (h)** | **R01** | **R02** | **R03** | **Mean** | ± | **SD** | **CV (%)** |
| **168** | 33100 | 34200 | 31800 | 33033 | ± | 1201 | 3.64 |
| **336** | 17100 | 16200 | 14300 | 15867 | ± | 1429 | 9.01 |
| **504** | 14300 | 13600 | 12000 | 13300 | ± | 1179 | 8.86 |
| **PK Parameters** | **R01** | **R02** | **R03** | **Mean** | ± | **SD** | **CV (%)** |
| **Rsq_adj** | 0.891 | 0.901 | 0.902 | -- | ± | -- | -- |
| **No. points used for $T_{1/2}$** | 4.00 | 4.00 | 4.00 | 4.00 | ± | -- | -- |
| **$C_0$ (ng/mL)** | 302388 | 283260 | 278205 | 287951 | ± | 12756 | 4.43 |
| **$T_{1/2}$ (h)** | 223 | 217 | 212 | 217 | ± | 5.78 | 2.66 |
| **$Vd_{ss}$ (L/kg)** | 0.202 | 0.203 | 0.218 | 0.208 | ± | 0.00916 | 4.41 |
| **Cl(mL/min/kg)** | 0.0113 | 0.0117 | 0.0130 | 0.0120 | ± | 0.000880 | 7.32 |
| **$T_{last}$ (h)** | 504 | 504 | 504 | 504 | ± | -- | -- |
| **$AUC_{0\text{-}last}$ (ng.h/mL)** | 17455740 | 17069680 | 15546000 | 16690473 | ± | 1009765 | 6.05 |
| **$AUC_{0\text{-}inf}$ (ng.h/mL)** | 22060600 | 21327000 | 19210400 | 20866000 | ± | 1479966 | 7.09 |
| **$MRT_{0\text{-}last}$ (h)** | 157 | 157 | 155 | 156 | ± | 1.42 | 0.907 |
| **$MRT_{0\text{-}inf}$ (h)** | 297 | 289 | 280 | 289 | ± | 8.66 | 3.00 |
| **$AUC_{Extra}$ (%)** | 20.9 | 20.0 | 19.1 | 20.0 | ± | 0.899 | 4.50 |
| **$AUMC_{Extra}$ (%)** | 58.1 | 56.5 | 55.2 | 56.6 | ± | 1.43 | 2.53 |

From the pharmacokinetic data of rats, it could be observed that Conjugate 4 exhibited a relatively long half-life, reaching more than 190 hours. Additionally, Conjugate 4 demonstrated good exposure *in vivo.* These data suggested that Conjugate 4 had a relatively long half-life and high exposure in clinical settings, potentially exhibiting extremely excellent antiviral activity.

**Example 28: Evaluation of antiviral efficacy in vivo of test substance (Conjugate 4) using a mouse infection model of influenza virus**

**1. Experimental materials and methods**

**1) Animals:** Female BALB/c mice (6-7 weeks old, 18-20 g) of specific pathogen-free grade, were purchased from Shanghai Jihui Experimental Animal Breeding Co., Ltd. They arrived at the animal room of the Biological Department of Nantong WuXi AppTec Pharmaceutical Technology Co., Ltd. on November 3, 2023, and were housed in individual ventilated cages. The quality certificate number for the experimental animals is 20220009014323. The mice were housed and used in accordance with the experimental protocol approved by WuXi AppTec IACUC-QD (Animal Use Protocol Number: ID01-QD031-2022v1.0). After at least 3 days of environmental adaptation, qualified animals were used for the experiment.

**2) Solvent and test substance**

10 mM histidine buffer solution (pH = 5.5). Taking the preparation of 1 L solution as an example, 0.372 g of histidine and 1.593 g of histidine hydrochloride were added to 1 L of $ddH_2O$, vortexed and mixed evenly for later use.

**Test substance information:**

| Test compound name | Appearance | Purity (%) | Concentration (mg/mL) | Dosage (μL) | Storage condition |
|---|---|---|---|---|---|
| Conjugate 4 | Liquid | 98.57 | 6.22 | 350 | -80°C |
| hIgG Fc SEQ No.71 | Liquid | 99.68 | 23.62 | 100 | |

**3) Virus**

Influenza A virus, A/California/07/2009(H1N1). The original strain was purchased from ATCC, Cat No.: VR1843. The working strain was amplified by WuXi AppTec Biologics, Lot No.: 20201228YNR. Titer: 7.0E+06 PFU/mL.

**4) Reagents and instruments**

**Main reagents**

| Name | Supplier and Cat No. |
|---|---|
| Phosphate buffer solution | Corning, 21031082 |
| 96-well microplate | Corning, 9018 |
| ADC-capturing antibody | Sino Biological# 40017-VNAHC |
| ELISA coating solution | Biolegend-421701 |
| TWEEN-20 | Sigma-P1379 |
| BSA | Beyotime ST023-50g |
| Peroxidase AffiniPure $F(ab')_2$ Fragment Goat Anti-Human IgG, Fcγ fragment specific | Jackson Immuno Research 709-036-098 |
| SureBlue™ TMB 1-Component Microwell Peroxidase Substrate | SeraCare 5120-0077 |
| Hydrochloric acid | Kunshan Rier Chemical Co., Ltd- |
| | 20230406 |
| Urea Assay kit (Colorimetric) | Abcam /ab83362 |

**Main instrument**

| Instrument name | Brand | Model |
|---|---|---|
| Analytical balance | Mettler Toledo | XSE205DU |
| Electronic balance | Changzhou Keyuan | YH-2000 |
| Level 2 Biosafety Cabinet | Suzhou Antai | BSC-1604IIA2 |
| Centrifuge | Eppendorf | 5427R |
| Microplate Reader | Molecular Devices | SpectraMax iD3 |
| Microplate mixer | Thermo Fisher | 88882006 |

### 2. Experimental methods and procedures

1) The day of the first drug administration was defined as Day 0 of the experiment, with the day before as Day -1 and the day after as Day 1, and so on;

2) **Virus inoculation:** On day 28, mice were deeply anesthetized via intraperitoneal injection with Zoletil 50 (30 mg/kg) and Xylazine hydrochloride (6 mg/kg) (injection volume 6 mL/kg). The virus was then inoculated via nasal drip, with an inoculation dose of 500 PFU per mouse and an inoculation volume of 50 $\mu$L.

3) **Grouping:** According to the experimental design, the animals were randomly divided into 11 groups, with 10 mice in each group. Adjustments were made based on the animal weights to ensure that there was no statistical difference in the average weight between groups.

4) **Drug .administration:** Except for the healthy control group (Group 1), mice in the remaining groups were treated with either vehicle or test substance via subcutaneous injection. The administration time was 28 days before virus inoculation, with a single dose.

### In vivo experimental design - dosing regimen

<table>
<tr><th rowspan="2">Group</th><th rowspan="2">Test compound</th><th colspan="5">Dosing regimen</th></tr>
<tr><th>Dosage (mg/kg/dose)</th><th>Dosing volume (mL/kg)</th><th>Mode of administration</th><th>Dosing frequency</th><th>Time of first administration</th></tr>
<tr><td>1</td><td>NA</td><td colspan="5">NA</td></tr>
<tr><td>2</td><td>Vehicle</td><td>0</td><td rowspan="6">10</td><td rowspan="6">Subcutaneous injection</td><td rowspan="6">Single-dose administration</td><td rowspan="6">Day 0</td></tr>
<tr><td>3</td><td>Fc (LS) (SEQ ID No:71)</td><td>2.5</td></tr>
<tr><td>4</td><td>Conjugate 4</td><td>2.5</td></tr>
<tr><td>5</td><td>Conjugate 4</td><td>0.5</td></tr>
<tr><td>6</td><td>Conjugate 4</td><td>0.1</td></tr>
<tr><td>7</td><td>Reference conjugate*</td><td>0.5</td></tr>
<tr><td colspan="7">*Reference conjugate: Prepared according to the synthesis and characterization of conjugate 9 in Example 3 of patent WO2023/125806A1.</td></tr>
</table>

**5) Sample collection:** For mice of groups 4-9, blood was collected from three mice in each group at 48, 336, 672, 885, or 1008 hours after administration to collect serum samples. The collected whole blood samples were incubated at 4°C for 8 hours, and then centrifuged at 3000 g for 10 min at 4 °C to separate the serum. Lung tissues and bronchoalveolar lavage fluid (BALF) were collected from groups 4 and 7 at 1008 hours after administration. All samples were stored at -80 °C in refrigerator.

**6) Health monitoring:** During the experiment, mice were weighed three times per week from day 0 to day 27, and once per day from day 28 to day 42. Their states were observed, and their body weight was recorded.

**7) Median survival time:** The survival time corresponding to a cumulative survival rate of 50%.

**8) Humane endpoint:** According to the IACUC protocol, during the experiment, any mouse that loses more than 35% (inclusive) of its initial body weight, or/and exhibits symptoms of imminent death, would be euthanized and recorded as a dead animal in the results. In actual experimental operations, the above criteria were followed.

### 3. Sample analysis

**ELISA experiment to detect the concentration of test substance in the sample:** 1) ADC-capturing antibody was diluted with the coating solution at a ratio of 1:400, and used to coat the 96-well plate at 100 μL/well overnight at 4 °C; 2) The liquid in the microplate was discarded, and the plate was washed three times with PBST (300 μL/well); 3) 200 μL of PBST containing 5% BSA was added to each well, and the plate was blocked at room temperature for 2 h; 4) The liquid in the microplate was discarded, and then the plate was washed three times with PBST (300 μL/well); 5) 100 μL of the sample to be tested was added to each well and incubated at room temperature for 2 h; 6) The liquid in the microplate was discarded, and then the plate was washed three times with PBST (300 μL/well); 7) 100 μL of HRP (see Example 24) (diluted in 1:20000) was added to each well, and the microplate was incubated at room temperature for 40 min; 8) The liquid in the microplate was discarded, and the plate was washed three times with PBST (300 μL/well); 9) 100 μL of TMB chromogenic substrate was added to each well, and then the plate was incubated in the dark at room temperature for 5-10 min. When the solution turned noticeably blue, 100 μL of stop solution was added to each well to terminate coloration; 10) The absorbance was measured at dual wavelengths of 450/620 nm using a microplate reader, and a standard curve was fitted using a four-parameter method. The content of the test substance in the sample was determined using the above method.

### 4. Data analysis

The data were statistically analyzed using Prism, and the survival status of mice was analyzed using the Log-rank (Mantel-Cox) test.

### 5. Results

The *in vivo* efficacy of the test compound in a mouse model of influenza A virus infection was evaluated based on changes in mouse body weight and survival rate. The pharmacokinetic characteristics of the test compound were analyzed by detecting its concentration in serum, lung tissue, and the lining fluid of lung epithelial cells. The tolerance of the test compound in mice was assessed by monitoring animal body weight.

1) The protective effect of the test substance on the body weight of mice in the model

**Healthy group:** During the experiment, the body weight of the healthy control group remained stable, with no significant decrease;

**Vehicle group:** The body weight of mice began to significantly decrease from day 31, and continued to decline until death or euthanasia, with an average maximum decrease of 37.77% (euthanasia was performed);

**hIgG1-Fc(LS) (2.5 mg/kg) group:** The body weight of mice began to significantly decrease from day 31 in the experiment, with an average maximum decrease of 36.08%. The body weight of the surviving mice began to recover from day 39;

**Conjugate 4 (2.5 mg/kg) group:** The body weight of mice began to significantly decrease from day 31 in the experiment, with an average maximum decrease of 16.83%. The body weight of the surviving mice began to recover from day 36 until it reached normal levels;

**Conjugate 4 (0.5 mg/kg) group:** The body weight of mice began to significantly decrease from day 31 in the experiment, with an average maximum decrease of 34.58%. The body weight of the surviving mice began to recover from day 38;

**Conjugate 4 (0.1 mg/kg) group:** The body weight of mice began to decrease significantly from day 31, and continued to decline until death or euthanasia, with an average maximum decrease of 33.00%;

**Reference conjugate (0.5 mg/kg) group:** The body weight of mice began to significantly decrease from day 31, and continued to decline until death or euthanasia, with an average maximum decrease of 37.03% (euthanasia was performed);

**2) The protective effect of the test substance on the survival of mice in the model**

After virus inoculation, the survival status of mice in each group was summarized as follows:
**Healthy group:** No mice died, with a final animal survival rate of 100% and a median survival time of >42 days;
**Vehicle group:** Mice began to die on day 35 and all died on day 37, with a final survival rate of 0% and a median survival time of 36 days;
**hIgG-Fc(LS)(2.5 mg/kg) group:** Mice began to die from day 36, with a final survival rate of 10% and a median survival time of 36.5 days;
**Conjugate 4 (2.5 mg/kg) group:** Mice began to die on day 38, with a final survival rate of 90% and a median survival time >42 days;
**Conjugate 4 (0.5 mg/kg) group:** Mice began to die from day 36, with a final survival rate of 50% and a median survival time of 39.5 days;
**Conjugate 4 (0.1 mg/kg) group:** Mice began to die from day 34 and all died by day 37, with a final survival rate of 0% and a median survival time of 36 days;
**Reference conjugate (0.5 mg/kg) group:** Mice began to die from day 36 and all died by day 38, with a final survival rate of 0% and a median survival time of 37 days;

| Group | Median survival time (days) | Prolonged # survival (%) | Survival rate (%) | Log-rank (Mantel-cox) test |
|---|---|---|---|---|
| | | | | # P value# |
| Group 1<br>Healthy control | Unknown | ∞ | 100 | ****, P< 0.0001 |
| Group 2<br>Vehicle | 36 | NA | 0 | NA |
| Group 3<br>Fc (LS), 2.5 mg/kg | 36.5 | 1.4 | 10 | NS, P=0.0551 |
| Group 4<br>Conjugate 4, 2.5 mg/kg | Unknown | ∞ | 90 | ****, P< 0.0001 |
| Group 5<br>Conjugate 4, 0.5 mg/kg | 39.5 | 9.7 | 50 | **, P=0.0017 |
| Group 6<br>Conjugate 4, 0.1 mg/kg | 36 | 0 | 0 | NS, P=0.4109 |
| Group 7<br>Reference conjugate, 0.5 mg/kg | 37 | 2.7 | 0 | *, P=0.0110 |
| Statistical method: Log-rank (Mantel-Cox) test; #, compared with the vehicle group; NS, P > 0.05; *, P < 0.05; **, P < 0.01; ***, P < 0.001, ***, P < 0.001. | | | | |

From the analysis of the results, it can be seen that Conjugate 4, obtained by the thiol site-specific conjugation technology described in this patent, exhibited excellent antiviral activity in a mouse model *in vivo.* At a high dose (2.5 mg/kg), the survival rate of mice reached 90%. When compared with the reference conjugate, at a dose of 0.5 mg/kg, the survival rate of mice treated with Conjugate 4 reached 50%, while the survival rate of mice treated with the reference conjugate molecule reached 0%. It could be found that Conjugate 4 demonstrated superior antiviral activity, indicating better clinical efficacy in the prevention and/or treatment of influenza in clinical settings.

The present invention has been illustrated by various specific examples. However, those skilled in the art would appreciate that the present invention was not limited to these specific examples. Those skilled in the art could make various modifications or variations within the scope of the invention, and the various technical features mentioned in this specification could be combined with each other, without departing from the spirit and scope of the present invention. Such modifications and variations were all within the scope of the invention.

## Claims

1. A conjugate represented by structure of Formula I-A or Formula I-B, or a pharmaceutically acceptable salt, ester, isomer, solvate, prodrug, or isotopically labeled compound thereof:

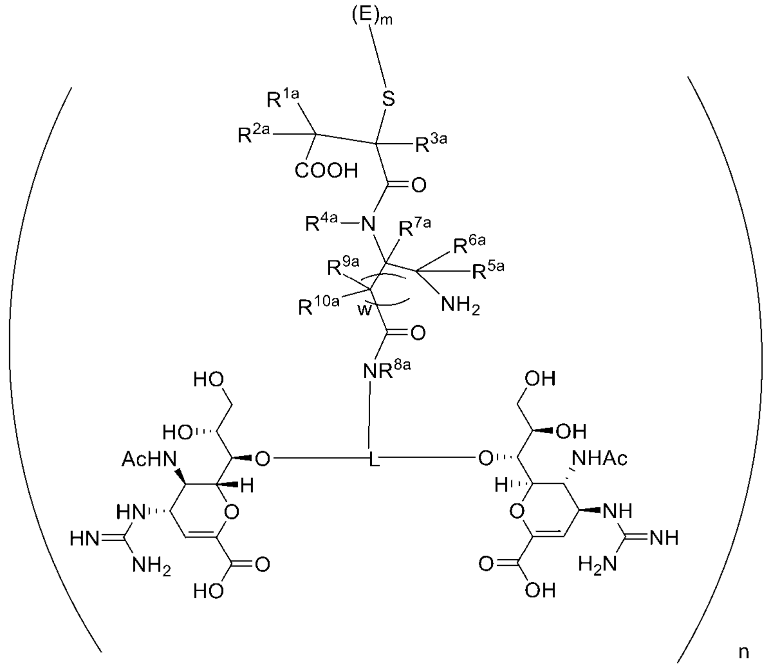

;

Formula I-A

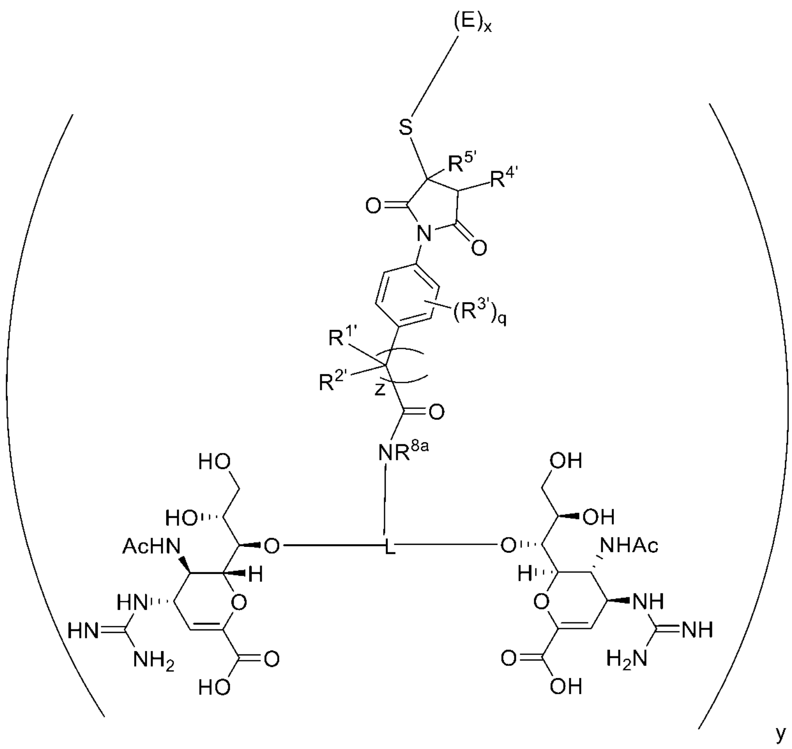

Formula I-B

wherein,

m is 1 or 2;
n is an integer from 1 to 20, and preferably an integer of 1, 2, 3, 4, 5, 6, 7, or 8;
w is an integer from 0 to 8; for example, 0, 1, 2, 3, 4, or 5;
L is a linker;
E is selected from an antibody or antibody fragment, Fc domain monomer, Fc domain, Fc binding peptide, albumin, or albumin-binding peptide;
each of $R^{1a}$, $R^{2a}$, $R^{3a}$, $R^{5a}$, $R^{6a}$, $R^{7a}$, $R^{9a}$ and $R^{10a}$ is independently selected from hydrogen, deuterium, hydroxyl, -O-$C_{1-6}$ alkyl, hydroxyl$C_{1-6}$ alkyl-, $C_{1-6}$ alkyl-, $C_{3-8}$ cycloalkyl-, $C_{3-8}$ heterocycloalkyl-, $C_6$-$C_{15}$ aryl, 5 to 15-membered heteroaryl, halogen, cyano, and amino; $R^{1a}$ and $R^{2a}$ may together form $C_{3-8}$ cycloalkyl or $C_{3-8}$ heterocycloalkyl;
or $R^{5a}$ and $R^{6a}$ may together form $C_{3-8}$ cycloalkyl or $C_{3-8}$ heterocycloalkyl;
or $R^{9a}$ and $R^{10a}$ may together form $C_{3-8}$ cycloalkyl or $C_{3-8}$ heterocycloalkyl, wherein said $C_{3-8}$ cycloalkyl or $C_{3-8}$ heterocycloalkyl may be optionally substituted with a substituent selected from halogen and hydroxyl;
each of $R^{4a}$ and $R^{8a}$ is independently selected from H, $C_{1-6}$ alkyl, $C_{3-8}$ cycloalkyl or $C_{3-8}$ heterocycloalkyl;
x is 1 or 2;
y is an integer from 1 to 20, and preferably an integer of 1, 2, 3, 4, 5, 6, 7, or 8;
z is an integer from 0 to 8; for example, 0, 1, 2, 3, 4, or 5;
q is 0, 1, 2, 3 or 4;
each of $R^{1'}$, $R^{2'}$, $R^{3'}$, $R^{4'}$ and $R^{5'}$ is independently selected from hydrogen, deuterium, hydroxyl, -O-$C_{1-6}$ alkyl, hydroxyl$C_{1-6}$ alkyl, $C_{1-6}$ alkyl, $C_{3-8}$ cycloalkyl, $C_{3-8}$ heterocycloalkyl, $C_6$-$C_{15}$ aryl, 5 to 15-membered heteroaryl, halogen, cyano, and amino; or $R^{1'}$ and $R^{2'}$ may together form $C_{3-8}$ cycloalkyl or $C_{3-8}$ heterocycloalkyl; or, two adjacent $R^{3'}$ may form $C_{3-8}$ cycloalkyl, $C_{3-8}$ heterocycloalkyl, $C_6$-$C_{15}$ aryl, or 5 to 15-membered heteroaryl.

**2.** The conjugate according to claim 1, or the pharmaceutically acceptable salt, ester, isomer, solvate, prodrug, or isotopically labeled compound thereof, wherein L is a linker represented by the structure of formula I-3a, comprising $L^1$ and $L^{2a}$, the left and right ends of $L^1$ are linked to the drug moiety, and the upper end of $L^{2a}$ is linked to - $NR^{8a}$,

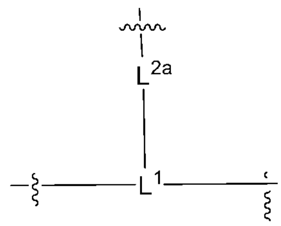

Formula I-3a

wherein,
$L^1$ is selected from the following structure:

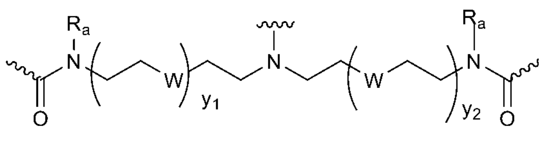

wherein,

W is selected from O, S, $NR^b$, $CH_{2-}$ or absence;
each of $R^a$ and $R^b$ is independently selected from H, optionally substituted $C_1$-$C_{20}$ alkyl, and optionally substituted $C_2$-$C_{20}$ alkenyl; and preferably selected from H and methyl, and more preferably methyl;
each of $y_1$ and $y_2$ is independently 0, 1, 2, 3, 4, 5, or 6;
preferably, $L^1$ is selected from the following structures:

| Structure of $L^1$ |
| --- |
| 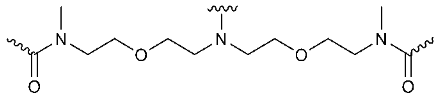 |
| 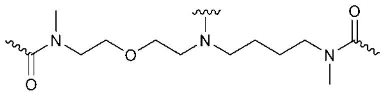 |
| 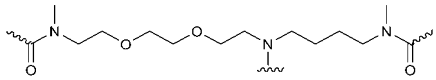 |
| 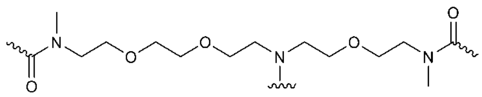 |
| 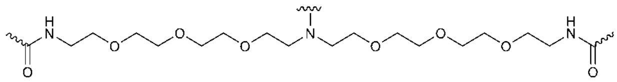 |
| 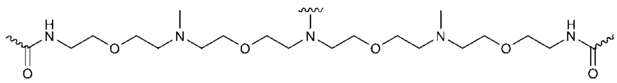 |
| 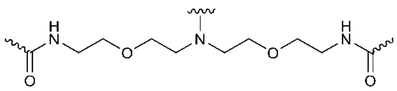 |
| 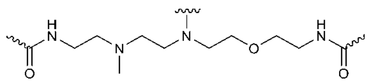 |
| 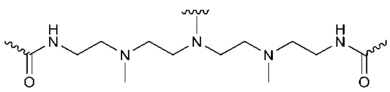 |
| 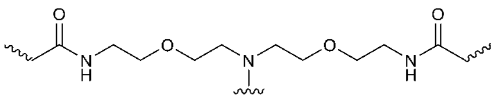 |
| 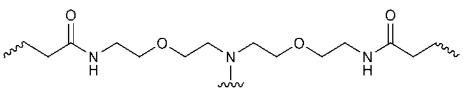 |
| 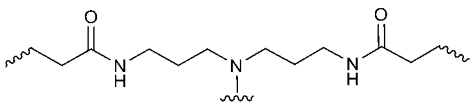 |
| 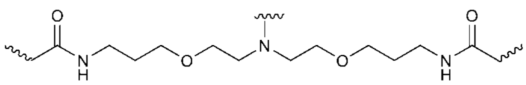 |
| 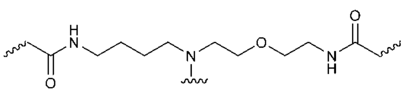 |
| 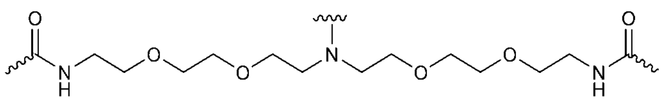 |

(continued)

| Structure of $L^1$ |
|---|
| 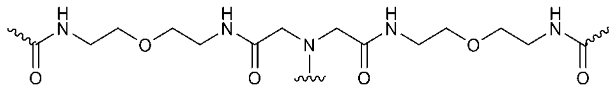 |
| 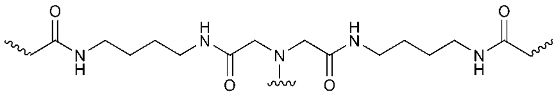 |
| 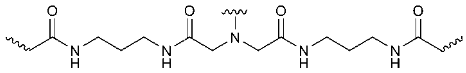 |
| 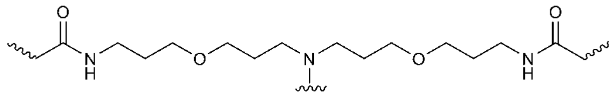 |
| 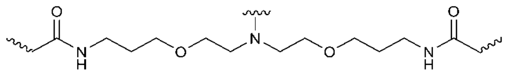 |
| 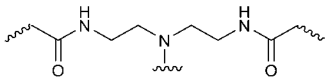 |
| 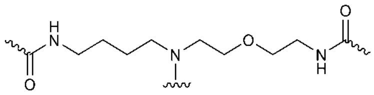 |
| 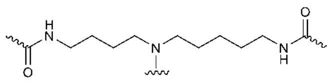 |
| 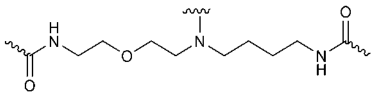 |
| 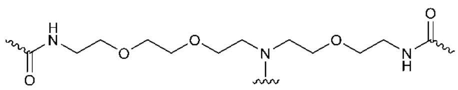 |

**3.** The conjugate according to claim 1 or 2, or the pharmaceutically acceptable salt, ester, isomer, solvate, prodrug, or isotopically labeled compound thereof, wherein $L^{2a}$ is of the structure of formulas L2-1a to L2-7a:

| $L^{2a}$ structure No. | Structure |
|---|---|
| Formula L2-1a | 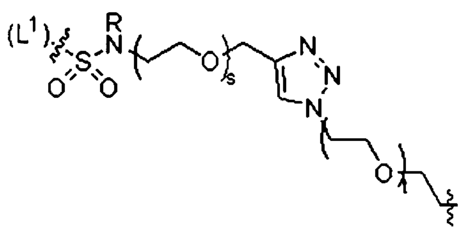 |

(continued)

| $L^{2a}$ structure No. | Structure |
|---|---|
| Formula L2-2a | 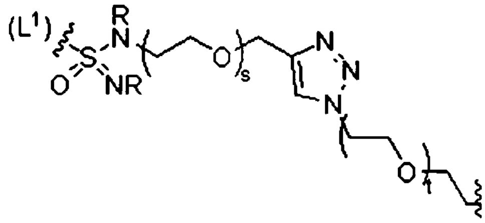 |
| Formula L2-3a | 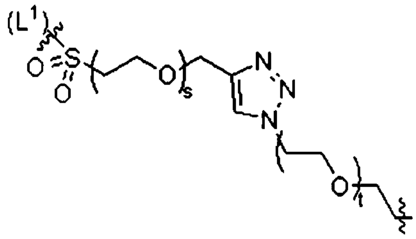 |
| Formula L2-4a | 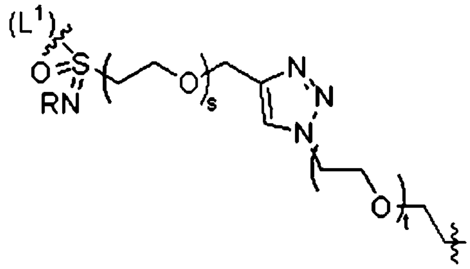 |
| Formula L2-5a | 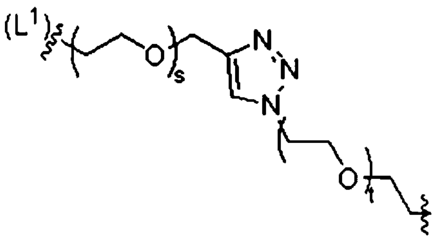 |
| Formula L2-6a | 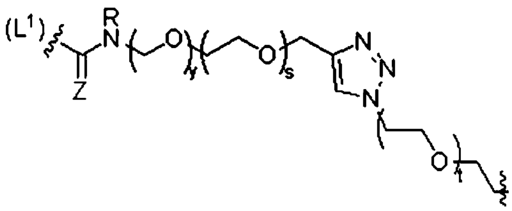 |
| Formula L2-7a | 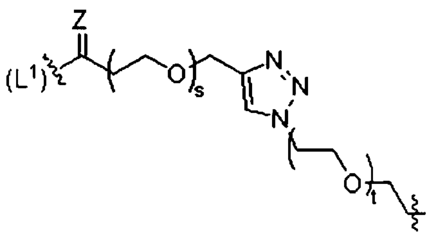 |

wherein,

Z is selected from NR, S and O;

R groups are each independently selected from hydrogen, deuterium, optionally substituted $C_1$-$C_{20}$ alkyl, optionally substituted $C_2$-$C_{20}$ alkenyl, optionally substituted $C_3$-$C_{20}$ cycloalkyl, optionally substituted 3 to 20-membered heterocycloalkyl, optionally substituted $C_6$-$C_{15}$ aryl, and optionally substituted 5 to 15-membered heteroaryl;
s and t are integers from 1 to 20; for example, 1, 2, 3, 4, 5, 6, 7, 8, 9, 10, 11, or 12; and
y is 0 or 1.

**4.** The conjugate according to any one of claims 1 to 3, or the pharmaceutically acceptable salt, ester, isomer, solvate, prodrug, or isotopically labeled compound thereof, wherein $L^{2a}$ is selected from Formula L2-1a, L2-5a, and L2-7a,

Z is O,
R is H,
s and t are integers selected from 3 to 10, preferably, s and t are 4 or 8, respectively.

**5.** The conjugate according to any one of claims 1 to 4, or the pharmaceutically acceptable salt, ester, isomer, solvate, prodrug, or isotopically labeled compound thereof,

wherein the S atom on E comes from the cysteine (cys) residue or disulfide bond in E;
preferably, E is an antibody or an antibody fragment thereof, a Fc domain monomer, a Fc domain, a Fc binding peptide, albumin, or an albumin-binding peptide, for example, the Fc domain monomer comprises or consists of any of the amino acid sequences set forth in SEQ ID Nos. 1-81, or an amino acid sequence having at least 95%, 96%, 97%, 98%, or 99% identity thereto;
preferably, it comprises or consists of an amino acid sequence selected from any one of SEQ ID NO: 9, SEQ ID NO: 10, SEQ ID NO: 11, SEQ ID NO: 33, SEQ ID NO: 36, SEQ ID NO: 37, SEQ ID NO: 38, SEQ ID NO: 41, SEQ ID NO: 42, SEQ ID NO: 43, SEQ ID NO: 44, SEQ ID NO: 45, SEQ ID NO: 46, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 59, SEQ ID NO: 65, SEQ ID NO: 66, SEQ ID NO: 67, SEQ ID NO: 68, SEQ ID NO: 69, SEQ ID NO: 70, SEQ ID NO: 71, and SEQ ID NO: 72.

**6.** The conjugate according to any one of claims 1 to 5, or the pharmaceutically acceptable salt, ester, isomer, solvate, prodrug, or isotopically labeled compound thereof, wherein the conjugate is of a structure of formula I-A.

**7.** The conjugate according to any one of claims 1 to 5, or the pharmaceutically acceptable salt, ester, isomer, solvate, prodrug, or isotopically labeled compound thereof, wherein the conjugate is of a structure of formula I-B.

**8.** The conjugate according to claim 6, or the pharmaceutically acceptable salt, ester, isomer, solvate, prodrug, or isotopically labeled compound thereof, wherein the structure between L and E is selected from the following structure:

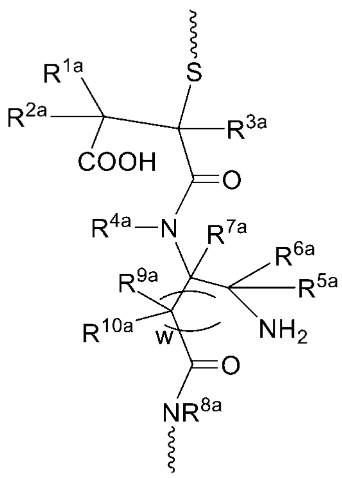

Formula I-4a

wherein, each of $R^{1a}$, $R^{2a}$, $R^{3a}$, $R^{5a}$, $R^{6a}$, $R^{7a}$, $R^{9a}$ and $R^{10a}$ is independently selected from hydrogen, deuterium, -O-$C_{1-6}$ alkyl, hydroxyl$C_{1-6}$ alkyl-, $C_{1-6}$ alkyl- and $C_{3-8}$ cycloalkyl-; or, $R^{1a}$ and $R^{2a}$ may together form $C_{3-8}$ cycloalkyl;
or, $R^{5a}$ and $R^{6a}$ may together form $C_{3-8}$ cycloalkyl;
or, $R^{9a}$ and $R^{10a}$ may together form $C_{3-8}$ cycloalkyl, wherein said $C_{3-8}$ cycloalkyl is optionally substituted with a

substituent selected from halogen and hydroxyl;
each of $R^{4a}$ and $R^{8a}$ is independently selected from H, $C_{1-6}$ alkyl, and $C_{3-8}$ cycloalkyl; and
w is 0, 1 or 2;
preferably, each of $R^{1a}$, $R^{3a}$, $R^{4a}$, $R^{5a}$, $R^{7a}$, $R^{9a}$, and $R^{10a}$ is independently hydrogen;
each of $R^{2a}$, $R^{6a}$ and $R^{8a}$ is independently selected from hydrogen, $C_{1-6}$ alkyl, and $C_{3-8}$ cycloalkyl; and
w is 0;
more preferably, the structure of formula I-4a is selected from:

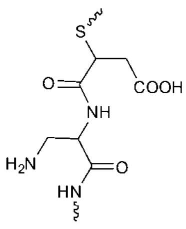 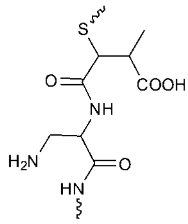 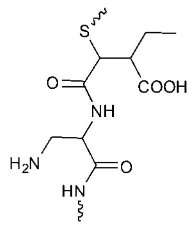 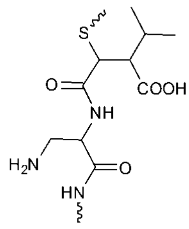 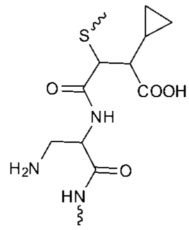

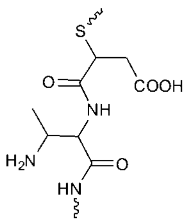 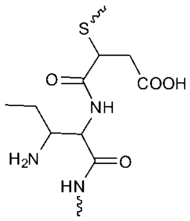 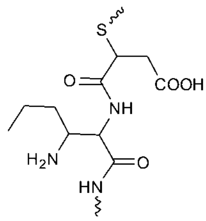 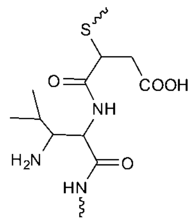 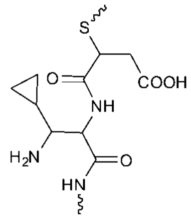

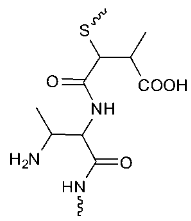 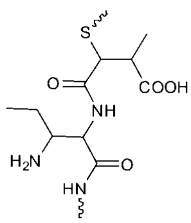 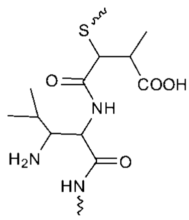 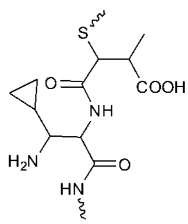 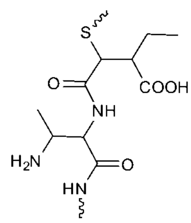

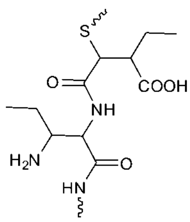 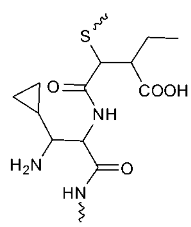 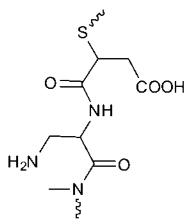 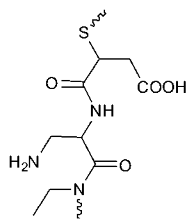 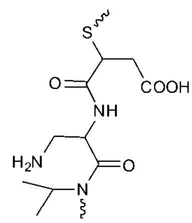

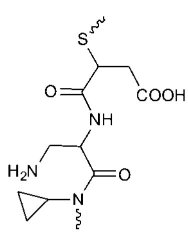 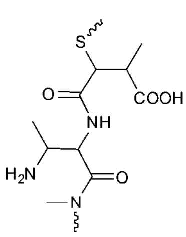 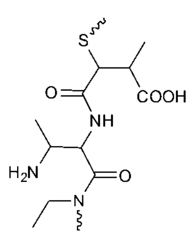 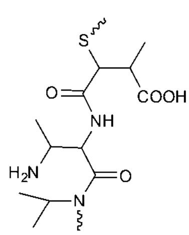 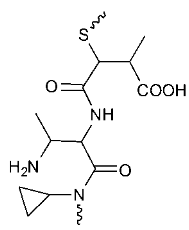
.

**9.** The conjugate according to any one of claims 1 to 5, or the pharmaceutically acceptable salt, ester, isomer, solvate, prodrug, or isotopically labeled compound thereof, characterized that the conjugate is selected from the compounds represented by the structures of formula C-1a to formula C-27a:

| No. | Structure of conjugate |
|---|---|
| C-1a | 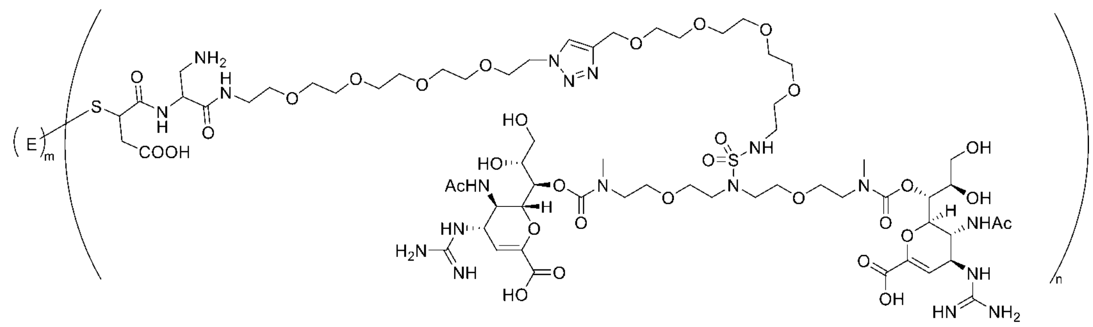 |
| C-2a | 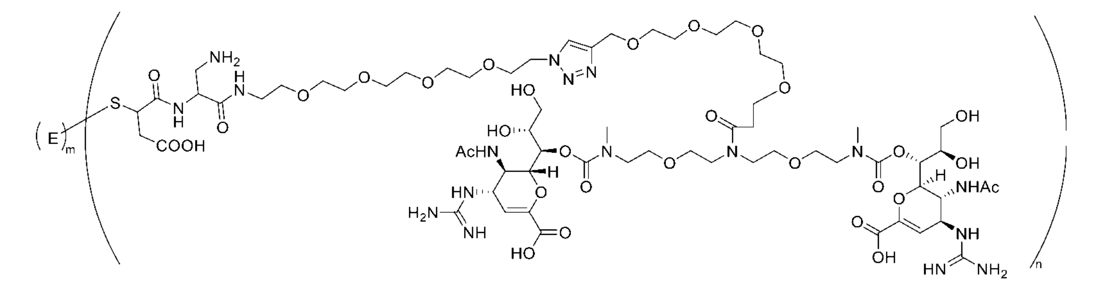 |
| C-3a | 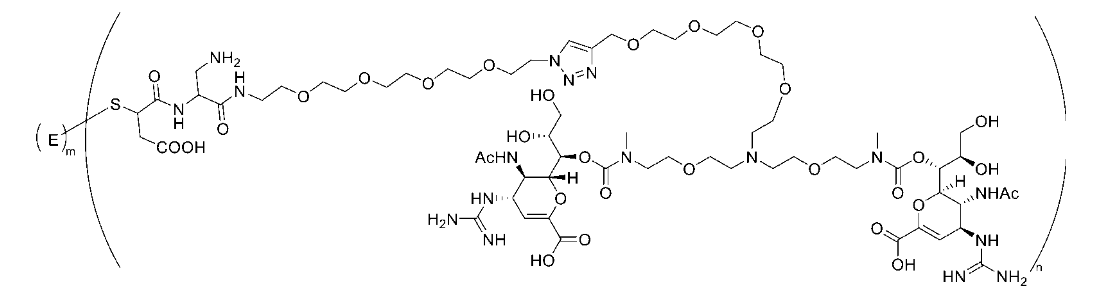 |
| C-4a | 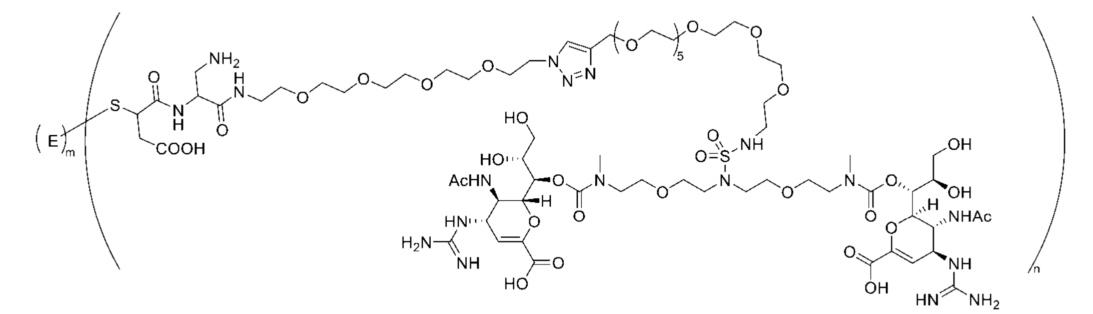 |
| C-5a | 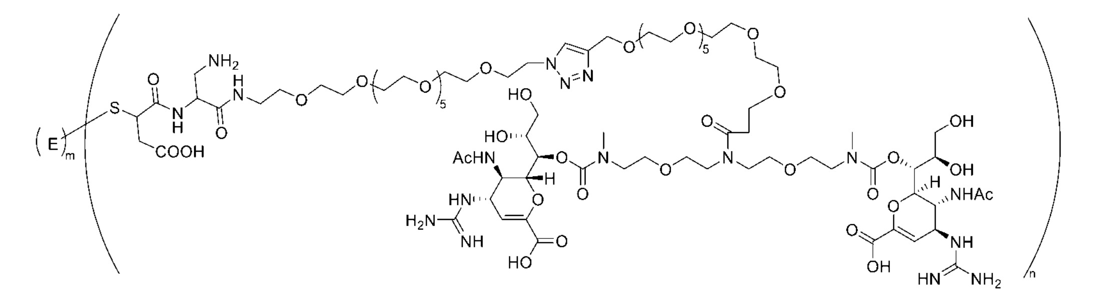 |

(continued)

| No. | Structure of conjugate |
|---|---|
| C-6a | 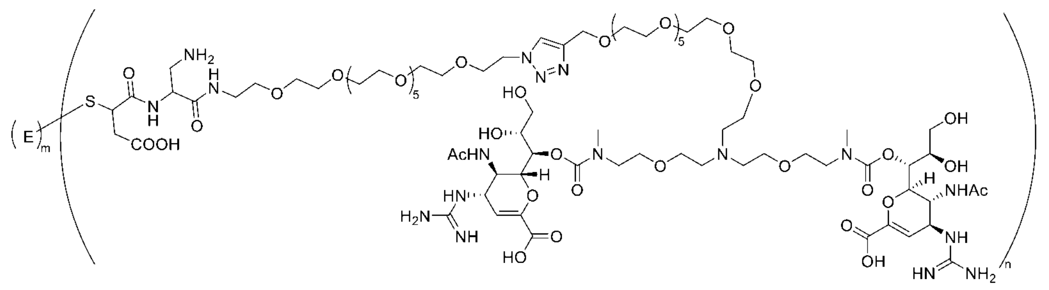 |
| C-7a | 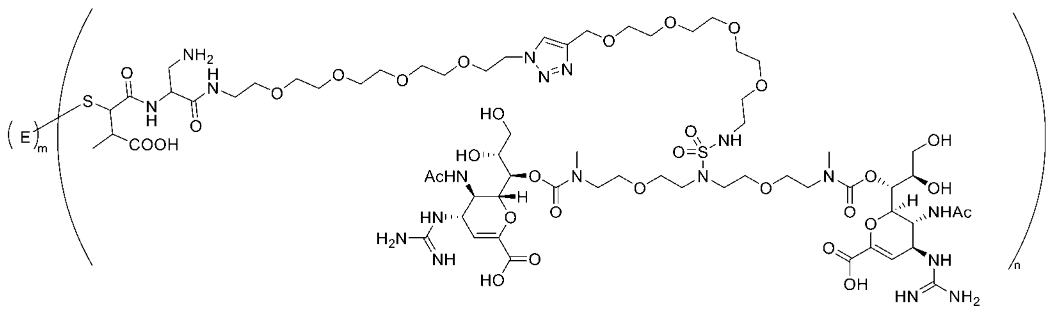 |
| C-8a | 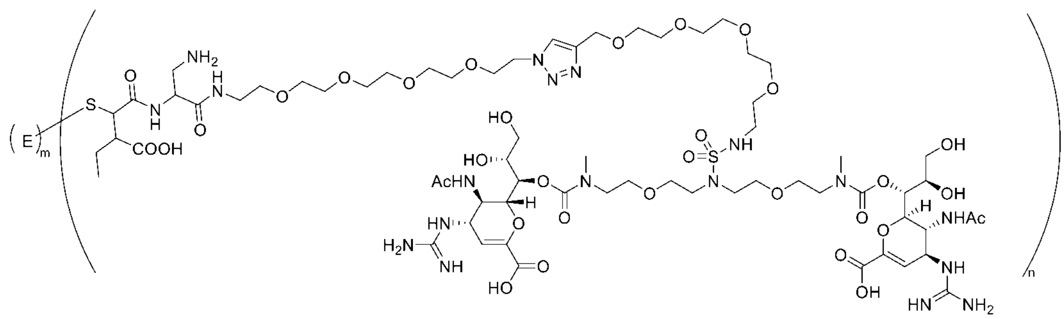 |
| C-9a | 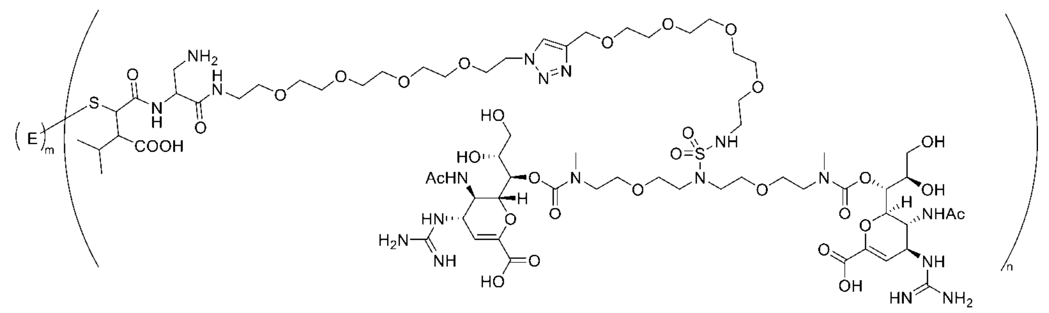 |

(continued)

| No. | Structure of conjugate |
|---|---|
| C-10a | 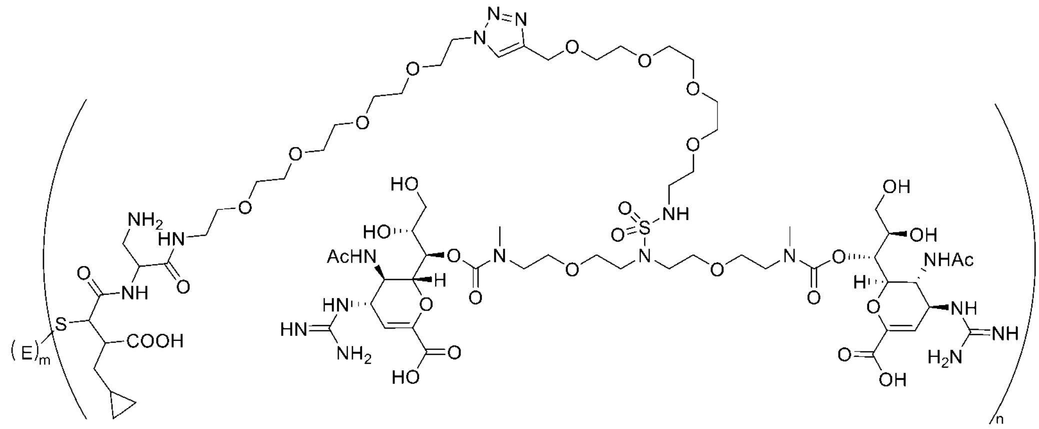 |
| C-11a | 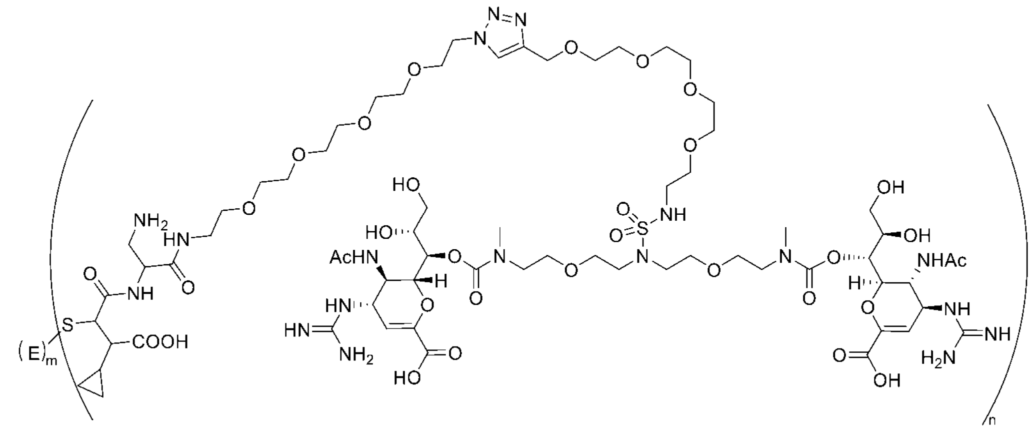 |
| C-12a | 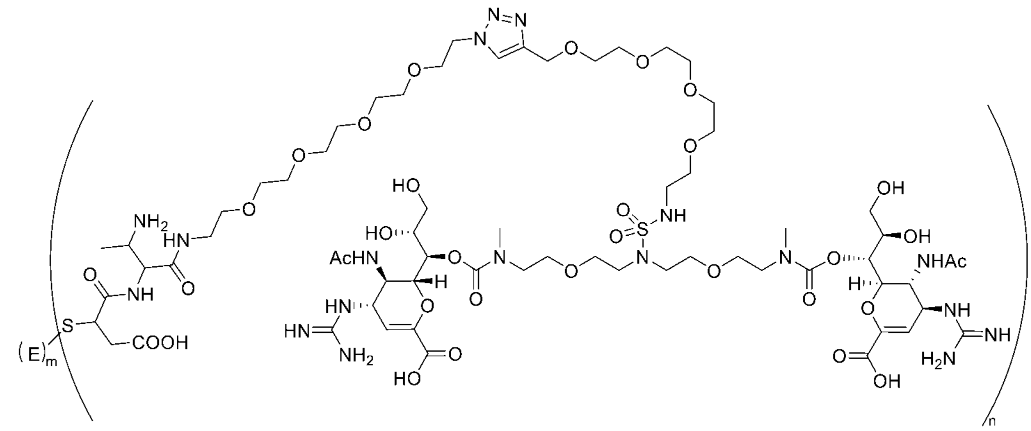 |

(continued)

| No. | Structure of conjugate |
| --- | --- |
| C-13a | 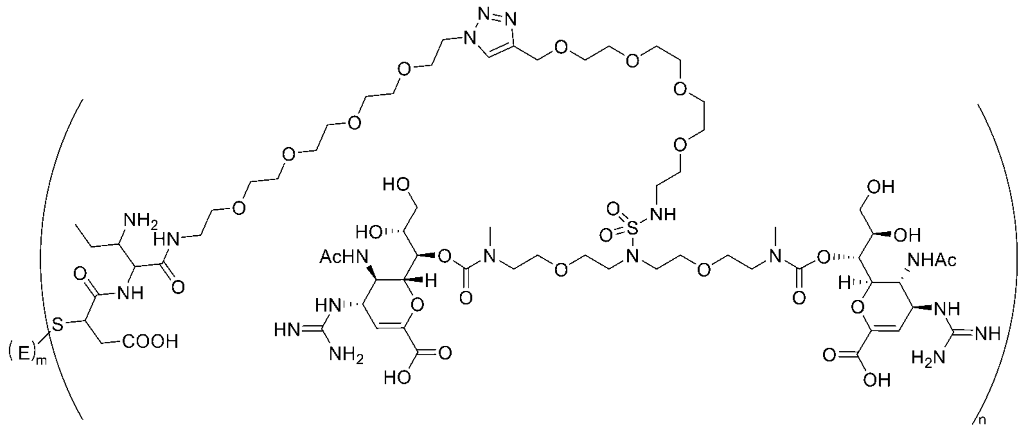 |
| C-14a | 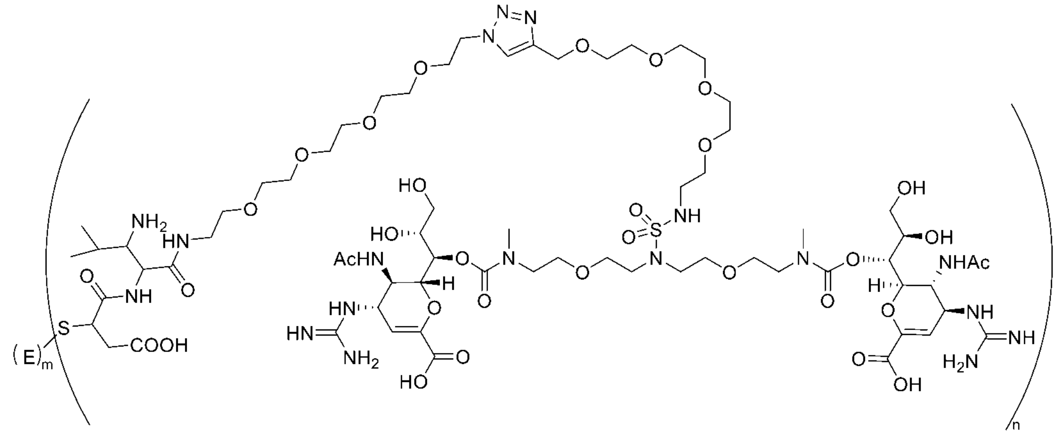 |
| C-15a | 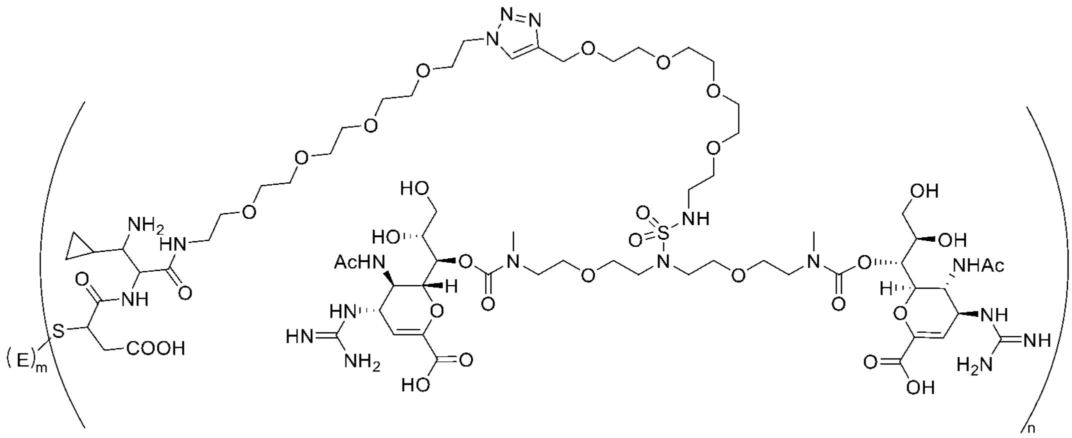 |

(continued)

| No. | Structure of conjugate |
|---|---|
| C-16a | 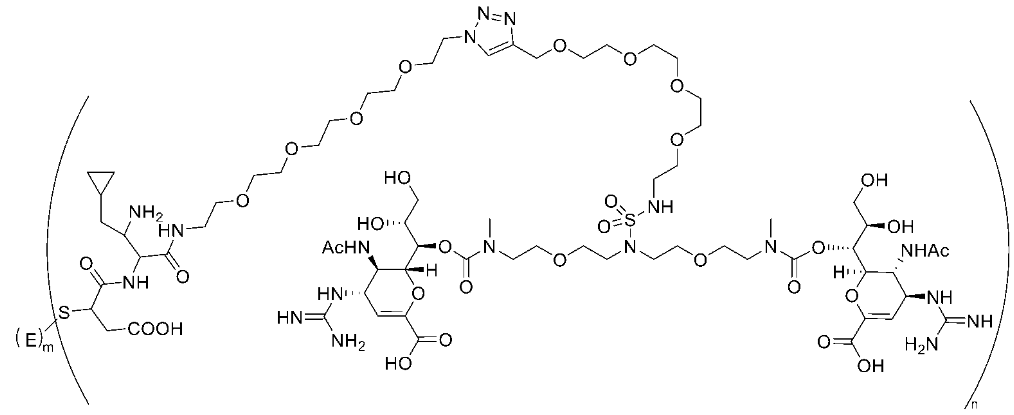 |
| C-17a | 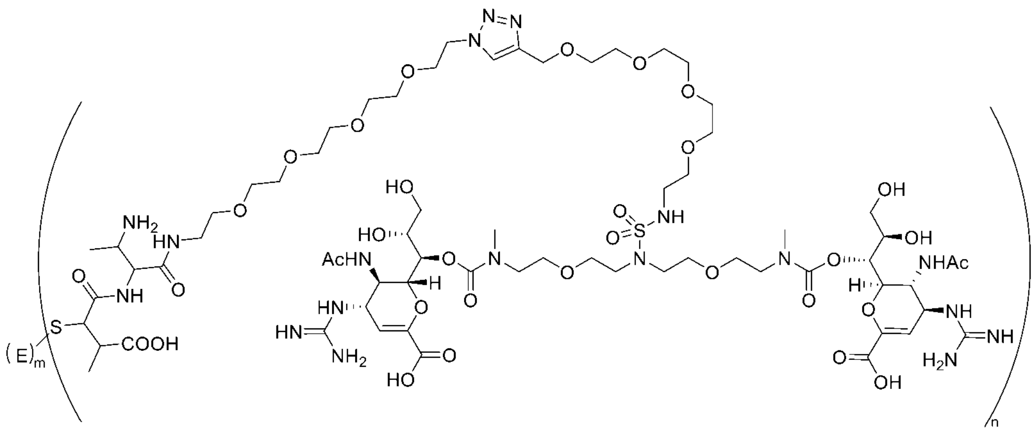 |
| C-18a | 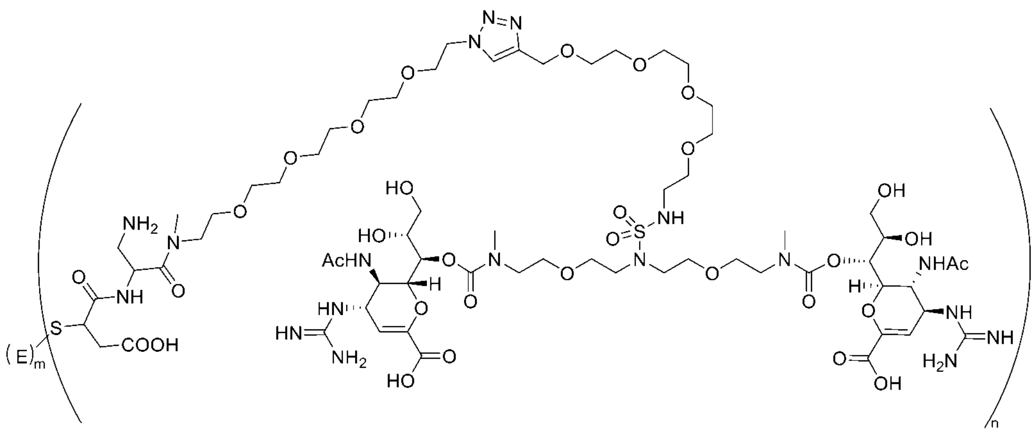 |

(continued)

| No. | Structure of conjugate |
|---|---|
| C-19a | 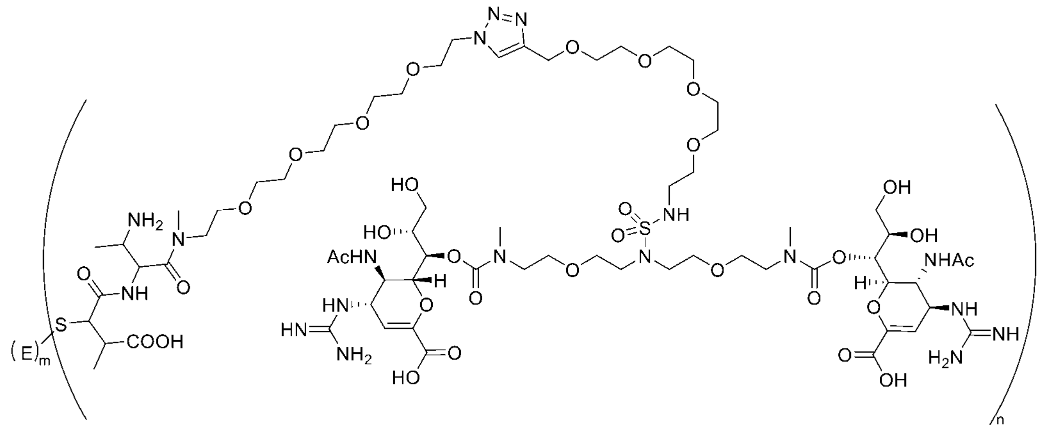 |
| C-20a | 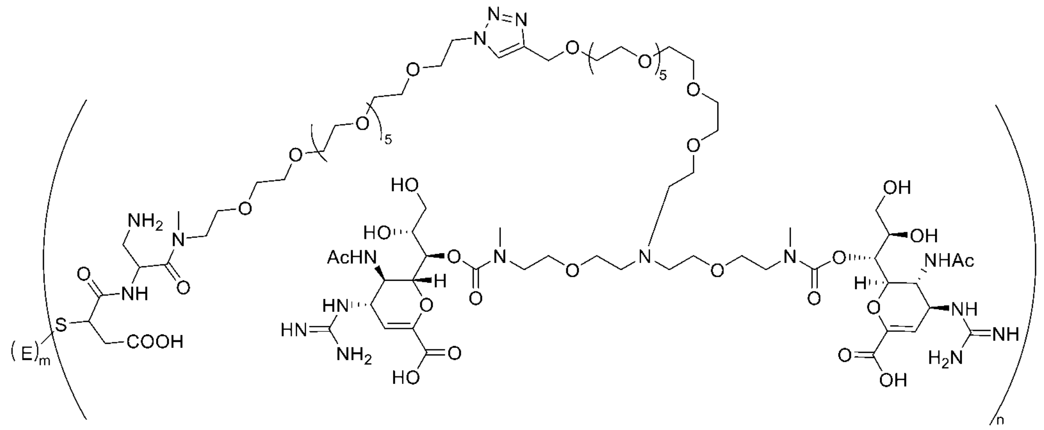 |
| C-21a | 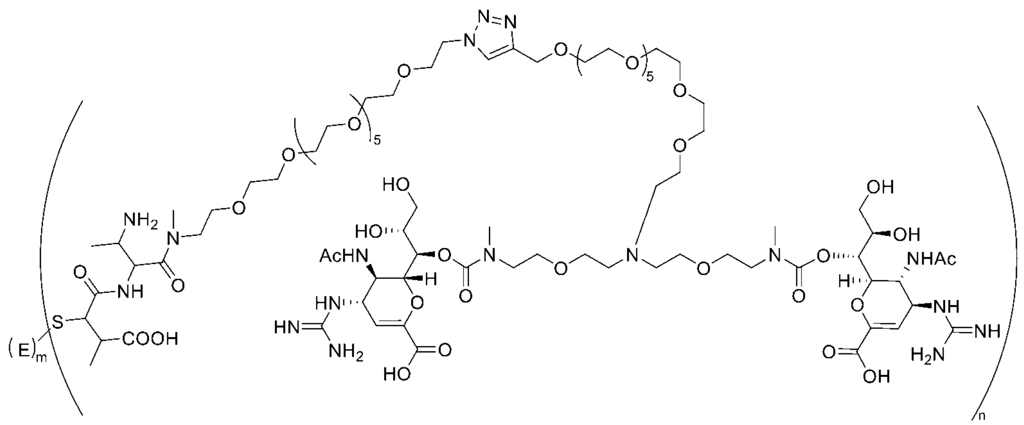 |

(continued)

| No. | Structure of conjugate |
|---|---|
| **C-22a** | 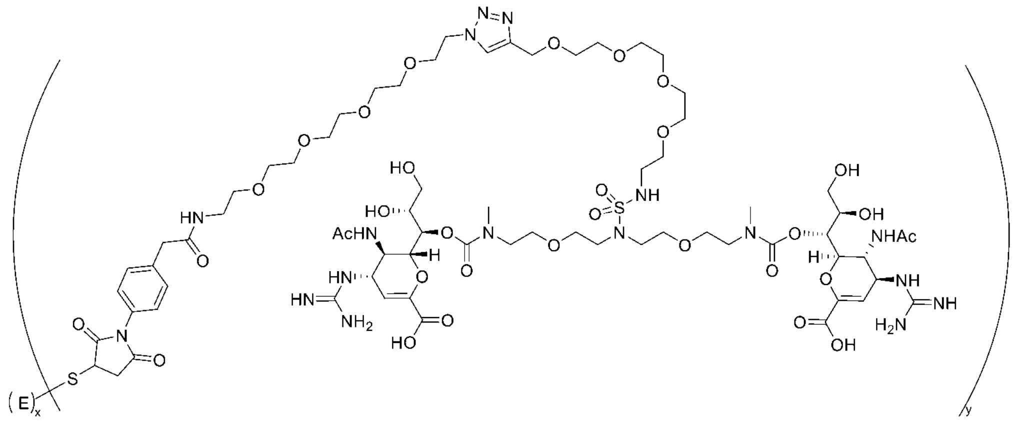 |
| **C-23a** | 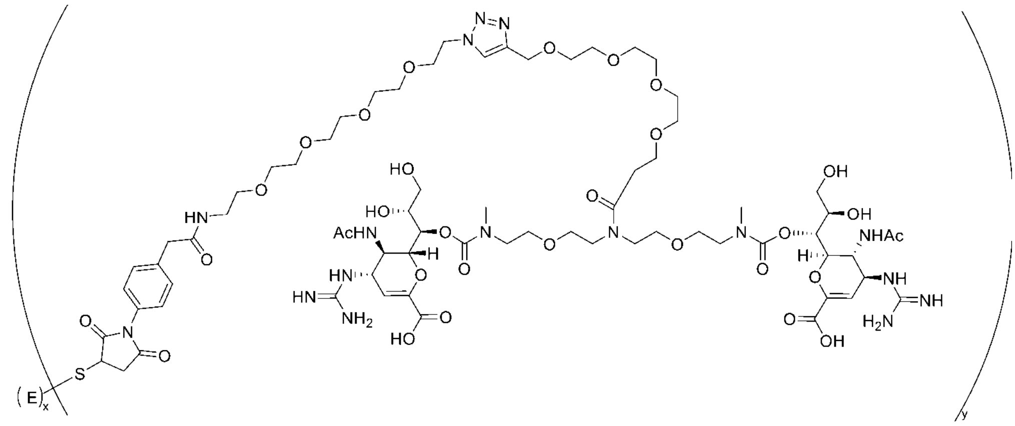 |
| **C-24a** | 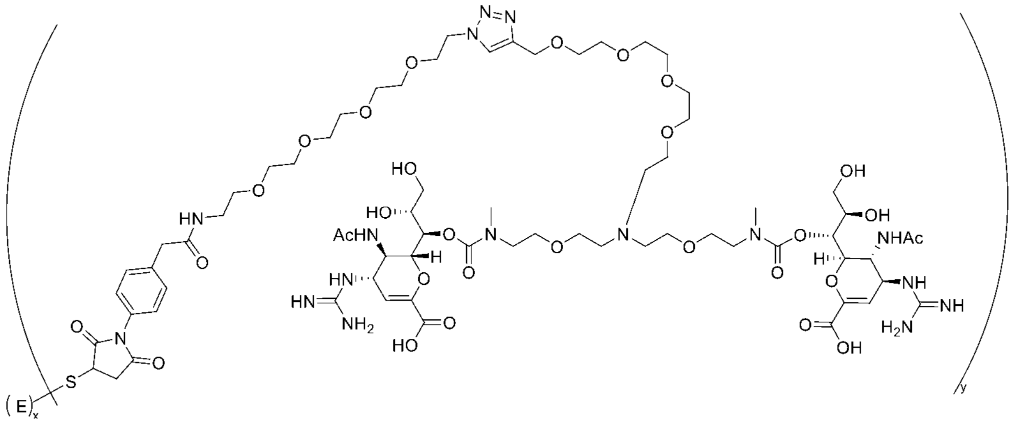 |

(continued)

| No. | Structure of conjugate |
|---|---|
| C-25a | 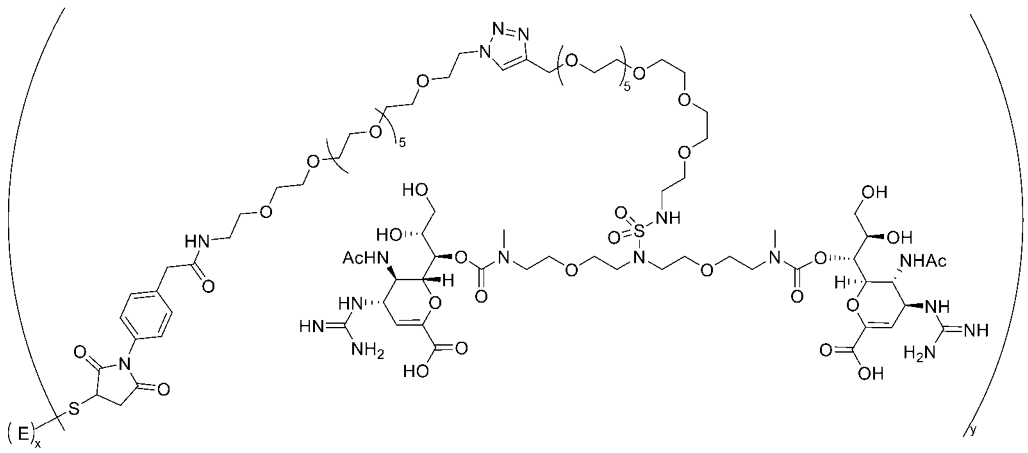 |
| C-26a | 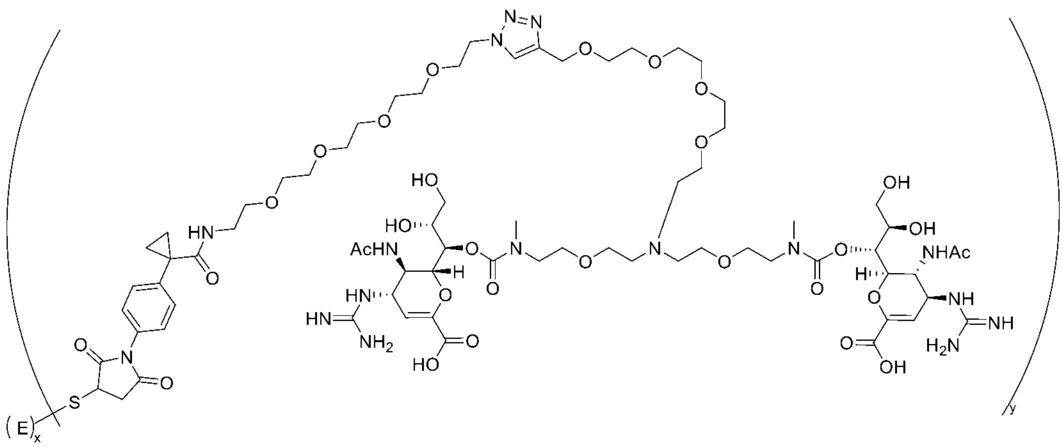 |
| C-27a | 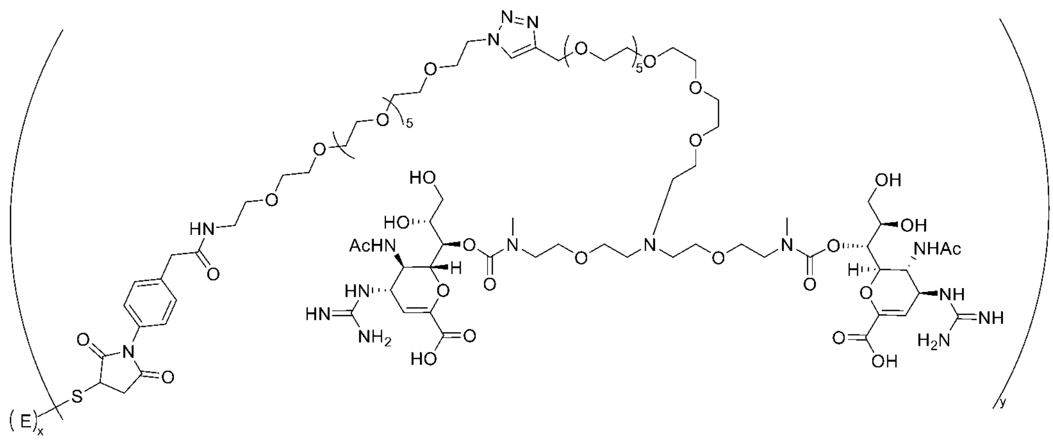 |

wherein, m, n, x, y, and E are as defined in any one of claims 1-5.

**10.** The conjugate according to any one of claims 1 to 9, or the pharmaceutically acceptable salt, ester, isomer, solvate, prodrug, or isotopically labeled compound thereof, wherein the ratio of n to m ranges from 1 to 20, preferably from 2 to 10, such as 2, 3, 4, 5, 6, 7, 8, 9, or 10.

**11.** The conjugate according to any one of claims 1 to 10, or the pharmaceutically acceptable salt, ester, isomer, solvate, prodrug, or isotopically labeled compound thereof, which has an average DAR value between 0.5 and 10.0, such as 2.0-4.5 or 4.8-8.5.

12. The conjugate according to any one of claims 1 to 11, or the pharmaceutically acceptable salt, ester, isomer, solvate, prodrug, or isotopically labeled compound thereof, wherein E comprises an Fc domain monomer or an Fc domain comprising said Fc domain monomer, wherein said Fc domain monomer comprises or consists of: any one of the amino acid sequences set forth in SEQ ID Nos. 1-81, or an amino acid sequence having at least 95%, 96%, 97%, 98% or 99% identity thereto.

13. The conjugate according to any one of claims 1 to 12, or the pharmaceutically acceptable salt, ester, isomer, solvate, prodrug, or isotopically labeled compound thereof, wherein E is capable of recognizing viral surface antigens, such as CR6261, CR8020, MEDI8897, Palivizumab, SD38, etc.; or
the Fc domain monomer may be an Fc domain monomer of any antibody subtype of immunoglobulin (e.g., IGHG1*01 (such as G1m(za)), IGHG1*07 (such as G1m(zax)), IGHG1*04 (such as G1m(zav)), IGHG1*03 (G1m(f)), IGHG1*08 (such as G1m(fa)), IGHG2*01, IGHG2*02, IGHG2*06, IGHG3*01, IGHG3*04, IGHG3*05, IGHG3*09, IGHG3*10, IGHG3*11, IGHG3*12, IGHG3*06, IGHG3*07, IGHG3*08, IGHG3*13, IGHG3*03, IGHG3*14, IGHG3*15, IGHG3*16, IGHG3*17, IGHG3*18, IGHG3*19, IGHG2*04, IGHG4*01, IGHG4*02, IGHG4*03).

14. The conjugate according to any one of claims 1 to 13, or the pharmaceutically acceptable salt, ester, isomer, solvate, prodrug, or isotopically labeled compound thereof, wherein the conjugate has the following structure:

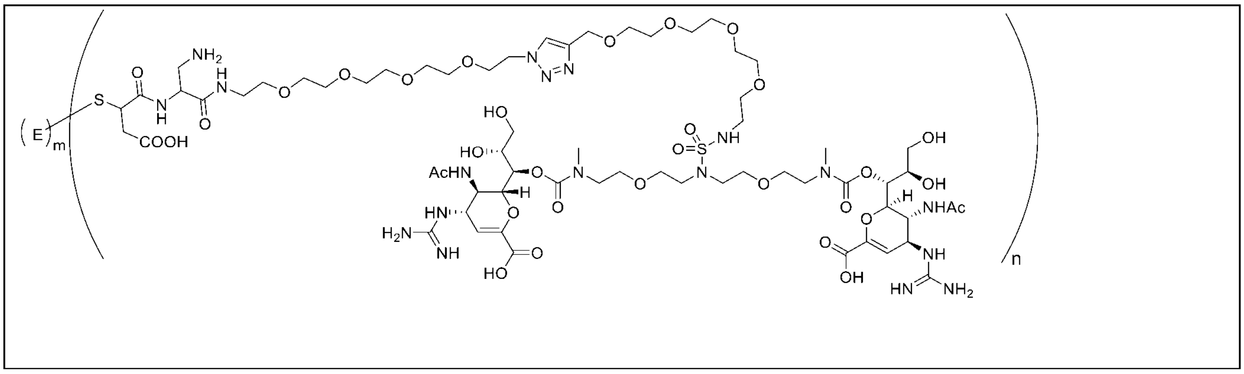

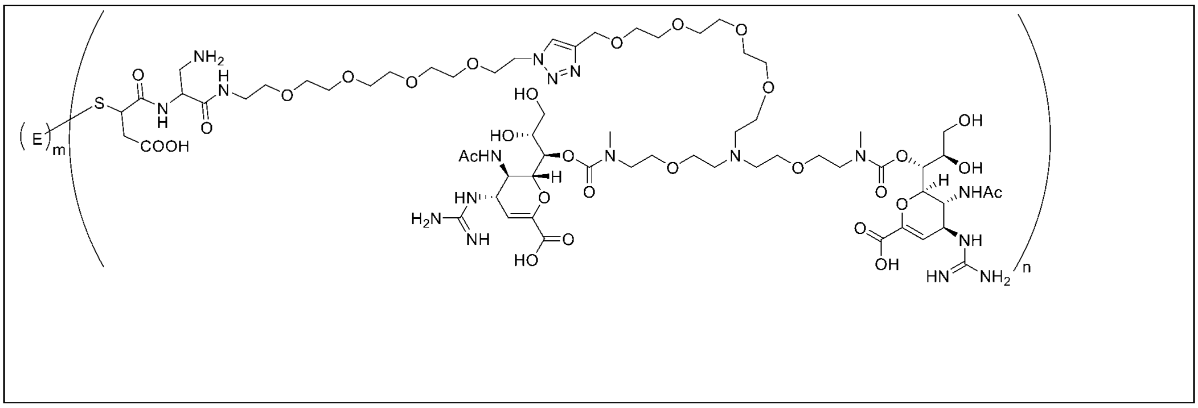

wherein E is as defined in any one of claims 1-13;
preferably, wherein E is SEQ ID NO: 67, SEQ ID NO: 69 or SEQ ID NO: 71;
and wherein m and n are as defined in claim 1, and m is preferably 1.

15. The conjugate according to any one of claims 1 to 14, or the pharmaceutically acceptable salt, ester, isomer, solvate, prodrug, or isotopically labeled compound thereof, wherein the carbon to which the

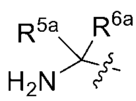

group is linked or the carbon to which the group corresponding to

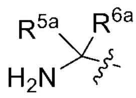

is linked is in R or S configuration, and preferably R configuration.

**16.** A compound represented by structure of formula I-5a or I-6a, or a pharmaceutically acceptable salt, ester, isomer, solvate, prodrug, or isotopically labeled compound thereof,

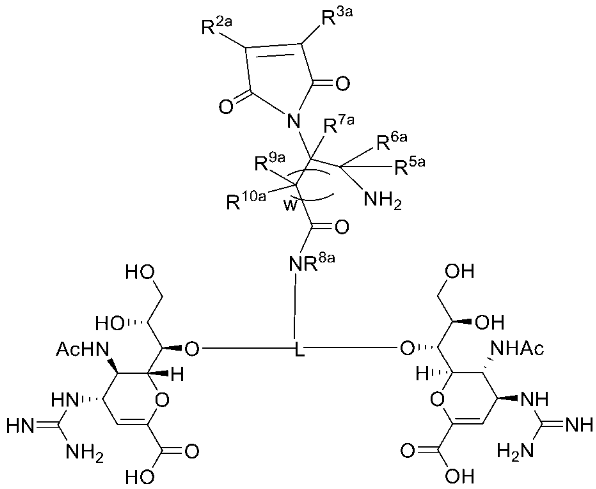

I-5a

wherein each symbol is as defined in any one of claims 1-15;

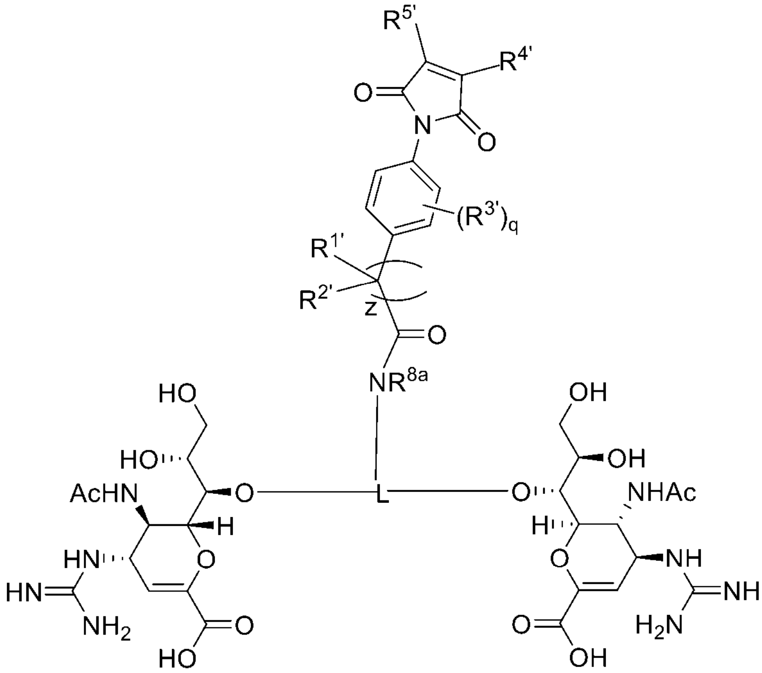

I-6a

wherein each symbol is as defined in any one of claims 1-15.

**17.** The compound according to claim 16, or the pharmaceutically acceptable salt, ester, isomer, solvate, prodrug, or isotopically labeled compound thereof, wherein the compounds have the structures of formulas C-Inter-1 to formula C-Inter-12:

| Intermediate No. | Structure of intermediate |
|---|---|
| C-Inter-1 | 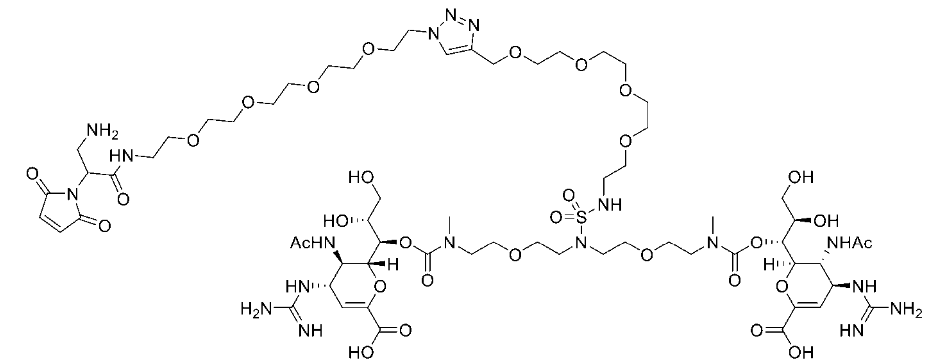 |
| C-Inter-2 | 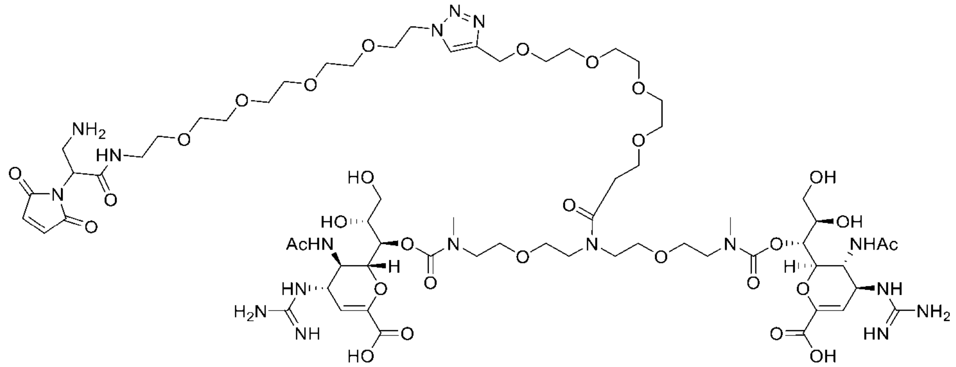 |
| C-Inter-3 | 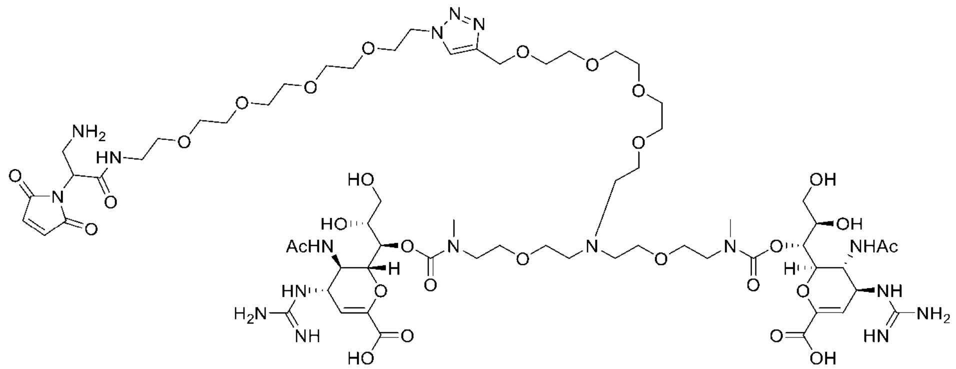 |
| C-Inter-4 | 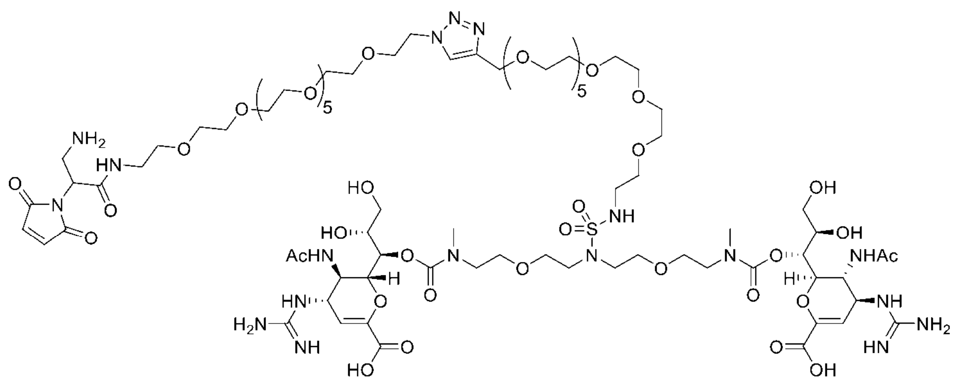 |

(continued)

| Intermediate No. | Structure of intermediate |
|---|---|
| C-Inter-5 | 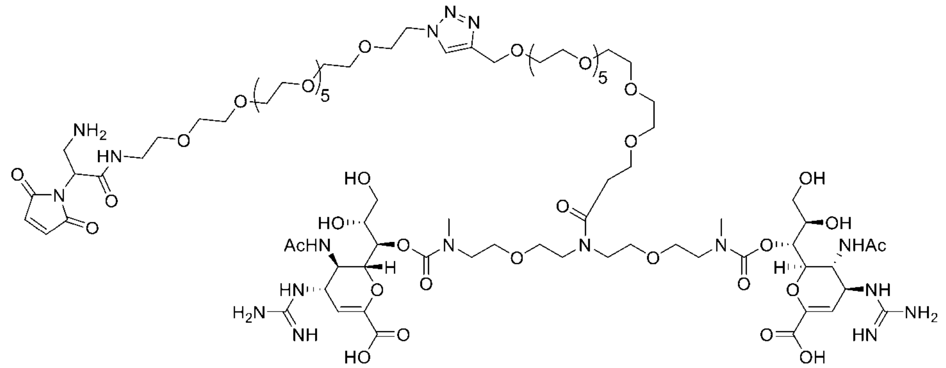 |
| C-Inter-6 | 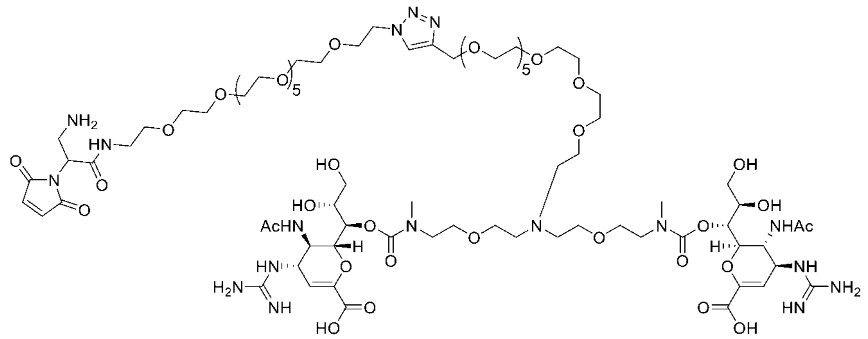 |
| C-Inter-7 | 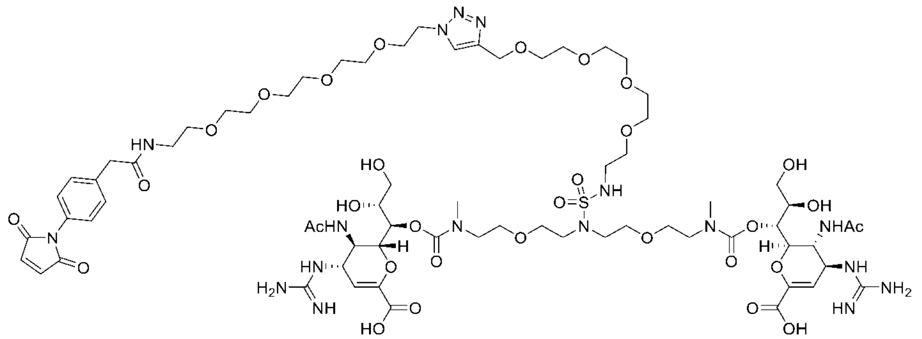 |
| C-Inter-8 | 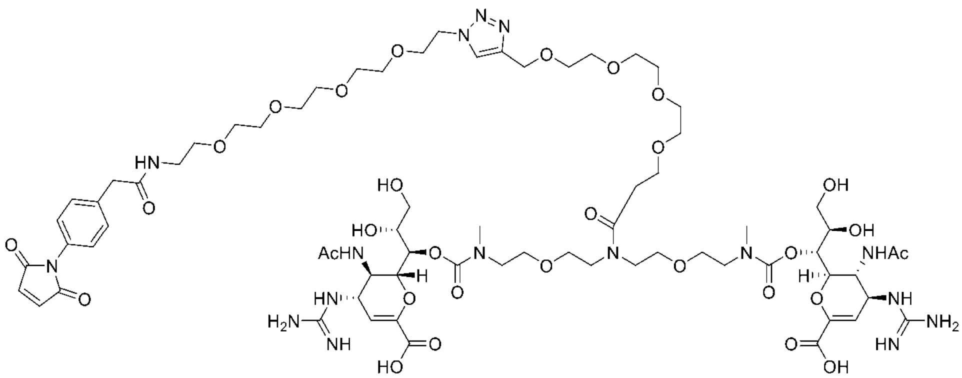 |

(continued)

| Intermediate No. | Structure of intermediate |
|---|---|
| **C-Inter-9** | 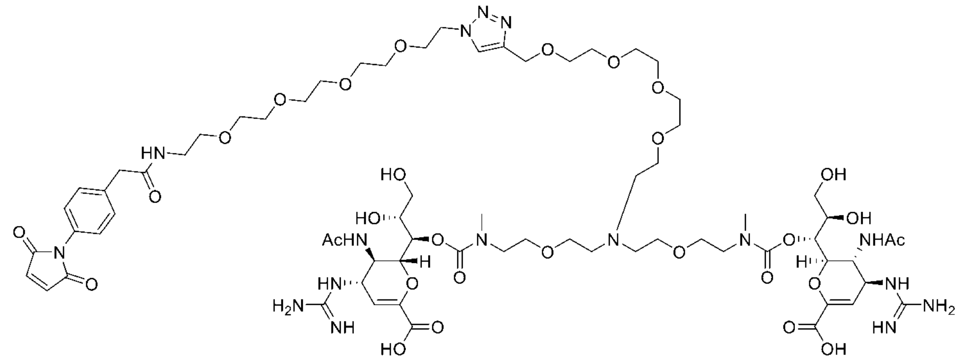 |
| **C-Inter-10** | 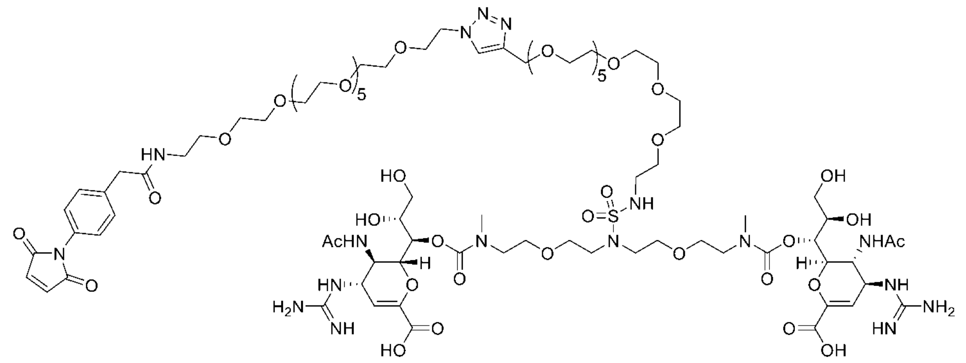 |
| **C-Inter-11** | 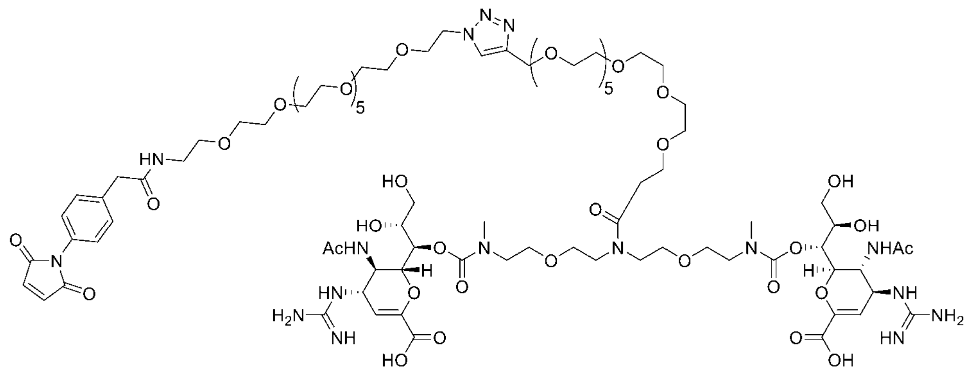 |
| **C-Inter-12** | 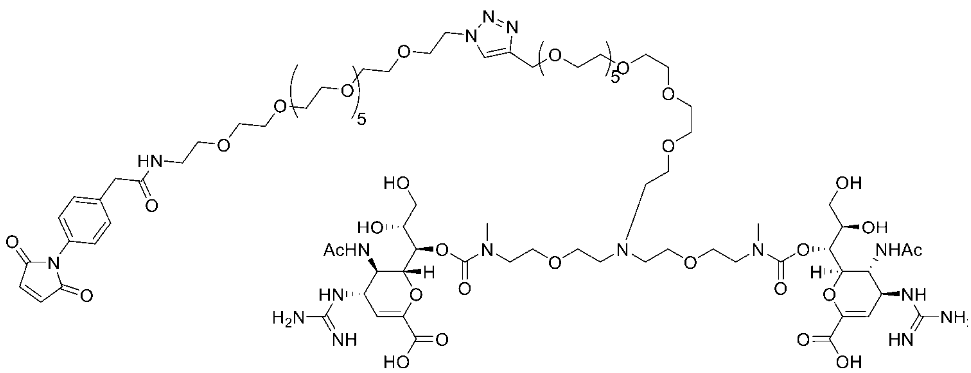 |

**18.** A method for preparing the conjugate of formula I-A according to any one of claims 1-15,

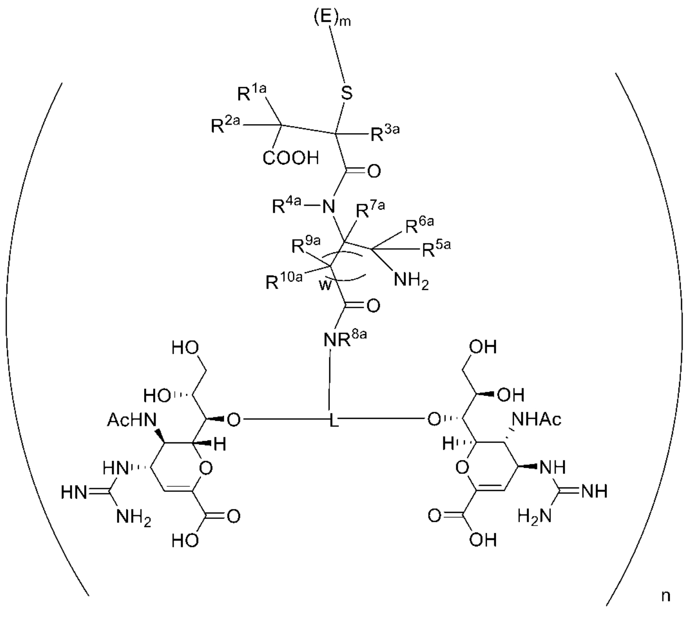 ;

Formula I-A

wherein each symbol is as defined in any one of claims 1-15;
the method comprises the following steps:

Step 1, dissolving $(E)_m$ in a buffer solution, wherein E and m are as defined in any one of claims 1-15; then, a reducing agent (such as TCEP, DTT) is added to reduce the disulfide bond in $(E)_m$, to obtain a compound of formula II-1 with thiol residues,

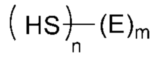

II-1

wherein E, m, and n are as defined in any one of claims 1-15;
Step 2, adding a buffer solution containing 6-14 molar equivalents of compound of formula I-5a to compound of formula II-1,

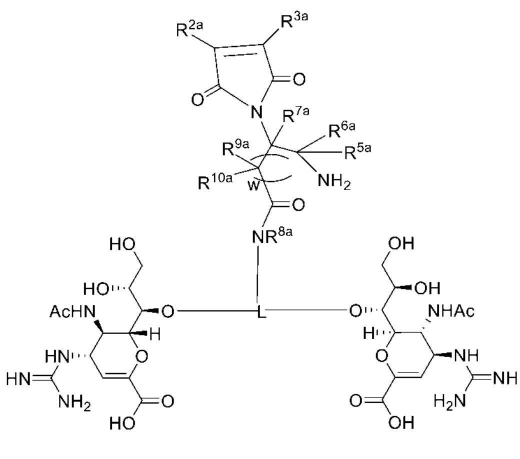

I-5a

wherein each symbol is as defined in formula I-A;
under the condition of pH 5.5-7.0 (e.g., 5.8-6.8, 6.0-6.6), the Michael addition reaction is carried out for

0.5-10 hours, preferably 0.5-2 hours; then, 3-7 molar equivalents of the buffer solution of compound of formula I-5a are added in batches (e.g., 1-2 batches), and the reaction is continued for 0.5-10 hours (preferably 0.5-2 hours) to obtain the conjugate of formula I-3b,

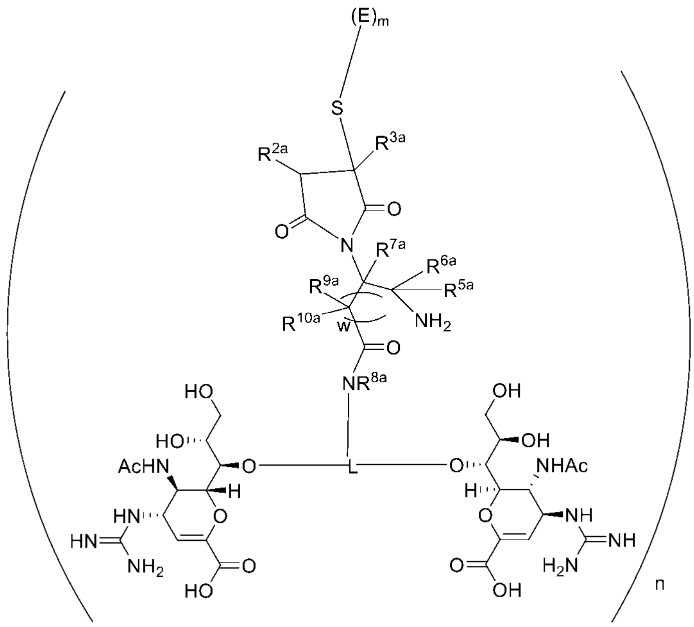

I-3b

wherein each symbol is as defined in formula I-A;

Step 3, adjusting the pH of the solution containing the conjugate of formula I-3b to 7-8 (e.g., 7.1-7.9, 7.3-7.7), and then performing a hydrolysis reaction to obtain the final conjugate of formula I-A;
or,

the method for preparing the conjugate of formula I-B according to any one of claims 1-15,

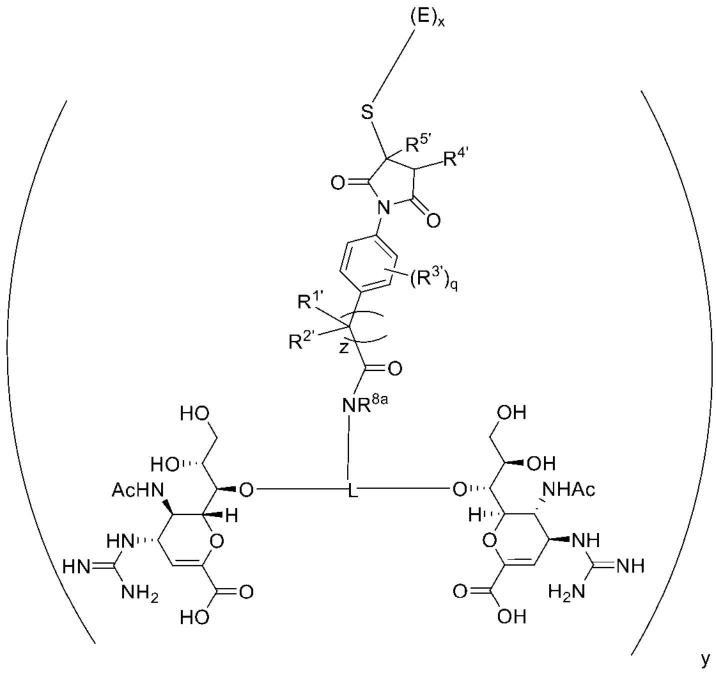

I-B

wherein each symbol is as defined in any one of claims 1-15;
the method comprises the following steps:

Step 1, dissolving $(E)_m$ in a buffer solution, wherein E and m are as defined in any one of claims 1-15, and adding a reducing agent (such as TCEP, DTT) to reduce the disulfide bond in $(E)_m$, to obtain a compound of formula II-1 with thiol residues,

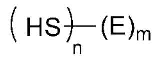

II-1

wherein E, m, and n are as defined in any one of claims 1-15;
Step 2, adding a buffer solution containing 6-14 molar equivalents of compound of formula I-6a to compound of formula II-1,

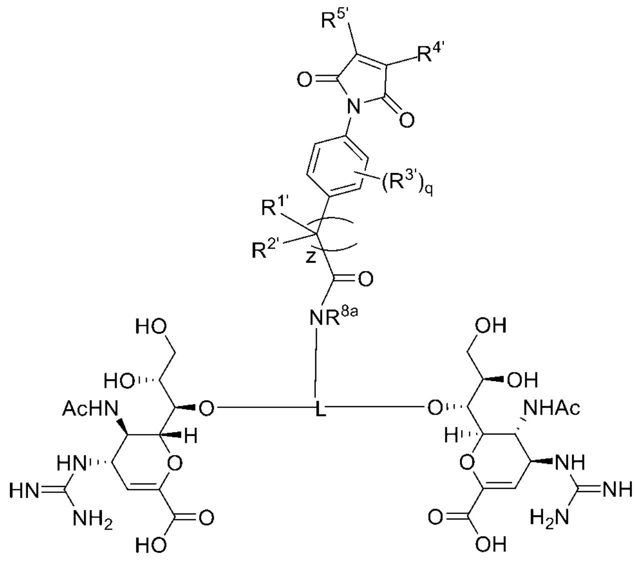

;

I-6a

wherein each symbol is as defined in the formula I-B;
the Michael addition reaction is carried out under the condition of pH 5.5-8 (e.g., 5.8-7.6, 6.0-7.5), to obtain the conjugate of formula I-B.

**19.** A pharmaceutical composition, comprising the conjugate of formula I-A or formula I-B according to any one of claims 1-15, or the compound of formula I-5a or I-6a according to claim 16 or claim 17, or the pharmaceutically acceptable salt, ester, isomer, solvate, prodrug, or isotopically labeled compound thereof, and optionally one or more other therapeutic agents, such as chemotherapeutic agents, angiogenesis inhibitors, cytokines, cytotoxic agents, other antibodies, small molecule drugs, or immunomodulators (e.g., immune checkpoint inhibitors or agonists), and optionally a pharmaceutically acceptable excipient.

**20.** A method for preventing or treating patients infected with viruses or patients who may be at risk of being infected with viruses, comprising the step of administering to the patients, for example, by injection, an effective dose of any of the conjugates of formula I-A or formula I-B according to any one of claims 1-15, or the compound of formula I-5a or I-6a according to claim 16 or claim 17, or the pharmaceutically acceptable salt, ester, isomer, solvate, prodrug, or isotopically labeled compound thereof.

**21.** The use of the conjugates of formula I-A or formula I-B according to any one of claims 1-15, or the compound of formula I-5a or I-6a according to claim 16 or claim 17, or the pharmaceutically acceptable salt, ester, isomer, solvate, prodrug, or isotopically labeled compound thereof in the manufacture of a medicament for preventing or treating viral infections in patients infected with viruses or patients who may be at risk of being infected with viruses;

preferably, the viral infection is an infection caused by influenza virus or parainfluenza virus;
preferably, the viral infection is an infection caused by influenza virus A, B, or C, or parainfluenza virus;
preferably, the patient who is infected with the virus or may be at risk of being infected with the virus may be a patient with immune system deficiencies;
preferably, the patient who is infected with the virus or may be at risk of being infected with the virus may be a patient who is receiving or will receive immunosuppressant treatment;
preferably, the patient who is infected with the virus or may be at risk of being infected with the virus may be a patient diagnosed with a disease that causes immunosuppression;
preferably, the patient diagnosed with a disease that causes immunosuppression suffers from cancer or acquired immune deficiency syndrome;
preferably, the cancer in the patient diagnosed with a disease that cause immunosuppression is selected from leukemia, lymphoma, humoral immunodeficiency, T-cell deficiency, complement deficiency, or multiple myeloma;

preferably, the patient is a patient who is undergoing or will undergo hematopoietic stem cell transplantation;
preferably, the patient is a patient who is undergoing or will undergo organ transplantation; and/or
preferably, the patient may be at risk of secondary infection.

**22.** The method for preventing a risk of secondary infection caused by influenza virus infection in patients, comprising administering to the patient, for example, by injection, an effective dose of any of the conjugates of formula I-A or formula I-B according to any one of claims 1-15, or the compound of formula I-5a or I-6a according to claim 16 or claim 17, or the pharmaceutically acceptable salt, ester, isomer, solvate, prodrug, or isotopically labeled compound thereof,

preferably, the secondary infection is a respiratory infection;
preferably, the secondary infection is related to pneumonia;
preferably, the secondary infection is bacterial, viral, or fungal infection;
preferably, the bacterial infection is an infection caused by methicillin-resistant *Staphylococcus aureus;* preferably, the bacterial infection is an infection caused by *Streptococcus pneumoniae*;
preferably, the conjugate of said formula I-A or formula I-B, the compound of said formula I-5a or I-6a, or the pharmaceutically acceptable salt, ester, isomer, solvate, prodrug, or isotopically labeled compound may be administered by intramuscular injection, intravenous injection, intradermal injection, intra-arterial injection, intraperitoneal injection, intralesional injection, intracranial injection, intra-articular injection, intrapleural injection, endotracheal injection, intraprostatic injection, intranasal injection, intravitreal injection, intravaginal injection, intrarectal injection, topical, intratumoral injection, intraperitoneal injection, subcutaneous injection, subconjunctival injection, intracapsular injection, mucosal injection, pericardial injection, intraumbilical injection, intraocular injection, oral administration, topical inhalation, injection, or infusion;
preferably, the conjugate of said formula I-A or Formula I-B, compound of formula I-5a or I-6a, or pharmaceutically acceptable salt, ester, isomer, solvate, prodrug, or isotopically labeled compound are administered in combination with an additional therapeutic agent or used to prepare a medicament in combination with an additional therapeutic agent;
preferably, the additional therapeutic agent is an antiviral drug;
preferably, the antiviral drug is baloxavir, pimodivir, oseltamivir, zanamivir, peramivir, laninamivir, amantadine, MEDI8852, or rimantadine;
preferably, the additional therapeutic agent used by the patient is an antiviral vaccine;
preferably, the antiviral drug and the conjugate of said formula I-A or formula I-B, the compound of said formula I-5a or I-6a, or pharmaceutically acceptable salt, ester, isomer, solvate, prodrug, or isotopically labeled compound are sequentially administered, for example, by injection, to the patient; and/or
preferably, the antiviral drug and the conjugate of formula I-A or formula I-B, the compound of formula I-5a or I-6a, or pharmaceutically acceptable salt, ester, isomer, solvate, prodrug, or isotopically labeled compound are administered simultaneously, for example, by injection to the patient.

**23.** A drug combination, comprising the conjugate of formula I-A or formula I-B according to any one of claims 1-15, or the compound of formula I-5a or I-6a according to claim 16 or claim 17, or the pharmaceutically acceptable salt, ester, isomer, solvate, prodrug, or isotopically labeled compound thereof, and optionally one or more other therapeutic agents, such as chemotherapeutic agents, angiogenesis inhibitors, cytokines, cytotoxic agents, other antibodies, small molecule drugs, or immunomodulators (e.g., immune checkpoint inhibitors or agonists).

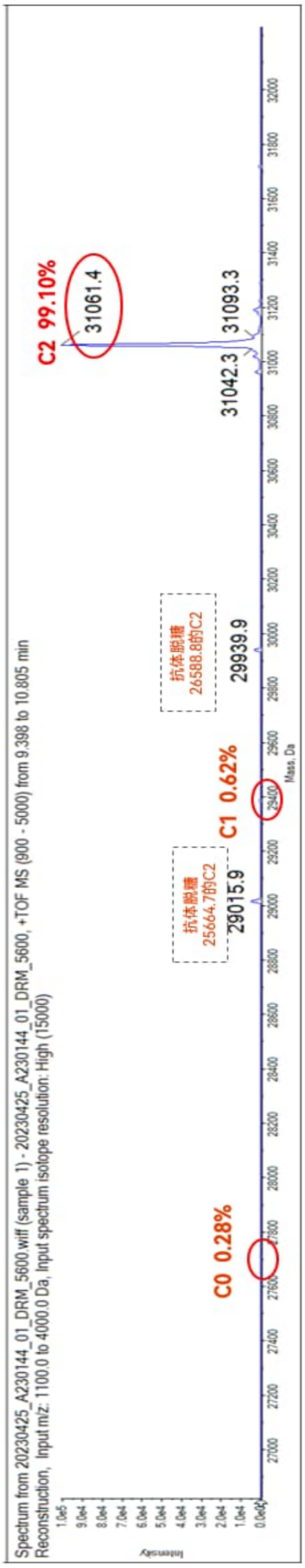
Spectrum from 20230425_A230144_01_DRM_5600.wiff (sample 1) - 20230425_A230144_01_DRM_5600, +TOF MS (900 - 5000) from 9.398 to 10.805 min
Reconstruction, Input m/z: 1100.0 to 4000.0 Da, Input spectrum isotope resolution: High (15000)
C2 99.10%
31061.4
31093.3
31042.3
抗体脱糖
26588.8的C2
29939.9
C1 0.62%
抗体脱糖
25664.7的C2
29015.9
C0 0.28%
Mass, Da
Intensity

Figure 1

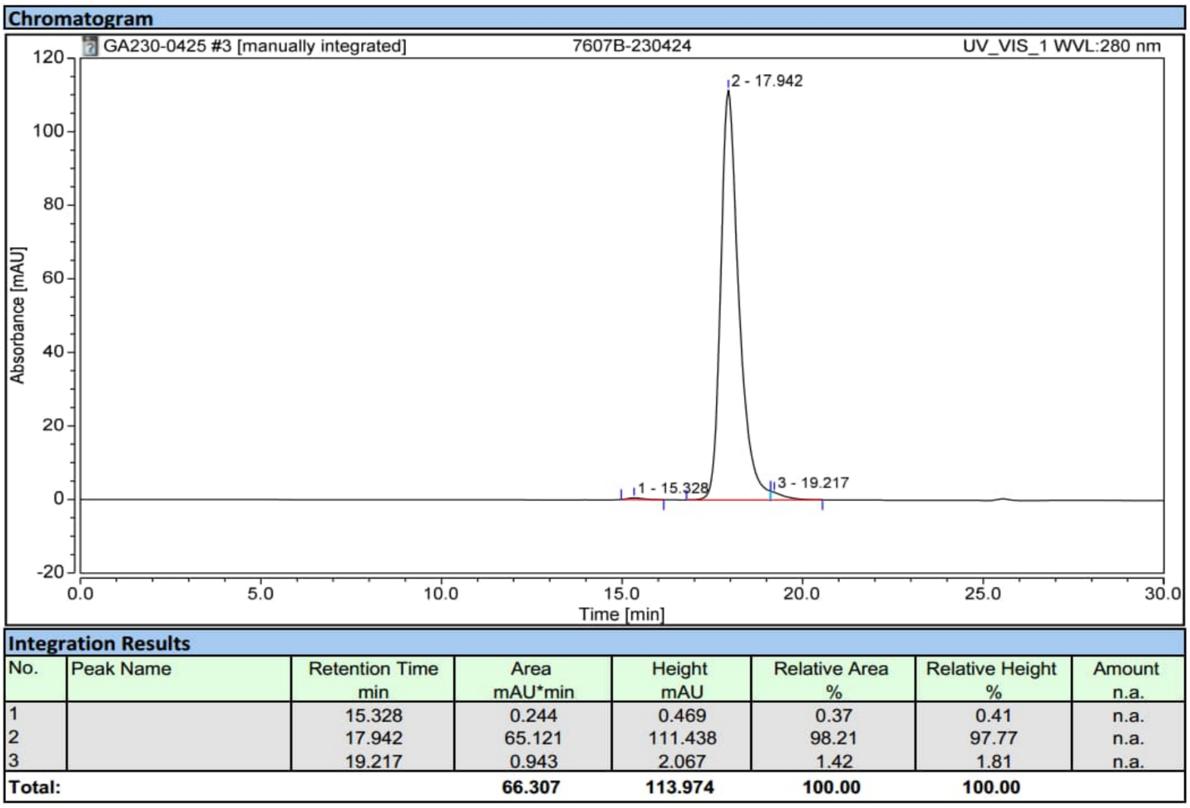


**Integration Results**

| No. | Peak Name | Retention Time min | Area mAU*min | Height mAU | Relative Area % | Relative Height % | Amount n.a. |
|---|---|---|---|---|---|---|---|
| 1 | | 15.328 | 0.244 | 0.469 | 0.37 | 0.41 | n.a. |
| 2 | | 17.942 | 65.121 | 111.438 | 98.21 | 97.77 | n.a. |
| 3 | | 19.217 | 0.943 | 2.067 | 1.42 | 1.81 | n.a. |
| **Total:** | | | **66.307** | **113.974** | **100.00** | **100.00** | |

Figure 2

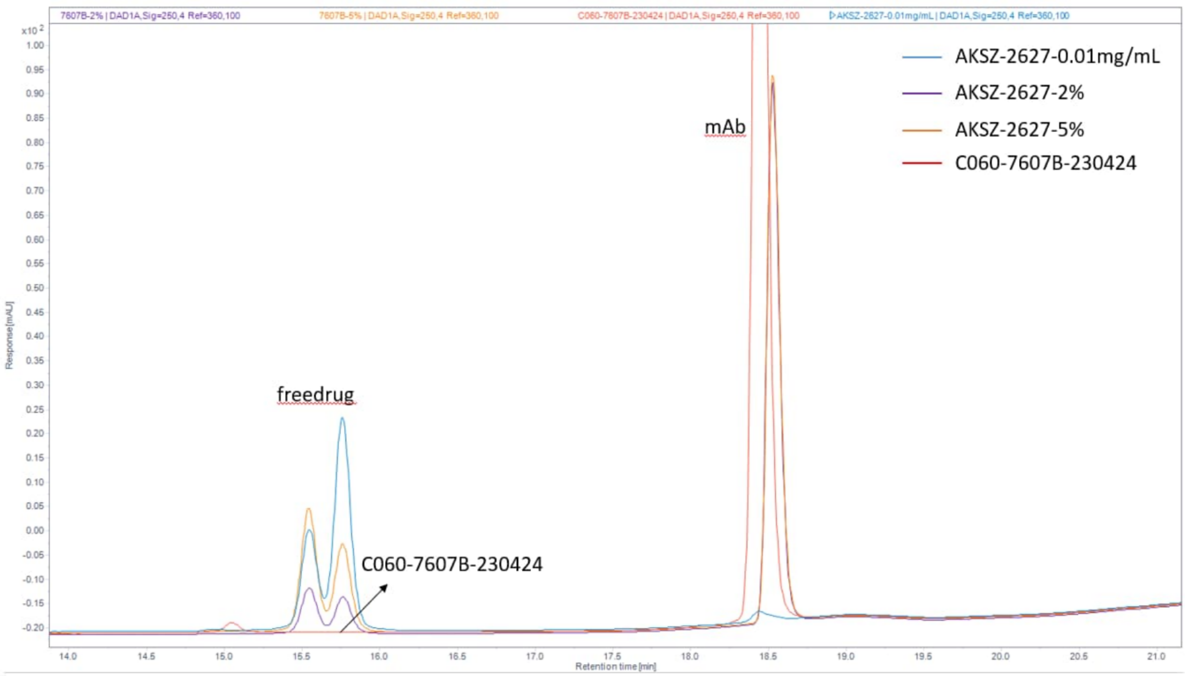


Figure 3

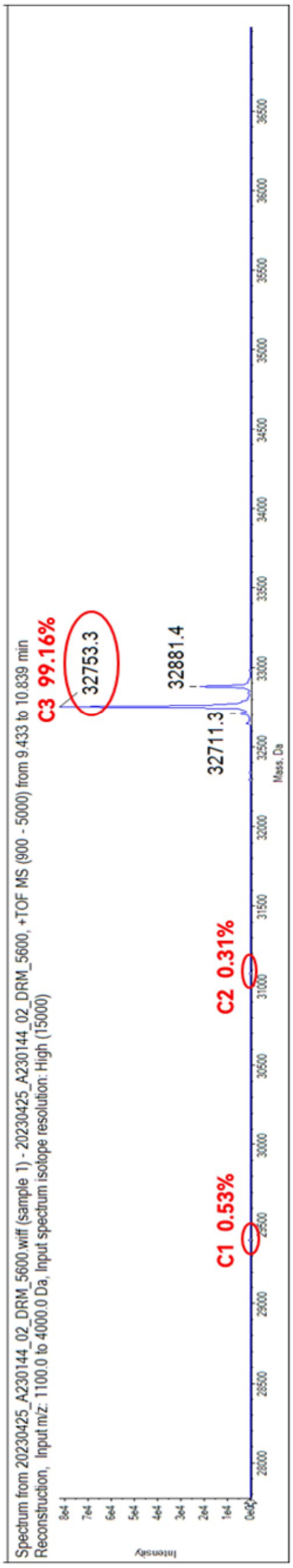
Spectrum from 20230425_A230144_02_DRM_5600.wiff (sample 1) - 20230425_A230144_02_DRM_5600, +TOF MS (900 - 5000) from 9.433 to 10.839 min
Reconstruction, Input m/z: 1100.0 to 4000.0 Da, Input spectrum isotope resolution: High (15000)
C3 99.16%
32753.3
32881.4
32711.3
C2 0.31%
C1 0.53%
Intensity
Mass, Da

Figure 4

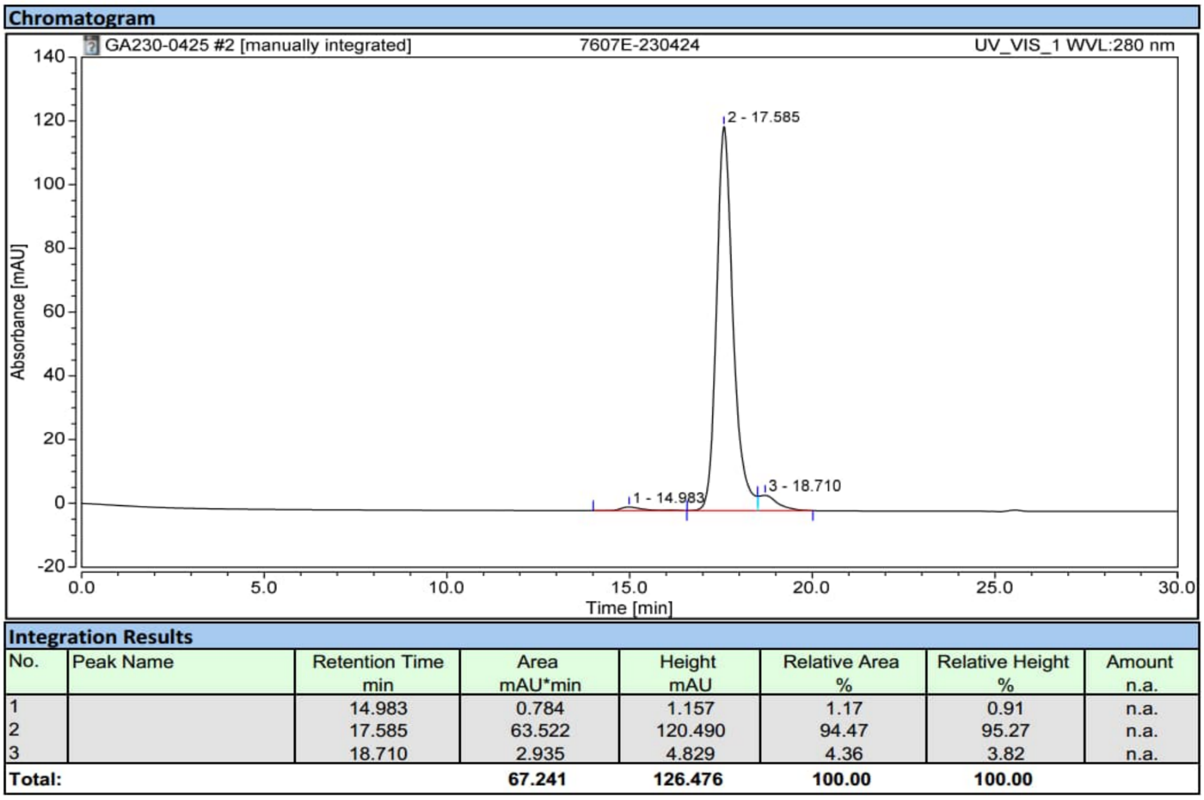


**Integration Results**

| No. | Peak Name | Retention Time min | Area mAU*min | Height mAU | Relative Area % | Relative Height % | Amount n.a. |
|---|---|---|---|---|---|---|---|
| 1 | | 14.983 | 0.784 | 1.157 | 1.17 | 0.91 | n.a. |
| 2 | | 17.585 | 63.522 | 120.490 | 94.47 | 95.27 | n.a. |
| 3 | | 18.710 | 2.935 | 4.829 | 4.36 | 3.82 | n.a. |
| **Total:** | | | **67.241** | **126.476** | **100.00** | **100.00** | |

Figure 5

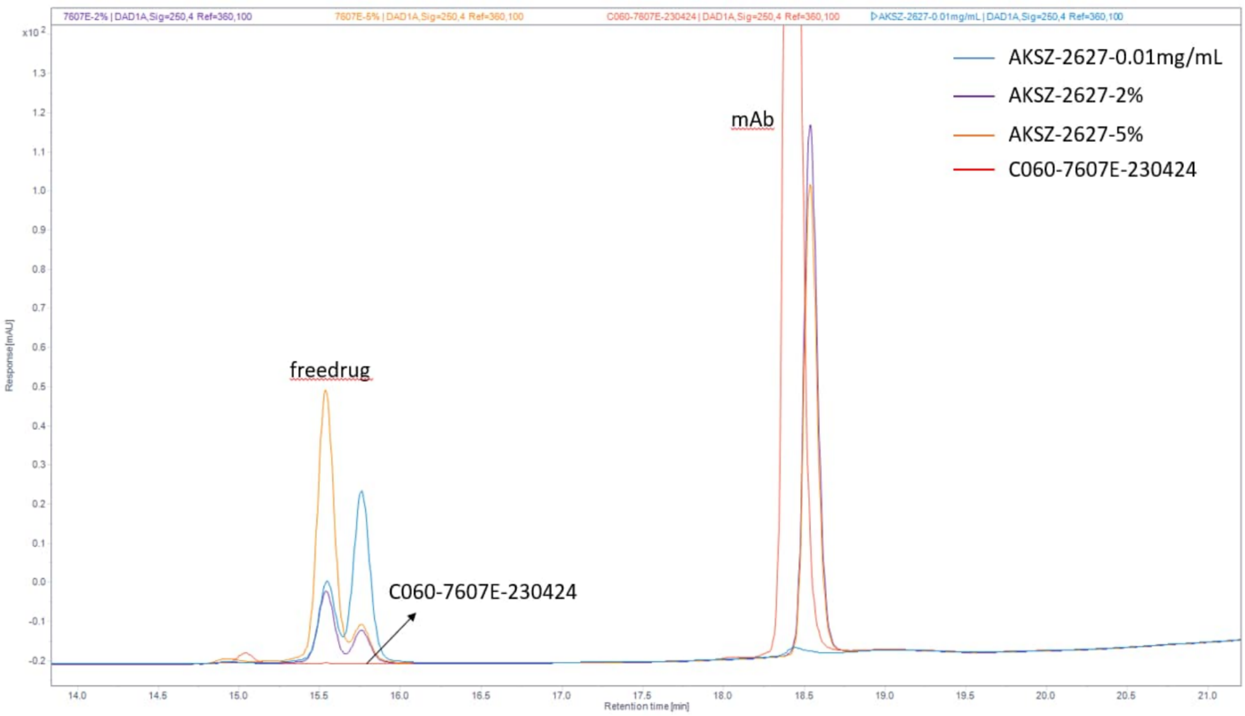


Figure 6

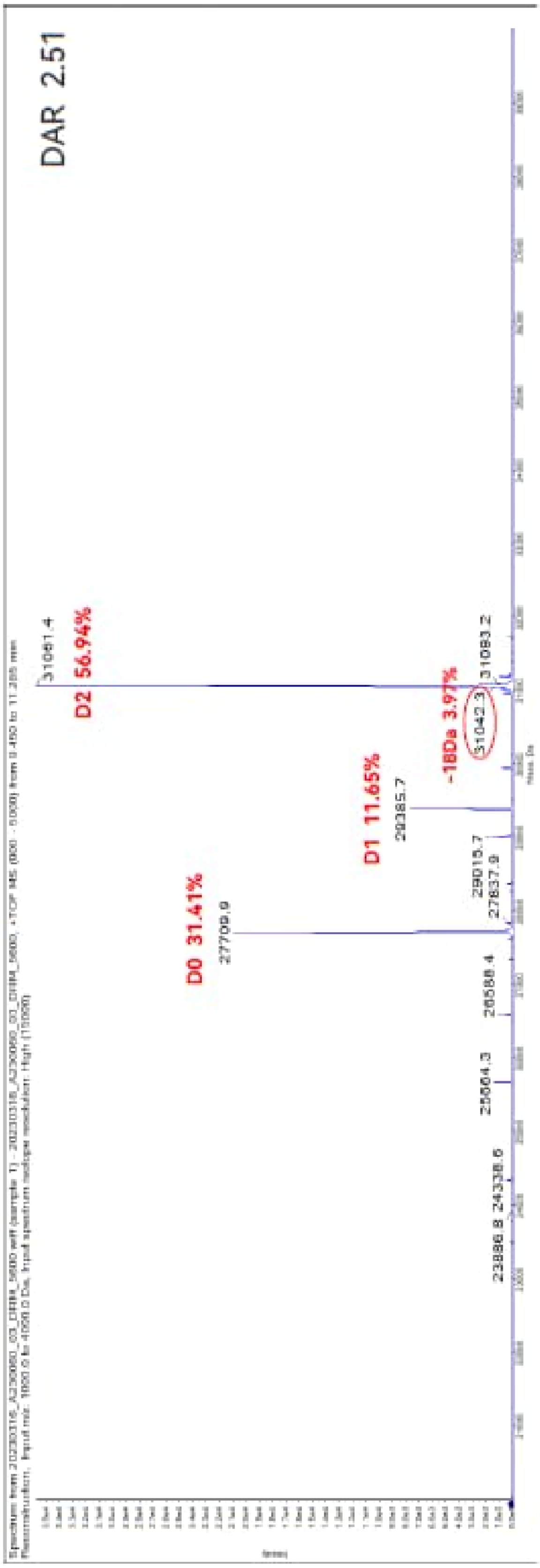
DAR 2.51
31061.4
D2 56.94%
-18Da 3.97%
31042.3
31083.2
D1 11.65%
29385.7
29015.7
27837.9
D0 31.41%
27709.9
26588.4
25564.3
23886.8 24338.6

Figure 7

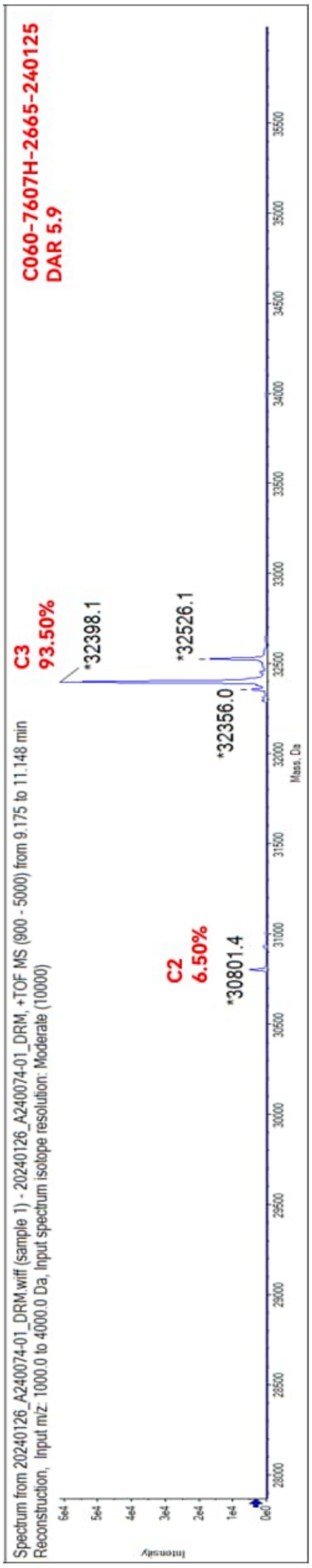
Spectrum from 20240126_A240074-01_DRM.wiff (sample 1) - 20240126_A240074-01_DRM, +TOF MS (900 - 5000) from 9.175 to 11.148 min
Reconstruction, Input m/z: 1000.0 to 4000.0 Da, Input spectrum isotope resolution: Moderate (10000)
C060-7607H-2665-240125
DAR 5.9
C3
93.50%
*32398.1
*32526.1
*32356.0
C2
6.50%
*30801.4
Intensity
Mass, Da

Figure 8

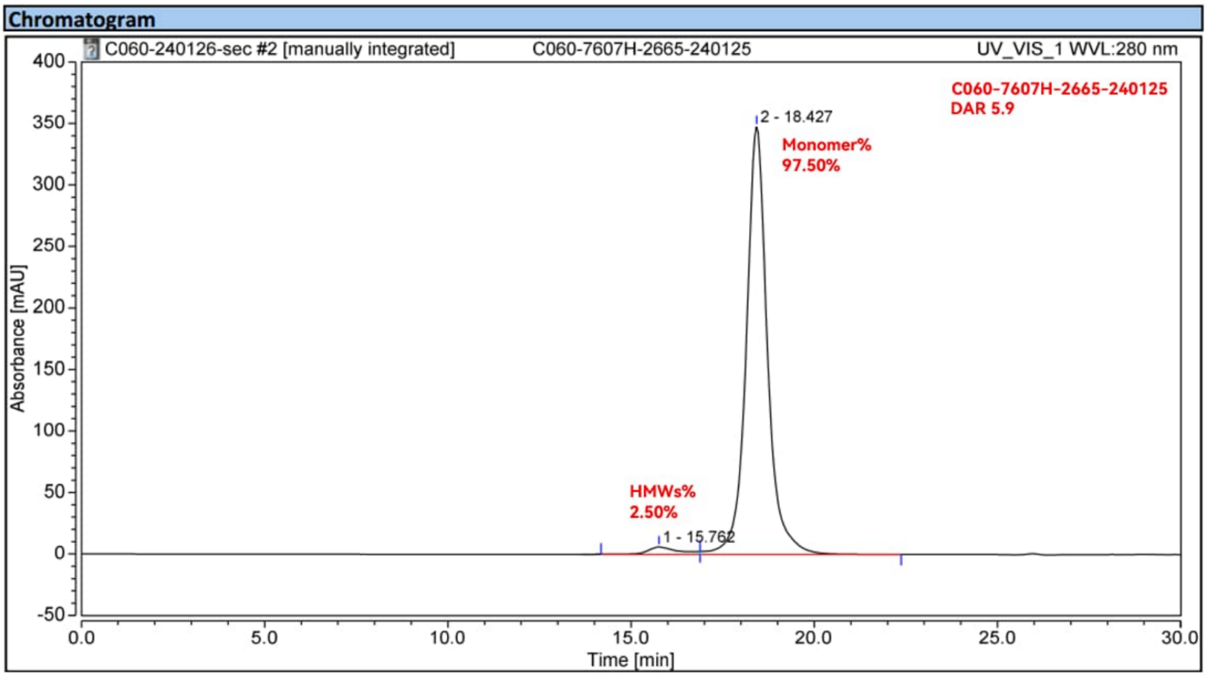


Figure 9

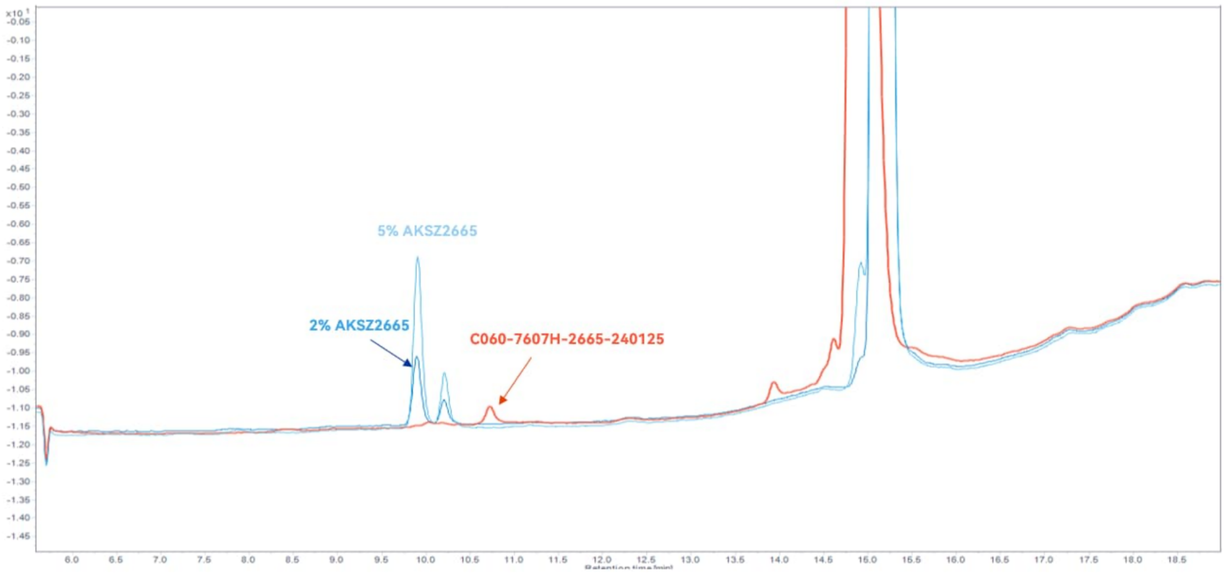


Figure 10

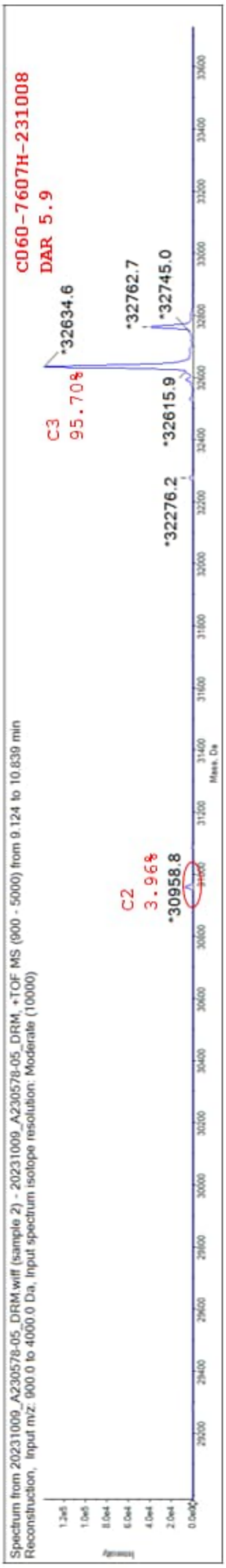
Spectrum from 20231009_A230578-05_DRM.wiff (sample 2) - 20231009_A230578-05_DRM, +TOF MS (900 - 5000) from 9.124 to 10.839 min
Reconstruction, Input m/z: 900.0 to 4000.0 Da, Input spectrum isotope resolution: Moderate (10000)
C060-7607H-231008
DAR 5.9
C3
95.70%
C2
3.96%
*32634.6
*32762.7
*32745.0
*32615.9
*32276.2
*30958.8
Intensity
Mass, Da

Figure 11

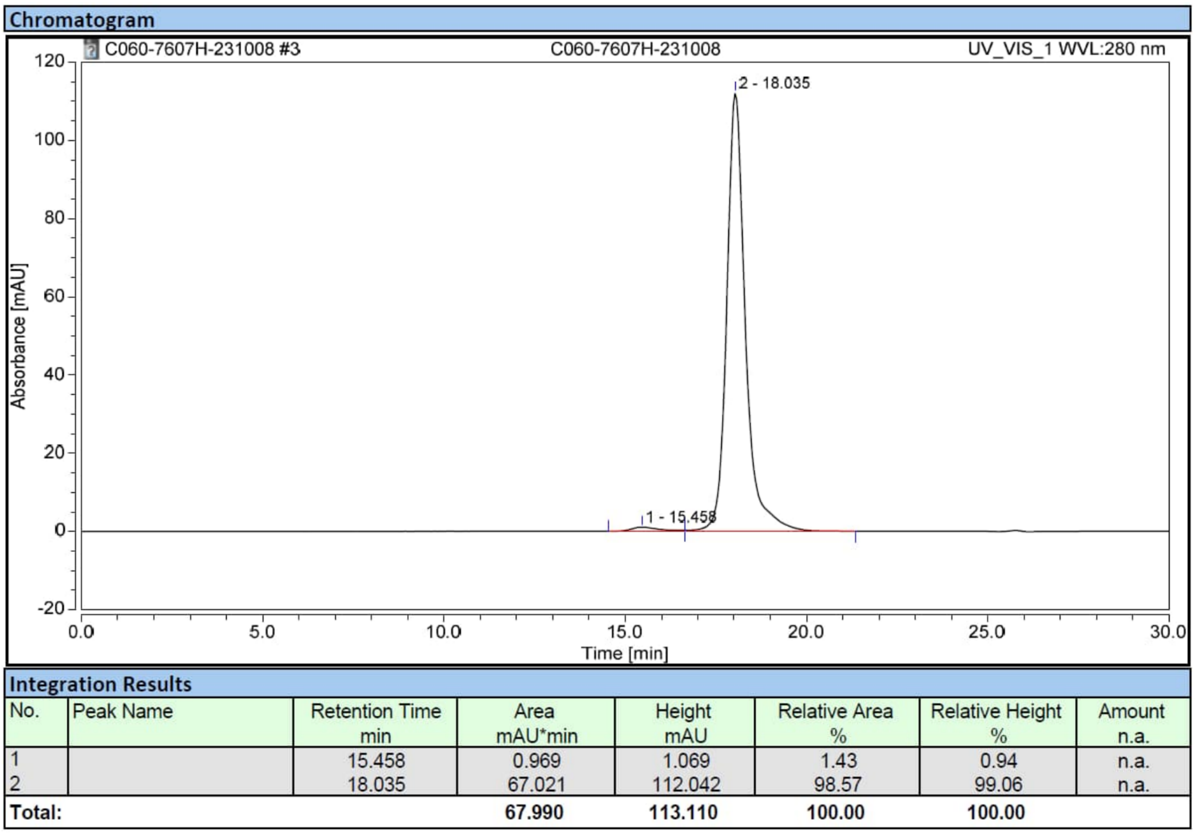


Integration Results

| No. | Peak Name | Retention Time min | Area mAU*min | Height mAU | Relative Area % | Relative Height % | Amount n.a. |
|---|---|---|---|---|---|---|---|
| 1 | | 15.458 | 0.969 | 1.069 | 1.43 | 0.94 | n.a. |
| 2 | | 18.035 | 67.021 | 112.042 | 98.57 | 99.06 | n.a. |
| **Total:** | | | **67.990** | **113.110** | **100.00** | **100.00** | |

Figure 12

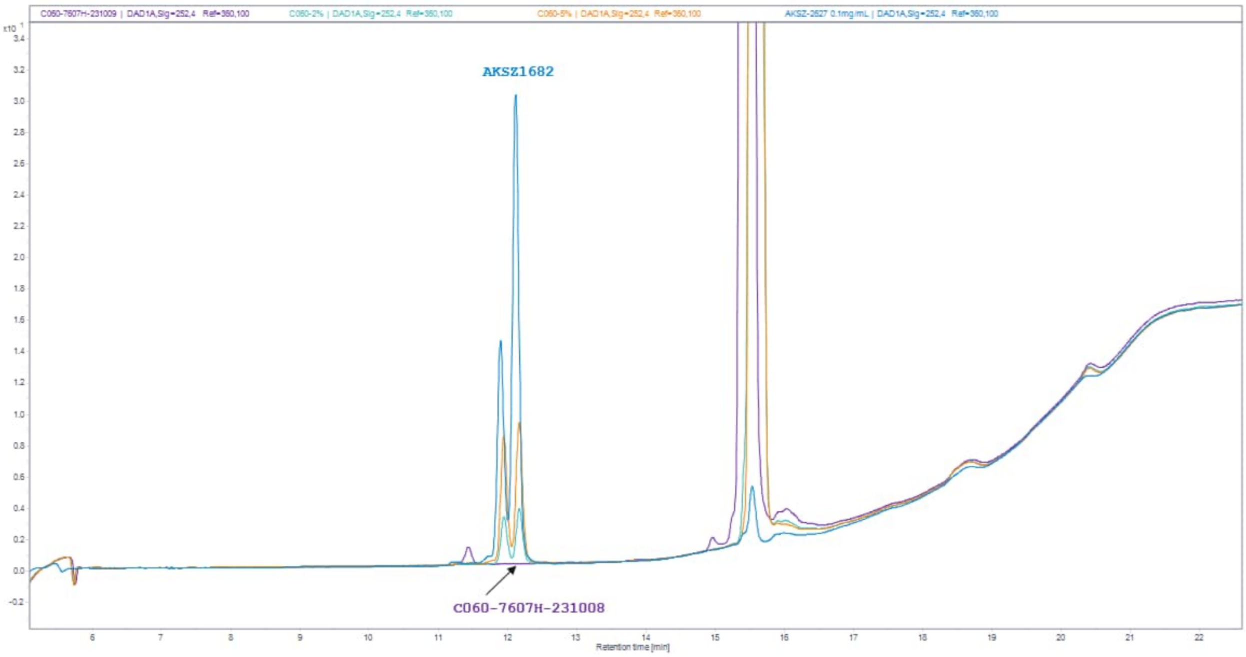


Figure 13

## INTERNATIONAL SEARCH REPORT

International application No.
**PCT/CN2024/102634**

**A. CLASSIFICATION OF SUBJECT MATTER**

A61K 47/64(2017.01)i； A61P31/16(2006.01)i

According to International Patent Classification (IPC) or to both national classification and IPC

**B. FIELDS SEARCHED**

Minimum documentation searched (classification system followed by classification symbols)

IPC:A61K A61P

Documentation searched other than minimum documentation to the extent that such documents are included in the fields searched

Electronic data base consulted during the international search (name of data base and, where practicable, search terms used)

CNTXT; ENTXTC; VEN; STN CAPLUS, REGISTRY, MARPAT; BING; 百度, Baidu: 上海爱科百发生物医药技术股份有限公司, 宋国伟, 宋治东, 彭程, 高广林, 武进, 高凡, 高熙, 邹罡, 叶军民, 邬征, 秦笑非, 结构扩展检索, extended search for structure, 扎那米韦, 缀合, 偶联, 流感, 流行性感冒, 病毒, 139110-80-8扩展, 139110-80-8 extended, ZANAMIVIR, CONJUGATE, ADC, INFLU, H1N1, H5N1, VIRUS

**C. DOCUMENTS CONSIDERED TO BE RELEVANT**

| Category* | Citation of document, with indication, where appropriate, of the relevant passages | Relevant to claim No. |
|---|---|---|
| A | CN 113194983 A (CIDARA THERAPEUTICS, INC.) 30 July 2021 (2021-07-30)<br>claim 79, and description, paragraph [0005] | 1-23 |
| A | WO 2023109942 A1 (JACOBIO PHARMACEUTICALS CO., LTD.) 22 June 2023 (2023-06-22)<br>claim 52 | 1-23 |
| A | CN 109843334 A (SEATTLE GENETICS INC.) 04 June 2019 (2019-06-04)<br>claim 53 | 1-23 |
| A | US 2016120998 A1 (UNIV. NAT. CENTRAL) 05 May 2016 (2016-05-05)<br>description, paragraph [0047] | 1-23 |
| A | CN 101287500 A (BARNES & THORNBURG LLP) 15 October 2008 (2008-10-15)<br>description, page 12, paragraph [0002] | 1-23 |
| A | CN 102008732 A (WUHAN HUAYAO BIOPHARMACEUTICAL CO., LTD.) 13 April 2011 (2011-04-13)<br>description, paragraph [0081] | 1-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 August 2024** | **27 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/102634

C. DOCUMENTS CONSIDERED TO BE RELEVANT

| Category* | Citation of document, with indication, where appropriate, of the relevant passages | Relevant to claim No. |
|---|---|---|
| A | US 2008280937 A1 (LEMAON, Christopher Paul et al.) 13 November 2008 (2008-11-13) description, paragraph [0115] | 1-23 |
| A | VLAHOV, Iontcho R. et al. "Design and Regioselective Synthesis of a New Generation of Targeted Therapeutics. Part 3: Folate Conjugates of Aminopterin Hydrazide for the Treatment of Inflammation" *Bioorganic & Medicinal Chemistry Letters*, No. no. 21, 22 December 2010 (2010-12-22), pp. 1202-1205 | 1-23 |

Form PCT/ISA/210 (second sheet) (July 2022)

**INTERNATIONAL SEARCH REPORT**

International application No.
**PCT/CN2024/102634**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/102634**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20, 22**
because they relate to subject matter not required to be searched by this Authority, namely:

Claim 20 sets forth a method for preventing or treating a patient suffering from viral infections or a patient possibly being at risk of being infected by a virus, and claim 22 sets forth a method for preventing a patient from the risk of a secondary infection caused by influenza virus infections, which both relate to a method for treatment of the human body, and thus said claims do not comply with PCT Rule 39.1(iv). The search report is provided on the basis that the designation of the subject matter of claim 20 is "the use of the conjugate of formula I-A or formula I-B according to any one of claims 1-15, or the compound of formula I-5a or I-6a according to claim 16 or 17, or a pharmaceutically acceptable salt, ester, isomer, solvate, prodrug or isotope marker thereof in the preparation of a drug for preventing or treating a patient suffering from viral infections or a patient possibly being at risk of being infected by a virus", and the designation of the subject matter of claim 22 is "the use of the conjugate of formula I-A or formula I-B of any one of claims 1-15, or the compound of formula I-5a or I-6a of claim 16 or 17, or a pharmaceutically acceptable salt, ester, isomer, solvate, prodrug or isotope marker thereof in the preparation of a drug for preventing a patient from the risk of a secondary infection caused by influenza virus infections".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/102634**

| Patent document cited in search report | Publication date (day/month/year) | Patent family member(s) | Publication date (day/month/year) |
|---|---|---|---|
| CN 113194983 A | 30 July 2021 | BR 112021004266 A2 | 03 August 2021 |
| | | EP 3846846 A1 | 14 July 2021 |
| | | EP 3846846 A4 | 03 August 2022 |
| | | JP 2021536483 A | 27 December 2021 |
| | | JP 7520816 B2 | 23 July 2024 |
| | | PH 12021550822 A1 | 04 October 2021 |
| | | AU 2019336230 A1 | 15 April 2021 |
| | | AU 2019336230 B2 | 07 September 2023 |
| | | US 2024156975 A1 | 16 May 2024 |
| | | SG 11202103313 RA | 29 April 2021 |
| | | MX 2021002569 A | 08 June 2021 |
| | | US 2021228727 A1 | 29 July 2021 |
| | | KR 20210079278 A | 29 June 2021 |
| | | TW 202104176 A | 01 February 2021 |
| | | IL 281277 A | 29 April 2021 |
| | | CA 3111803 A1 | 12 March 2020 |
| | | AU 2023278077 A1 | 18 January 2024 |
| | | WO 2020051498 A1 | 12 March 2020 |
| | | MA 53558 A | 15 September 2021 |
| | | ZA 202102158 B | 26 June 2024 |
| | | US 2022257787 A1 | 18 August 2022 |
| | | US 11833213 B2 | 05 December 2023 |
| | | CR 20210166 A | 27 July 2021 |
| | | ECSP 21023859 A | 30 June 2021 |
| WO 2023109942 A1 | 22 June 2023 | None | |
| CN 109843334 A | 04 June 2019 | KR 20230144096 A | 13 October 2023 |
| | | MA 46660 A | 28 August 2019 |
| | | MX 2023010327 A | 14 September 2023 |
| | | CA 3039589 A1 | 26 April 2018 |
| | | EP 3528850 A1 | 28 August 2019 |
| | | EP 3528850 A4 | 24 June 2020 |
| | | JP 2023038355 A | 16 March 2023 |
| | | SG 11201903013 SA | 30 May 2019 |
| | | KR 20190074292 A | 27 June 2019 |
| | | TW 201818970 A | 01 June 2018 |
| | | TWI 835714 B | 21 March 2024 |
| | | US 2019314519 A1 | 17 October 2019 |
| | | US 11638762 B2 | 02 May 2023 |
| | | BR 112019006778 A2 | 15 October 2019 |
| | | US 2024016953 A1 | 18 January 2024 |
| | | AU 2017345262 A1 | 06 June 2019 |
| | | EA 201990890 A1 | 31 October 2019 |
| | | JP 2019530697 A | 24 October 2019 |
| | | MX 2019004046 A | 21 August 2019 |
| | | IL 291810 A | 01 June 2022 |
| | | WO 2018075600 A1 | 26 April 2018 |
| | | IL 265837 A | 30 June 2019 |
| | | IL 265837 B | 01 May 2022 |
| US 2016120998 A1 | 05 May 2016 | TW 201603823 A | 01 February 2016 |
| | | TWI 538689 B | 21 June 2016 |

Form PCT/ISA/210 (patent family annex) (July 2022)

**INTERNATIONAL SEARCH REPORT**
**Information on patent family members**

International application No.
**PCT/CN2024/102634**

| Patent document cited in search report | | | Publication date (day/month/year) | Patent family member(s) | | | Publication date (day/month/year) |
|---|---|---|---|---|---|---|---|
| | | | | US | 10322188 | B2 | 18 June 2019 |
| CN | 101287500 | A | 15 October 2008 | None | | | |
| CN | 102008732 | A | 13 April 2011 | JP | 2013543849 | A | 09 December 2013 |
| | | | | CA | 2825083 | A1 | 18 May 2012 |
| | | | | WO | 2012062094 | A1 | 18 May 2012 |
| | | | | RU | 2013125317 | A | 20 December 2014 |
| | | | | EP | 2650309 | A1 | 16 October 2013 |
| | | | | EP | 2650309 | A9 | 11 December 2013 |
| | | | | EP | 2650309 | A4 | 18 May 2016 |
| | | | | AU | 2011328810 | A1 | 04 July 2013 |
| | | | | US | 2013259882 | A1 | 03 October 2013 |
| US | 2008280937 | A1 | 13 November 2008 | EP | 2382995 | A2 | 02 November 2011 |
| | | | | EP | 2382995 | A3 | 25 September 2013 |
| | | | | WO | 2007022493 | A3 | 24 January 2008 |
| | | | | US | 2008280937 | A1 | 13 November 2008 |
| | | | | JP | 2009504783 | A | 05 February 2009 |
| | | | | EP | 1948240 | A2 | 30 July 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021046549 A1
- WO 2023125806 A1


### Non-patent literature cited in the description


- **LULIANO et al.** *Lancet*, 2018, vol. 391, 1285-1300
- **VIDARSSON et al.** IgG subclasses and allotypes: from structure to effector function. *Frontiers in Immunology.*, 2014, vol. 5 (520), 1-17
- **R.C. ROWE** ; **P.J. SESKEY** ; **S.C. OWEN**. Handbook of Pharmaceutical Excipients. Pharmaceutical Press
- **KABAT et al.** Sequences of Proteins of Immunological Interest. National Institute of Health, 1991